# EUROPEAN PATENT SPECIFICATION

(11) **EP 0 829 476 B1**
(45) Date of publication and mention of the grant of the patent: **28.08.2002**
(21) Application number: 97110658.8
(22) Date of filing: 28.09.1990
(51) Int. Cl.: C07D 239/60, C07D 239/54, C07D 401/12, C07D 239/56, C07D 239/58, A61K 31/505

(54) **6-Substituted acyclopyrimidine nucleoside derivatives and antiviral agent containing the same as active ingredient thereof**
6-Substituierte Acylopyrimidin-Nucleoside-Derivate und diese als wirksamen Stoff enthaltende Antivirusmittel
Dérivés de nucléoside acylopyrimidine 6-substitués et agent antiviral les contenant comme ingrédient actif

(30) Priority: 29.09.1989 JP 25453189; 09.03.1990 JP 5970090; 27.07.1990 JP 20089590
(43) Date of publication of application: 18.03.1998
(62) Divisional of application: 90402693.7
(73) Proprietor: MITSUBISHI CHEMICAL CORPORATION, Chiyoda-ku Tokyo (JP)
(72) Inventor: Miyasaka, Tadashi, Yokohama-shi, Kanagawa-Ken (JP); Tanaka, Hiromichi, Yokohama-shi, Kanagawa-ken (JP); De Clercq, Erik Desire Alice, 3000 Leuven (BE); Baba, Masanori, Kagoshima-shi, Kagoshima 891-01 (JP); Walker, Richard Thomas, Selly Oak, Birmingham (GB); Ubasawa, Masaru, Yokohama-shi, Kanagawa-ken (JP)
(74) Representative: HOFFMANN - EITLE

(56) References cited:
- EP-A- 0 371 139
- WO-A-83/02891
- DE-A- 2 126 148
- DE-A- 2 142 317
- US-A- 3 497 515
- I.T. KAY, ET AL.: "A new synthesis of phenyl ketones by intramolecular "deoxybenzoylation" of enols and phenols" TETRAHEDRON LETTERS, vol. 27, no. 1, January 1986, pages 113-116, XP002069855 OXFORD, GB
- M. SAKO, ET AL.: "Reductive debromination of 5-bromouracils by 1-benzyl-1,4-dihydronicotinamide" TETRAHEDRON, vol. 39, no. 23, December 1983, pages 3919-2921, XP002069856 OXFORD, GB
- F. YONEDA, ET AL.: "Synthesis of 2H-chromeno[2,3-d]pyrimidine-2,4(3H)- diones (10-oxa-5-deazaflvins)" JOURNAL OF HETEROCYCLIC CHEMISTRY, vol. 19, no. 2, March 1982, pages 301-304, XP002069857 PROVO, US
- B.R. BAKER, ET AL.: "Irreversible enzyme inhibitors. LXXVII. Inhibitors of thymidine phosphorylase. III. Hydrophobic bonding by 1-substituted uracils containing additional substitutents at the 5 and 6 positions" JOURNAL OF MEDICINAL CHEMISTRY, vol. 10, no. 3, May 1967, pages 304-311, XP002069858 WASHINGTON, DC, US
- B.R. BAKER, ET AL.: "Irreversible enzyme inhibitors. LXXX. Inhibitors of thymidine phosphorylase. VI. Hydorphobic bonding with 6 substituents on the acidic 5-nitro- and 5-bromouracils" JOURNAL OF MEDICINAL CHEMISTRY, vol. 10, no. 3, May 1967, pages 316-320, XP002069862 WASHINGTON, DC, US
- M. YOSHIMOTO, ET AL.: "Correlation analysis of Baker's studies on enzyme inhibition. 2" JOURNAL OF MEDICINAL CHEMISTRY, vol. 19, no. 1, January 1976, pages 71-98, XP000609047 WASHINGTON, DC, US
- CHEMICAL ABSTRACTS, vol. 111, no. 17, 23 October 1989 Columbus, Ohio, US; abstract no. 153828m, page 728; XP002069859 & JP 61 205261 A (SDS BIOTECHNOLOGY) 28 November 1988
- CHEMICAL ABSTRACTS, vol. 106, no. 3, 19 January 1987 Columbus, Ohio, US; abstract no. 18608w, page 613; XP002069860 & JP 61 205261 A (SAGAMI CHEMICAL RESEARCH CENTRE) 11 September 1987

## Description

### FIELD OF THE INVENTION

The present invention relates to novel 6-substituted acyclopyrimidine derivatives and antiviral agents containing the derivative as the active ingredients.

### BACKGROUND OF THE INVENTION

Infectious diseases caused by human acquired immunodeficiency virus (HIV), which is a type of retrovirus, have recently become a serious social problem. A compound of 3'-deoxy-3'-azidothymidine is known as a nucleoside compound used in the clinical treatment for diseases caused by HIV-infection. However, this compound has side-effects since it also exhibits considerable strong toxicity in the host cells.

Although some 2',3'-dideoxyribonucleosides are known as nucleoside compounds exhibiting an anti-viral activity, it is still necessary to develop a substance possessing a higher activity and lower toxicity to the host cell (Hiroaki Mitsuya, Bodily Defense, Vol. 4, pp.213 - 223 (1987)).

On the other hand, various acyclonucleoside compounds have been synthesized since Acyclovir (acycloguanosine) was developed as an antiviral substance effective against herpes virus (C.K. Chu and S.J. Culter, J. Heterocyclic Chem., 23, p.289 (1986)). However, no acyclonucleoside compound having a sufficient activity especially against retroviruses has yet been discovered.

We have focussed our attention on 6-substituted acyclopyrimidine nucleoside compounds and have synthesized various novel 6-substituted acyclopyrimidine nucleoside derivatives and screened those compounds to detect effective antiviral agents, especially to the retrovirus, in order to provide antiviral agents exhibiting an effective activity particularly against retroviruses.

Some 6-substituted acyclopyrimidine nucleoside compounds such as 6-fluoro substituted derivatives, 6-alkylamino substituted derivatives (DD-A-232492) and 6-methyl substituted derivatives (C.A. 107, 129717w (1987)) are known; however, the antiviral activity of these compounds has not been described.

As a result of our researches for compounds exhibiting an effective antiviral activity, particularly anti-retroviral activity, we found that specific 6-substituted pyrimidine nucleoside compounds according to the invention satisfy the above demand to achieve the present invention.

### SUMMARY OF THE INVENTION

The present invention concerns 6-substituted acyclopyrimidine nucleoside derivatives represented by the following general formula I; 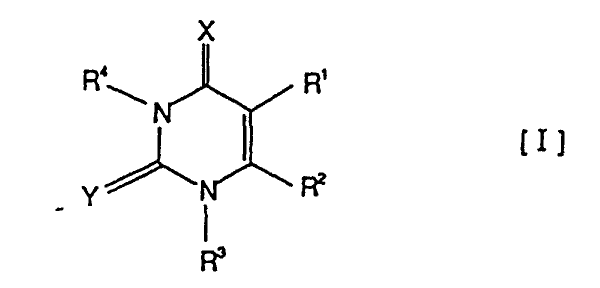 wherein
R¹ represents C₁ to C₁₀ alkyl; C₃ to C₁₀ cycloalkyl; C₂ to C₅ alkenyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkynyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₇ to C₁₃ arylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₈ to C₁₄ arylcarbonylalkyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₆ to C₁₂ arylthio optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; or C₇ to C₁₇ aralkyl group optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups,
R² represents C₆ to C₁₂ arylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₆ to C₁₂ arylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro. amino, cyano and C₂ to C₇ acyl groups; C₃ to C₁₀ cycloalkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkenyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkynyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₇ aralkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₃ arylcarbonyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₈ to C₁₄ arylcarbonylalkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; or C₆ to C₁₂ aryloxy group optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups,
R³ represents a hydrogen atom; methyl; C₃ to C₁₀ branched alkyl; or -CH₂-Z-(CH₂)ₙ-R⁵ group where R⁵ represents a hydrogen atom, halogen atom, 5- or 6-membered heterocyclic carbonyloxy, formyloxy, C₂ to C₁₁ alkylcarbonyloxy, C₄ to C₁₁ cycloalkylcarbonyloxy, C₈ to C₁₂ aralkylcarbonyloxy, C₇ to C₁₃ arylcarbonyloxy, azido, C₂ to C₁₁ alkoxycarbonyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₁ to C₁₀ alkoxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ aralkyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ branched alkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ cycloalkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, or C₆ to C₁₂ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5,
R⁴ represents a hydrogen atom; and
X and Y independently represent an oxygen or sulfur atom; or a pharmaceutically acceptable salt thereof, and antiviral agents containing the derivative or the salt thereof as an active ingredient.
The present invention further concerns the use of 6-substituted acyclopyrimidine nucleoside derivative represented by the general formula I; wherein:
R¹ represents a hydrogen atom; halogen atom; C₁ to C₁₀ alkyl; C₃ to C₁₀ cycloalkyl; C₂ to C₅ alkenyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkynyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₇ to C₁₃ arylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₈ to C₁₄ arylcarbonylalkyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₆ to C₁₂ arylthio optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; or C₇ to C₁₇ aralkyl group optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups,
R² represents C₆ to C₁₂ arylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₆ to C₁₂ arylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₃ to C₁₀ cycloalkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkenyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkynyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₇ aralkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₃ arylcarbonyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₈ to C₁₄ arylcarbonylalkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; or C₆ to C₁₂ aryloxy group optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino; cyano and C₂ to C₇ acyl groups,
R³ represents a hydrogen atom; methyl; C₃ to C₁₀ branched alkyl; or -CH₂-Z-(CH₂)ₙ-R⁵ group where R⁵ represents a hydrogen atom, halogen atom, 5- or 6-membered heterocyclic carbonyloxy, formyloxy, C₂ to C₁₁ alkylcarbonyloxy, C₄ to C₁₁ cycloalkylcarbonyloxy, C₈ to C₁₂ aralkylcarbonyloxy, C₇ to C₁₃ arylcarbonyloxy, azido, C₂ to C₁₁ alkoxycarbonyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₁ to C₁₀ alkoxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ aralkyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ branched alkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ cycloalkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, or C₆ to C₁₂ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5,
R⁴ represents a hydrogen atom, C₁ to C₁₃ alkyl or C₇ to C₁₃ aralkyl group; and
X and Y independently represent an oxygen or sulfur atom;
or a pharmaceutically acceptable salt thereof,
in a process for the production of a pharmaceutical composition for the treatment or prevention of diseases caused by viral, particularly retroviral infection, especially in human beings.

### DETAILED DESCRIPTION OF THE INVENTION

The 6-substituted acyclopyrimidine nucleoside derivatives according to the invention are represented by the general formula I.

The group of R¹ represents C₁ to C₁₀ alkyl group such as methyl, ethyl, n-propyl, i-propyl and n-butyl; C₃ to C₁₀ cycloalkyl group such as cyclopropyl, cyclobutyl, cyclopentyl, cyclohexyl and cycloheptyl; C₂ to C₅ alkenyl group such as vinyl, propenyl, butenyl, phenylvinyl, bromovinyl, cyanovinyl, alkoxycarbonylvinyl and carbamoylvinyl; C₂ to C₅ alkynyl group such as ethynyl, propynyl and phenylethynyl; C₂ to C₅ alkylcarbonyl group such as acetyl, propionyl, and i-butyryl; C₇ to C₁₃ arylcarbonyl group such as benzoyl and naphthoyl; arylcarbonylalkyl group such as phenacyl; C₆ to C₁₂ arylthio group such as phenylthio, tolylthio and naphthylthio; or C₇ to C₁₇ aralkyl group such as benzyl.

The group of R² represents C₆ to C₁₂ arylthio group such as phenylthio and naphthylthio; C₁ to C₁₀ alkylthio group such as methylthio, ethylthio, propylthio, butylthio and pentylthio; C₃ to C₁₀ cycloalkylthio group such as cyclopentylthio, cyclohexylthio and cycloheptylthio; C₆ ot C₁₂ arylsulfinyl group such as phenylsulfinyl; alkylsulfinyl group such as methylsulfinyl, ethylsulfinyl and butylsulfinyl; C₃ to C₁₀ cycloalkylsulfinyl group such as cyclopentylsulfinyl and cyclohexylsulfinyl; C₂ to C₅ alkenyl group such as vinyl, propenyl and phenylvinyl; C₂ to C₅ alkynyl group such as ethynyl, propynyl and phenylethynyl; C₇ to C₁₇ aralkyl group such as benzyl; C₇ to C₁₃ arylcarbonyl group such as benzoyl; C₈ to C₁₄ arylcarbonylalkyl group such as phenacyl; or C₆ to C₁₂ aryloxy group such as phenoxy, and those groups may be optionally substituted by one or more of substituents selected from a halogen atom such as chlorine, bromine, fluorine and iodine, C₁ to C₅ alkyl group such as methyl, ethyl, propyl, butyl and pentyl, a C₁ to C₅ halogenated alkyl group such as trifluoromethyl, C₁ to C₅ alkoxy group such as methoxy, ethoxy, propoxy and butoxy, hydroxyl group, nitro group, amino group, cyano group and C₂ to C₇ acyl group such as acetyl.

The group of R³ represents a hydrogen atom, methyl group, C₃ to C₁₀ branched alkyl group such as i-propyl and t-butyl or -CH₂-Z-(CH₂)ₙ-R⁵ group where R⁵ represents a hydrogen atom; halogen atom such as fluorine, chlorine, iodine and bromine; 5- or 6-membered heterocyclic carbonyloxy group such as nicotinoyloxy; formyloxy group; optionally branched C₂ to C₁₁ alkylcarbonyloxy group such as acetoxy, propyonyloxy, n-butyryloxy, i-butyryloxy, valeryloxy, hexanoyloxy, heptanoyloxy and decanoyloxy; C₄ to C₁₁ cycloalkylcarbonyloxy group such as cyclohexylcarbonyloxy; C₈ to C₁₂ aralkylcarbonyloxy group such as benzylcarbonyloxy; C₇ to C₁₃ arylcarbonyloxy group such as benzoyloxy, toluoylcarbonyloxy and naphthoylcarbonyloxy group; azido group; C₂ to C₁₁ alkoxycarbonyloxy group such as methoxycarbonyloxy, ethoxycarbonyloxy, n-propoxycarbonyloxy, i-propoxycarbonyloxy, n-butoxycarbonyloxy and t-butoxycarbonyloxy group, optionally substituted by one or more substituents selected from a halogen atom such as fluorine, chlorine, bromine and iodine, C₆ to C₁₂ aryl group such as phenyl, toluyl and naphthyl, C₁ to C₅ alkyl group such as methyl, ethyl, n-propyl, i-propyl, n-butyl and t-butyl, C₁ to C₅ alkoxy group such as methoxy, ethoxy, n-propoxy, i-propoxy, n-butoxy and t-butoxy and C₁ to C₅ halogenated alkyl group such as trifluoromethyl; C₂ to C₁₁ N-alkylcarbamoyloxy group such as N-methylcarbamoyloxy, N-ethylcarbamoyloxy, N-propylcarbamoyloxy, N-butylcarbamoyloxy and N-pentylcarbamoyloxy, optionally substituted by one or more substituents selected from a halogen atom such as fluorine, chlorine, bromine and iodine, C₆ to C₁₂ aryl group such as phenyl, toluyl and naphthyl, C₁ to C₅ alkyl group such as methyl, ethyl, n-propyl, i-propyl, n-butyl and t-butyl, C₁ to C₅ alkoxy group such as methoxy, ethoxy, n-propoxy, i-propoxy, n-butoxy and t-butoxy and C₁ to C₅ halogenated alkyl group such as trifluoromethyl; C₇ to C₁₃ N-arylcarbamoyloxy group such as N-phenylcarbamoyloxy and N-tolylcarbamoyloxy, optionally substituted by one or more substituents selected from a halogen atom such as fluorine, chlorine, bromine and iodine, C₆ to C₁₂ aryl group such as phenyl, toluyl and naphthyl, C₁ to C₅ alkyl group such as methyl, ethyl, n-propyl, i-propyl, n-butyl and t-butyl, C₁ to C₅ alkoxy group such as methoxy, ethoxy, n-propoxy, i-propoxy, n-butoxy and t-butoxy and C₁ to C₅ halogenated alkyl group such as trifluoromethyl; C₂ to C₁₁ N-alkylthiocarbamoyloxy group such as N-methythiocarbamoyloxy, N-ethylthiocarbamoyloxy, N-propylthiocarbamoyloxy, N-butylthiocarbamoyloxy and N-pentylthiocarbamoyloxy, optionally substituted by one or more substituents selected from a halogen atom such as fluorine, chlorine, bromine and iodine, C₆ to C₁₂ aryl group such as phenyl, toluyl and naphthyl, C₁ to C₅ alkyl group such as methyl, ethyl, n-propyl, i-propyl, n-butyl and t-butyl, C₁ to C₅ alkoxy group such as methoxy, ethoxy, n-propoxy, i-propoxy, n-butoxy and t-butoxy and C₁ to C₅ halogenated alkyl group such as trifluoromethyl; C₇ to C₁₃ N-arylthiocarbamoyloxy group such as N-phenylthiocarbamoyloxy and N-tolylthiocarbamoyloxy, optionally substituted by one or more substituents selected from a halogen atom such as fluorine, chlorine, bromine and iodine, C₆ to C₁₂ aryl group such as phenyl, toluyl and naphthyl, C₁ to C₅ alkyl group such as methyl, ethyl, n-propyl, i-propyl, n-butyl and t-butyl, C₁ to C₅ alkoxy group such as methoxy, ethoxy, n-propoxy, i-propoxy, n-butoxy and t-butoxy and C₁ to C₅ halogenated alkyl group such as trifluoromethyl; C₁ to C₁₀ alkoxy group such as methoxy, ethoxy, n-propoxy, i-propoxy, n-butoxy, t-butoxy, n-pentyloxy and n-hexyloxy group, optionally substituted by one or more substituents selected from a halogen atom such as fluorine, chlorine, bromine and iodine, C₆ to C₁₂ aryl group such as phenyl, toluyl and naphthyl, C₁ to C₅ alkyl group such as methyl, ethyl, n-propyl, i-propyl, n-butyl and t-butyl, C₁ to C₅ alkoxy group such as methoxy, ethoxy, n-propoxy, i-propoxy, n-butoxy and t-butoxy and C₁ to C₅ halogenated alkyl group such as trifluoromethyl; C₃ to C₁₀ branched alkyl group such as i-propyl, i-butyl, sec-bytyl, t-butyl, i-heptyl and i-hexyl, optionally substituted by one or more substituents selected from a halogen atom such as fluorine, chlorine, bromine and iodine, C₆ to C₁₂ aryl group such as phenyl, toluyl and naphthyl, C₁ to C₅ alkyl group such as methyl, ethyl, n-propyl, i-propyl, n-butyl and t-butyl, C₁ to C₅ alkoxy group such as methoxy, ethoxy, n-propoxy, i-propoxy, n-butoxy and t-butoxy and C₁ to C₅ halogenated alkyl group such as trifluoromethyl; C₃ to C₁₀ cycloalkyl group such as cyclopropyl, cyclobutyl, cyclopentyl, cyclohexyl and cycloheptyl, optionally substituted by one or more substituents selected from a halogen atom such as fluorine, chlorine, bromine and iodine, C₆ to C₁₂ aryl group such as phenyl, toluyl and naphthyl, C₁ to C₅ alkyl group such as methyl, ethyl, n-propyl, i-propyl, n-butyl and t-butyl, C₁ to C₅ alkoxy group such as methoxy, ethoxy, n-propoxy, i-propoxy, n-butoxy and t-butoxy and C₁ to C₅ halogenated alkyl group such as trifluoromethyl; or C₆ to C₁₂ aryl group such as phenyl, optionally substituted by one or more substituents selected from a halogen atom such as fluorine, chlorine, bromine and iodine, C₆ to C₁₂ aryl group such as phenyl, toluyl and naphthyl, C₁ to C₅ alkyl group such as methyl, ethyl, n-propyl, i-propyl, n-butyl and t-butyl, C₁ to C₅ alkoxy group such as methoxy, ethoxy, n-propoxy, i-propoxy, n-butoxy and t-butoxy and C₁ to C₅ halogenated alkyl group such as trifluoromethyl, and Z represents an oxygen, sulfur atom or methylene group, and n represents 0 or an integer of 1 to 5,
R⁴ represents a hydrogen atom;
X and Y represent oxygen or sulfur atom independently.

For the production of a pharmaceutical composition for the treatment or prevention of diseases caused by viral, particularly retroviral infection, especially in human beings the above 6-substituted acyclopyrimidine nucleoside derivatives represented by the general formula I can be used, wherein:
the group R¹ in addition to the above meaning represents a hydrogen atom; and halogen atom such as chlorine, iodine, bromine and fluorine; and
R⁴ represents a hydrogen atom, C₁ to C₁₃ alkyl or C₇ to C₁₃ aralkyl group.

The preferred compounds according to the invention have R¹ of C₁ to C₅ alkyl group or C₂ to C₅ alkenyl group, particularly C₁ to C₅ alkyl group; R² of C₆ to C₁₀ arylthio group, C₃ to C₁₀ cycloalkylthio group or C₇ to C₁₁ aralkyl group, particularly C₆ to C₁₀ arylthio, C₃ to C₁₀ cycloalkylthio or C₇ to C₁₁ aralkyl group, optionally substituted by one or more substituents selected from a halogen atom, C₁ to C₅ alkyl, C₁ to C₅ alkoxy and nitro groups; R³ of a hydrogen atom, methyl or -CH₂-Z-(CH₂)ₙ-R⁵ group where R⁵ represents a hydrogen atom, halogen atom, 5- or 6 membered heterocyclic carbonyloxy, C₂ to C₁₁ alkylcarbonyloxy, C₄ to C₁₀ cycloalkylcarbonyloxy, C₈ to C₁₂ aralkylcarbonyloxy, C₇ to C₁₃ arylcarbonyloxy, C₂ to C₁₁ alkoxycarbonyloxy, C₈ to C₁₀ aralkyloxycarbonyloxy, C₂ to C₈ N-alkylcarbamoyloxy, C₇ to C₁₃ N-arylcarbamoyloxy, C₂ to C₈ N-alkylthiocarbamoyloxy, C₇ to C₁₃ N-arylthiocarbamoyloxy, C₁ to C₁₀ alkoxy, C₇ to C₁₃ aralkyloxy, azido, C₃ to C₅ branched alkyl, C₅ to C₇ cycloalkyl or C₆ to C₁₀ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5; R⁴ of a hydrogen atom; X of an oxygen or sulfur atom; and Y of an oxygen or sulfur atom.

The preferred compounds used according to the invention have R¹ of a hydrogen atom, halogen atom, C₁ to C₅ alkyl group or C₂ to C₅ alkenyl group, particularly C₁ to C₅ alkyl group; R² of C₆ to C₁₀ arylthio group, C₃ to C₁₀ cycloalkylthio group or C₇ to C₁₁ aralkyl group, particularly C₆ to C₁₀ arylthio, C₃ to C₁₀ cycloalkylthio or C₇ to C₁₁ aralkyl group, optionally substituted by one or more substituents selected from a halogen atom, C₁ to C₅ alkyl, C₁ to C₅ alkoxy and nitro groups; R³ of a hydrogen atom, methyl or -CH₂-Z-(CH₂)ₙ-R⁵ group where R⁵ represents a hydrogen atom, halogen atom, 5- or 6 membered heterocyclic carbonyloxy, C₂ to C₁₁ alkylcarbonyloxy, C₄ to C₁₀ cycloalkylcarbonyloxy, C₈ to C₁₂ aralkylcarbonyloxy, C₇ to C₁₃ arylcarbonyloxy, C₂ to C₁₁ alkoxycarbonyloxy, C₈ to C₁₀ aralkyloxycarbonyloxy, C₂ to C₈ N-alkylcarbamoyloxy, C₇ to C₁₃ N-arylcarbamoyloxy, C₂ to C₈ N-alkylthiocarbamoyloxy, C₇ to C₁₃ N-arylthiocarbamoyloxy, C₁ to C₁₀ alkoxy, C₇ to C₁₃ aralkyloxy, azido, C₃ to C₅ branched alkyl, C₅ to C₇ cycloalkyl or C₆ to C₁₀ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5; R⁴ of a hydrogen atom, C₁ to C₁₃ alkyl or C₇ to C₁₁ aralkyl group; X of an oxygen or sulfur atom; and Y of an oxygen or sulfur atom.

Examples of the preferred compounds according to the present invention are listed in Table 1 below.

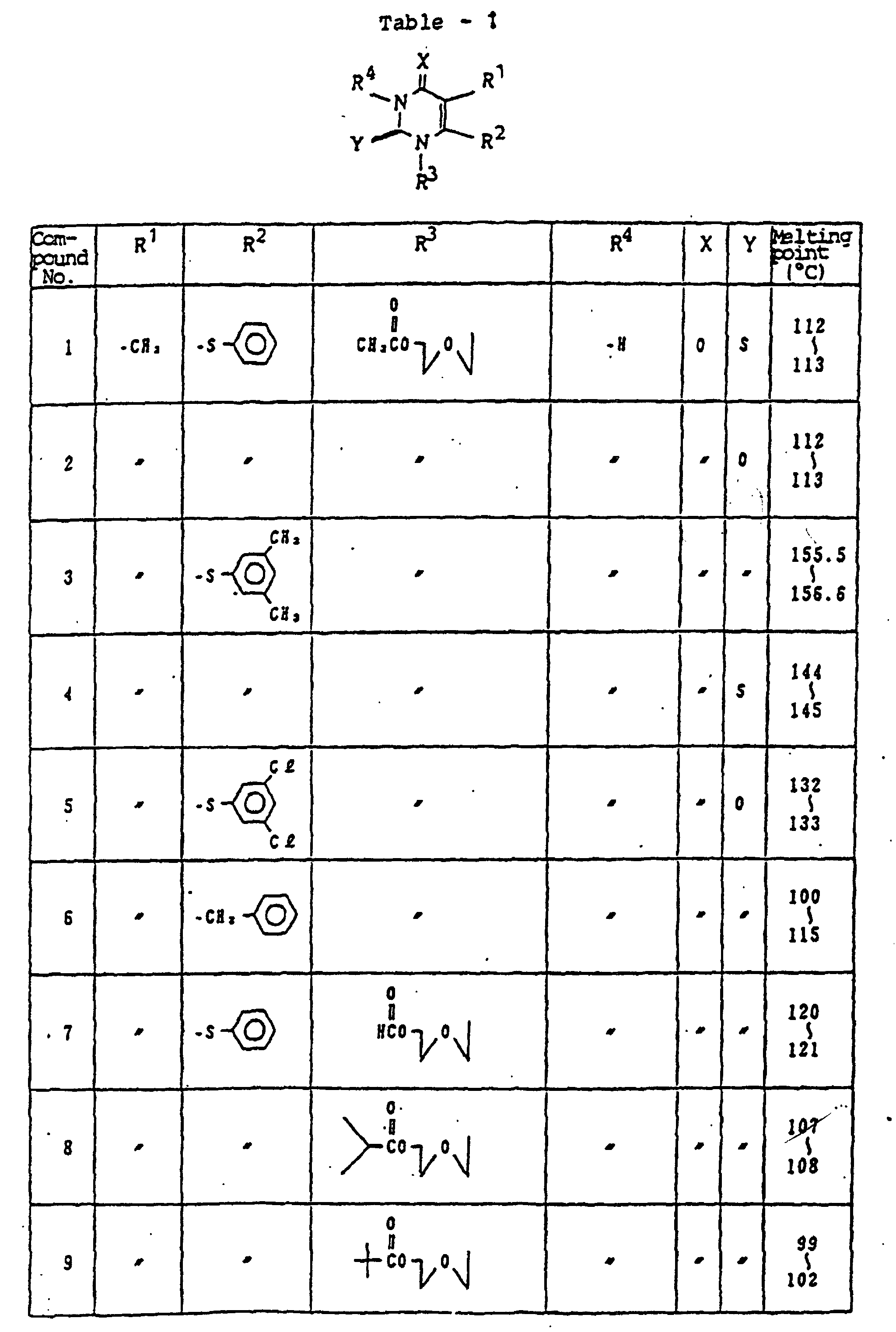

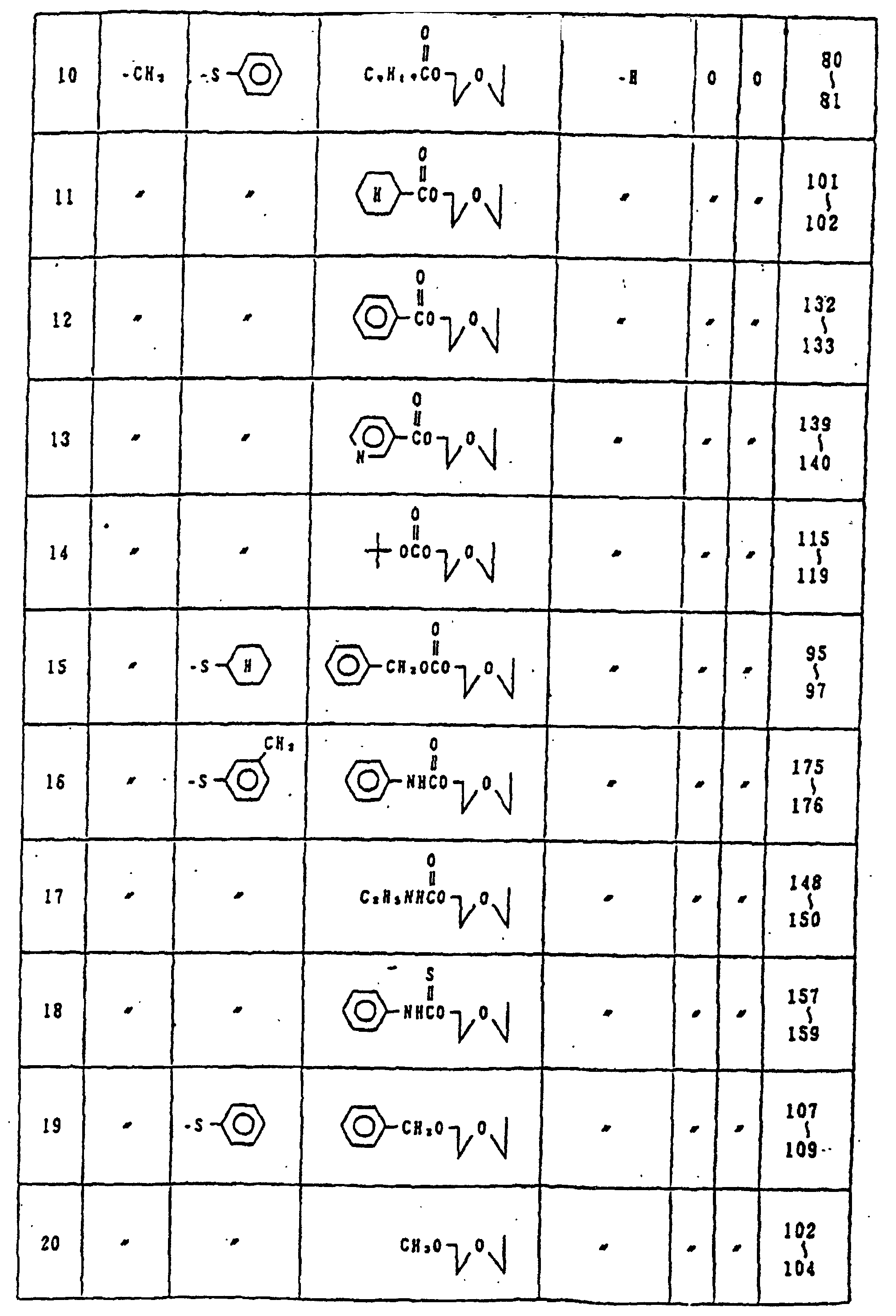

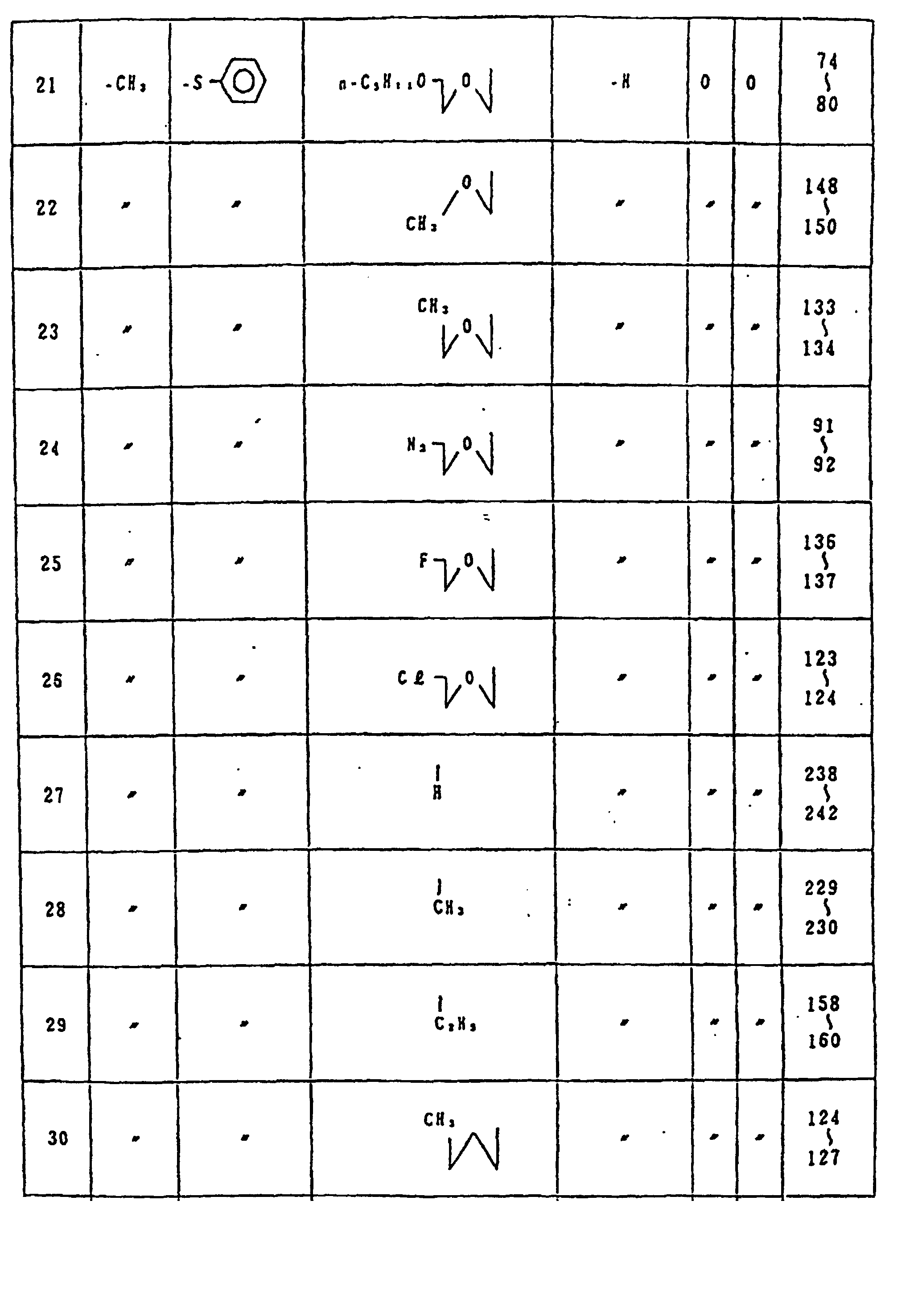

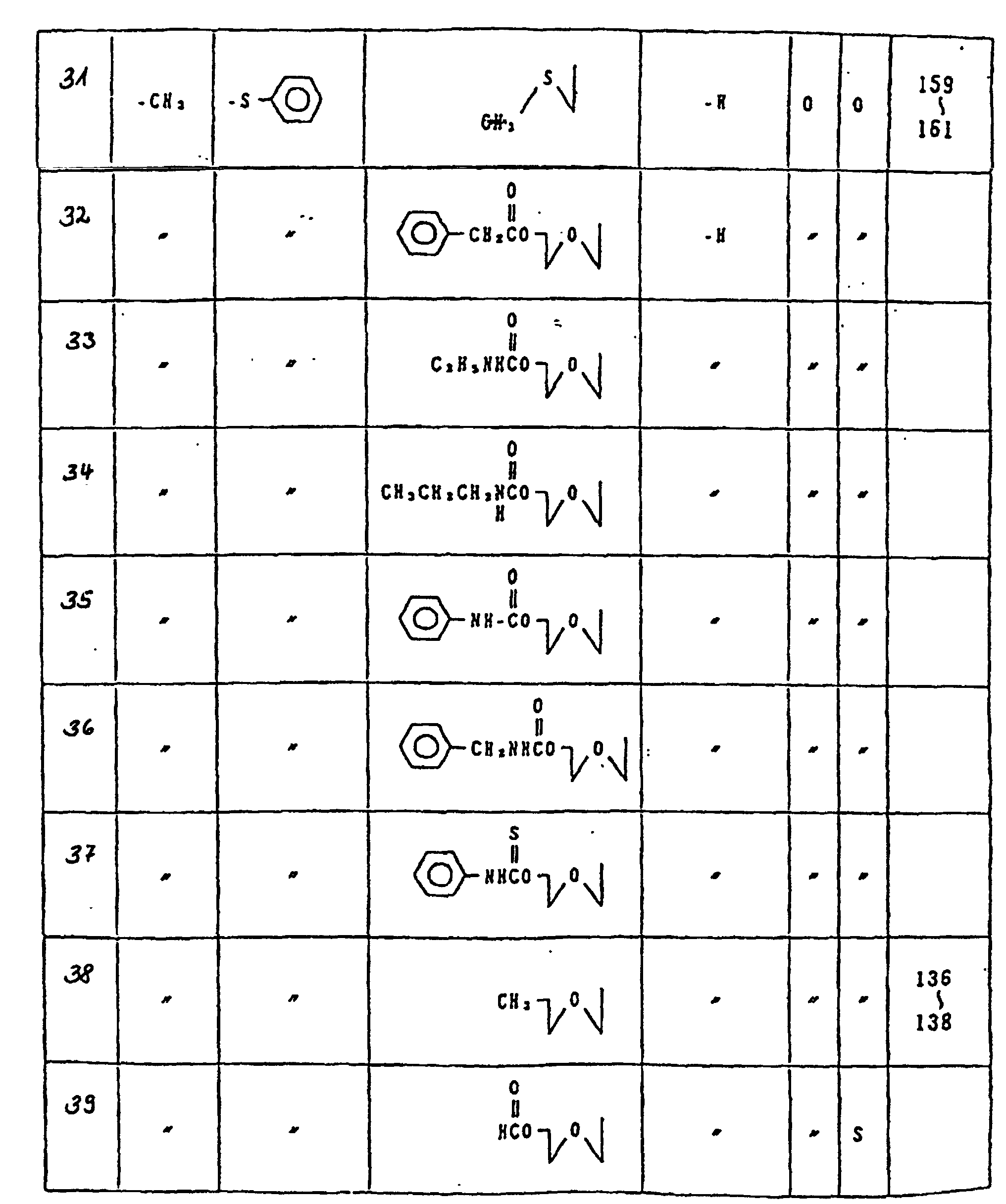

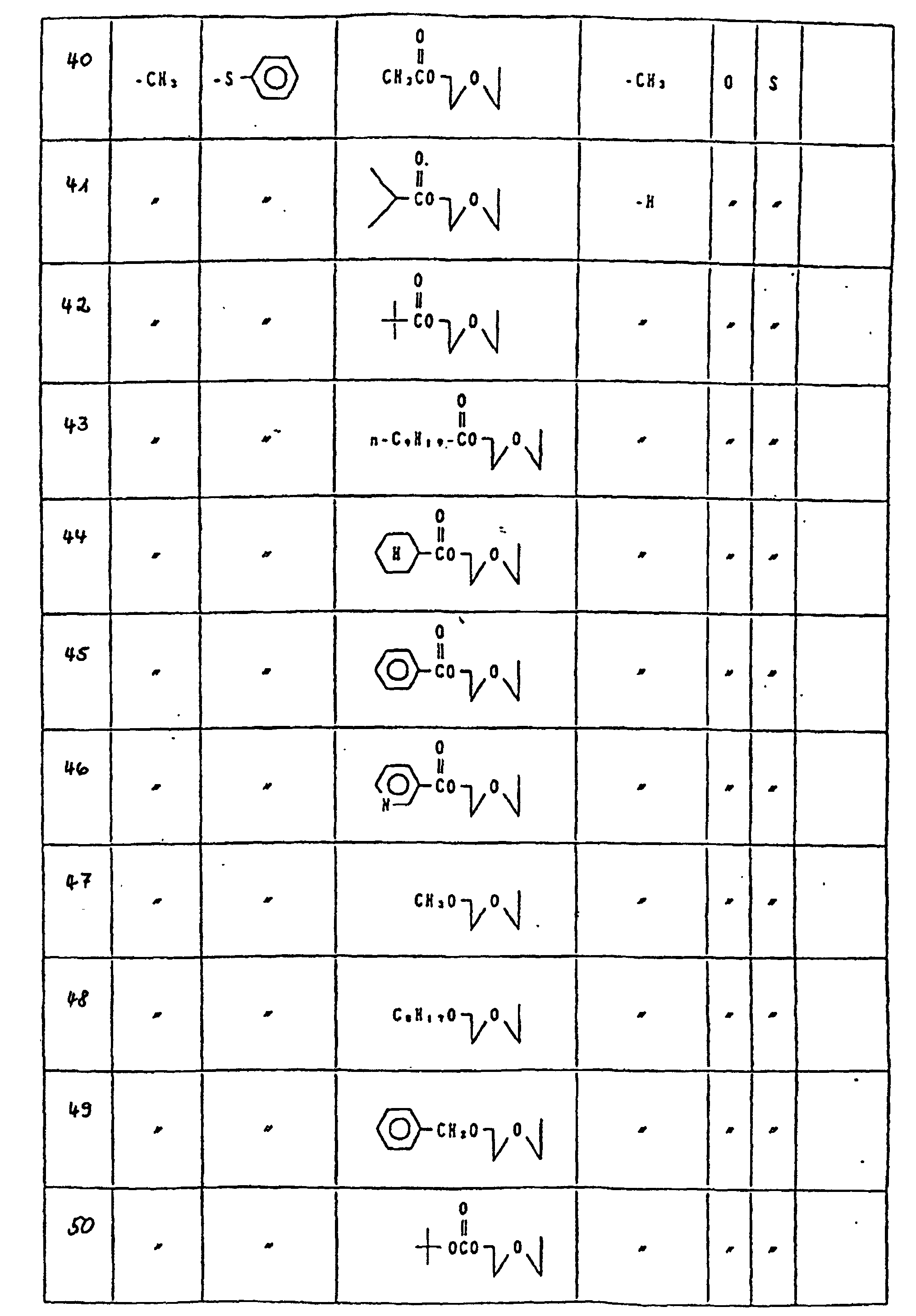

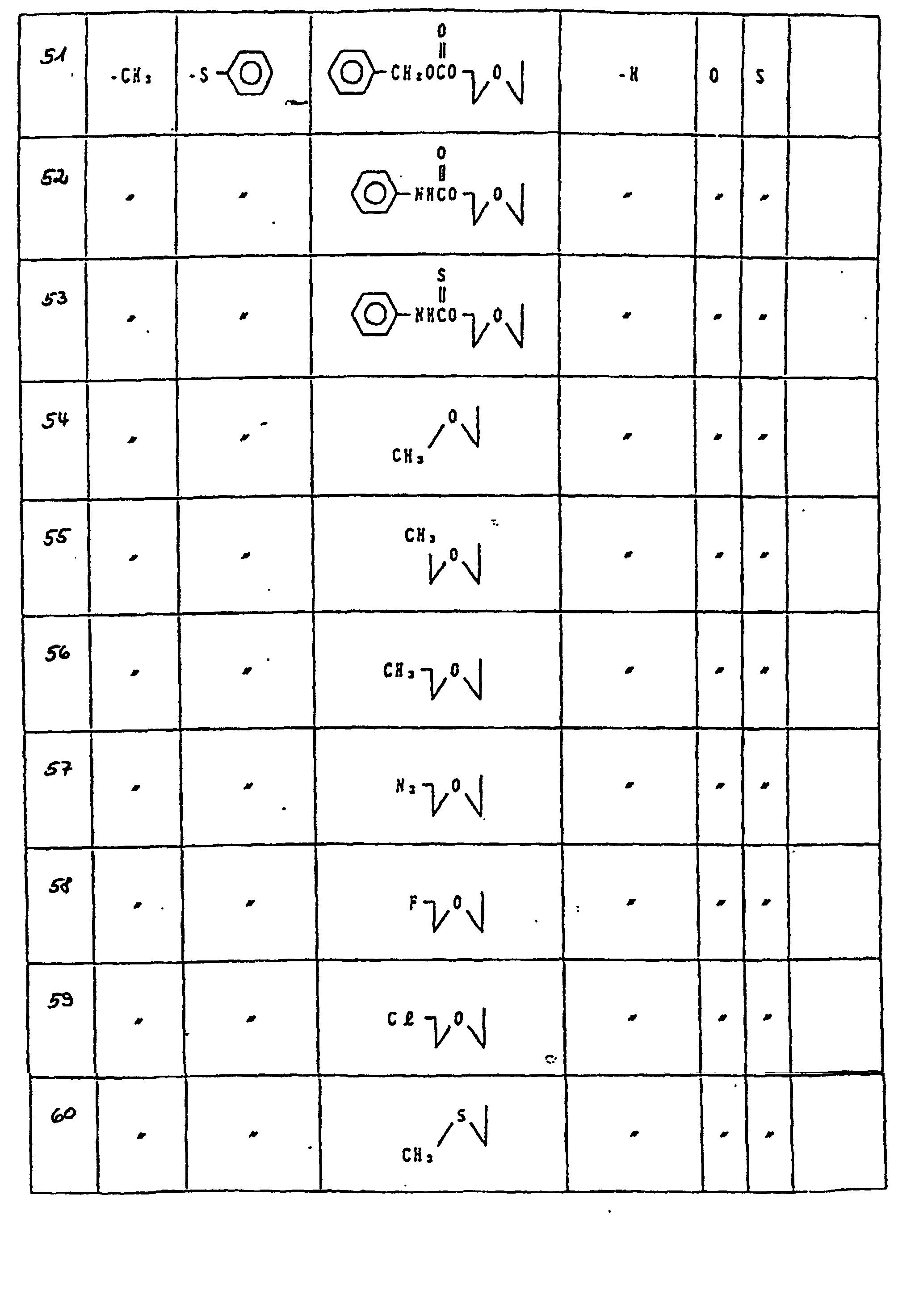

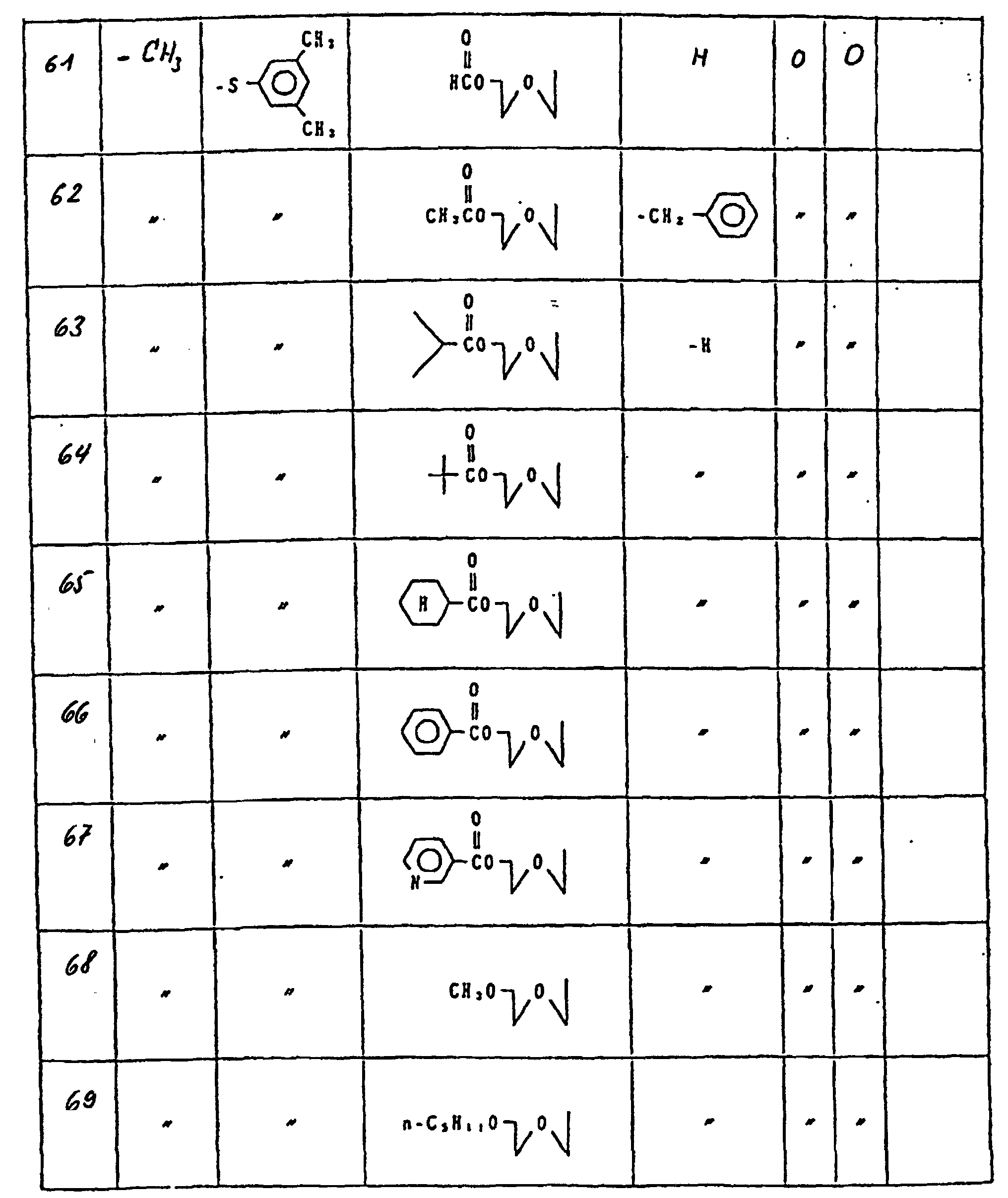

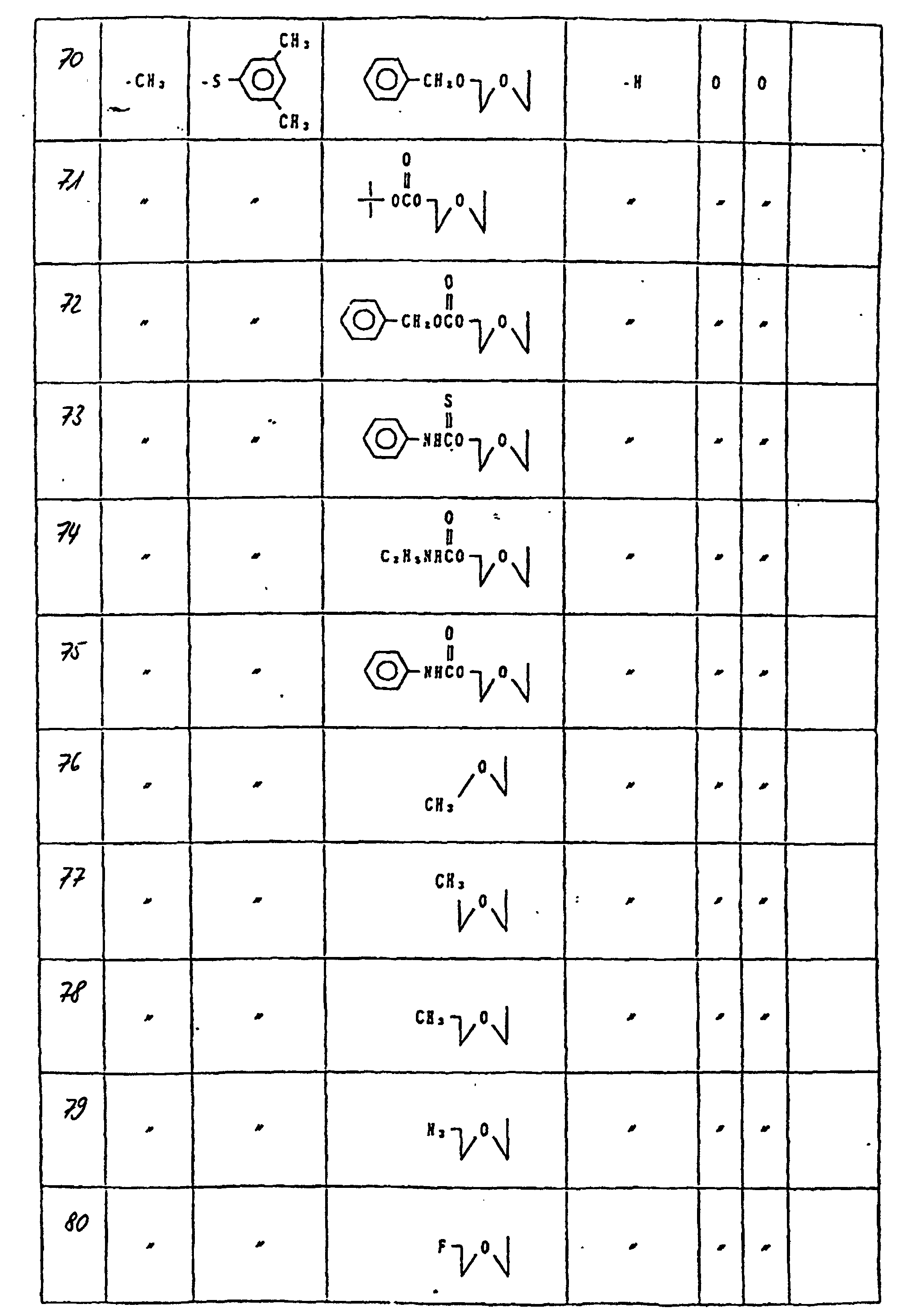

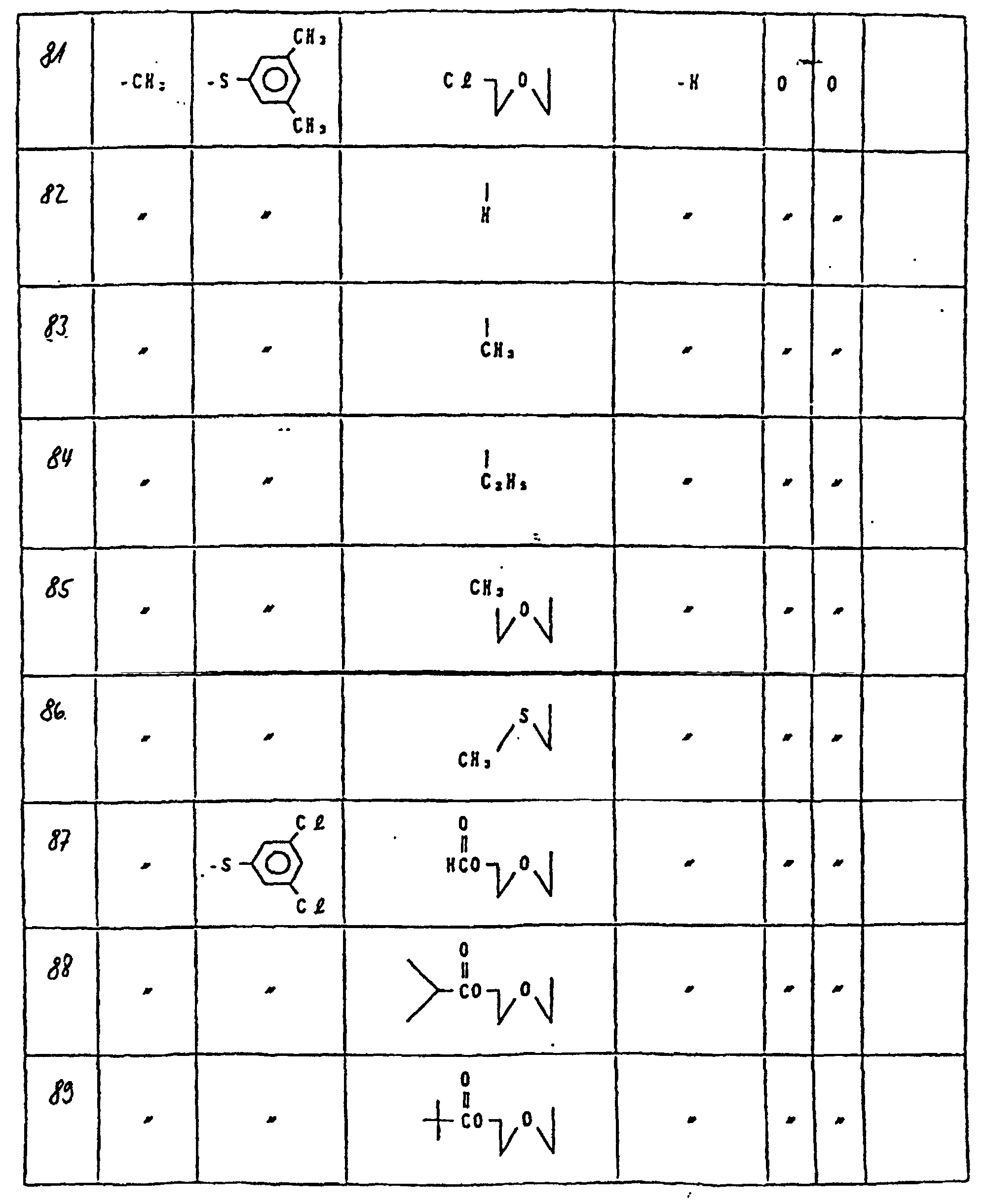

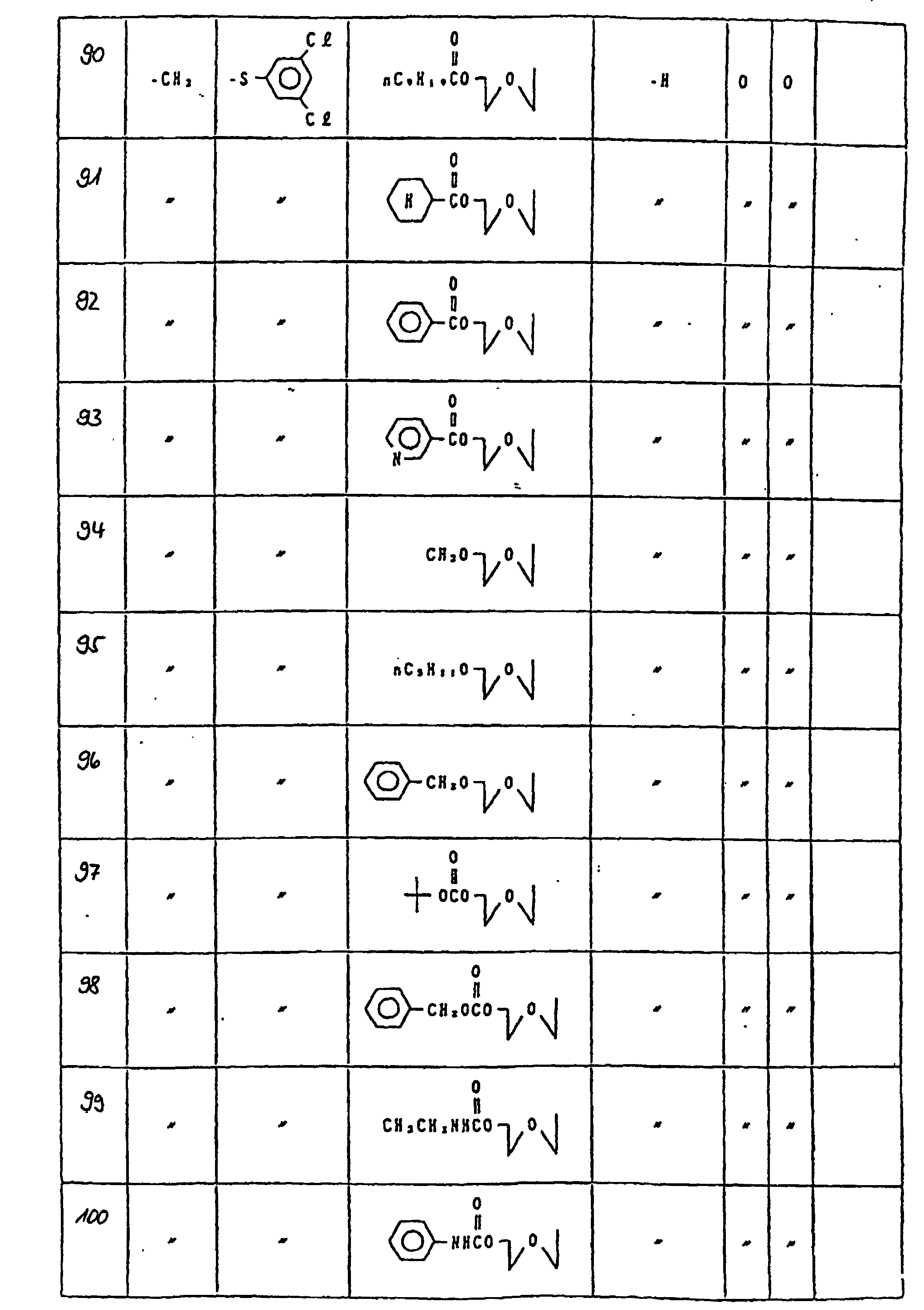

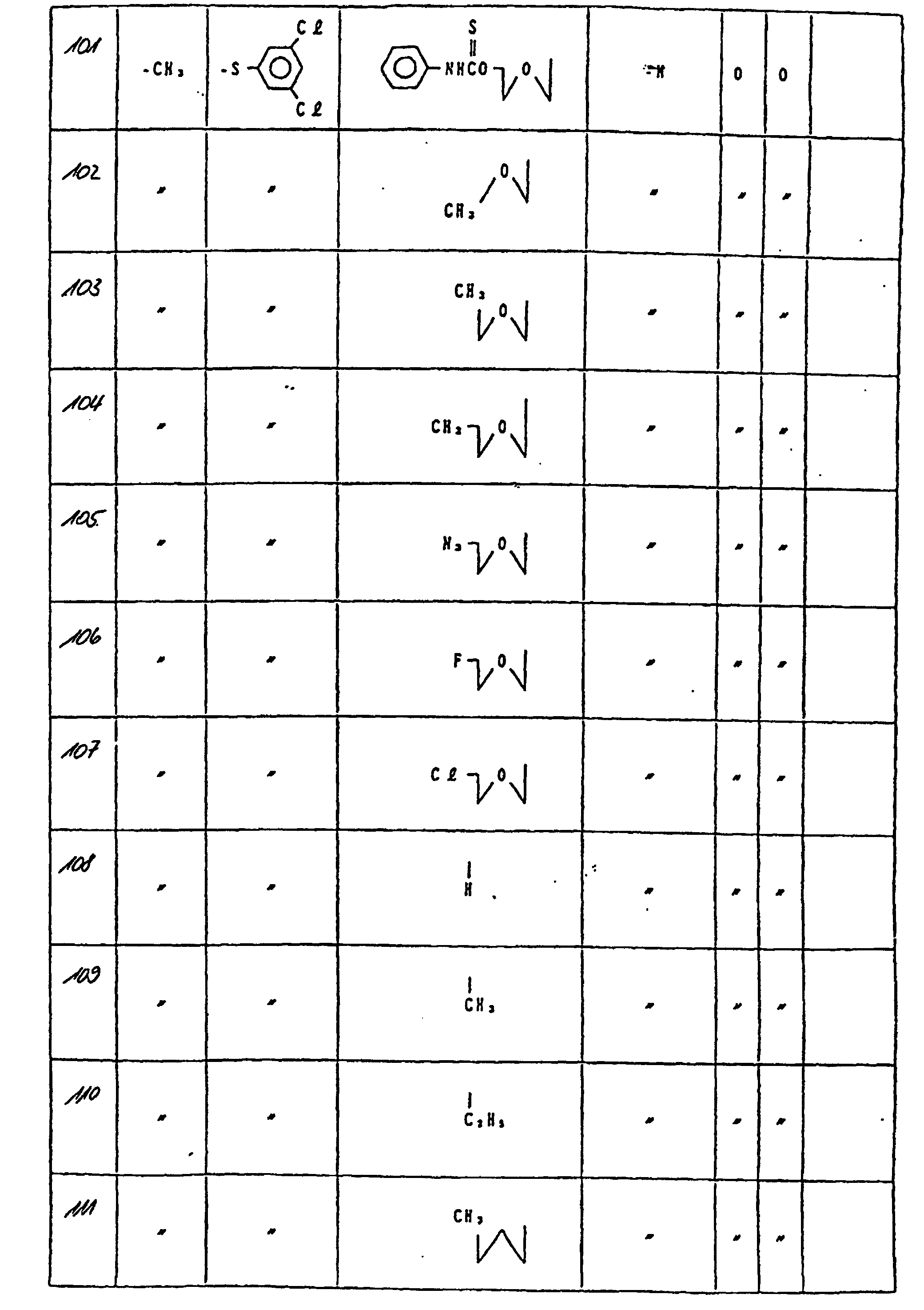

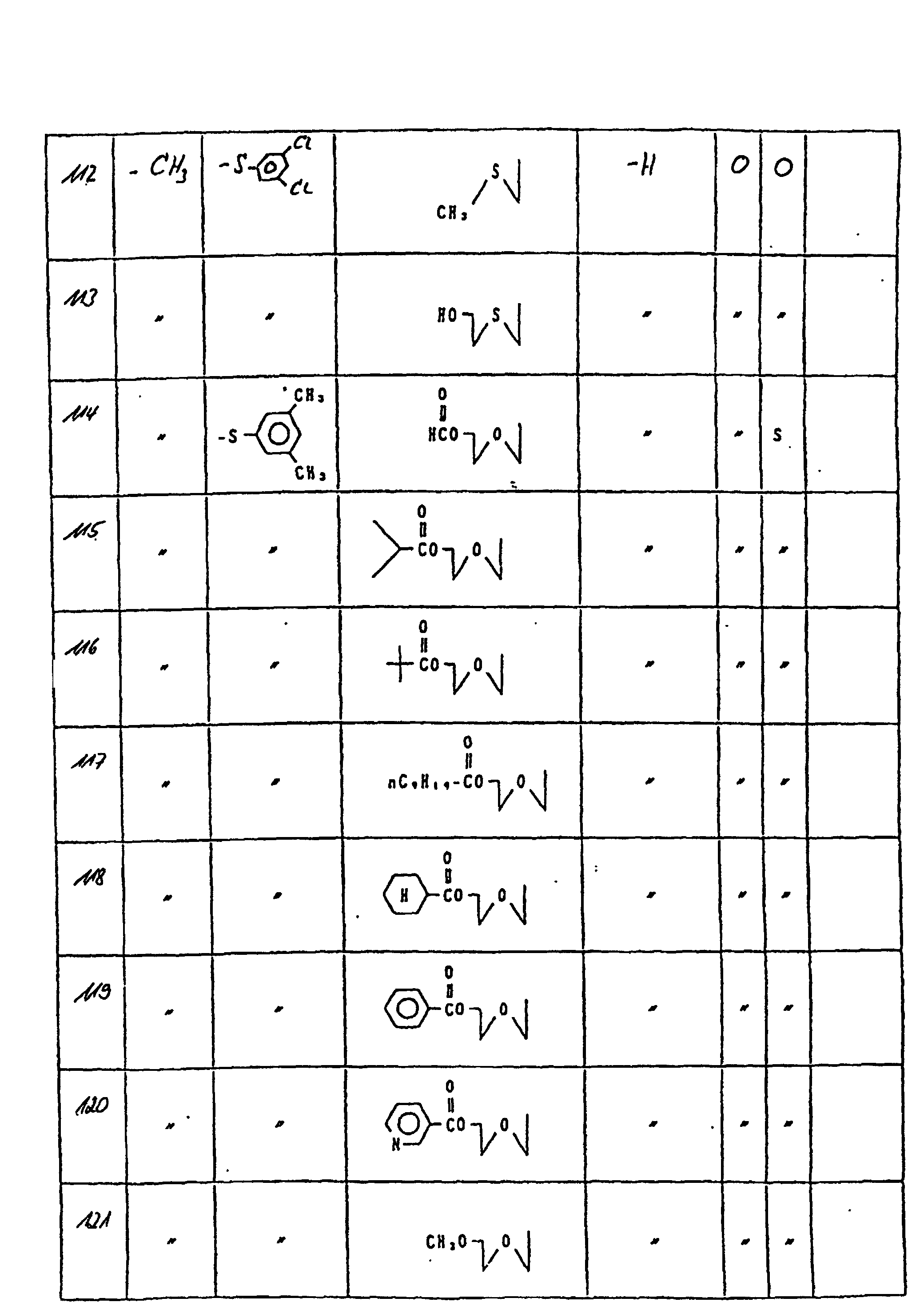

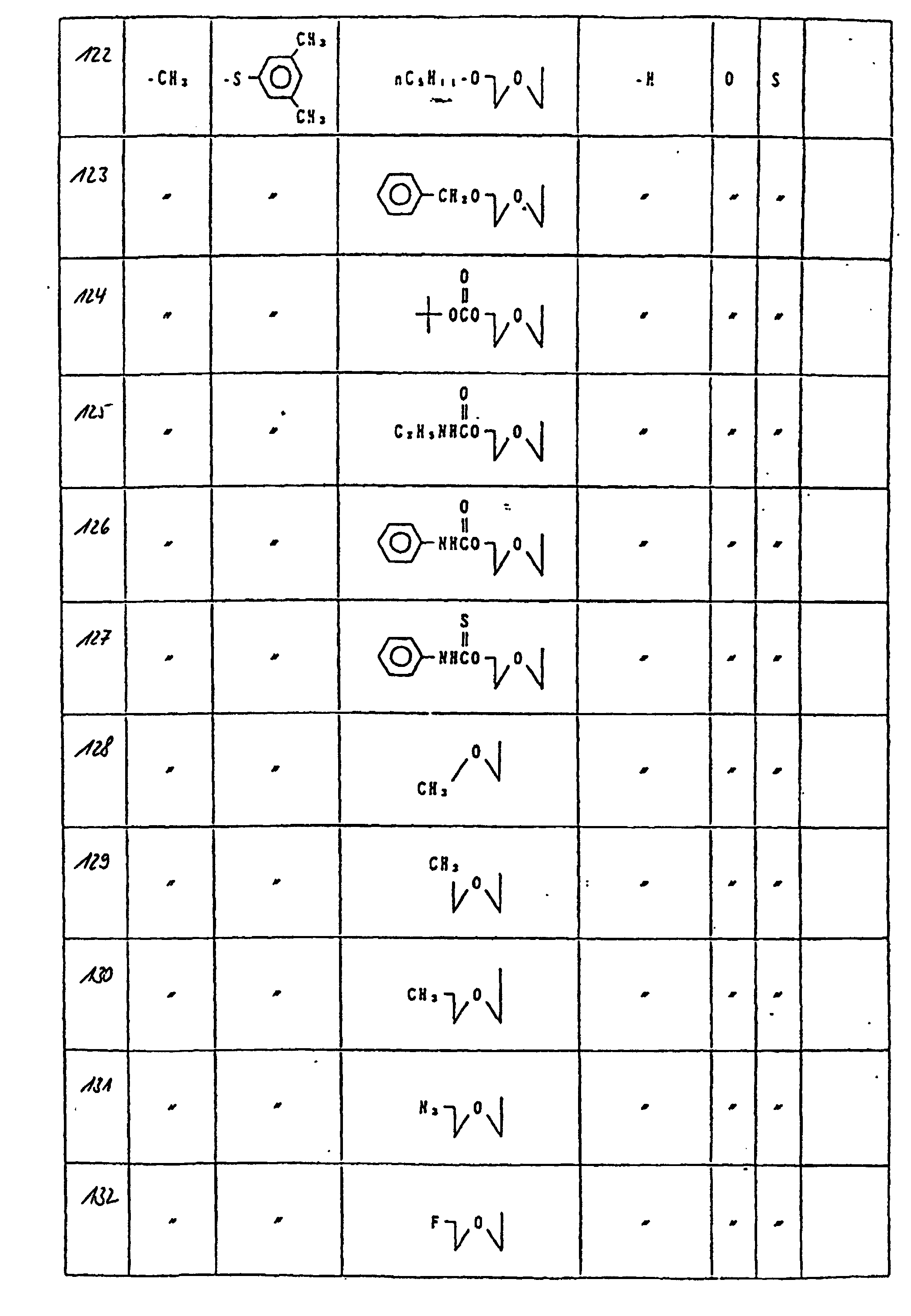

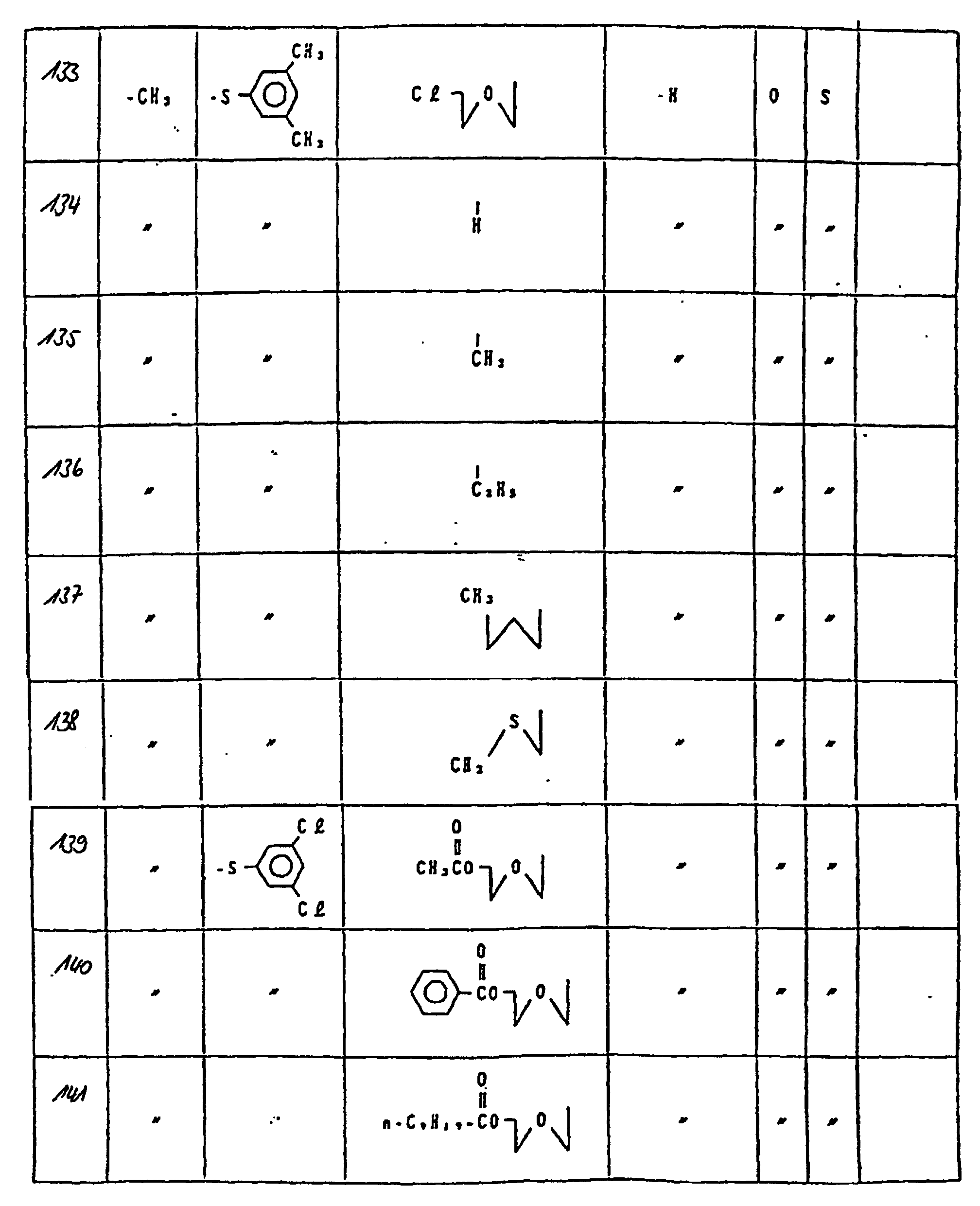

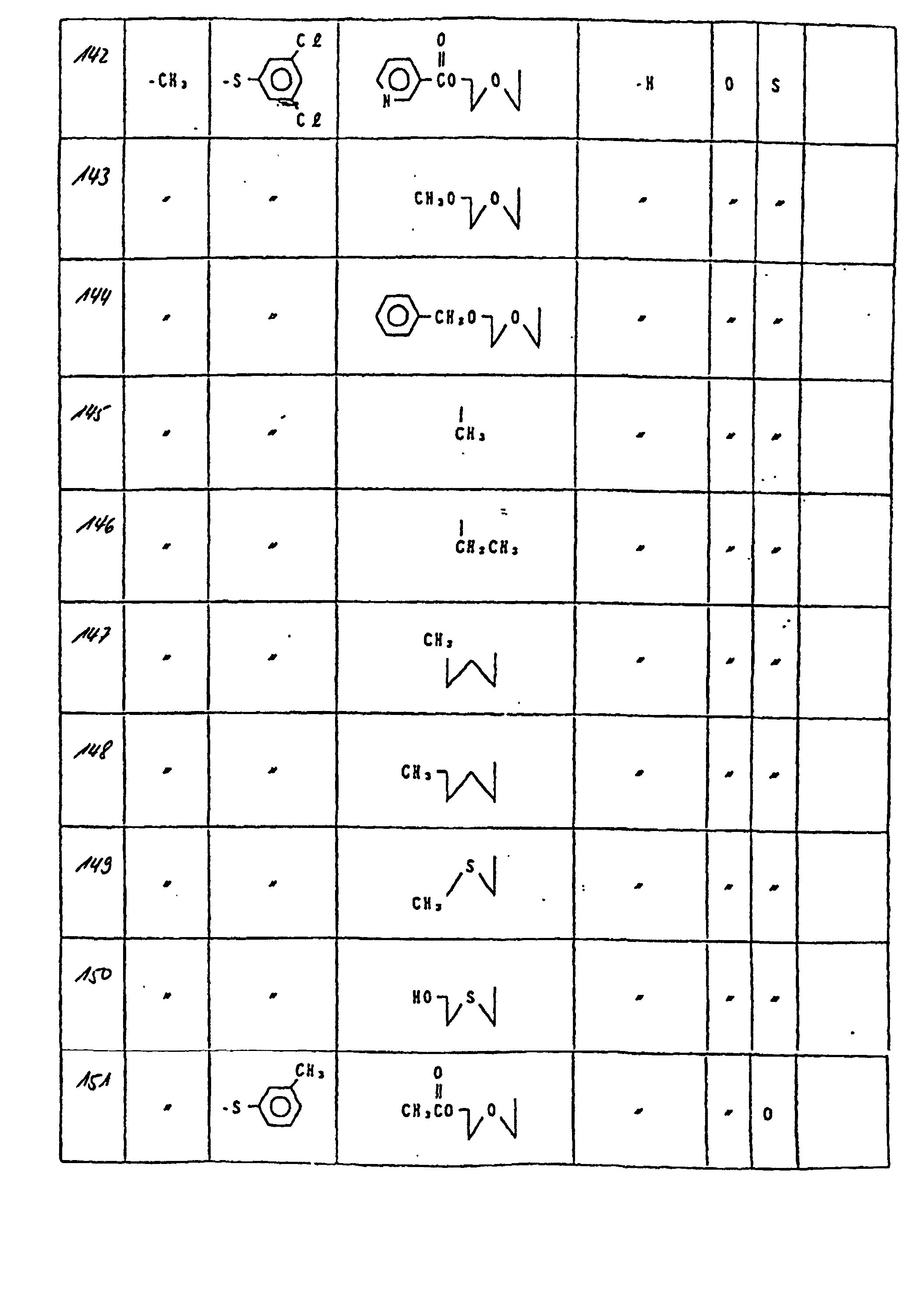

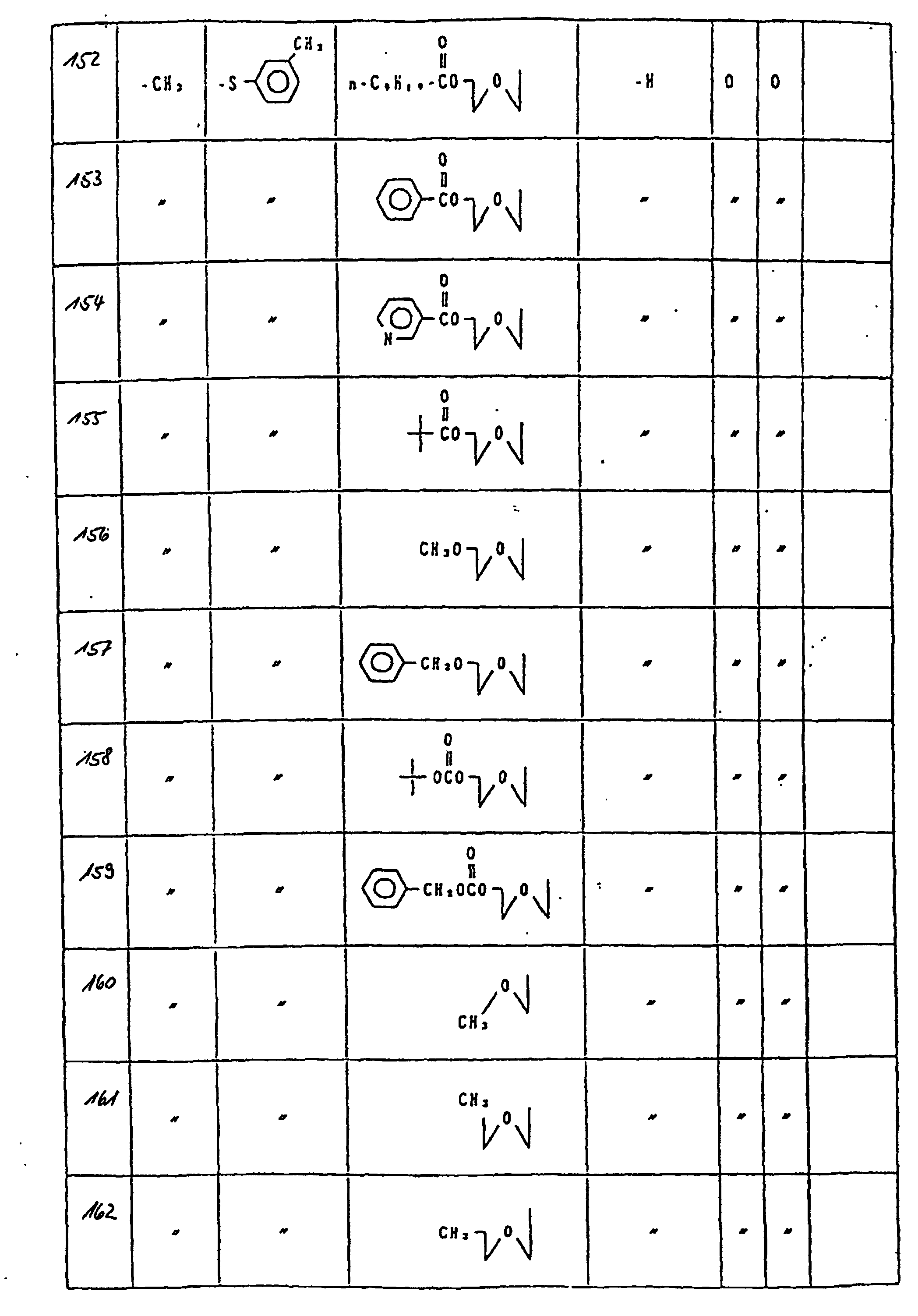

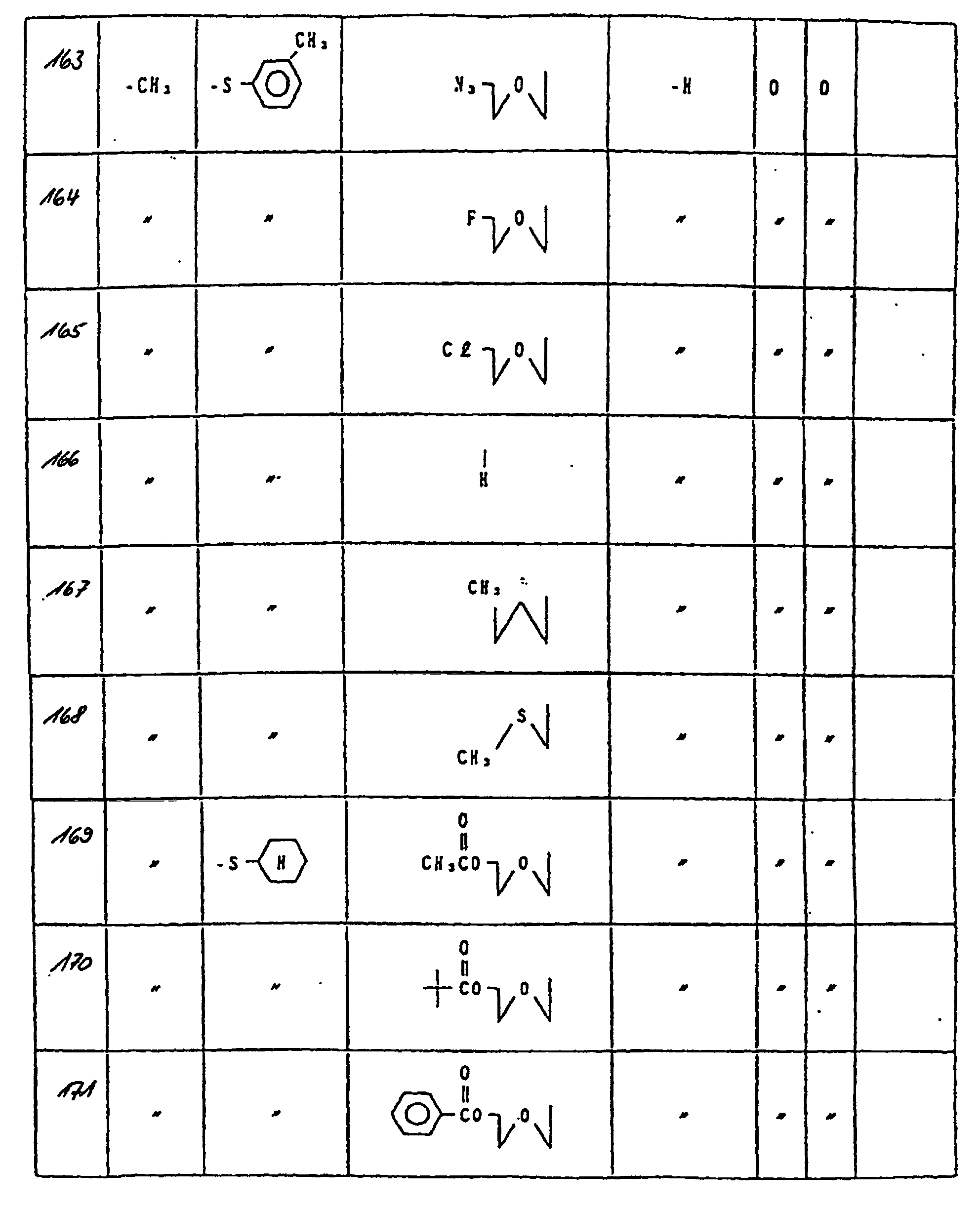

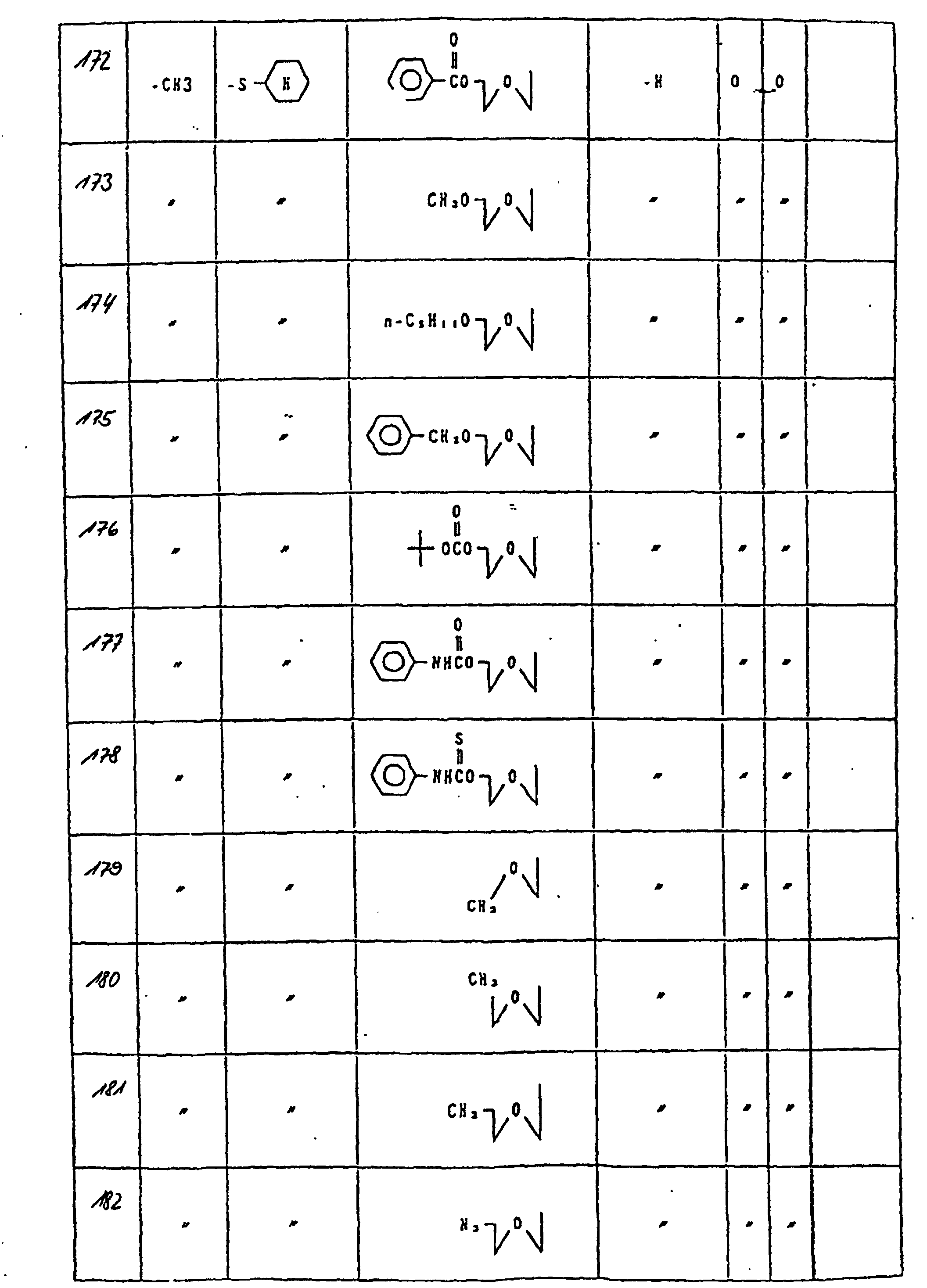

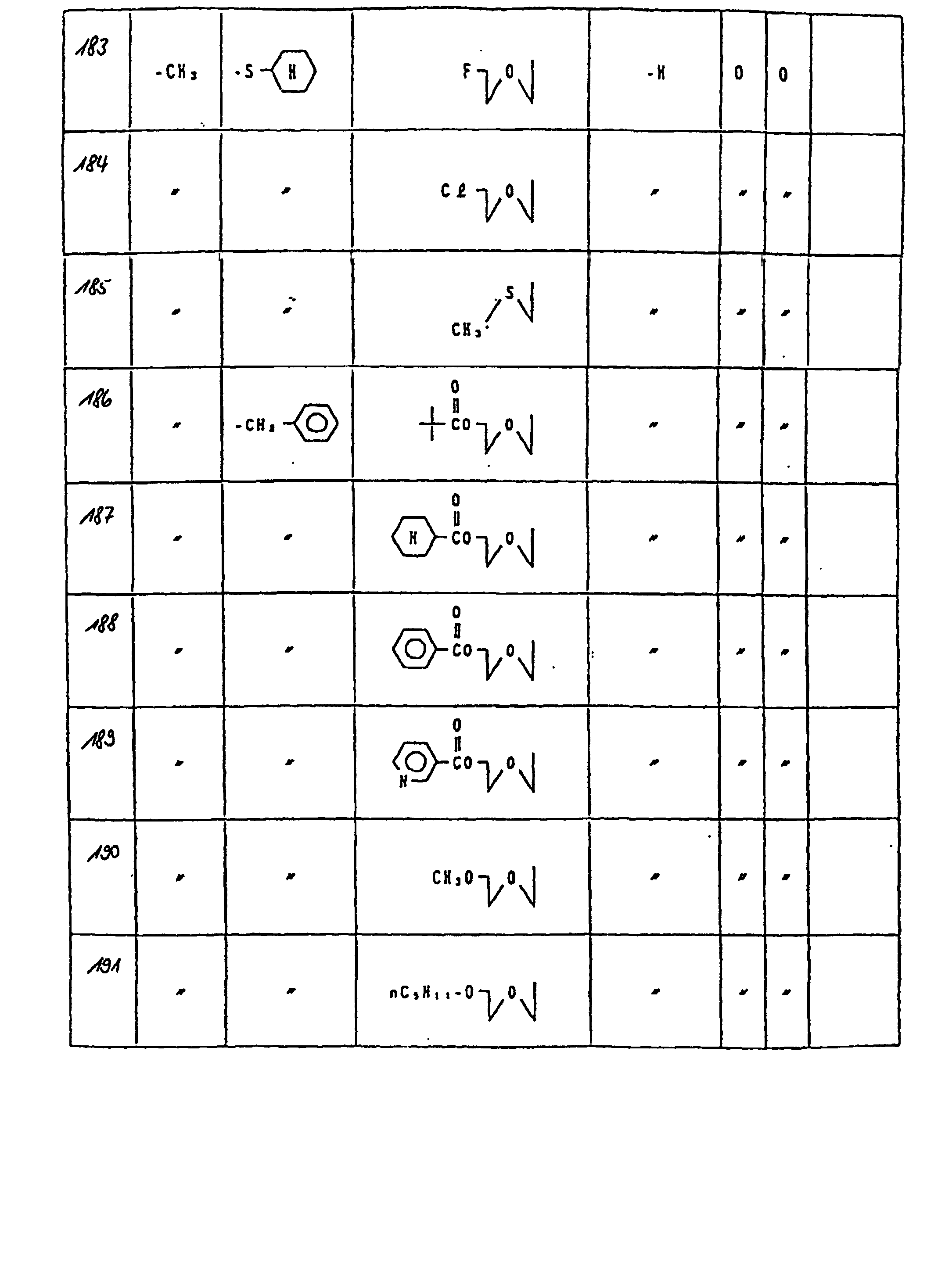

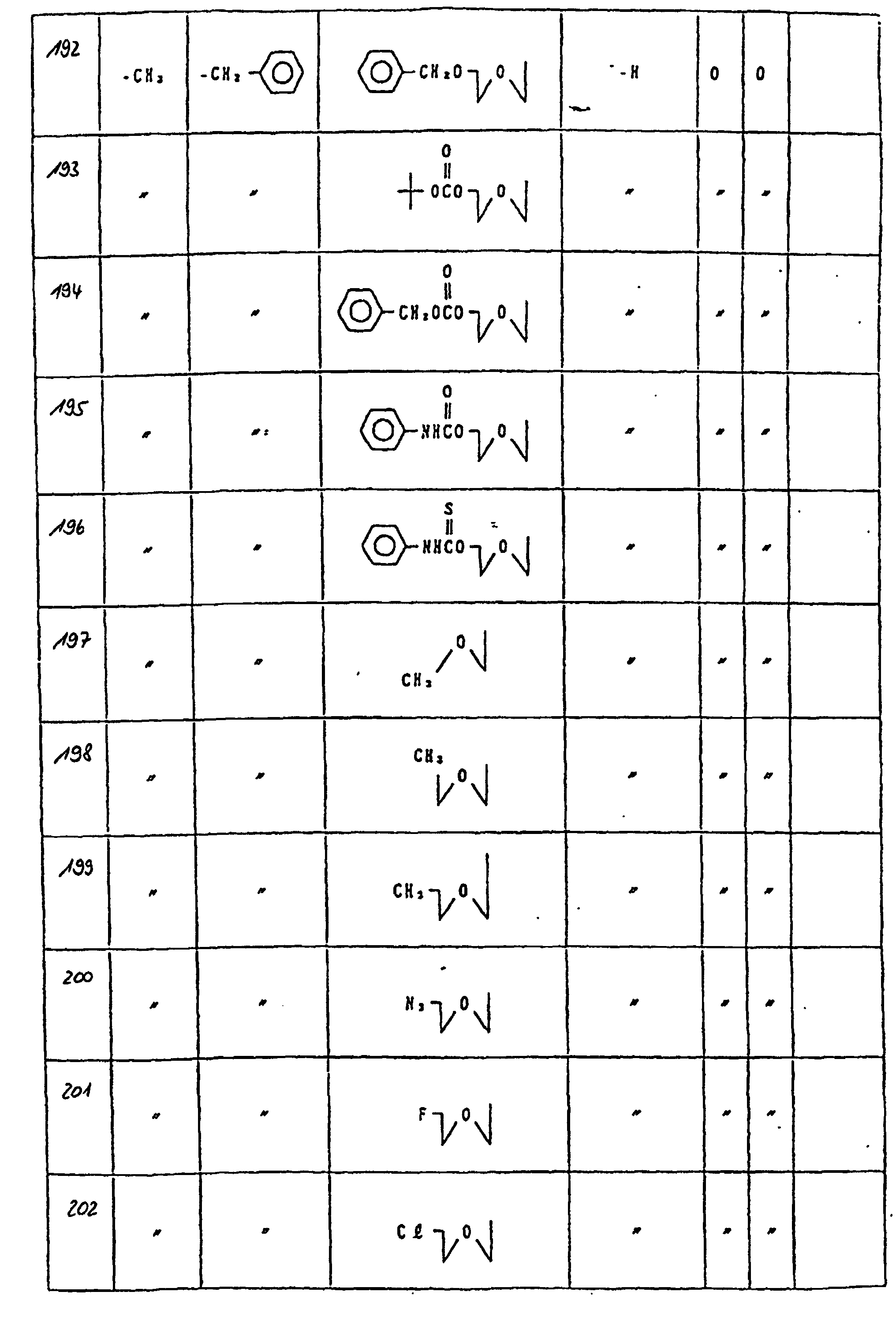

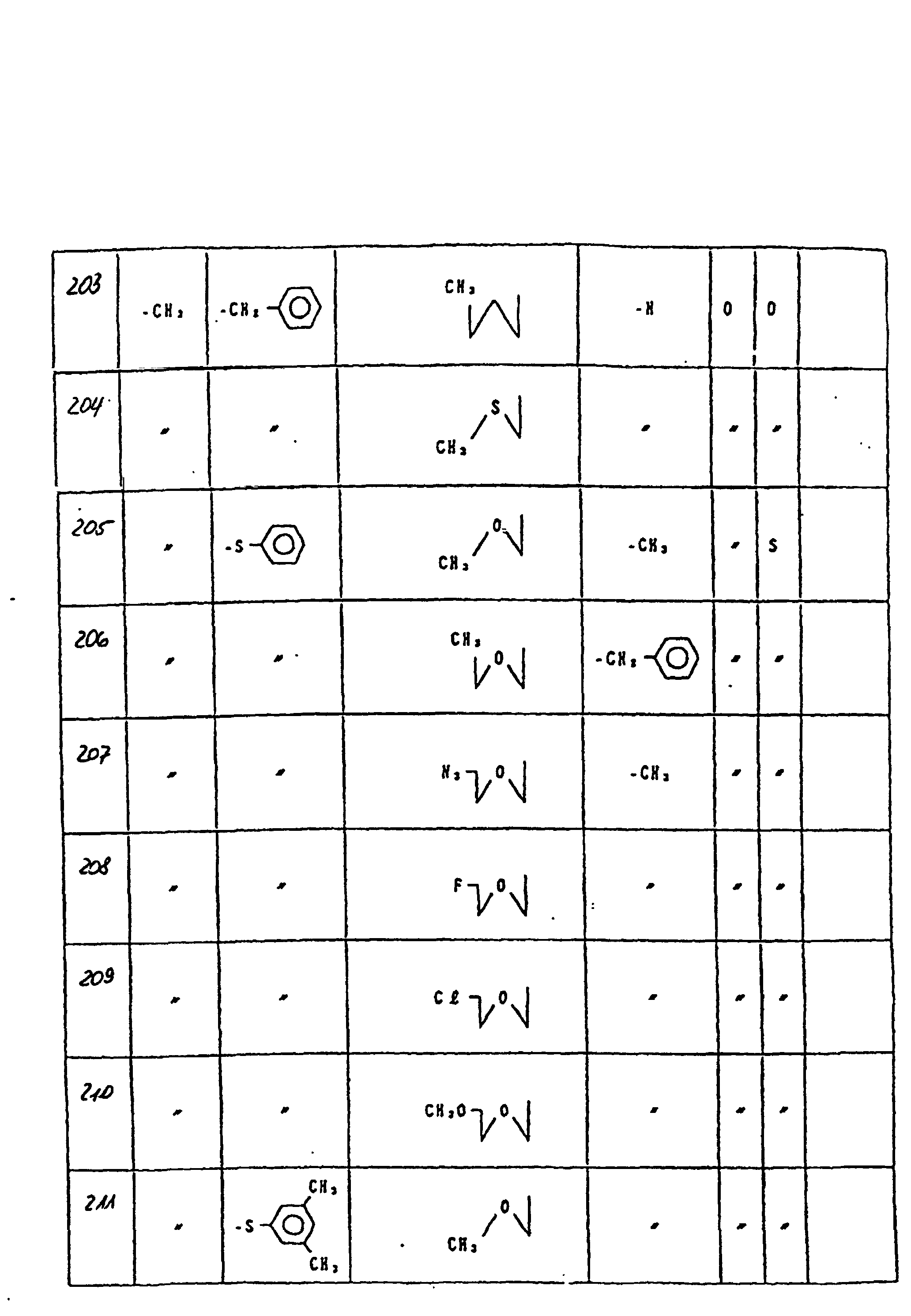

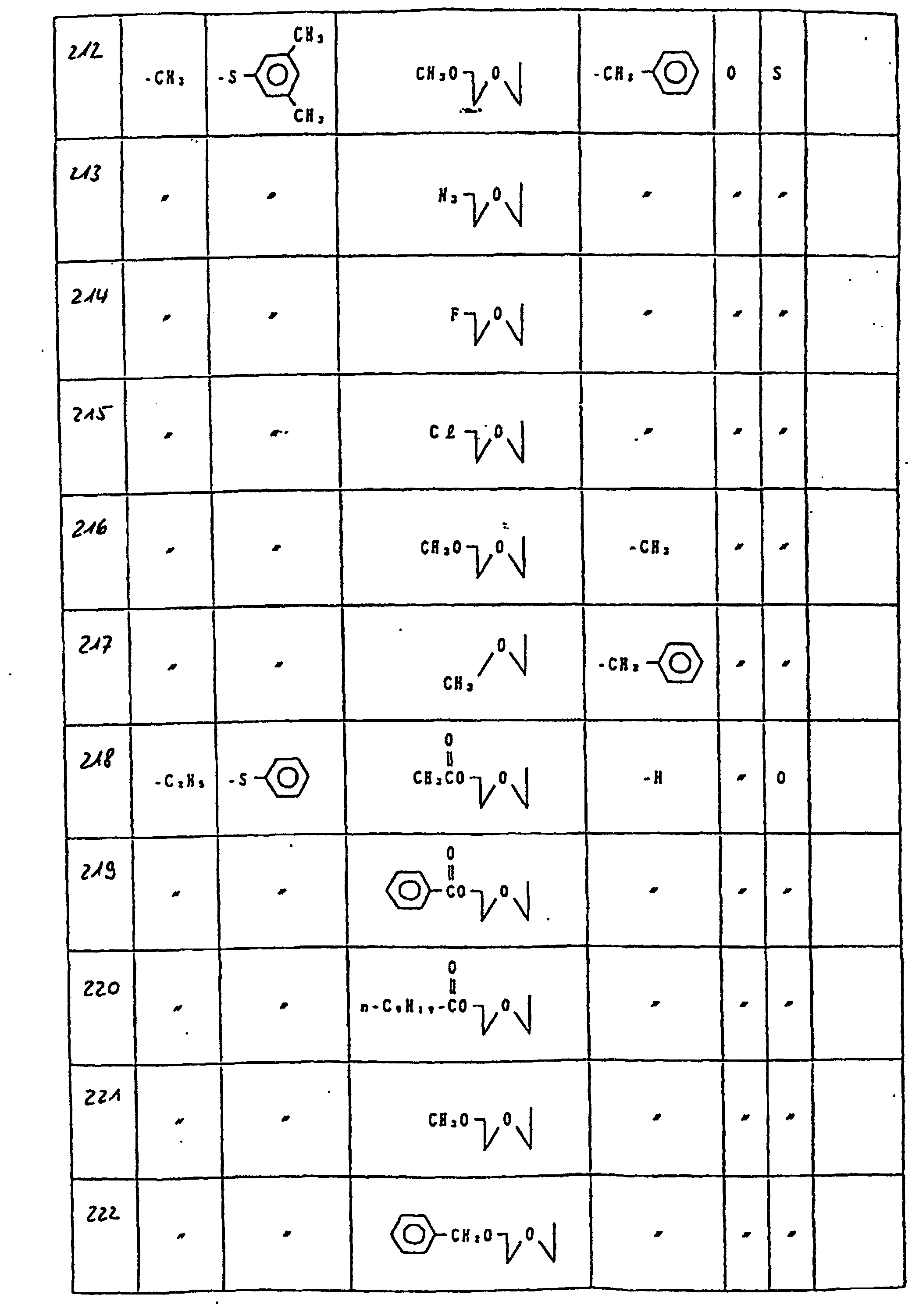

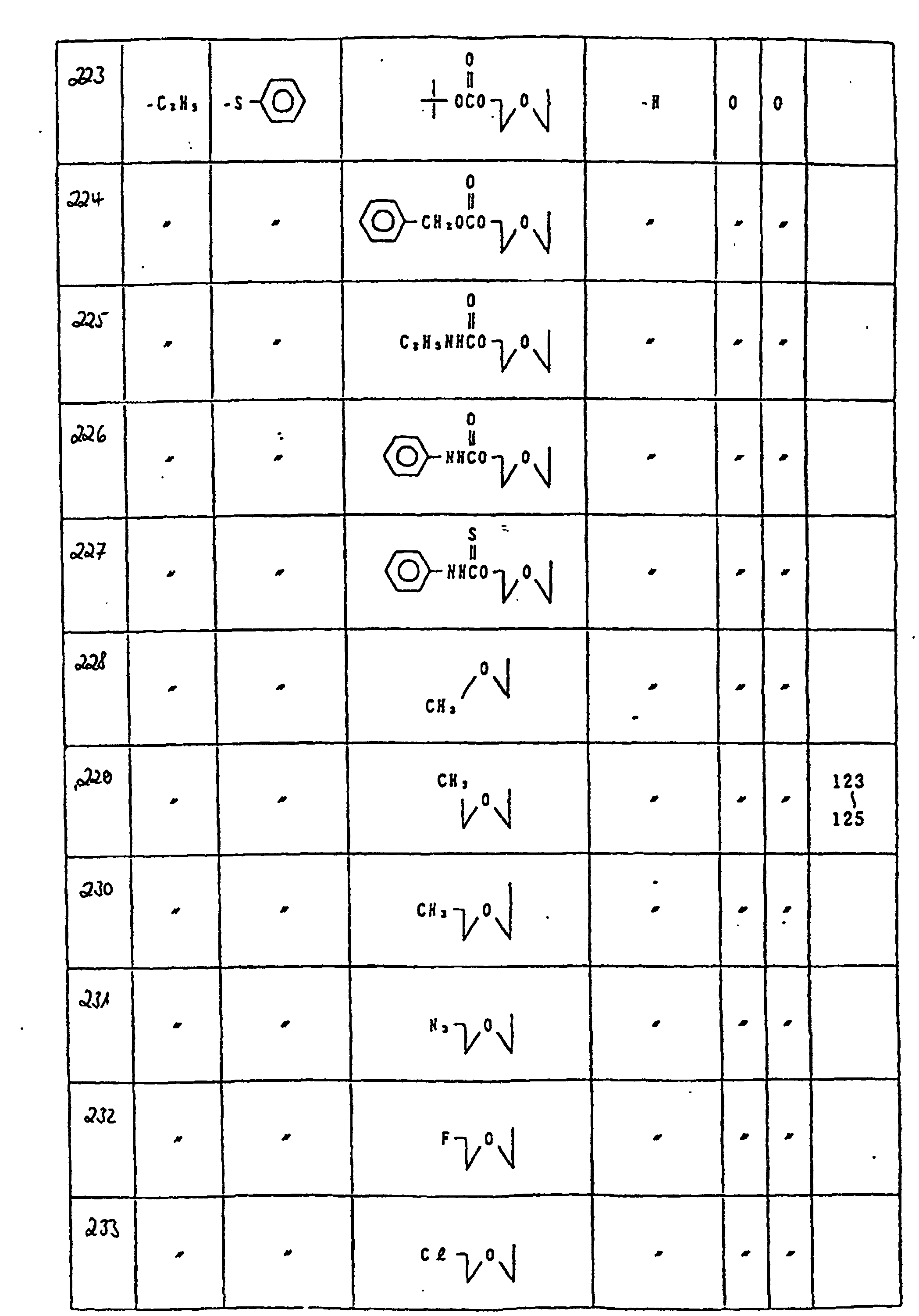

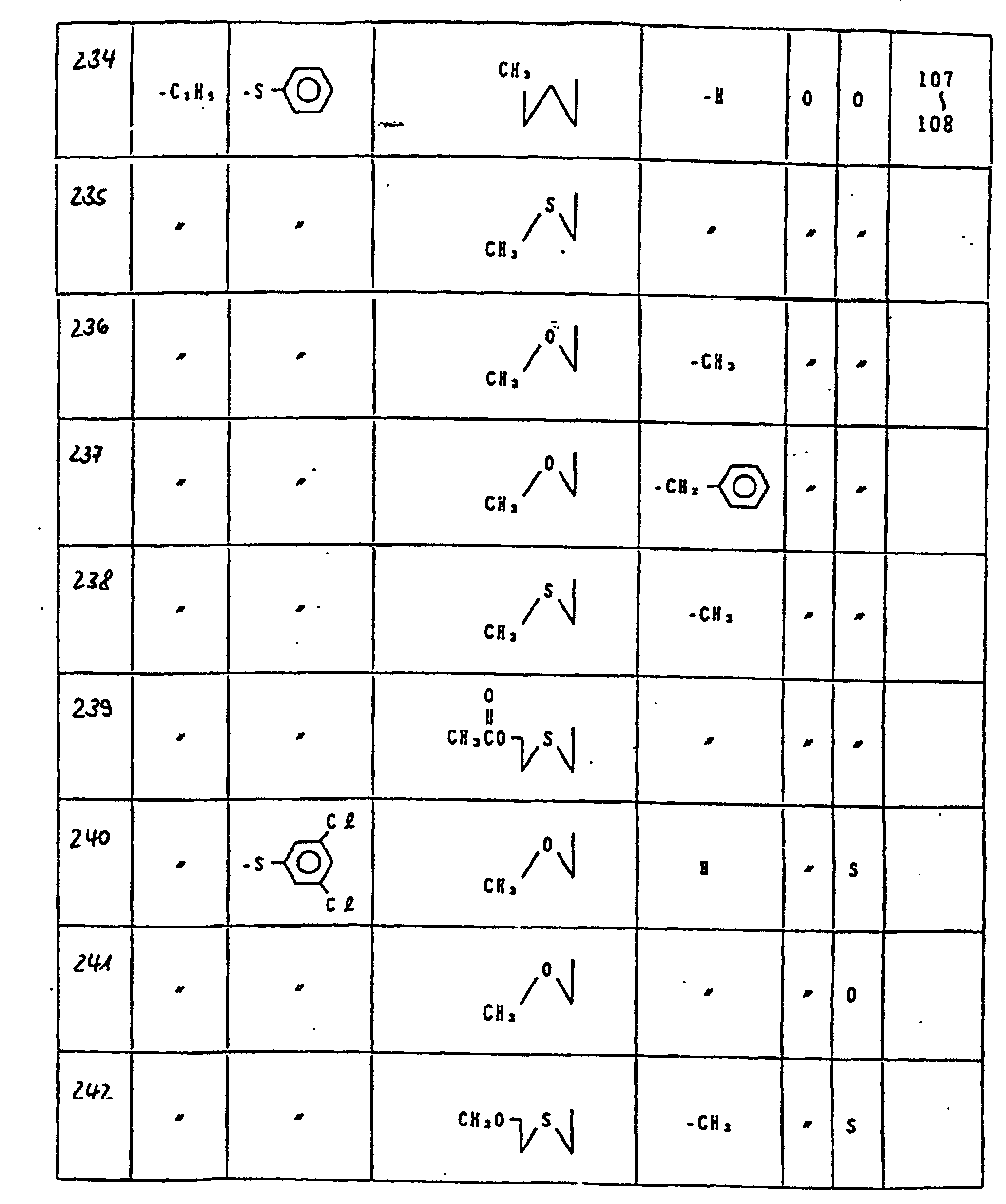

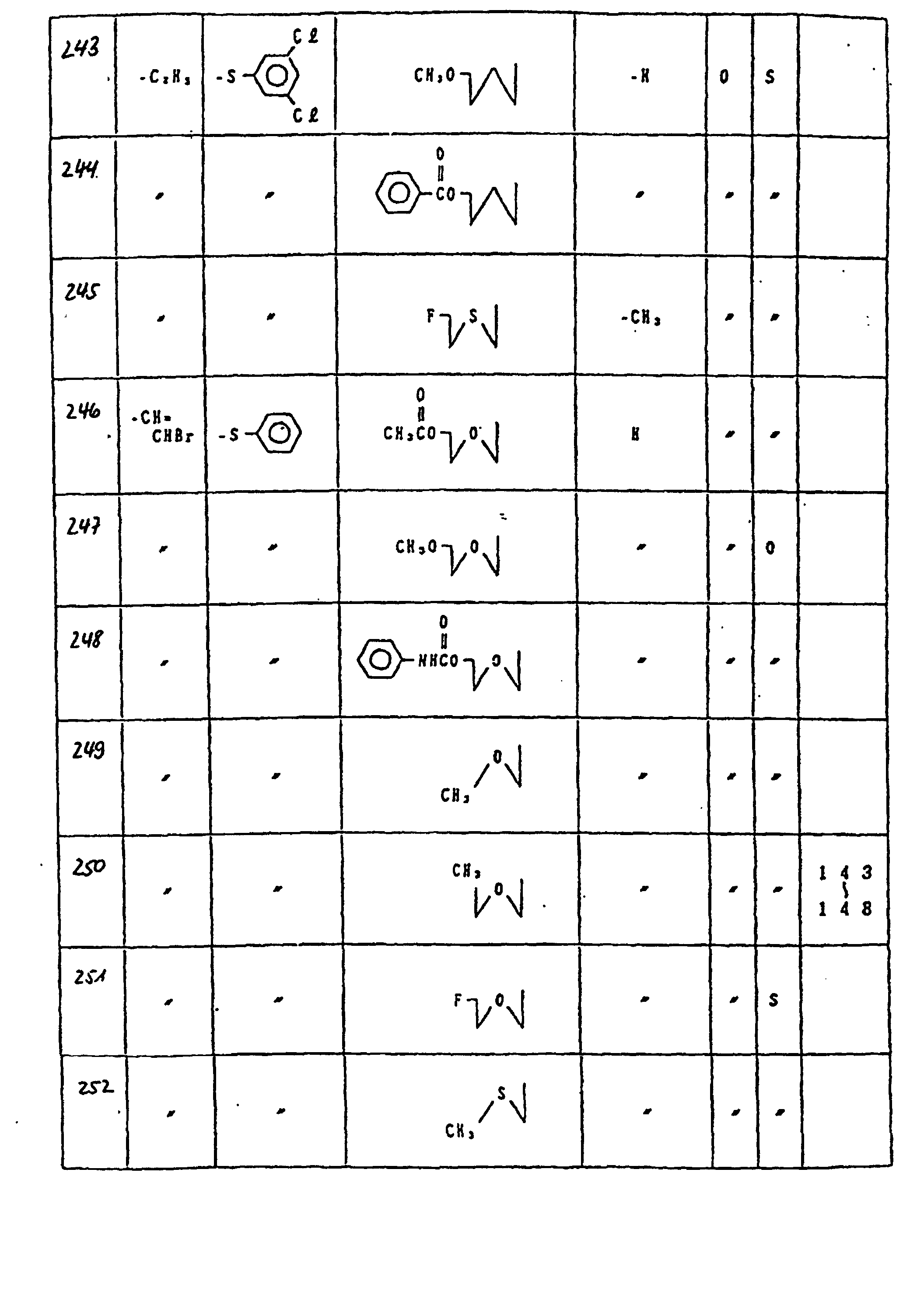

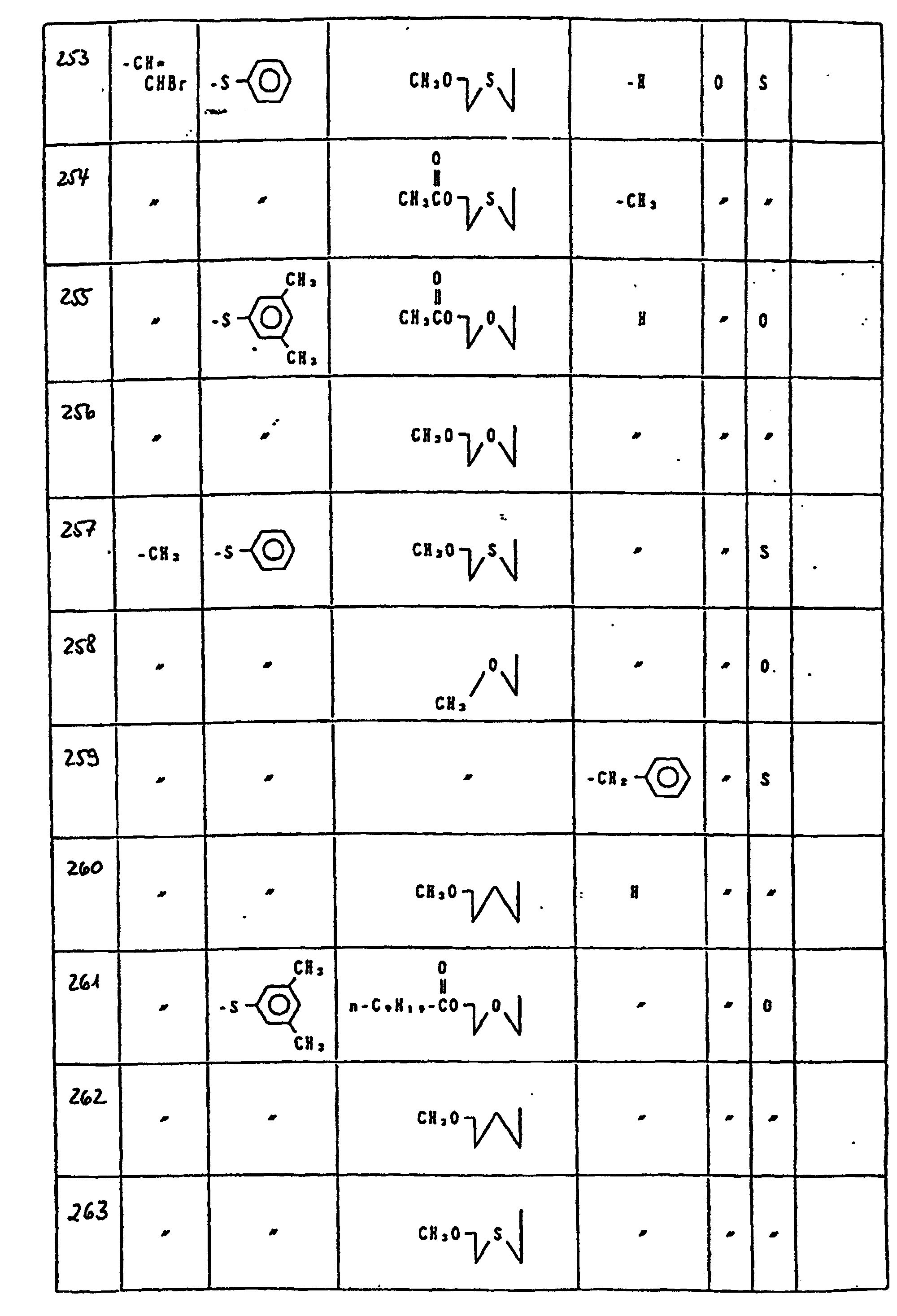

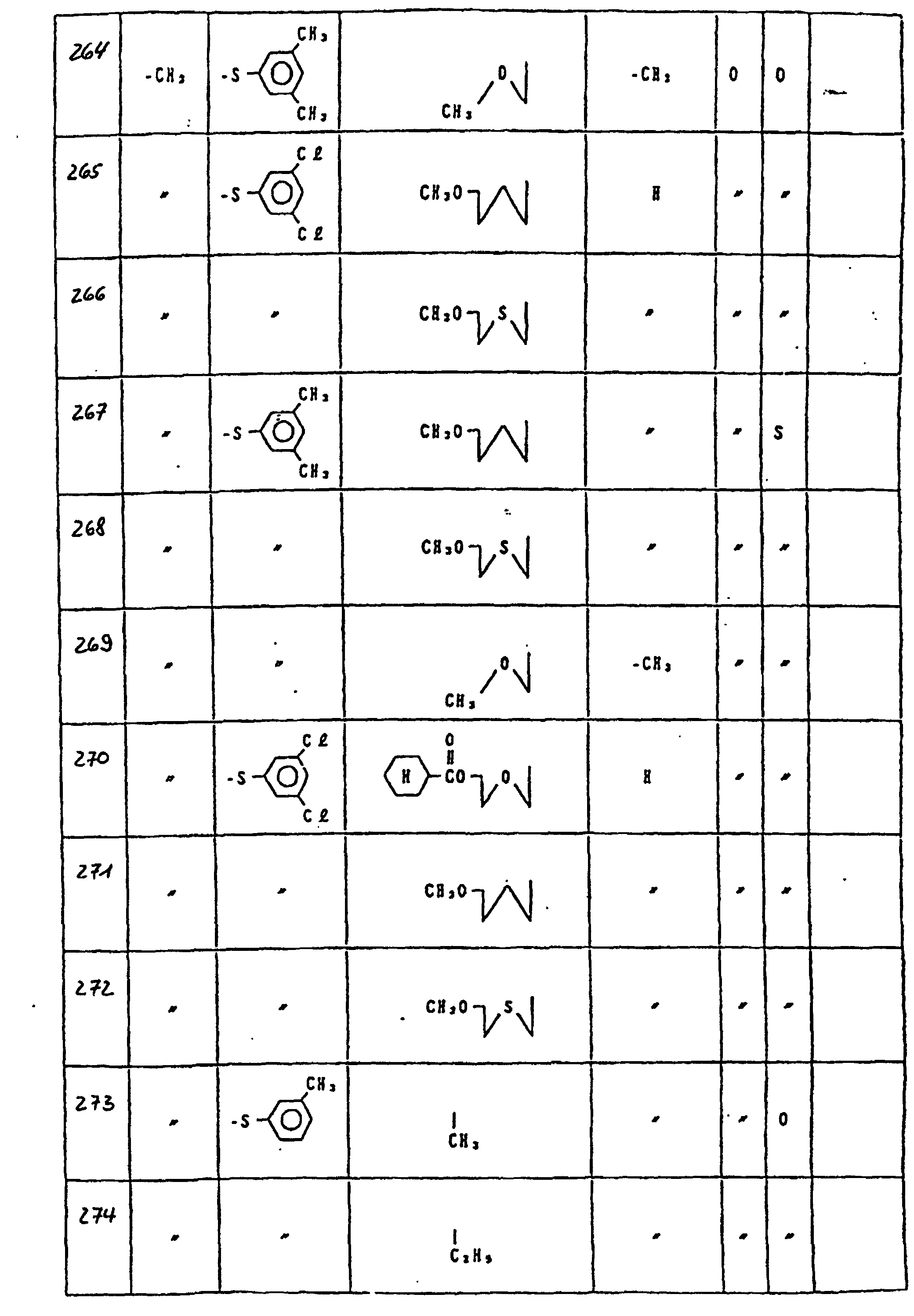

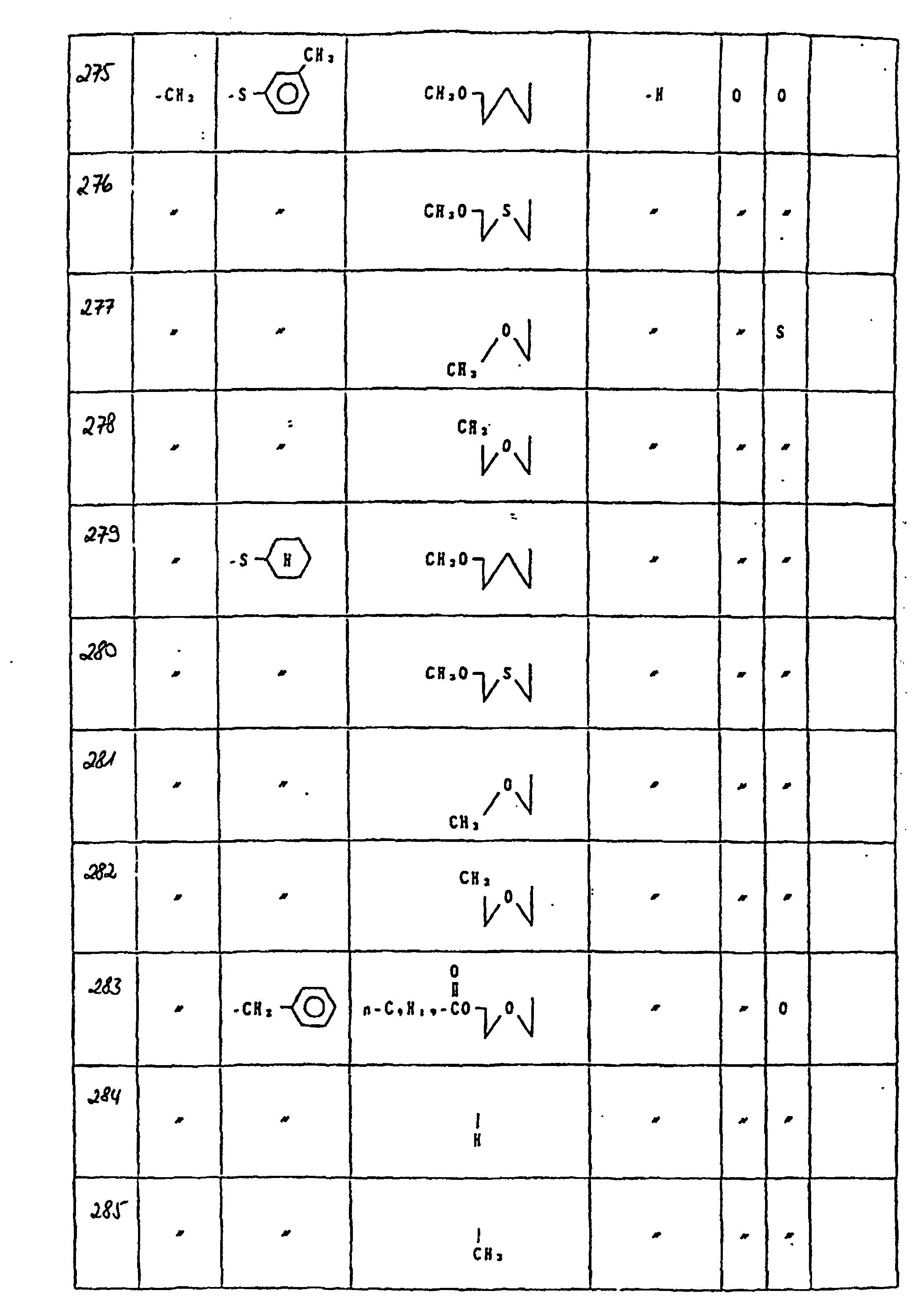

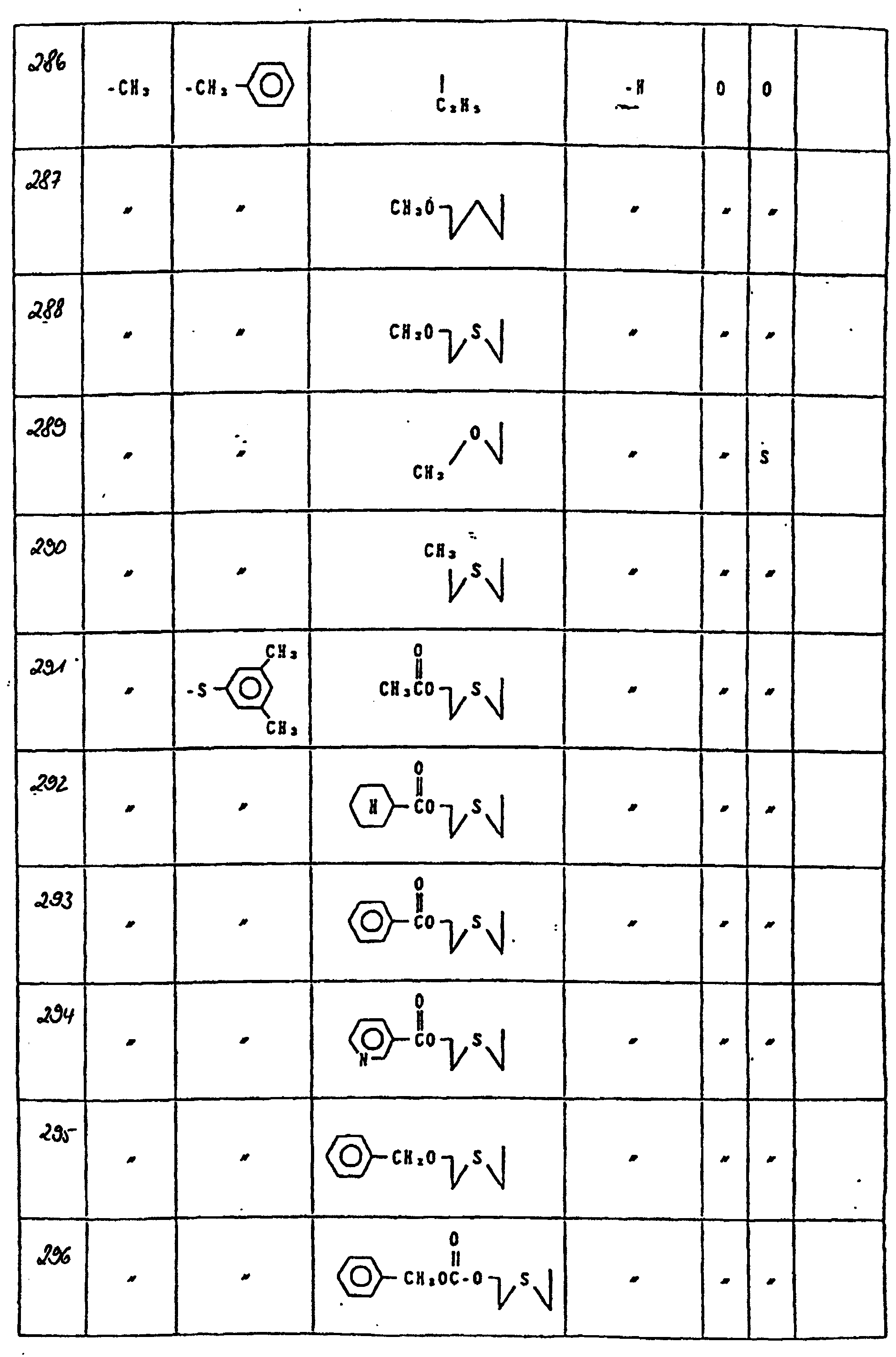

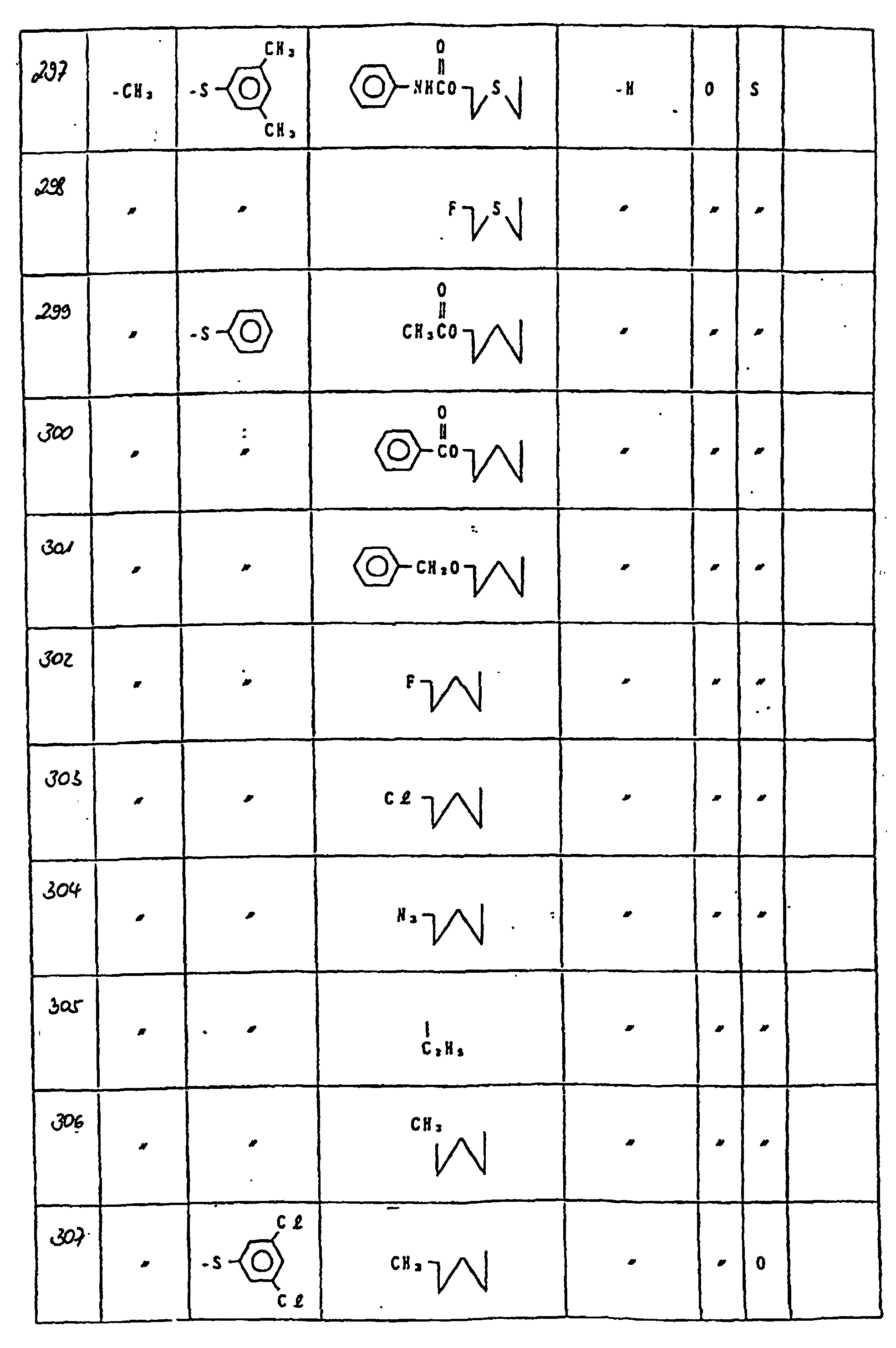

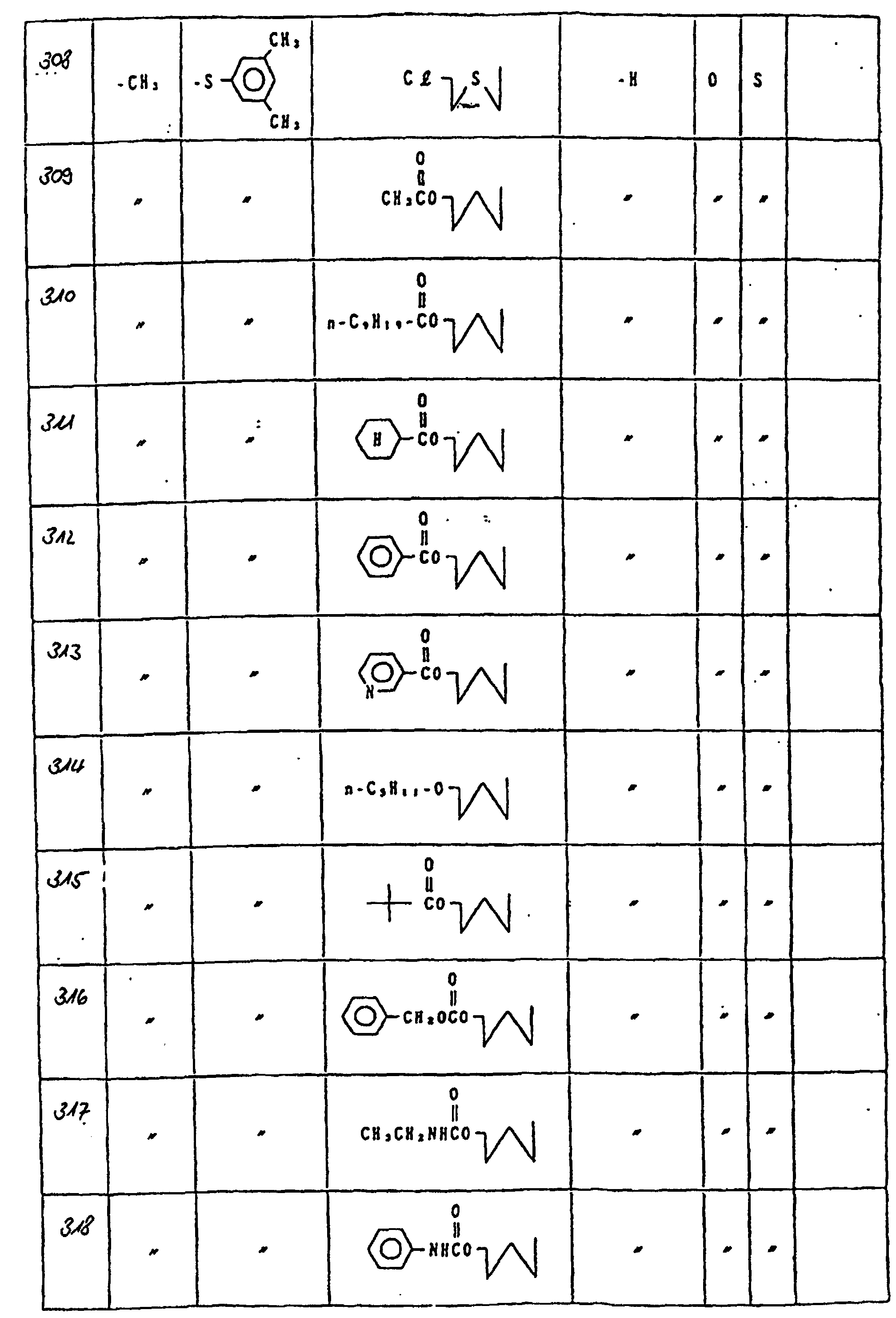

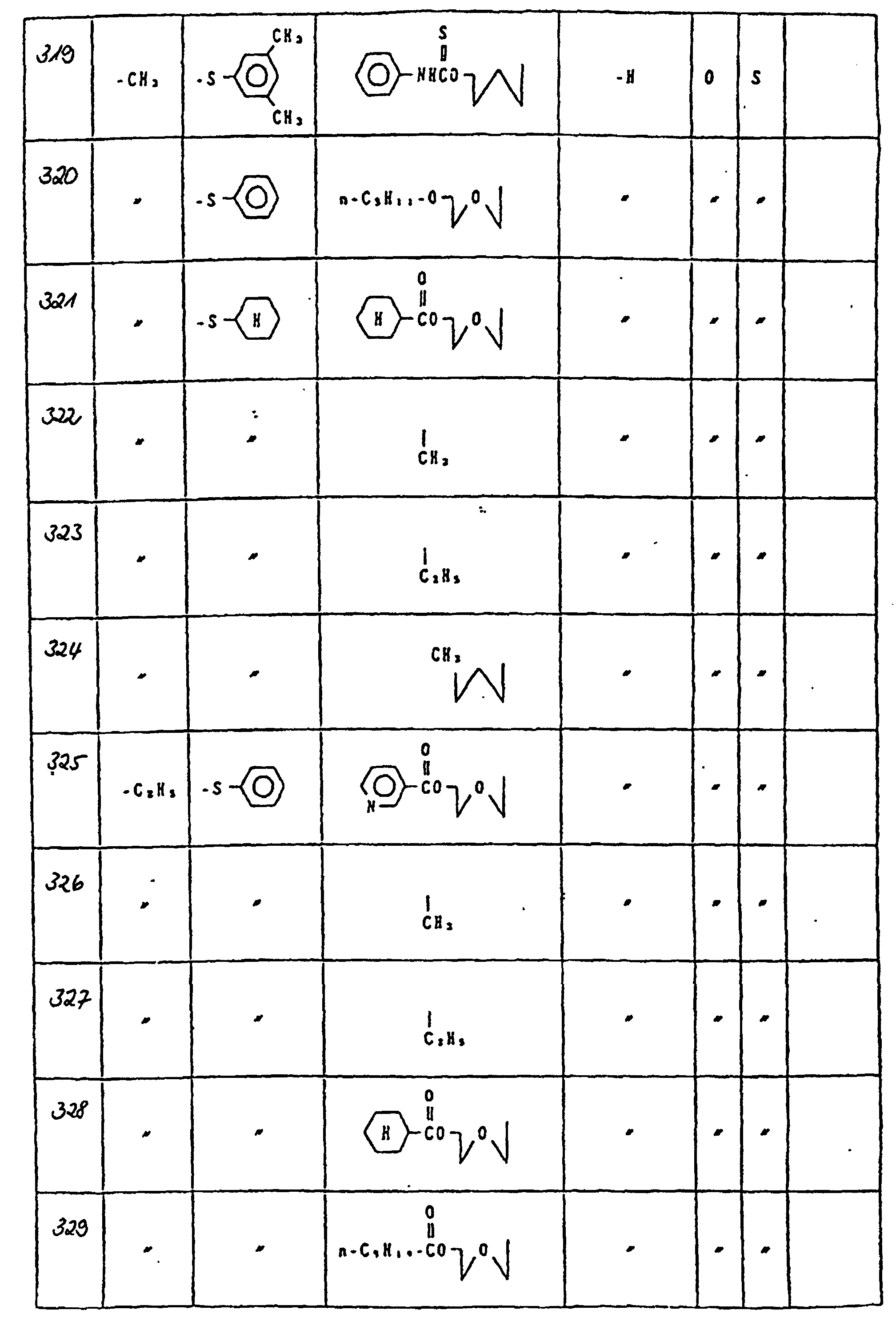

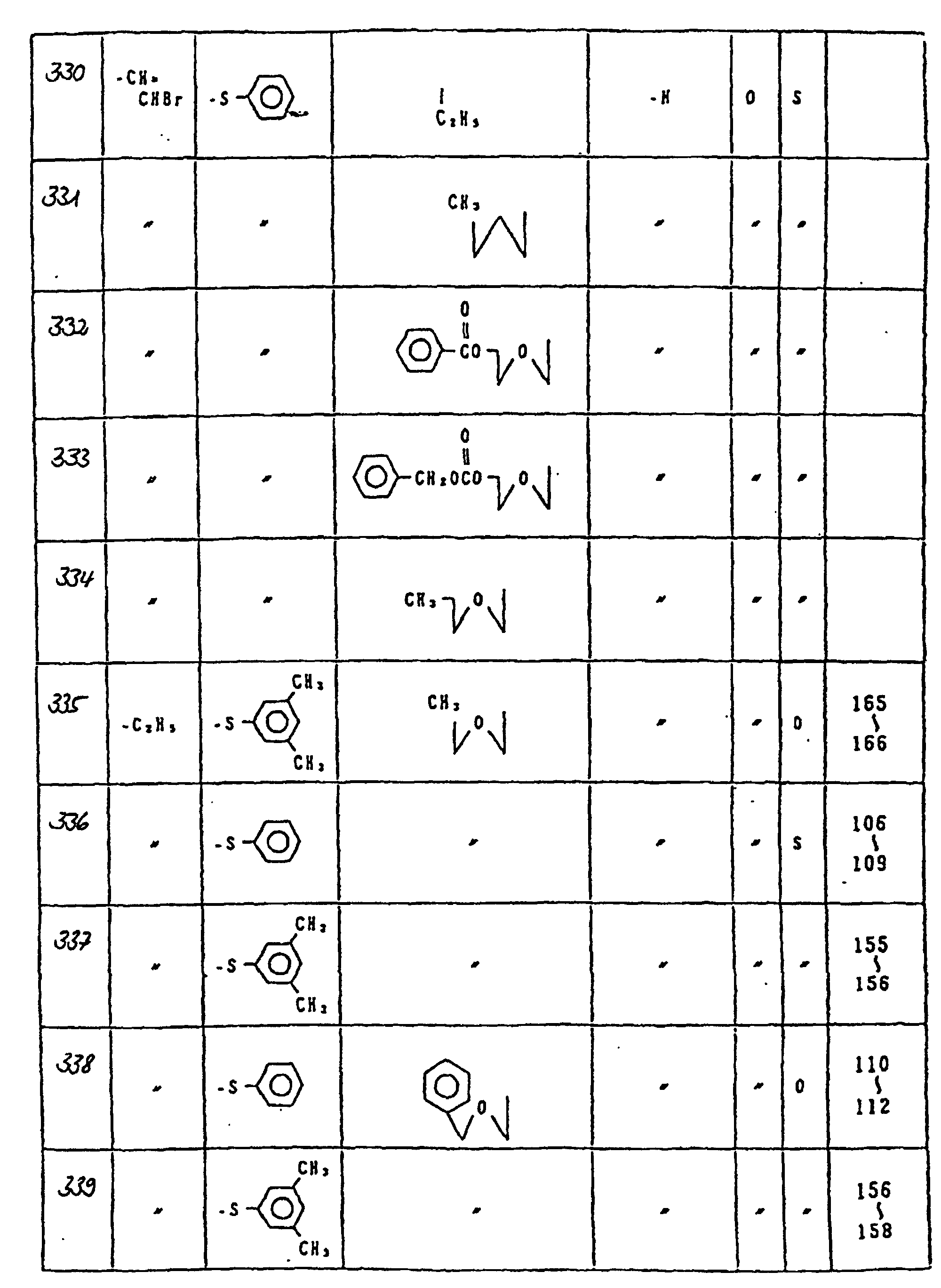

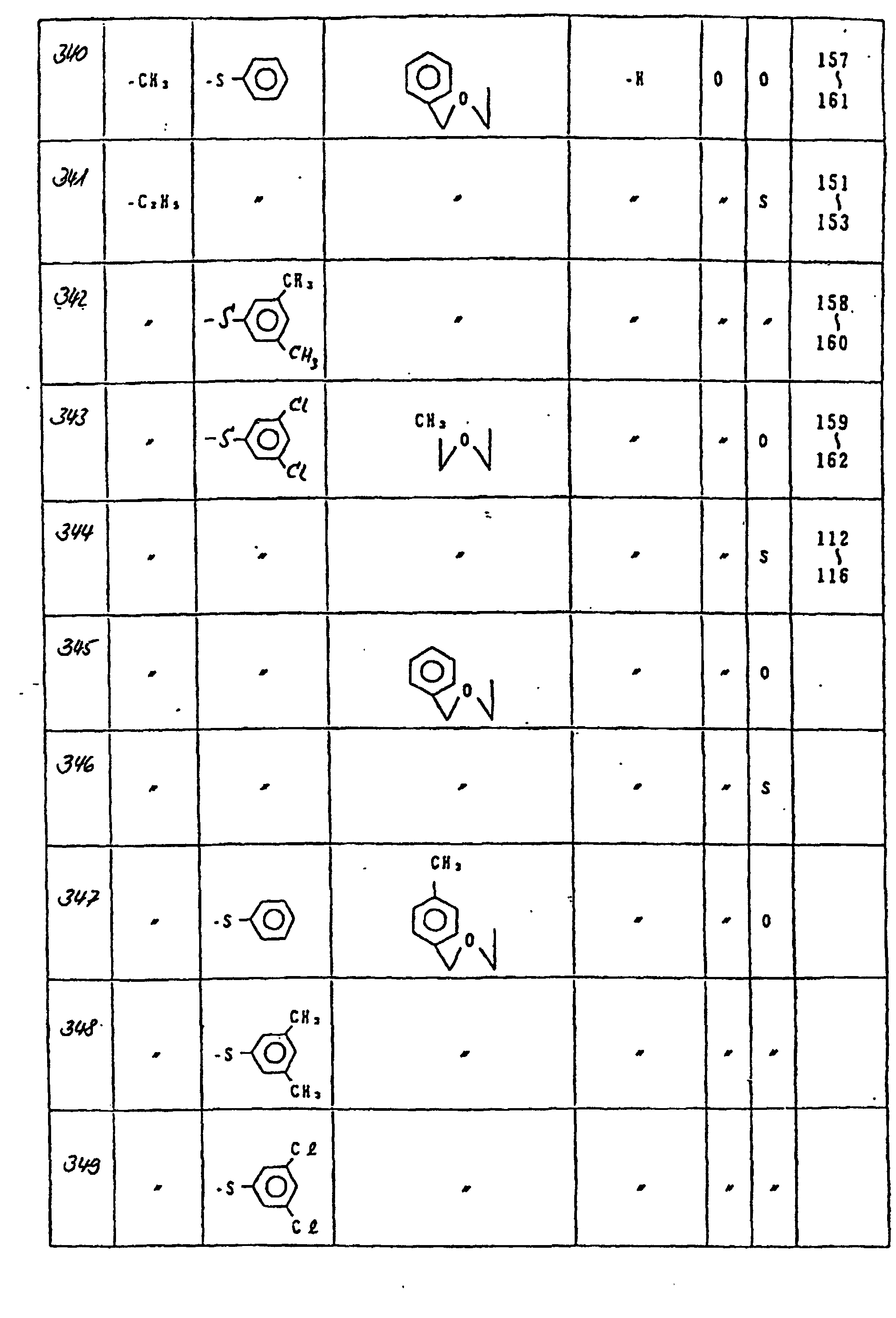

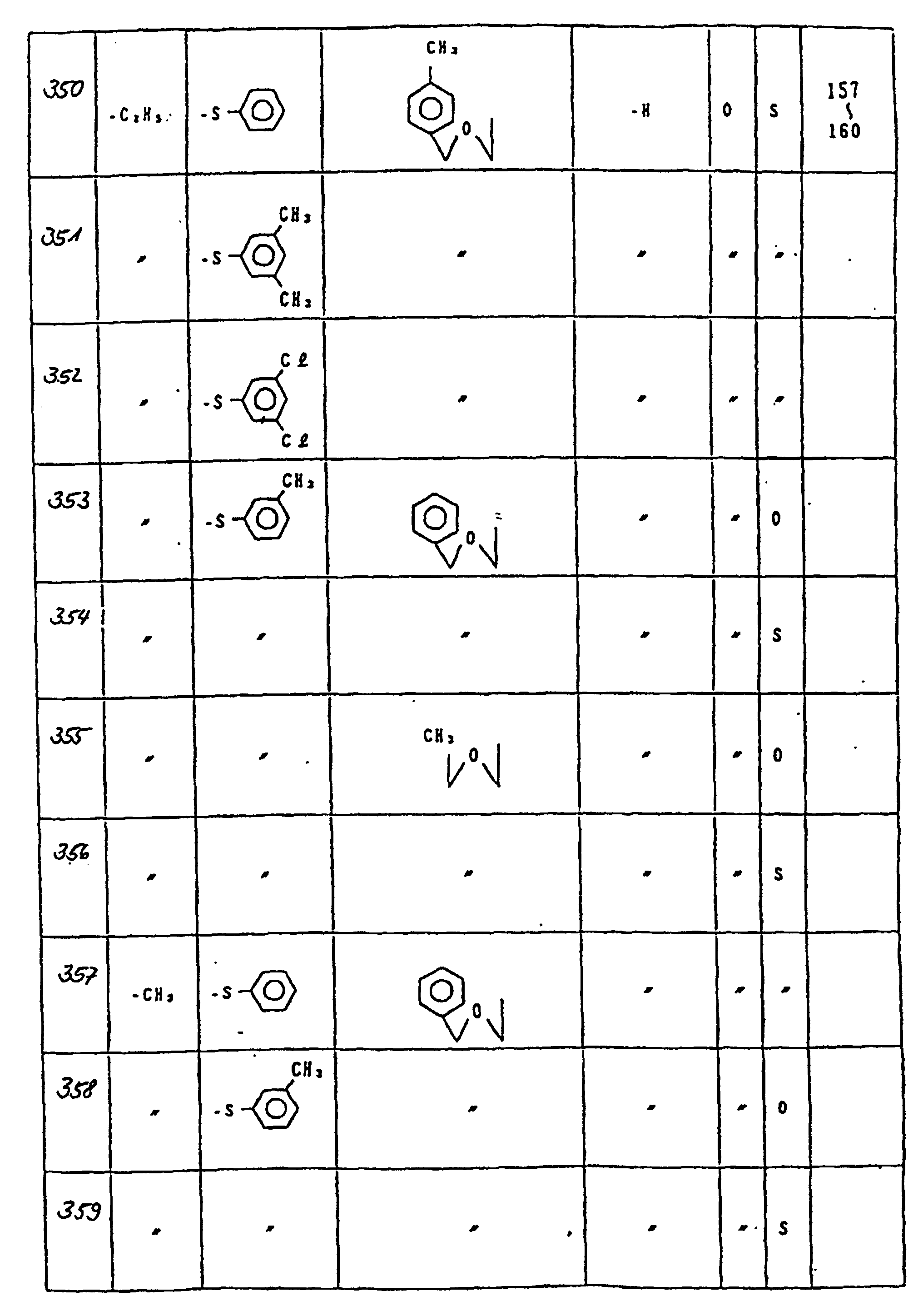

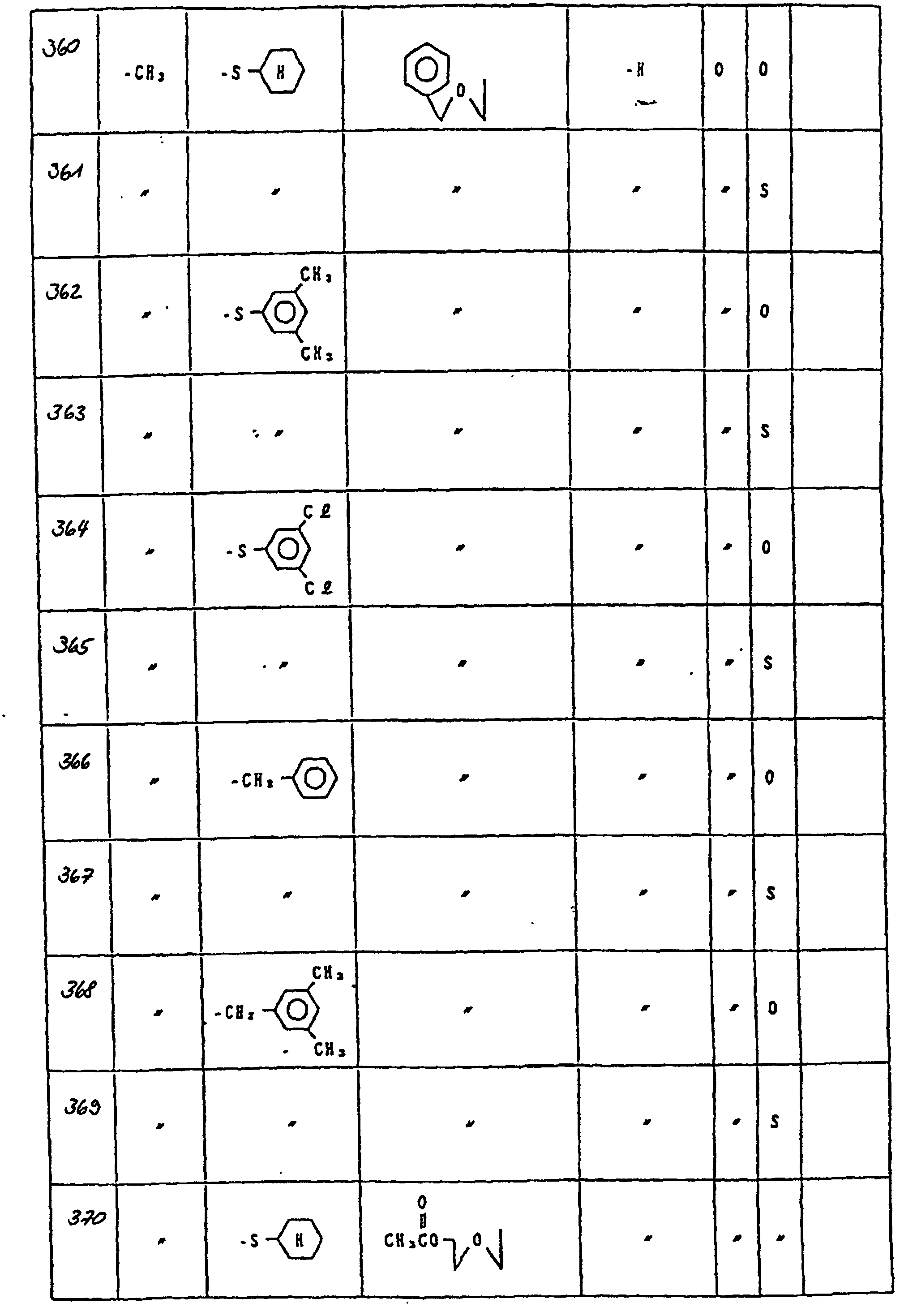

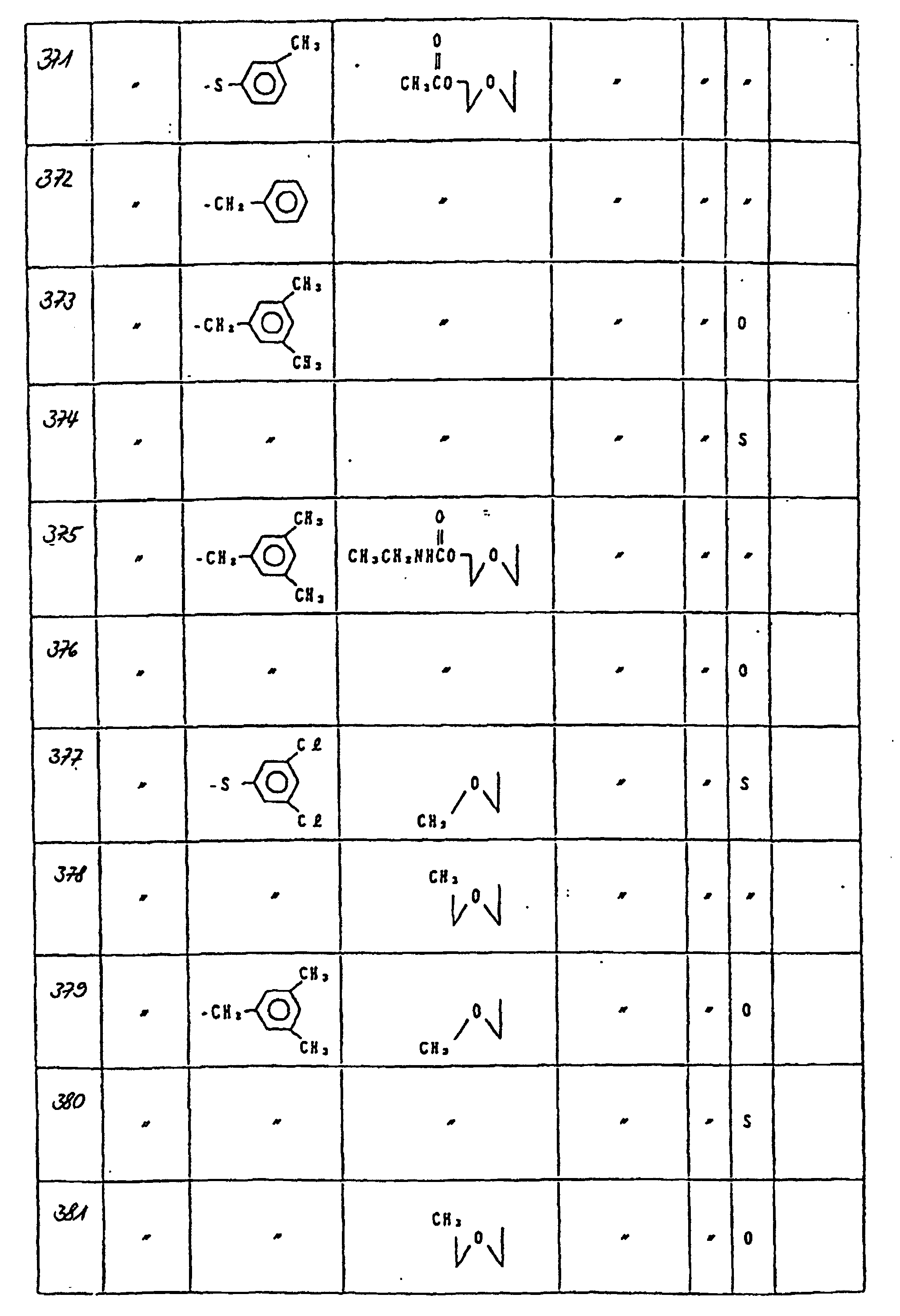

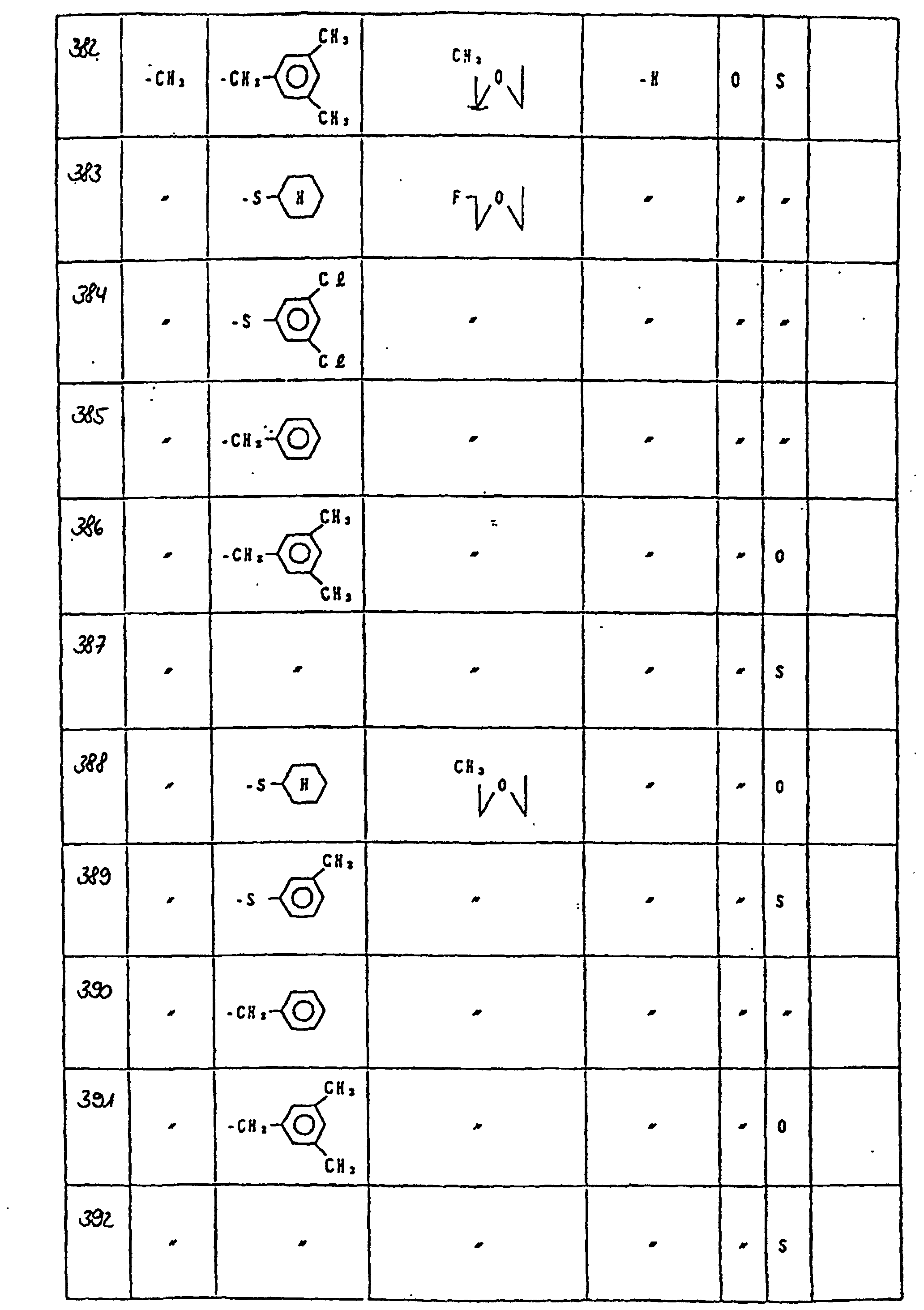

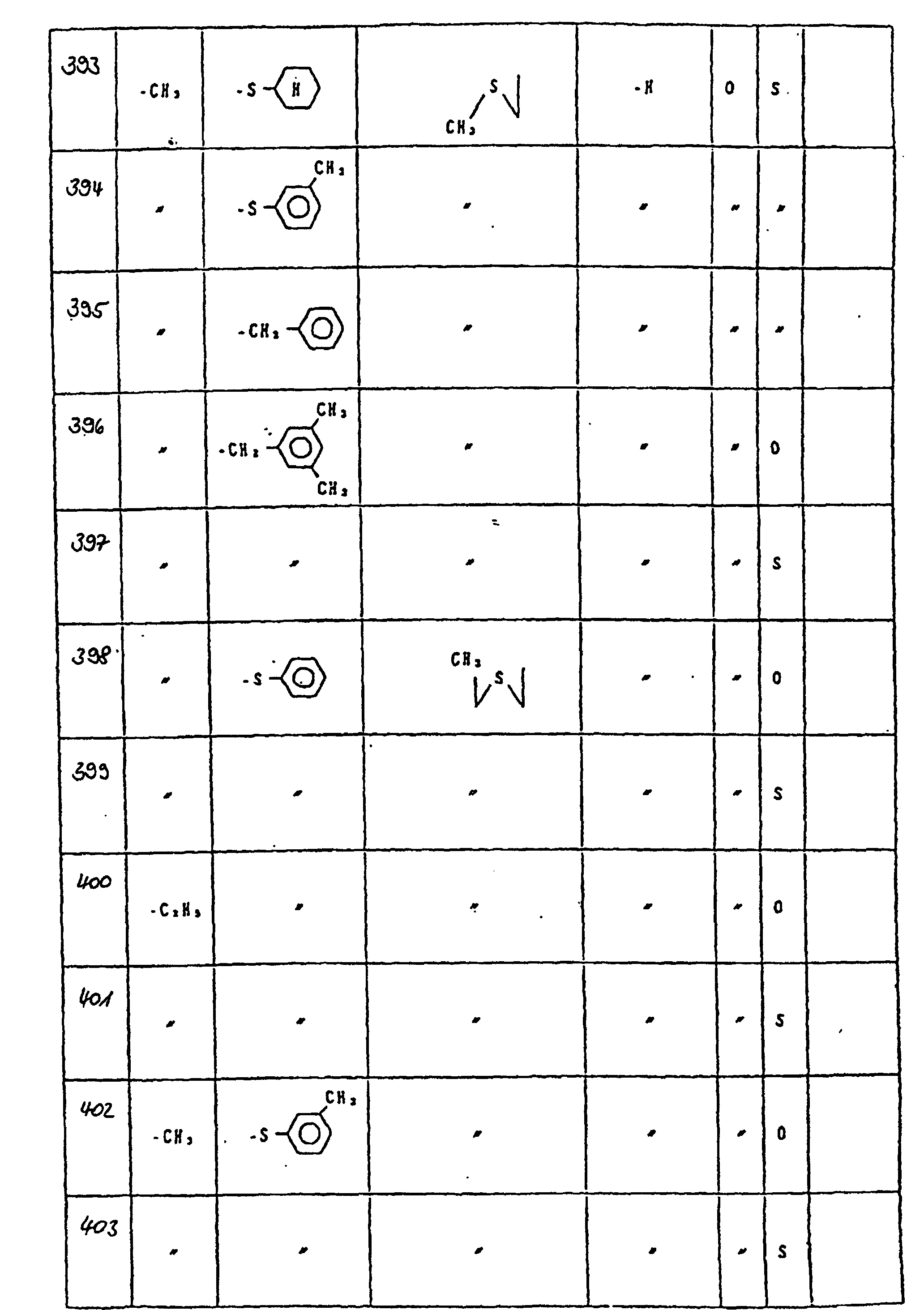

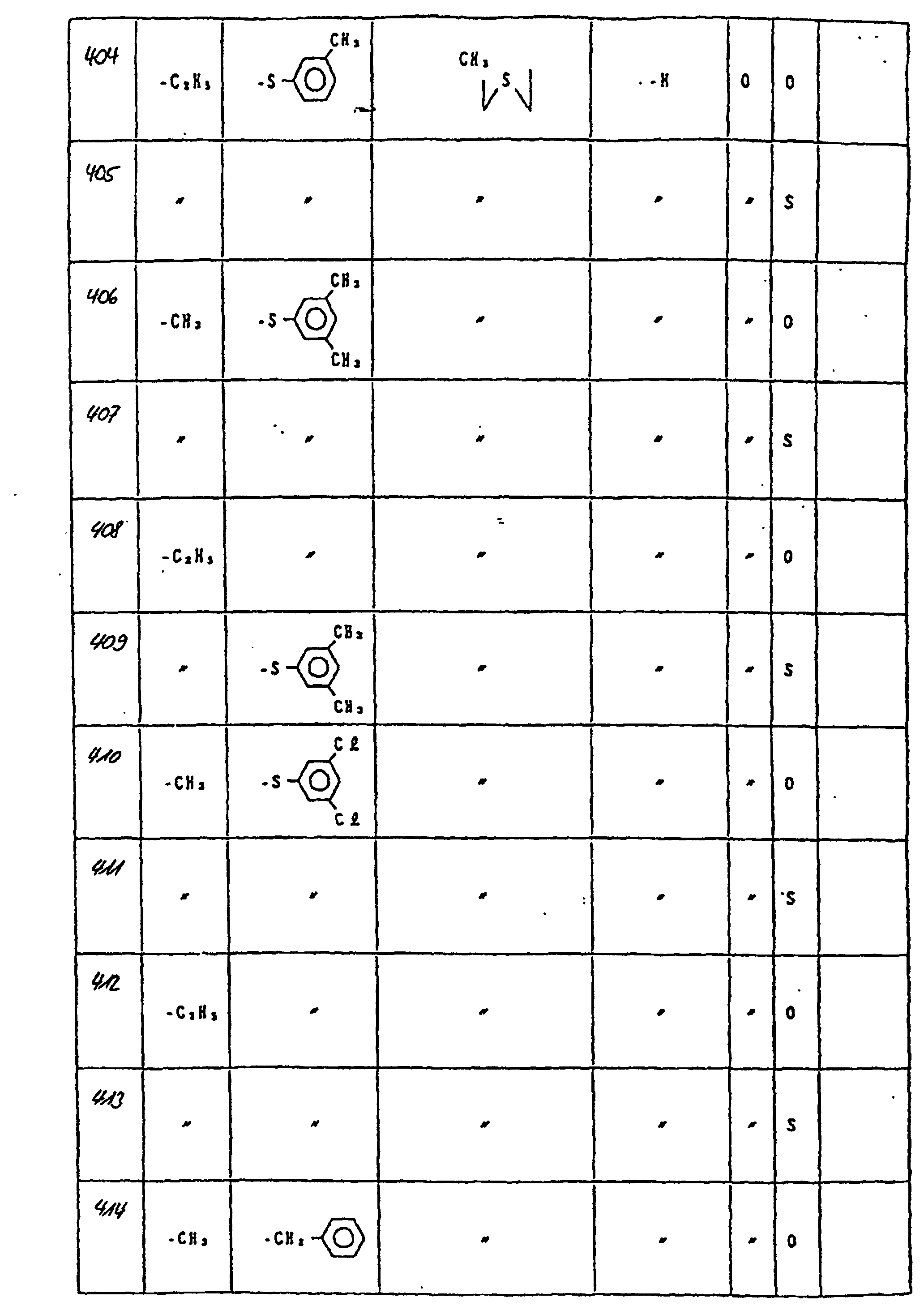

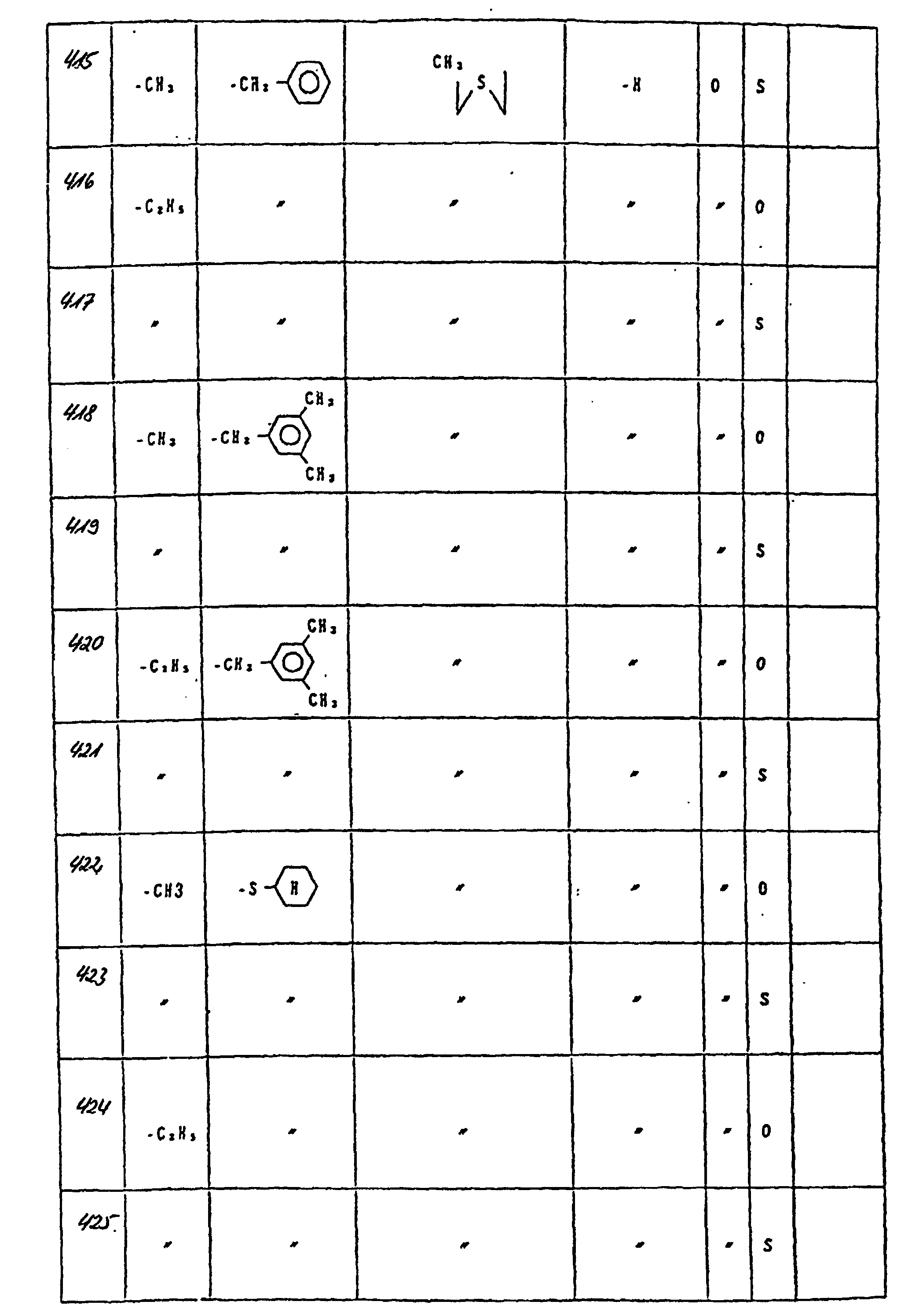

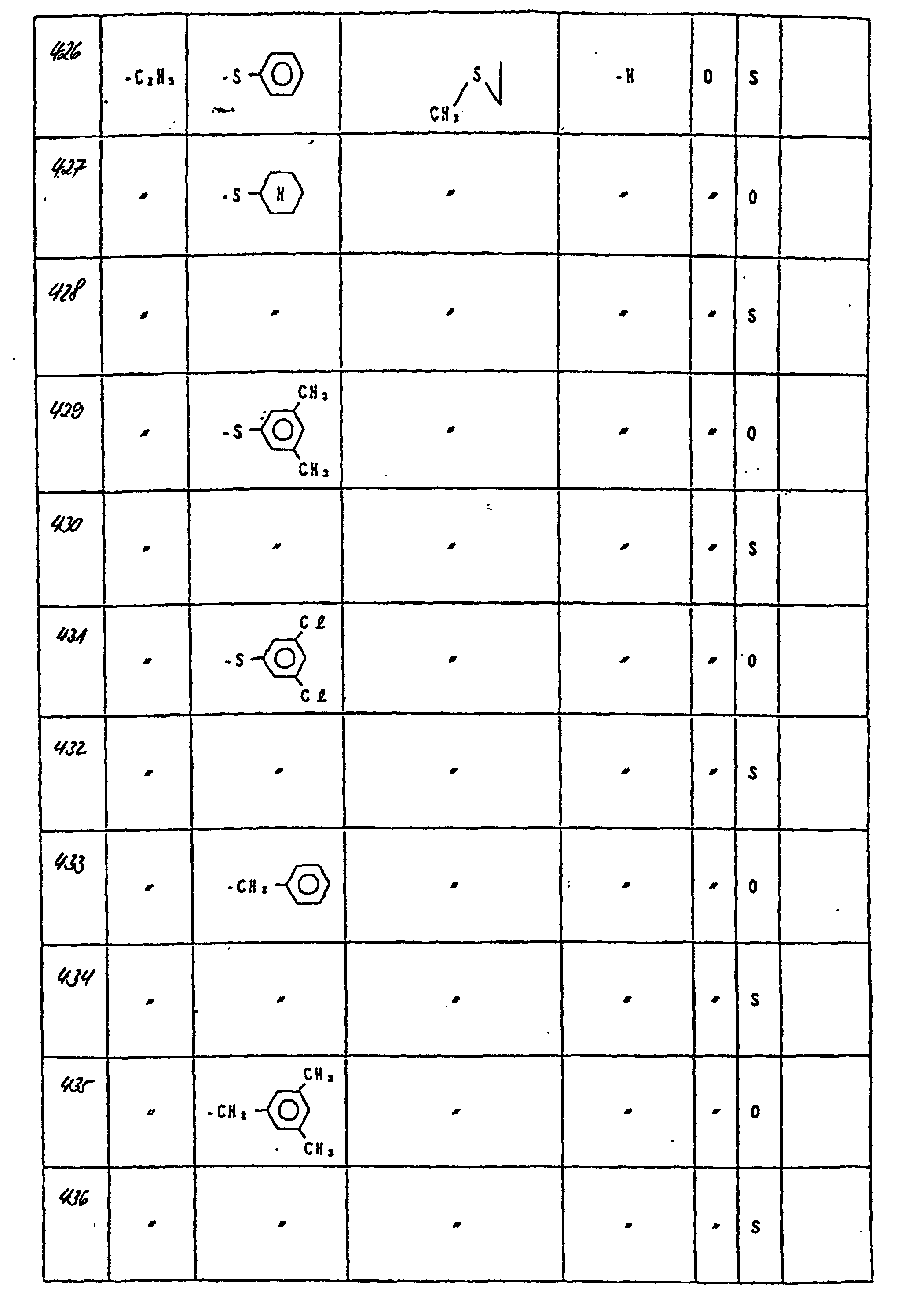

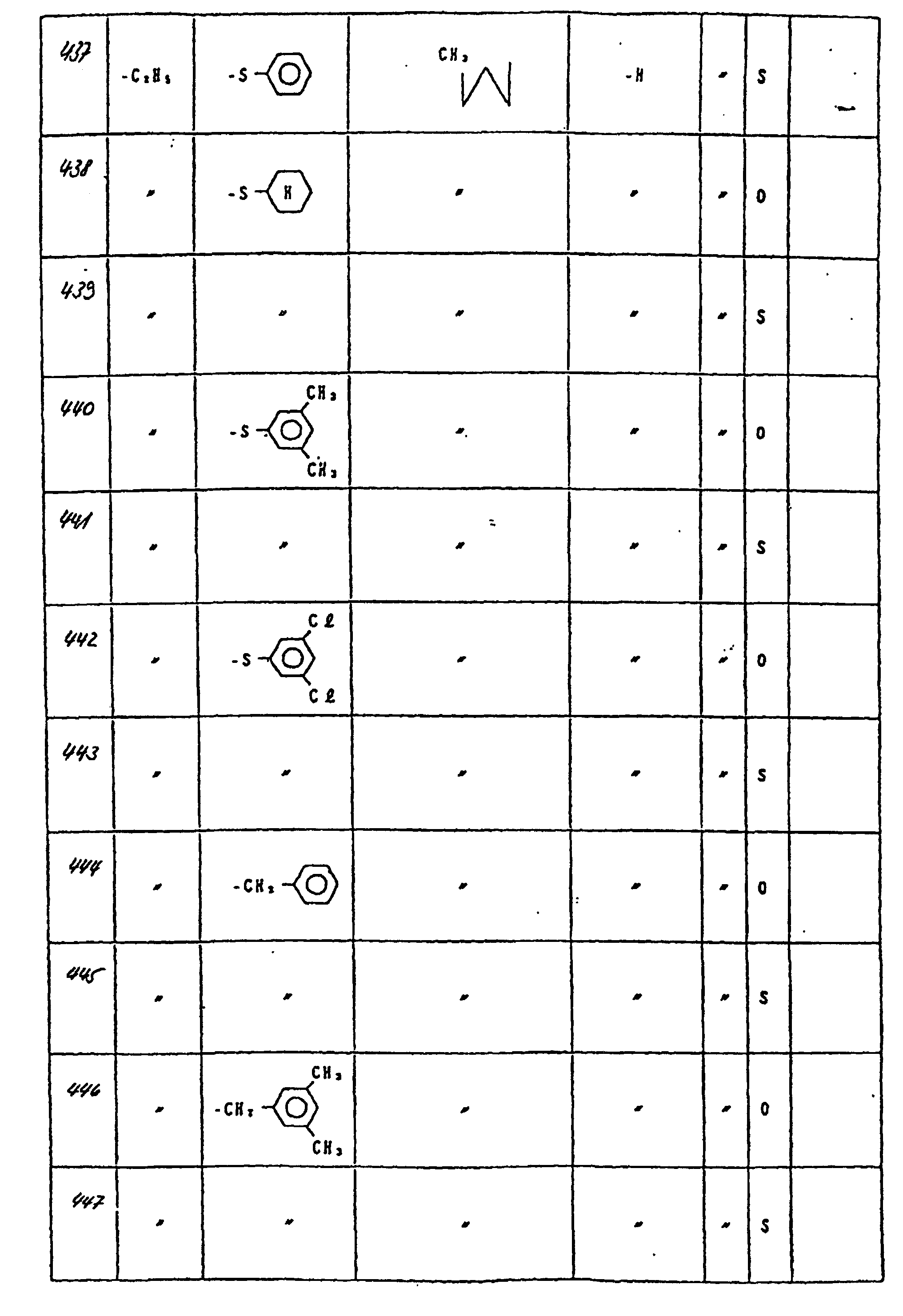

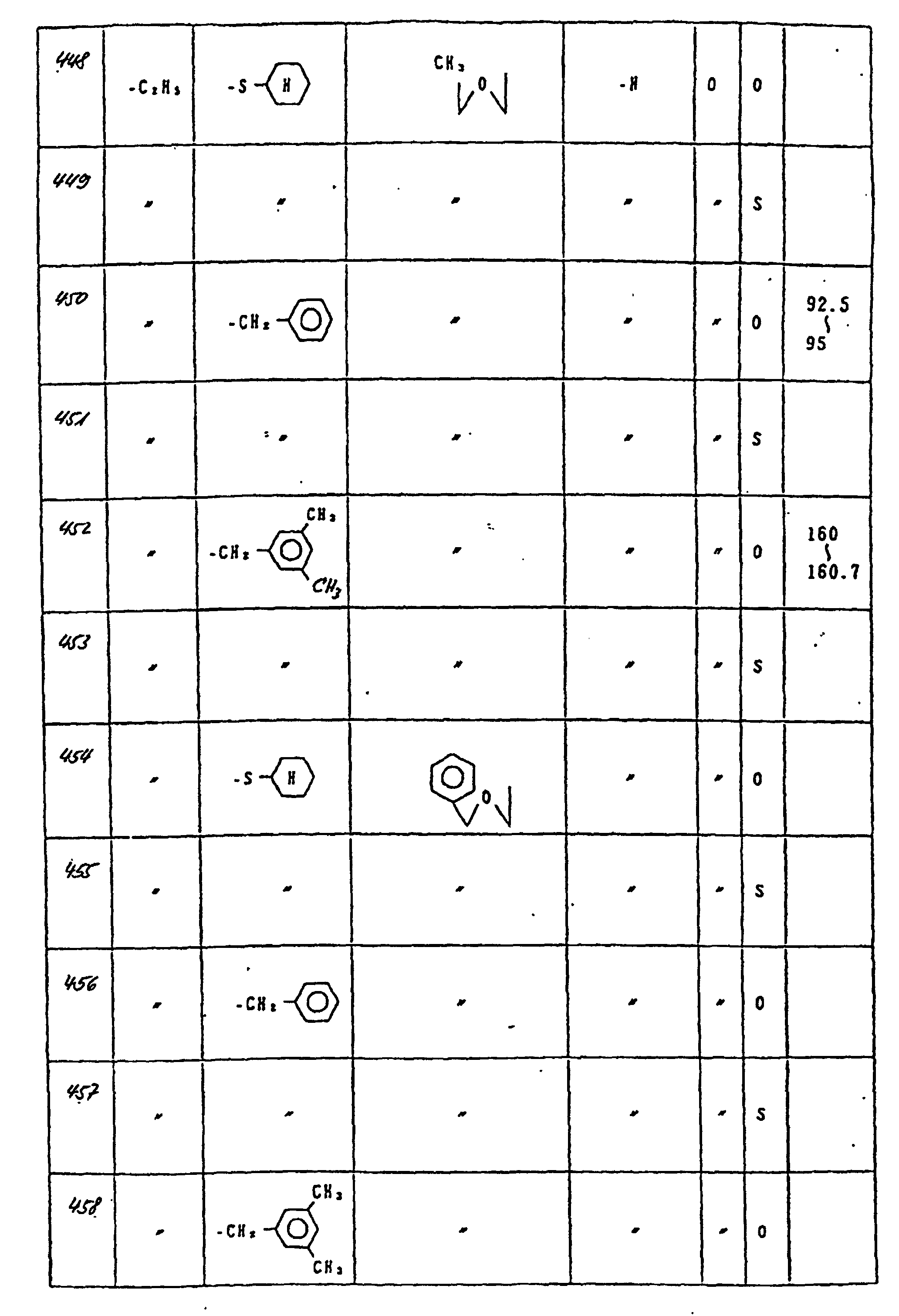

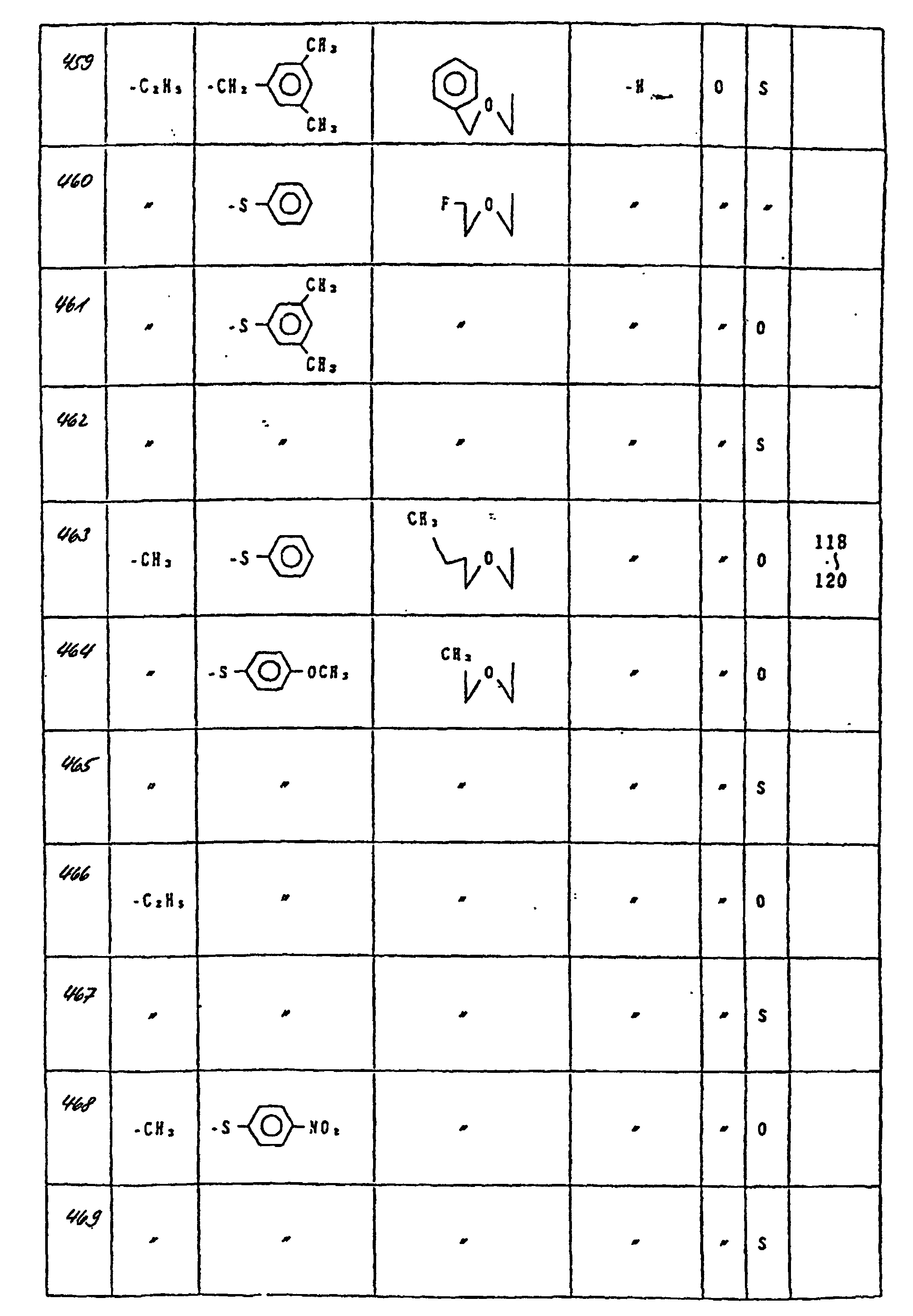

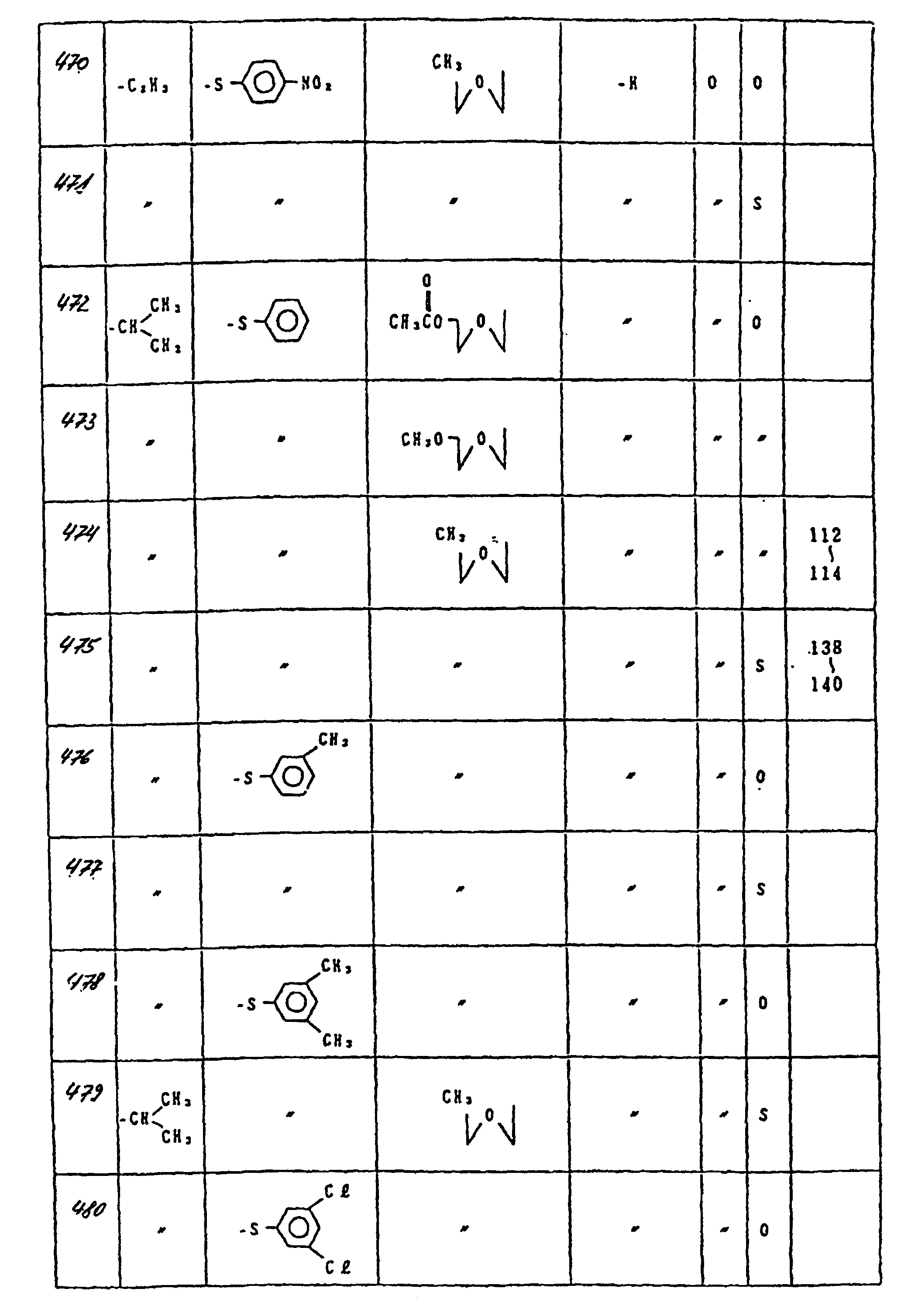

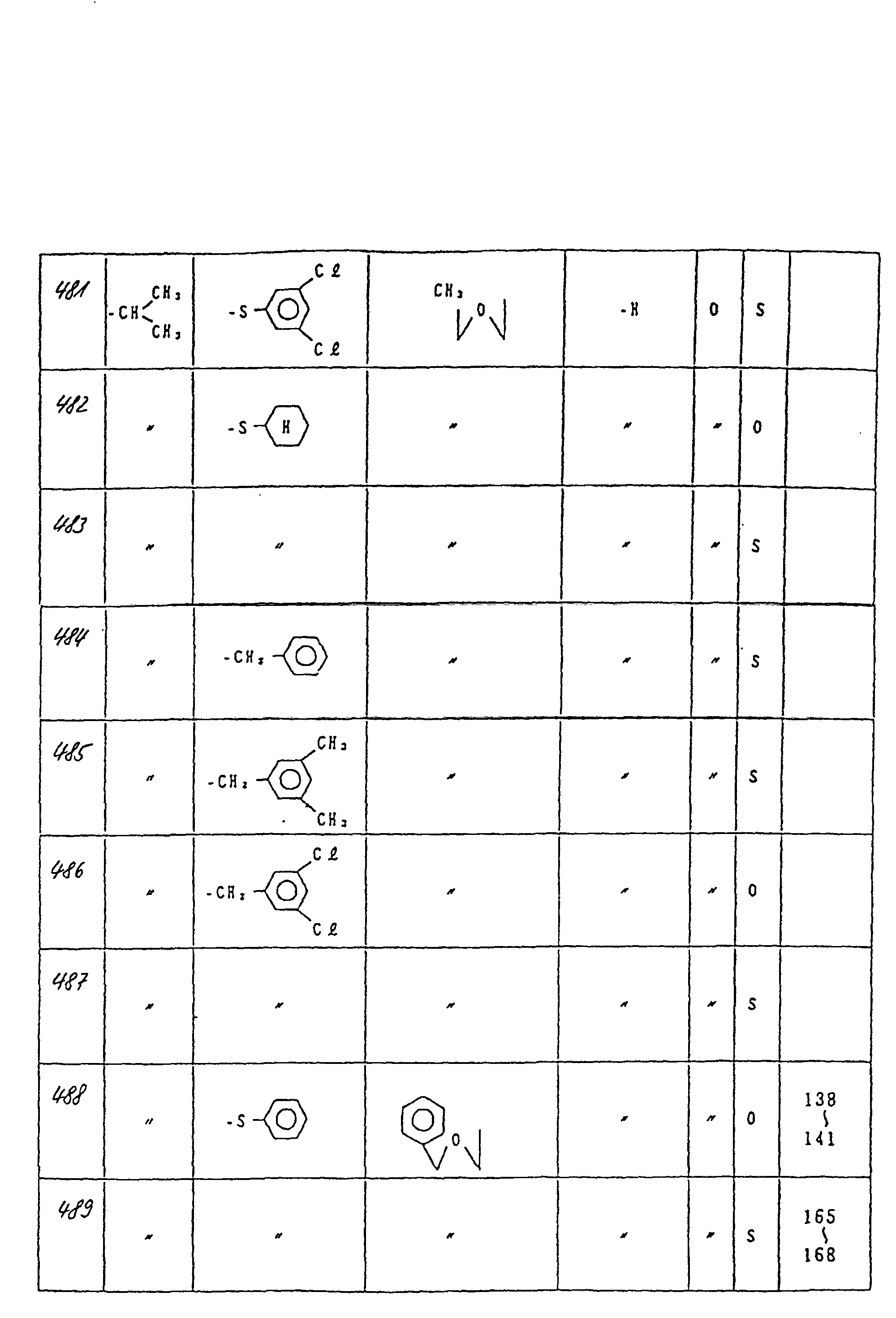

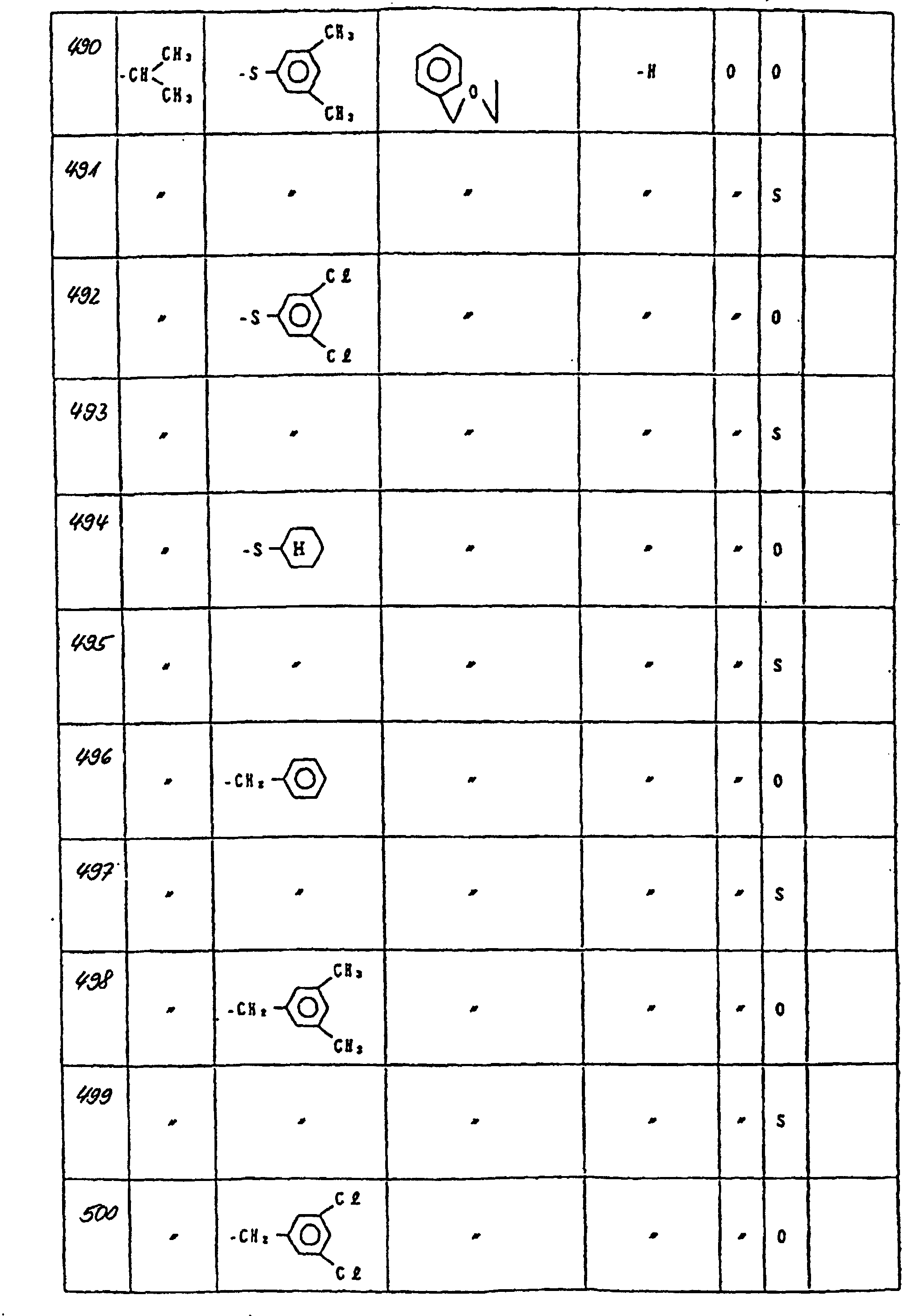

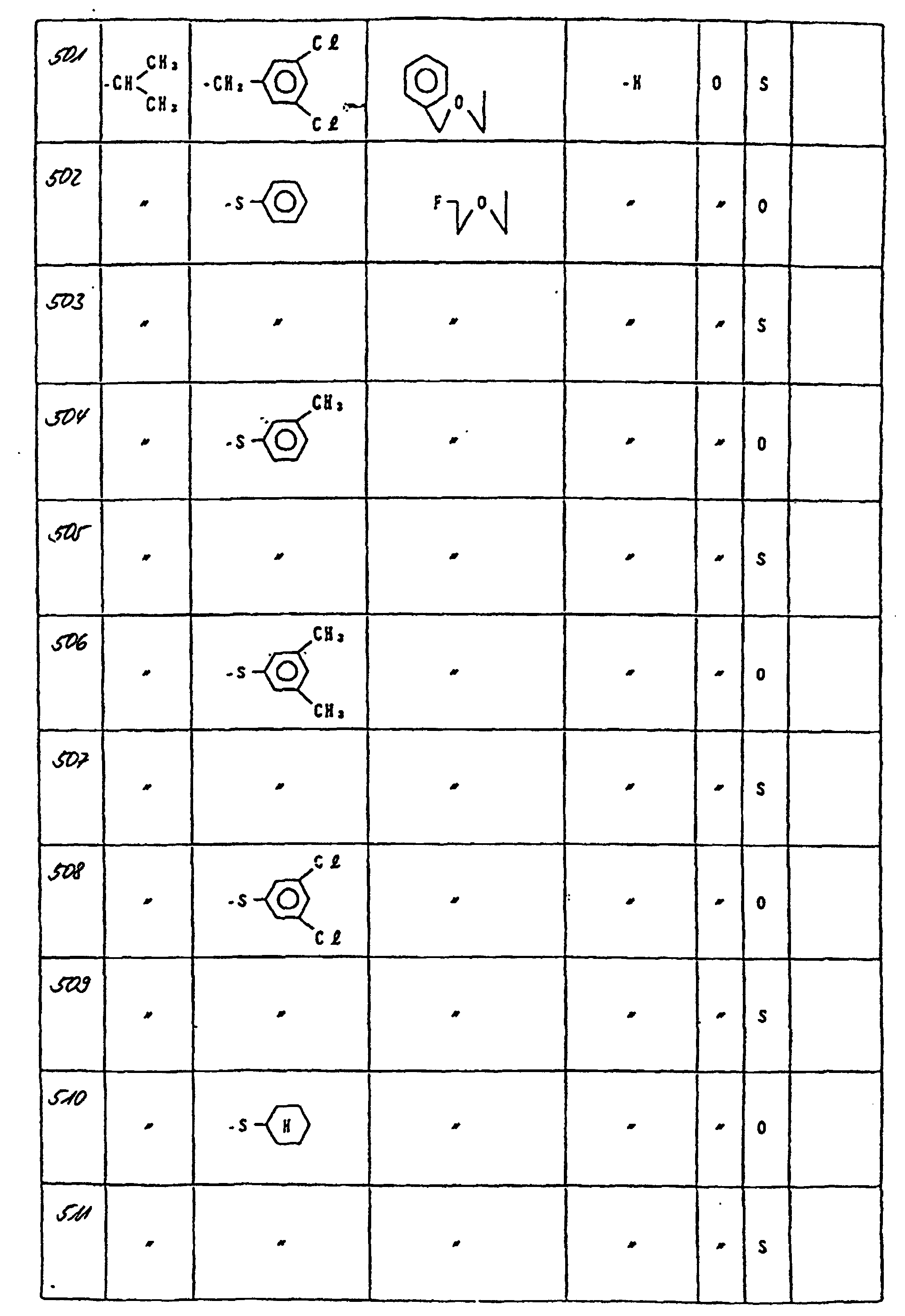

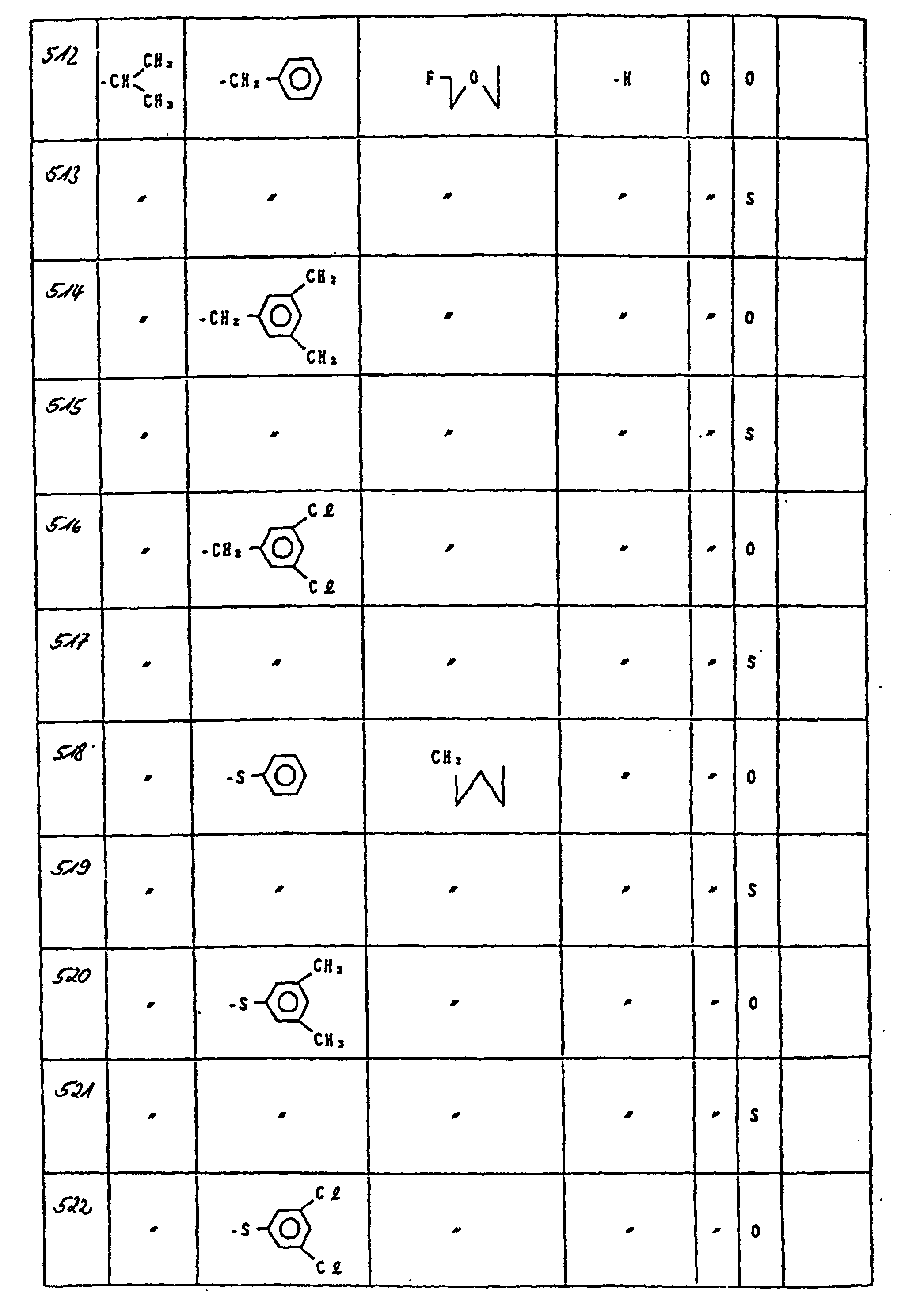

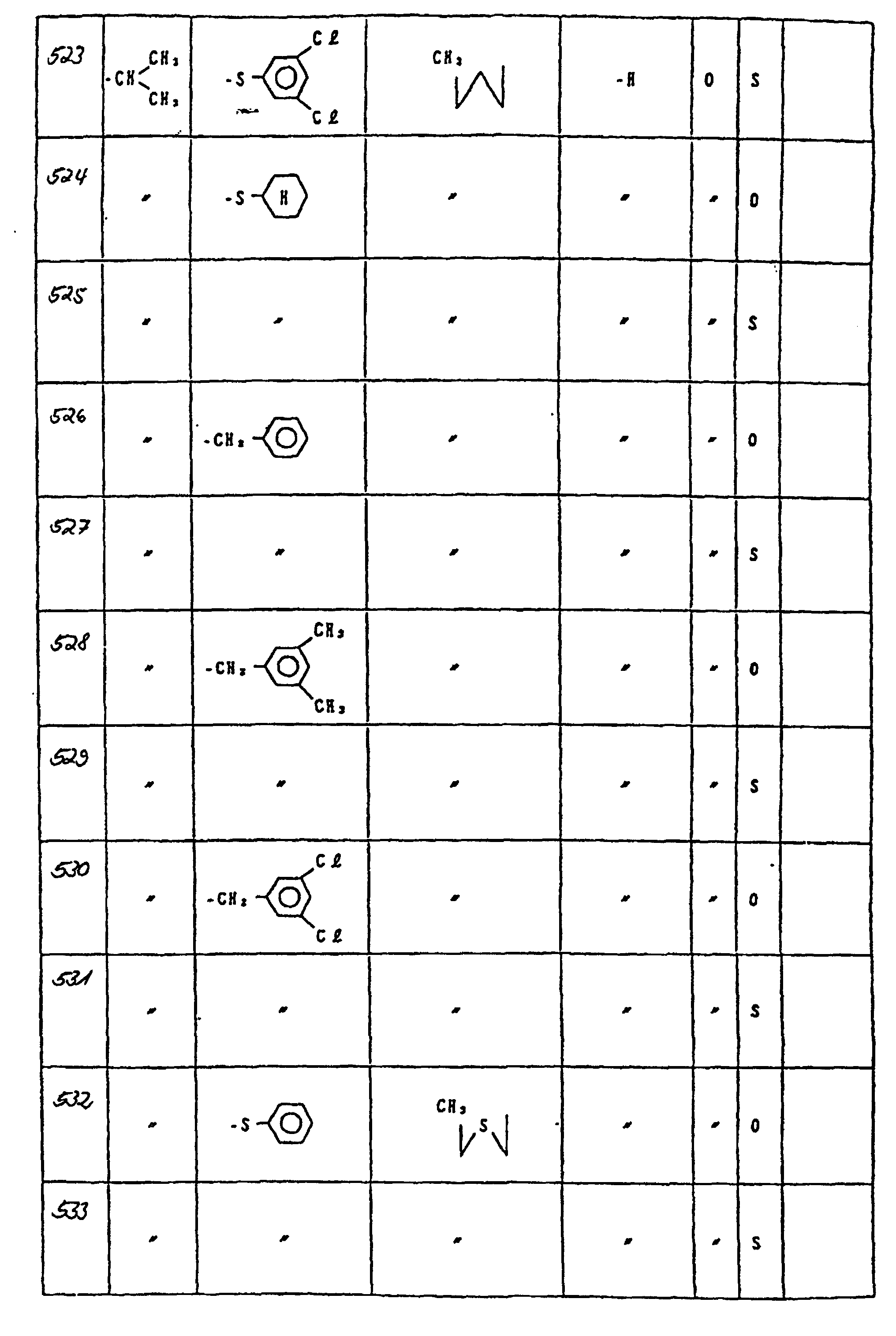

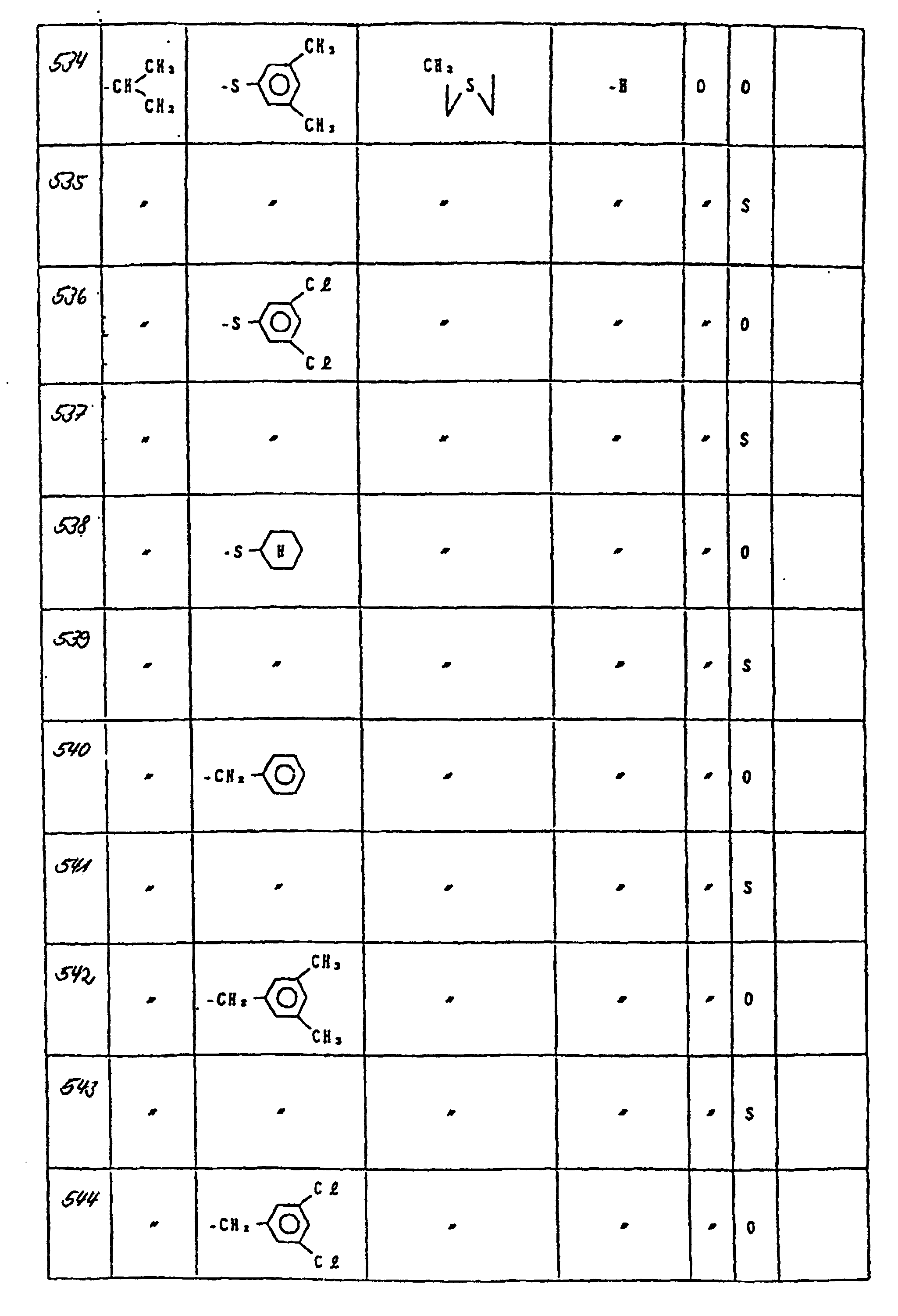

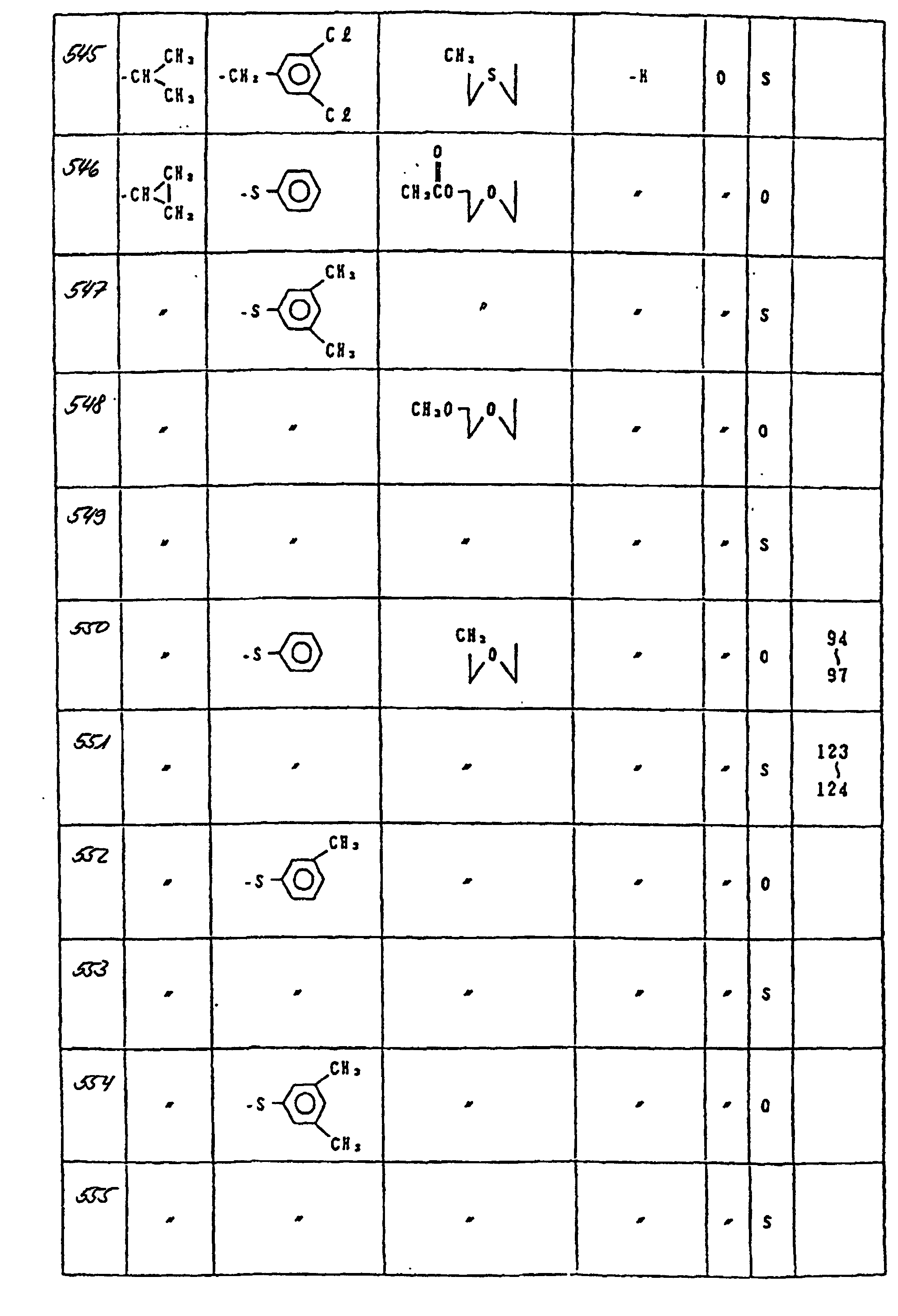

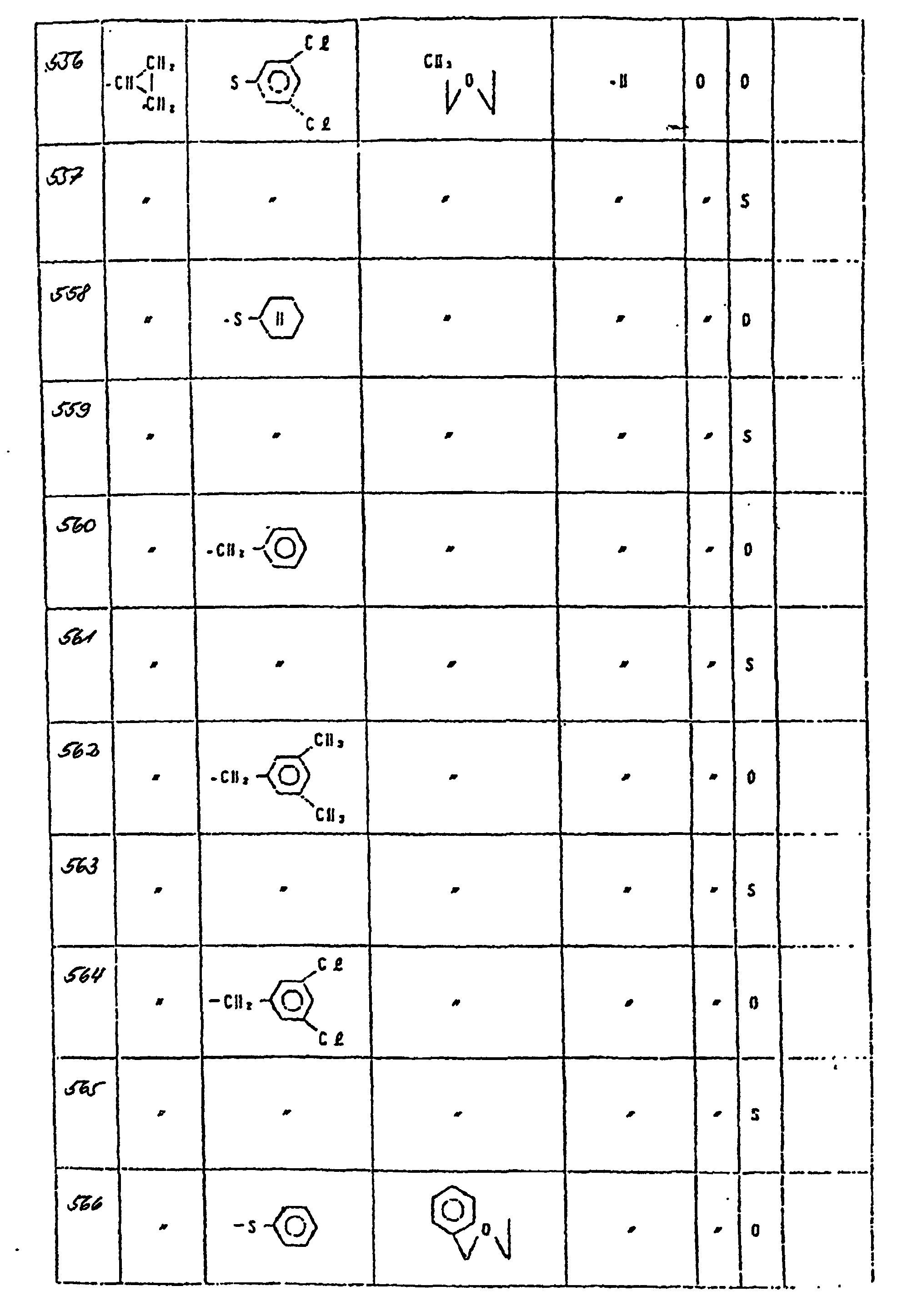

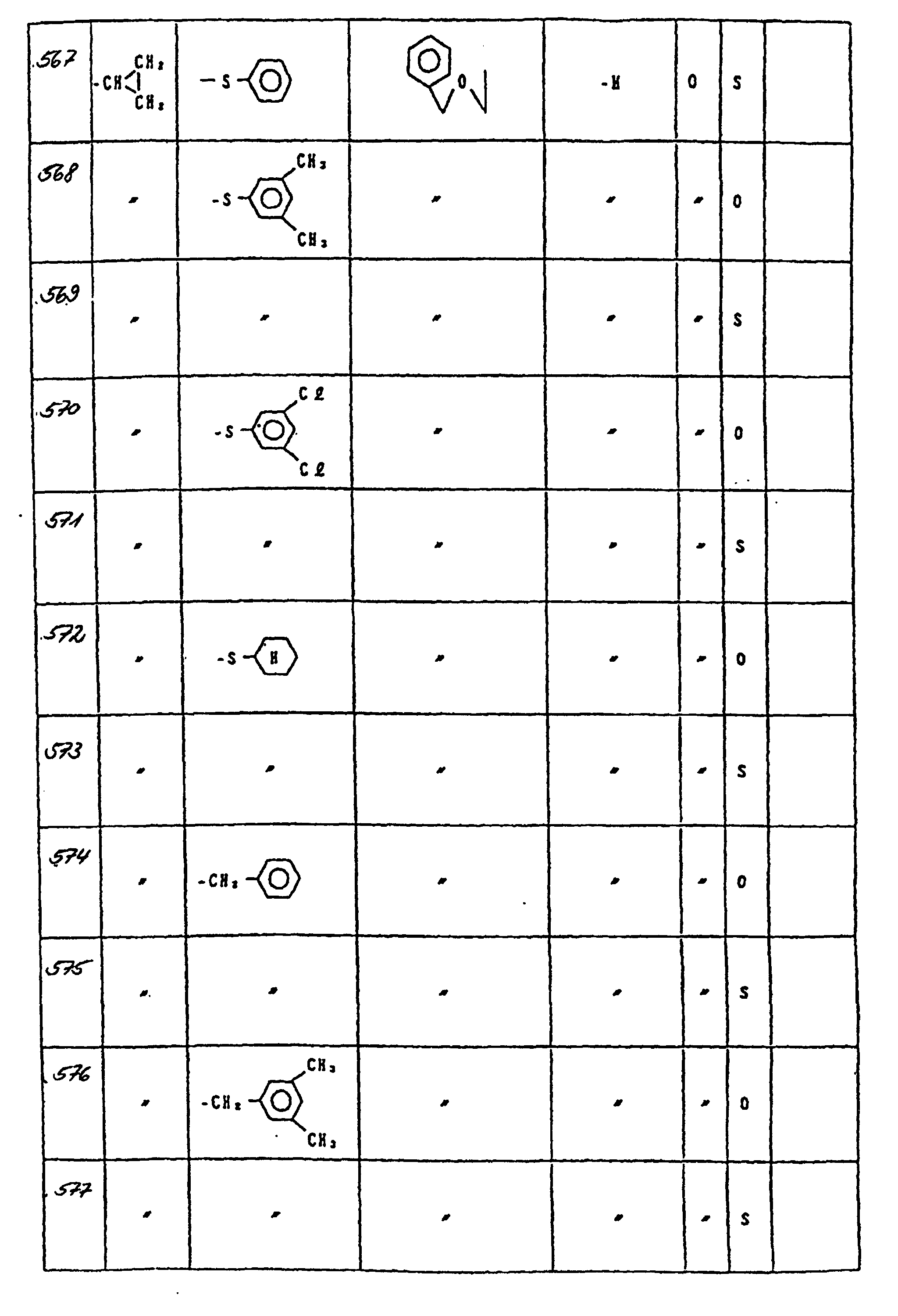

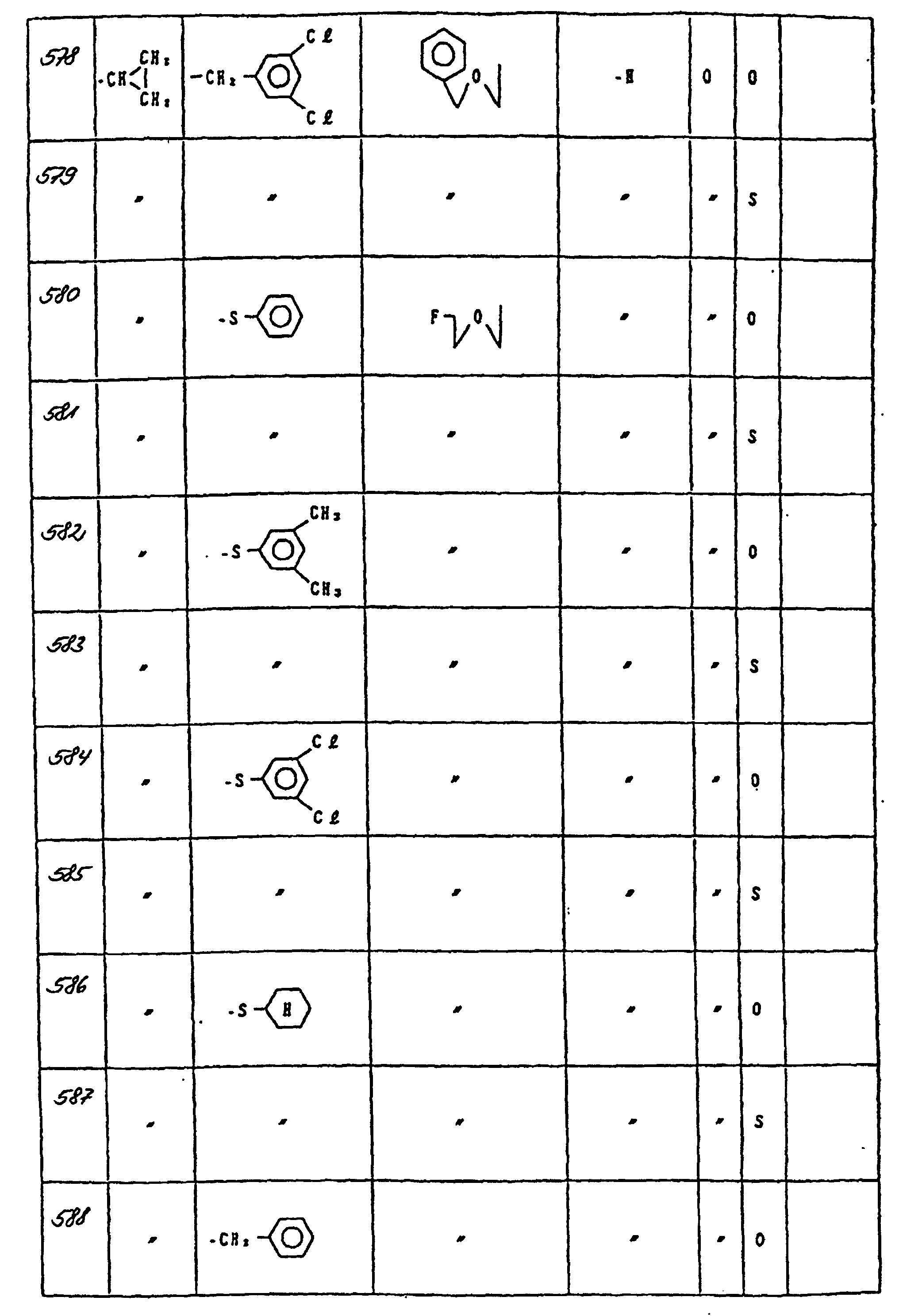

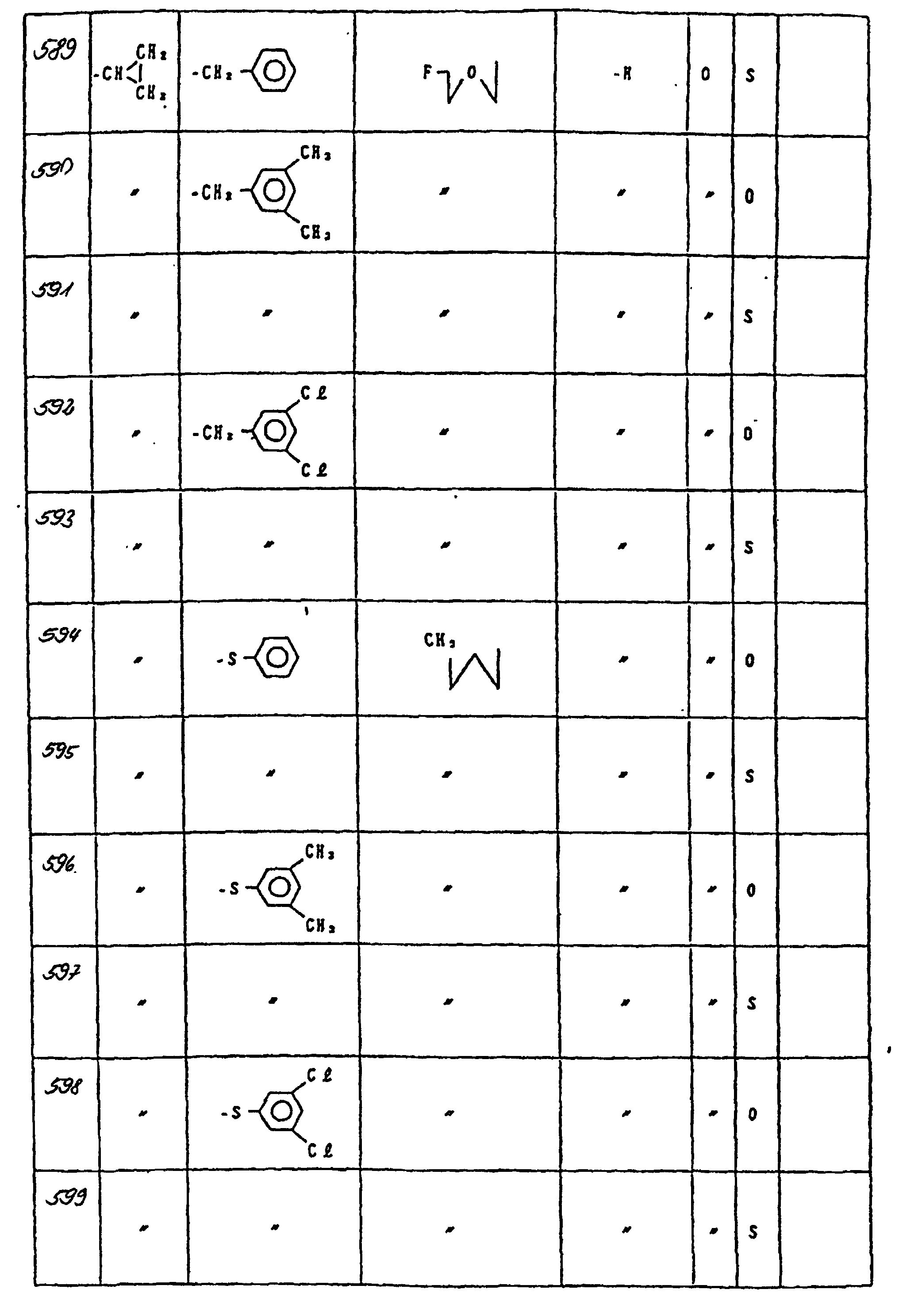

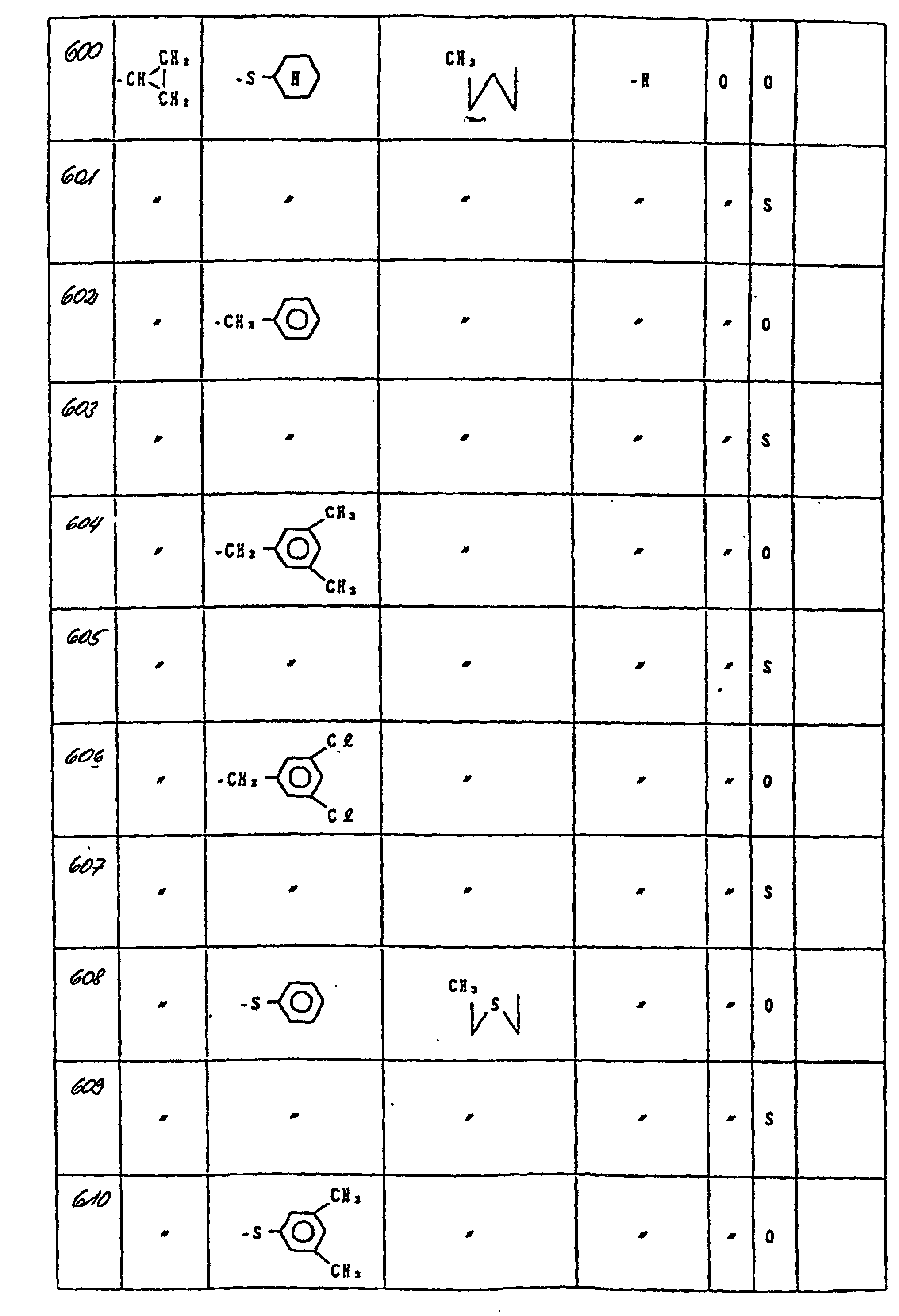

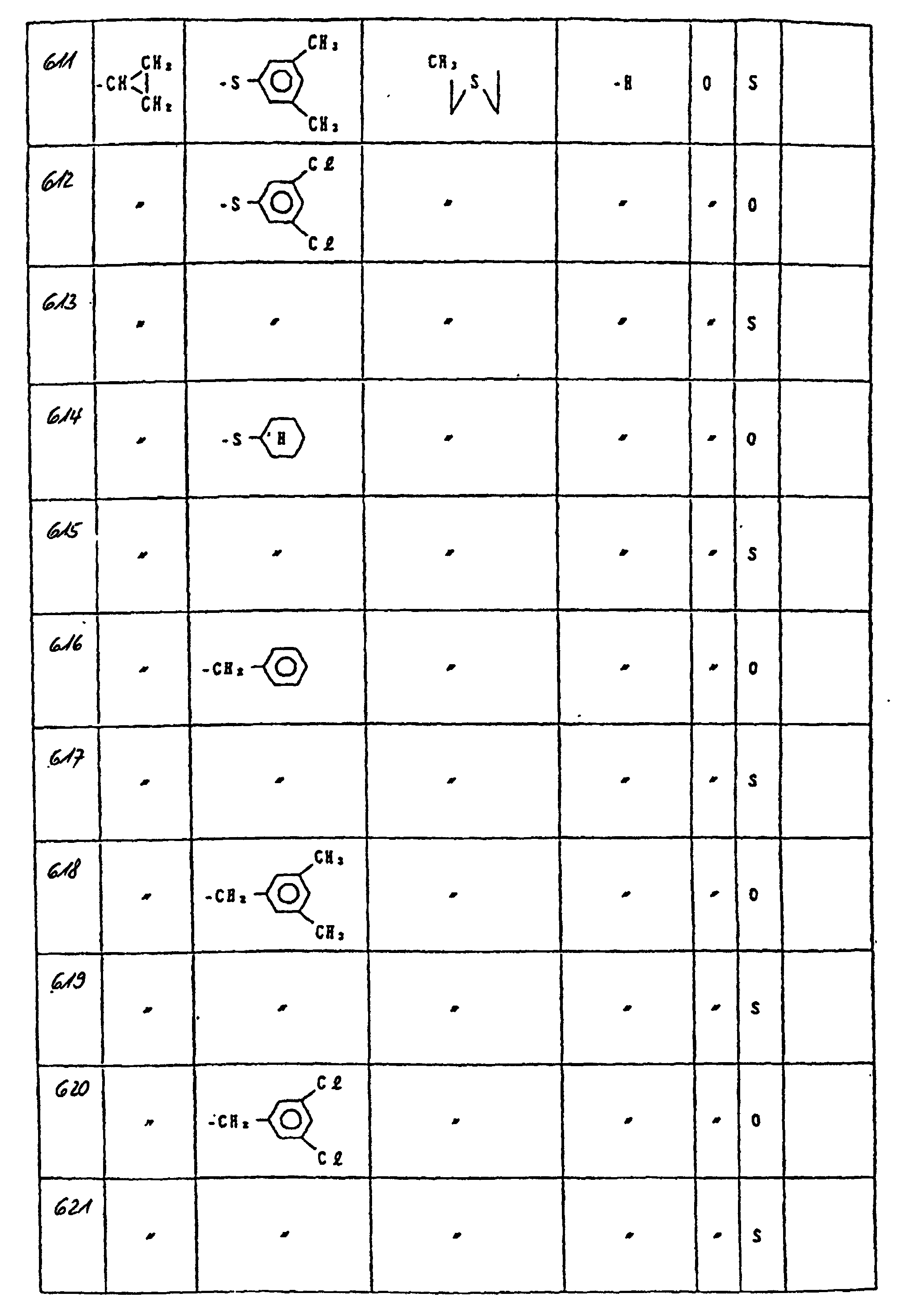

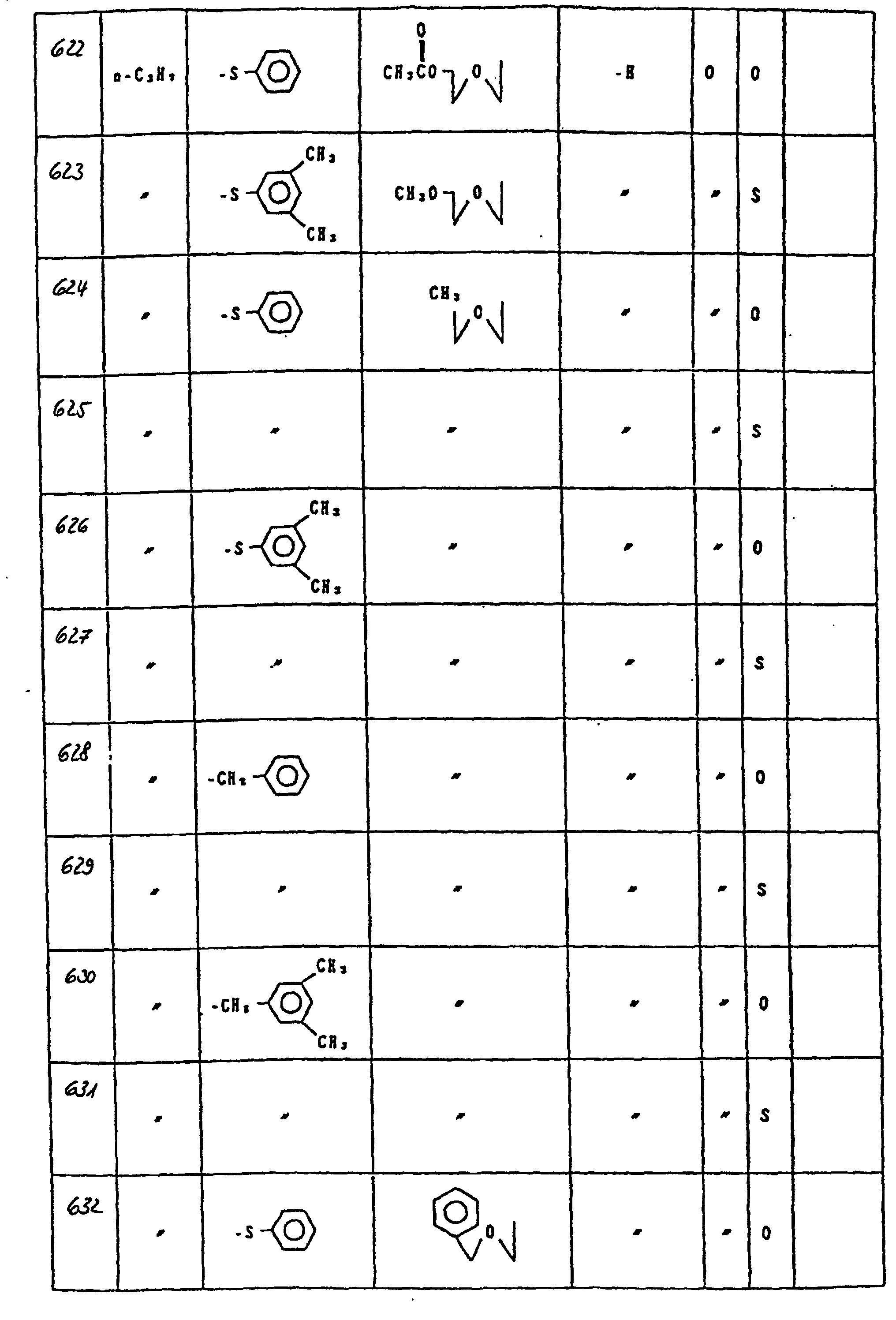

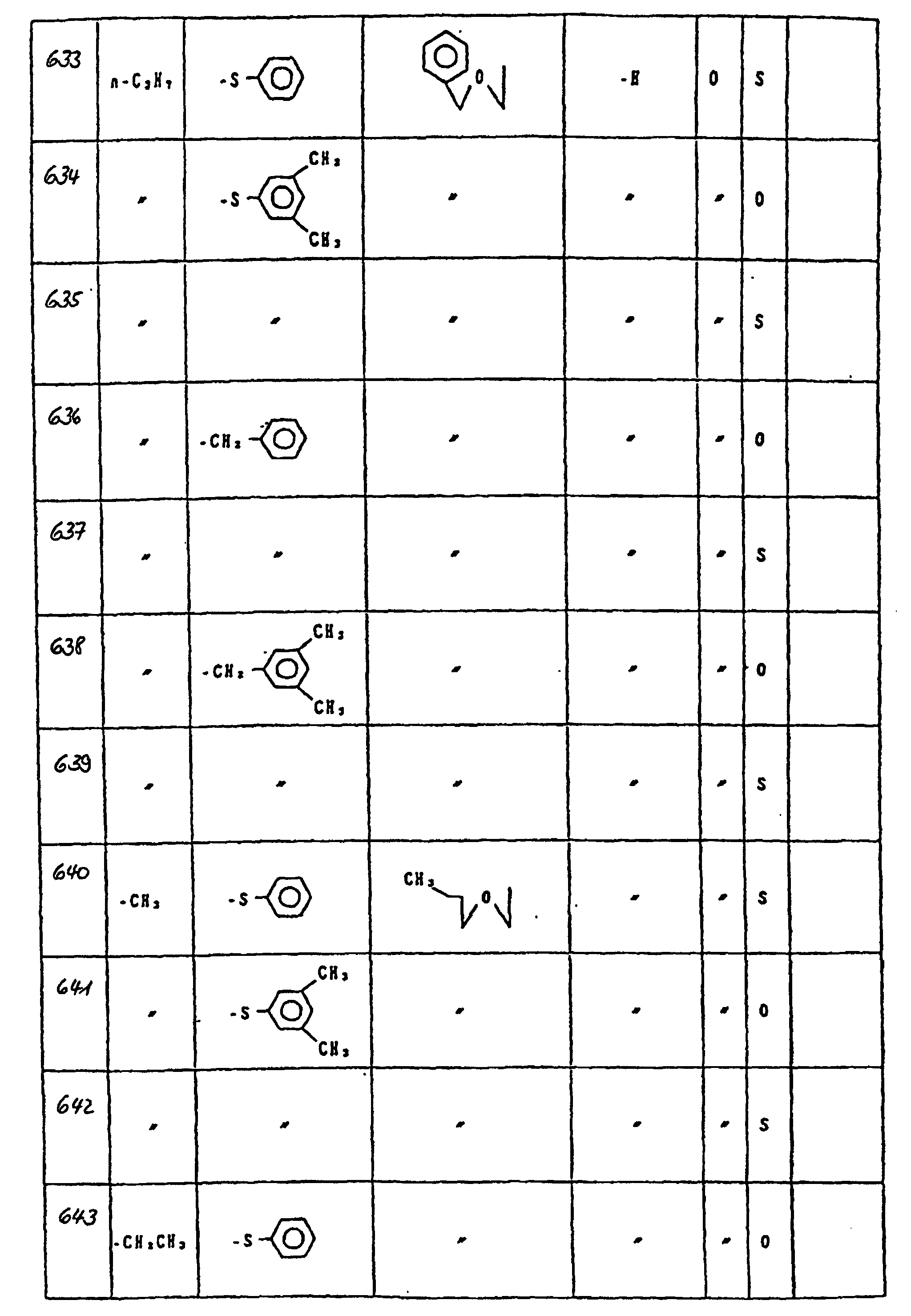

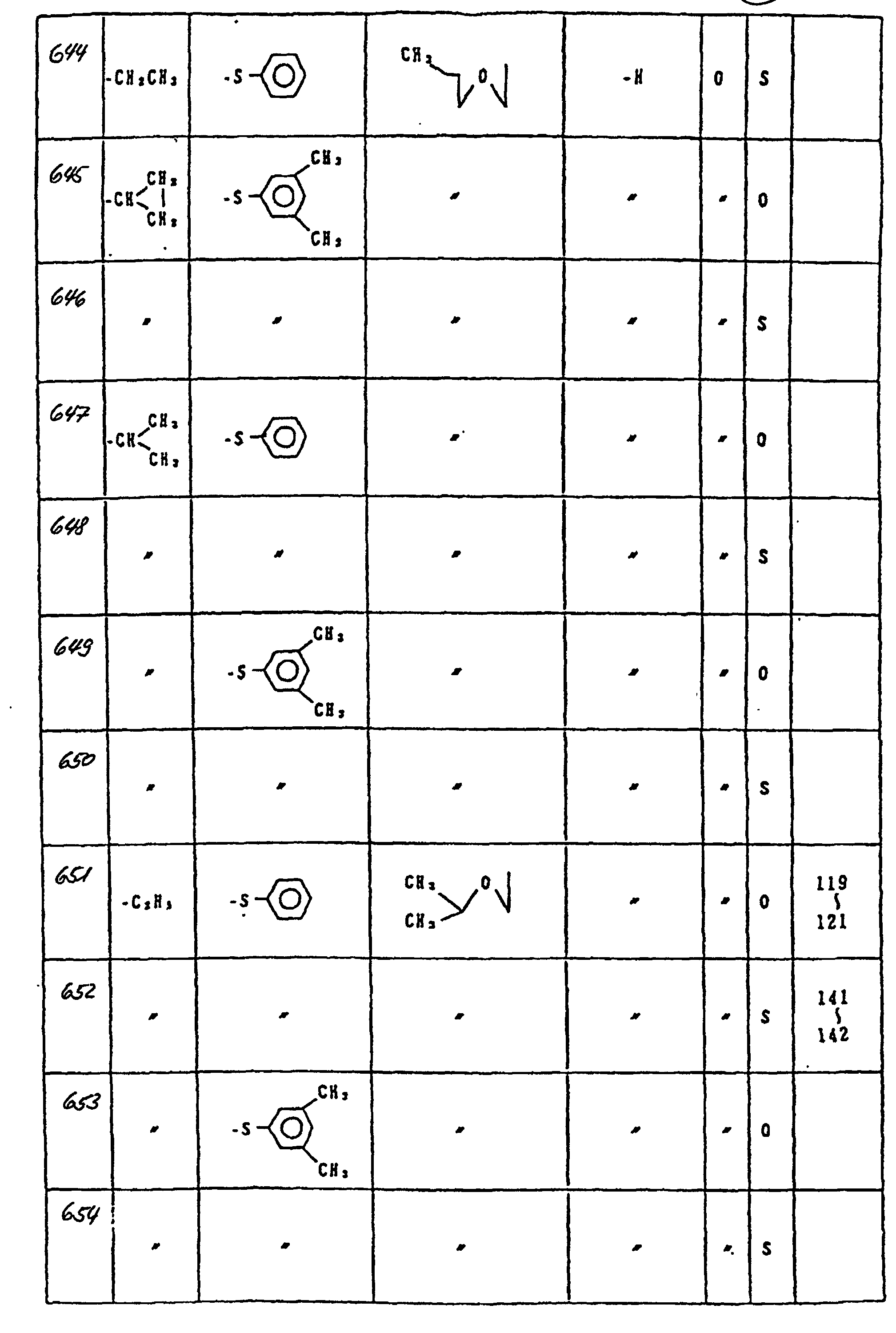

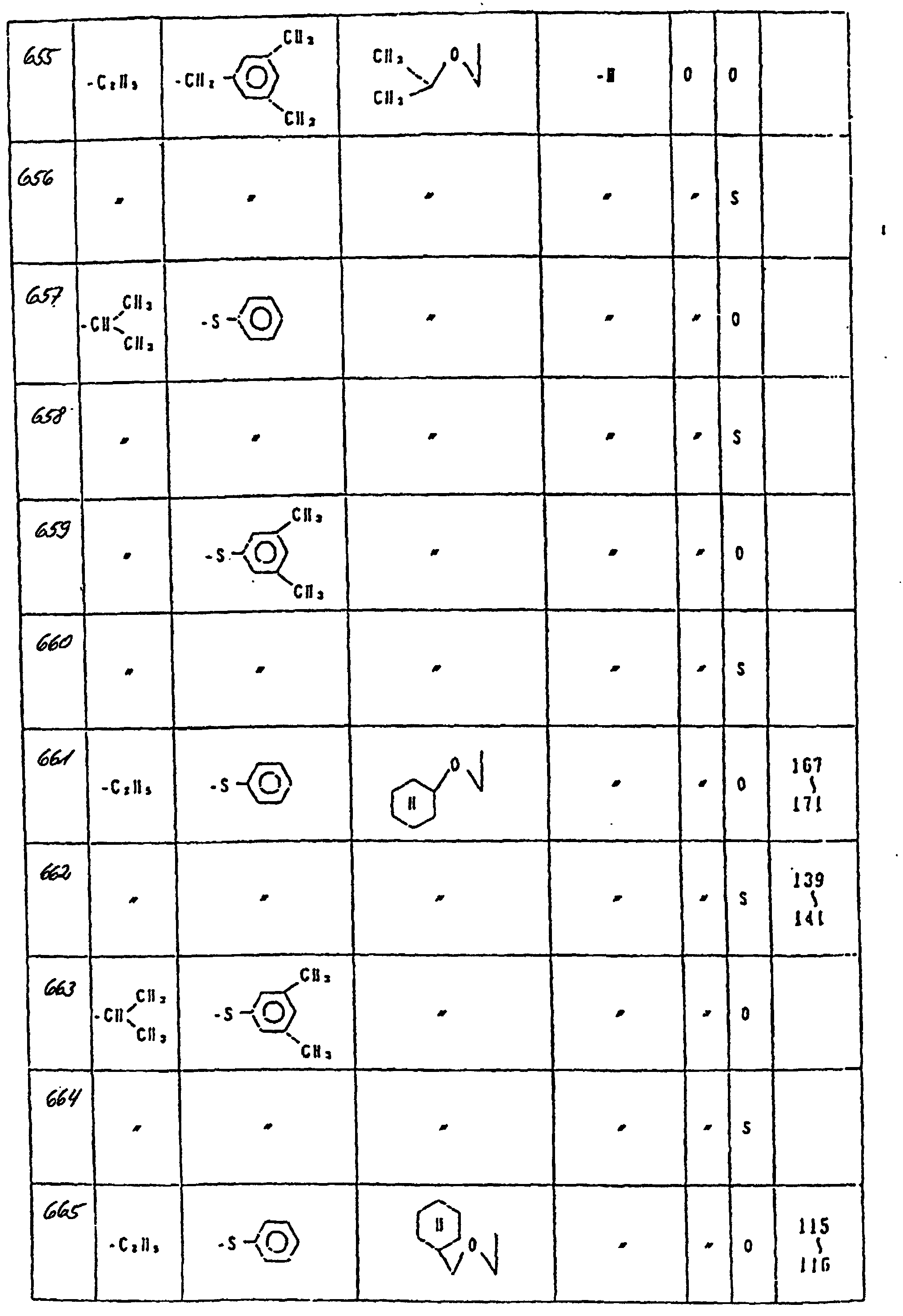

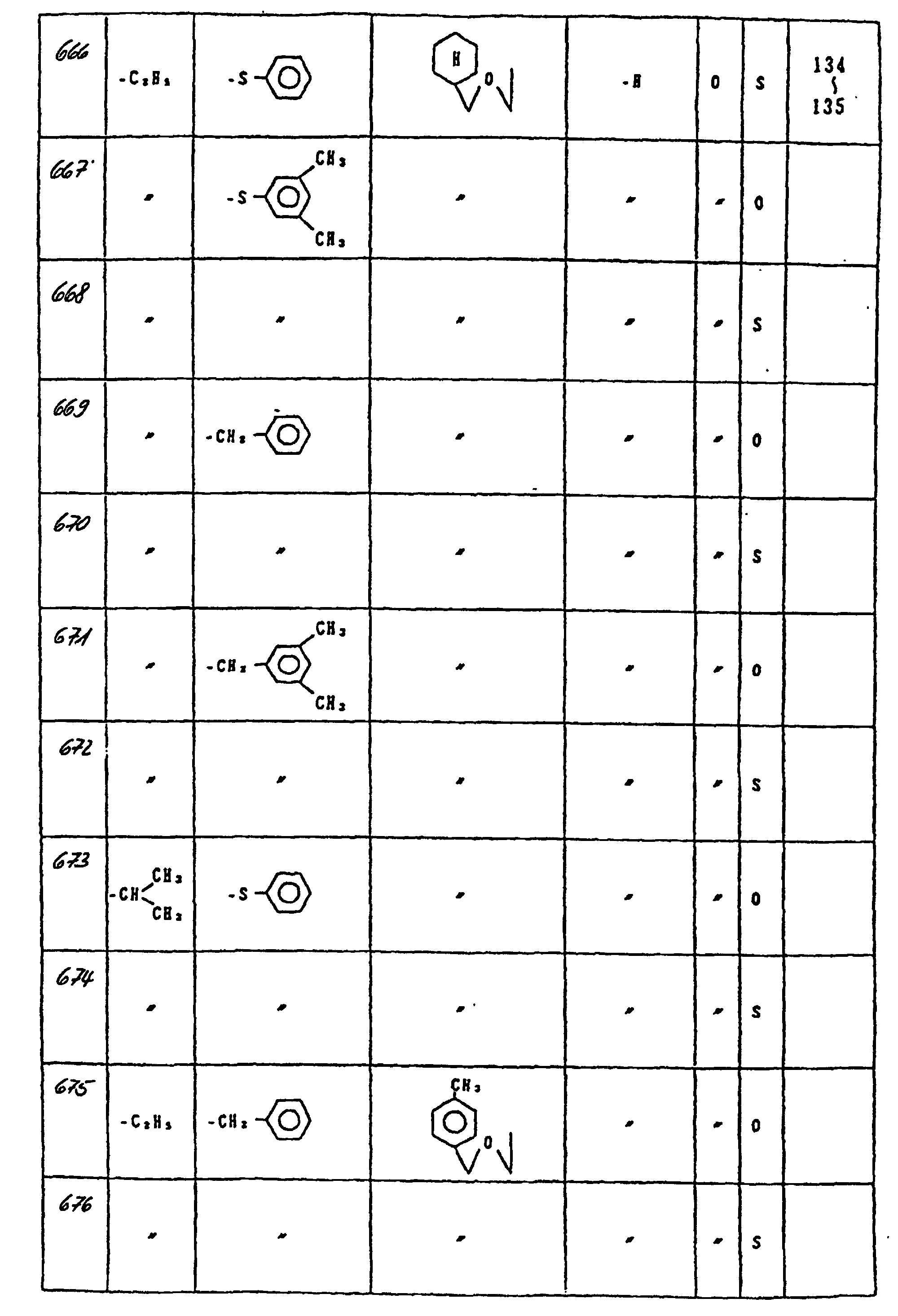

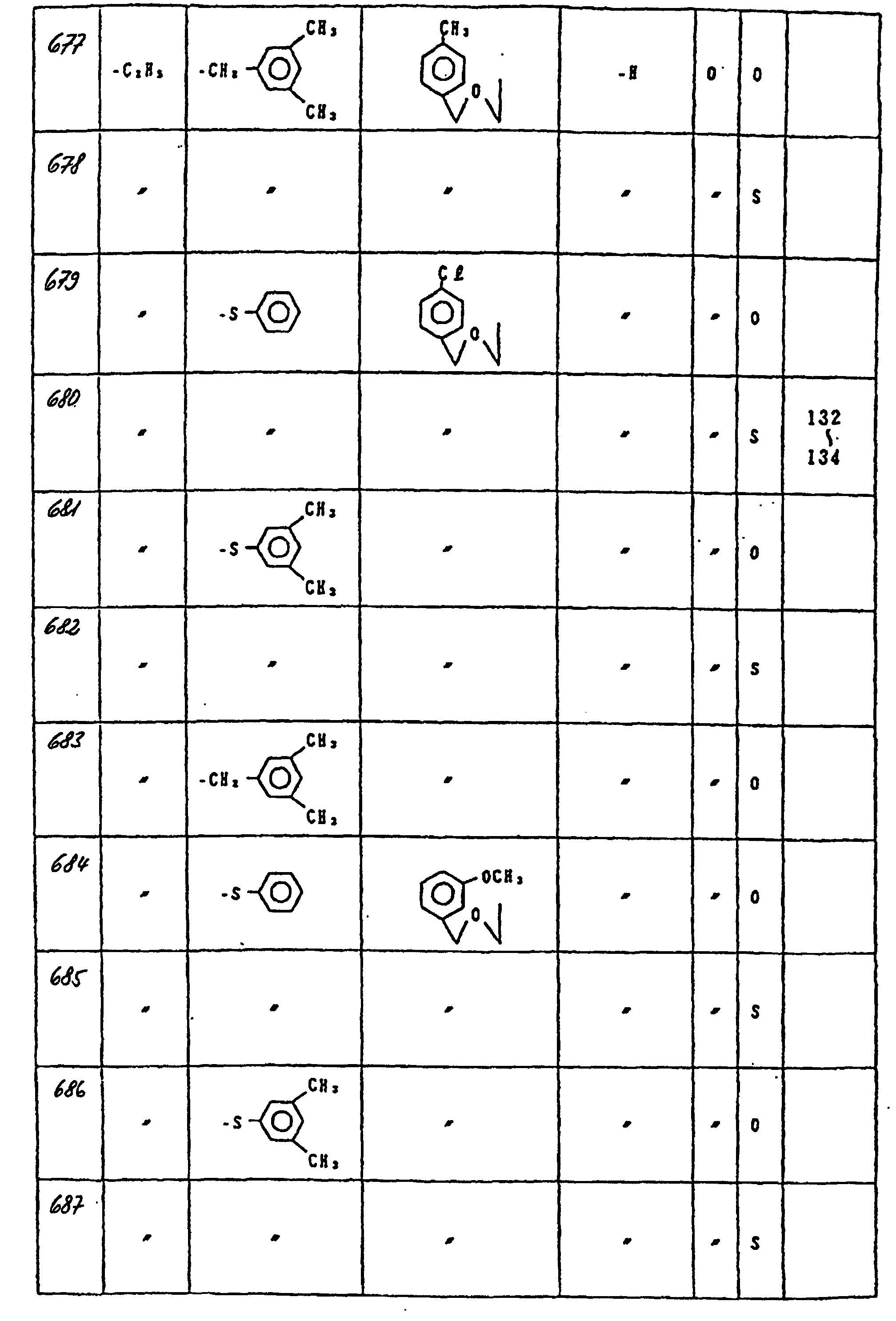

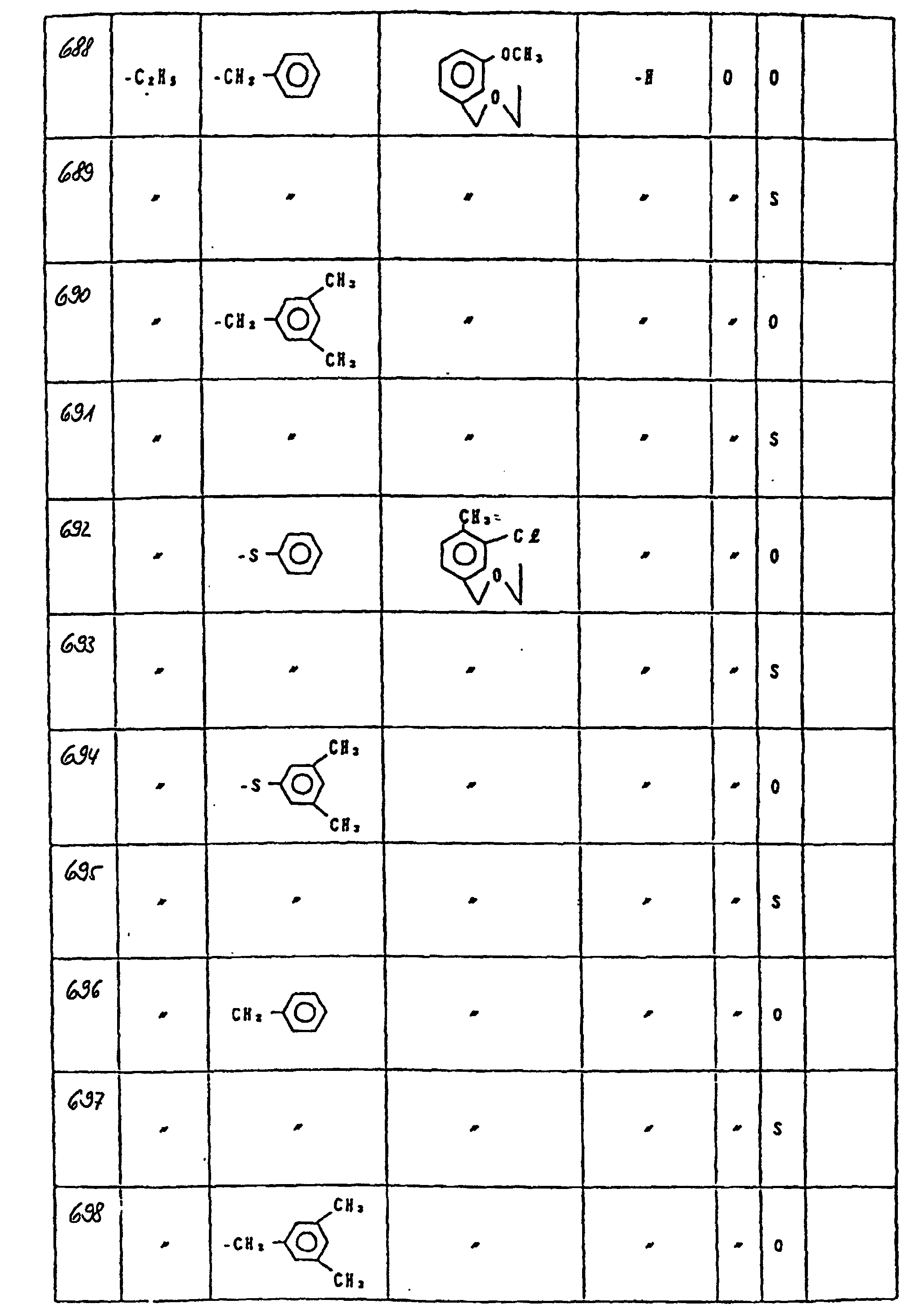

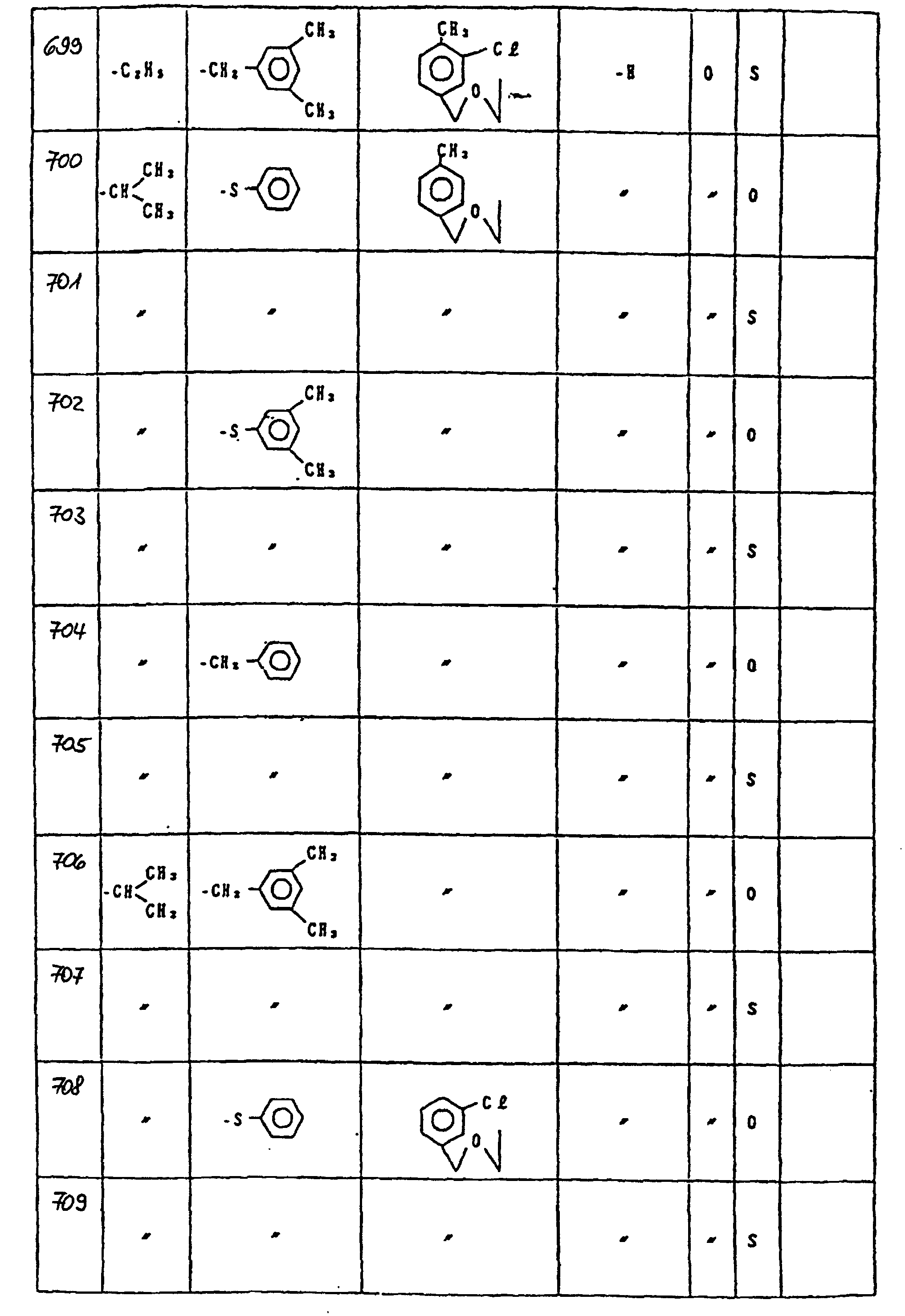

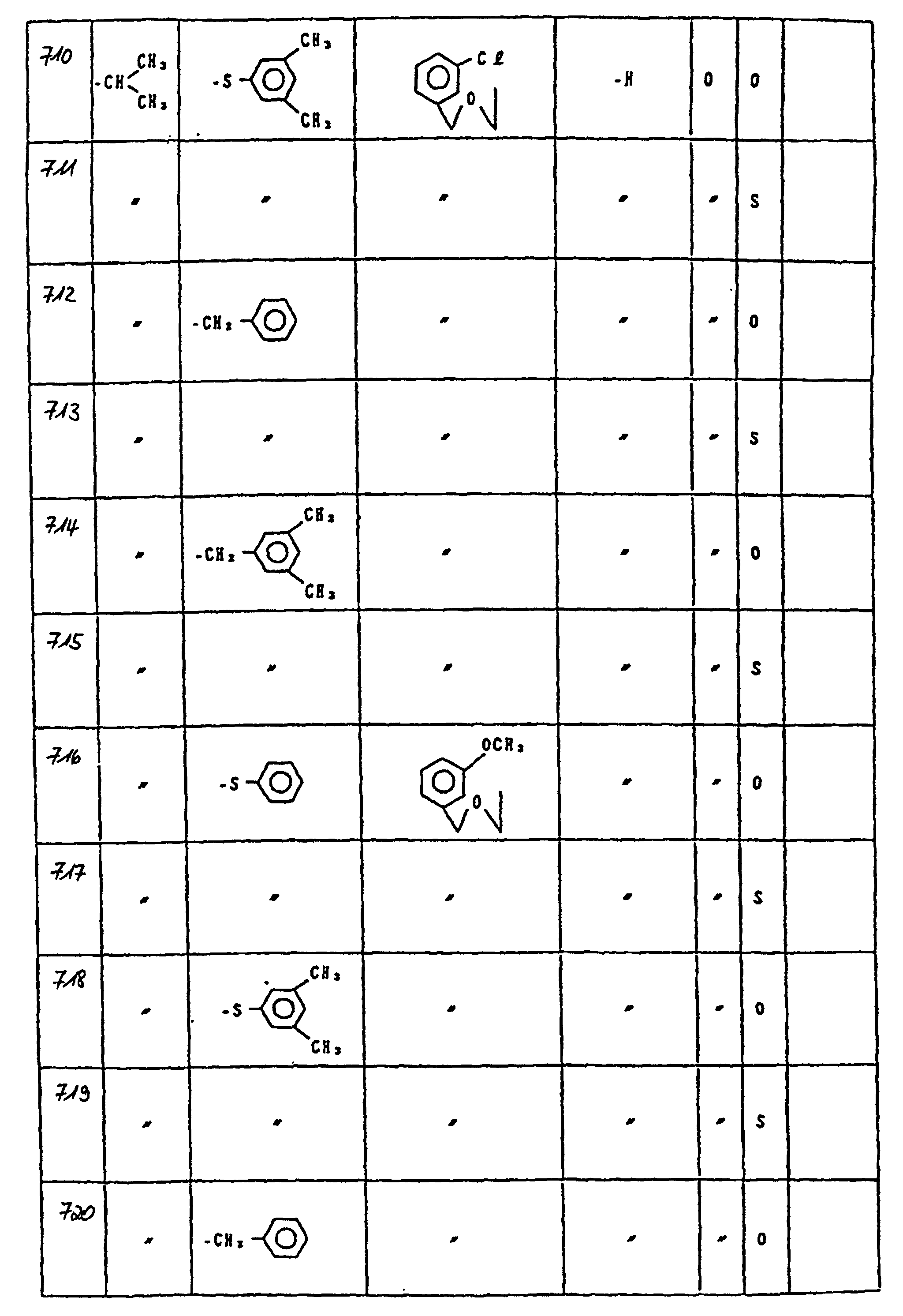

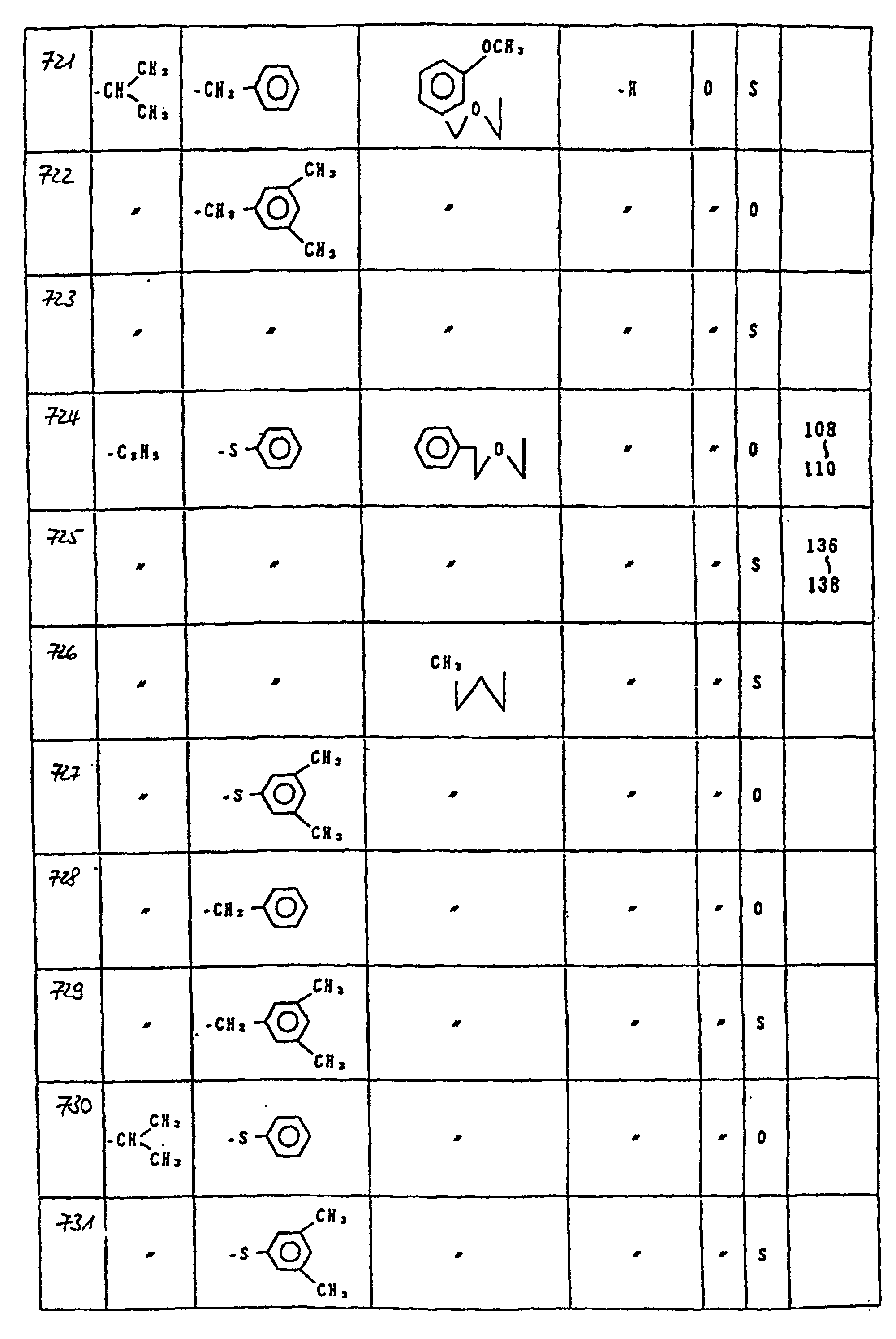

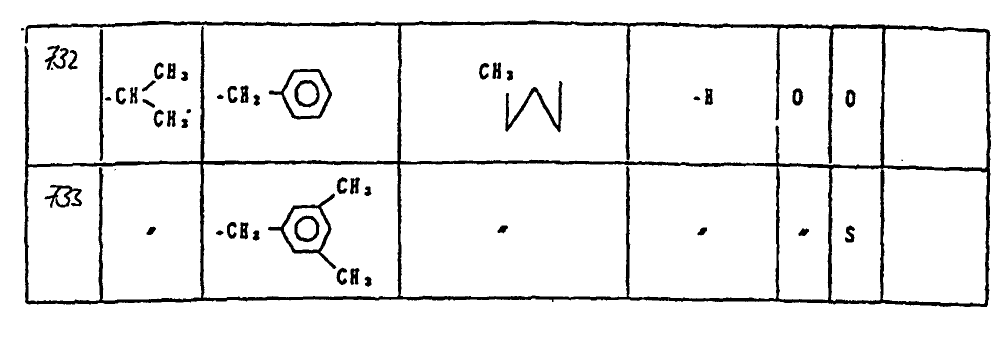

The compounds according to the invention of the formula I wherein R³ represents methyl or C₃ to C₁₀ branched alkyl or -CH2-Z(CH₂)n-R⁵ group where R⁵ represents a hydrogen, halogen atom, azido, C₁ to C₁₀ alkoxy, C₇ to C₁₃ aralkyloxy, optionally substituted C₆ to C₁₂ aryl group or the like may be prepared in accordance with the following reaction formula (1), (2) or (3): 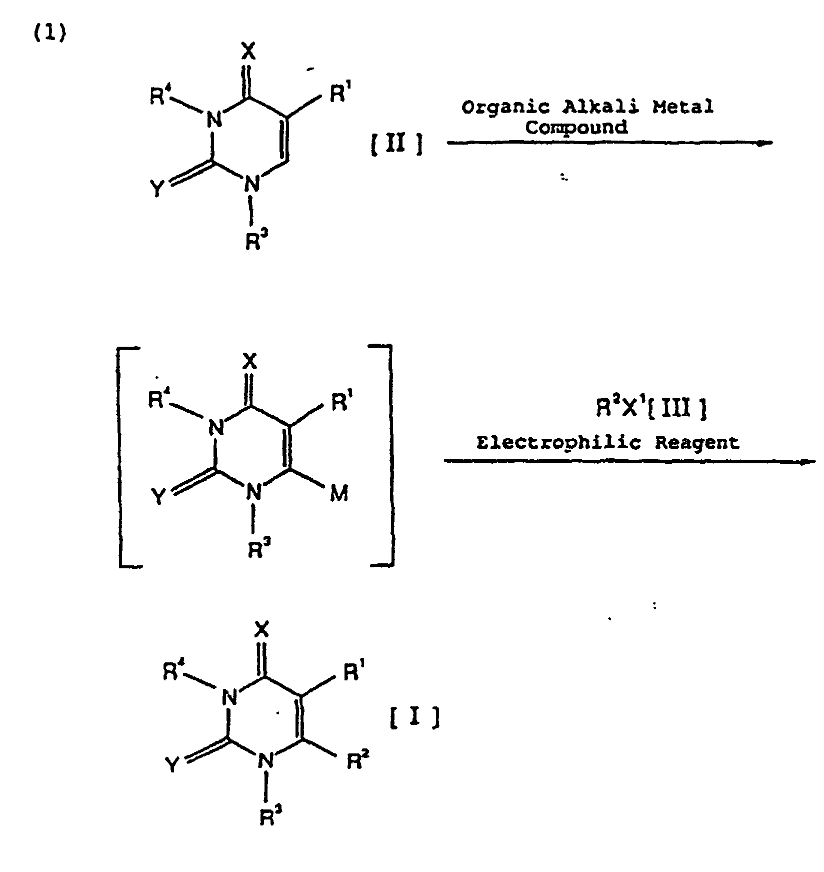 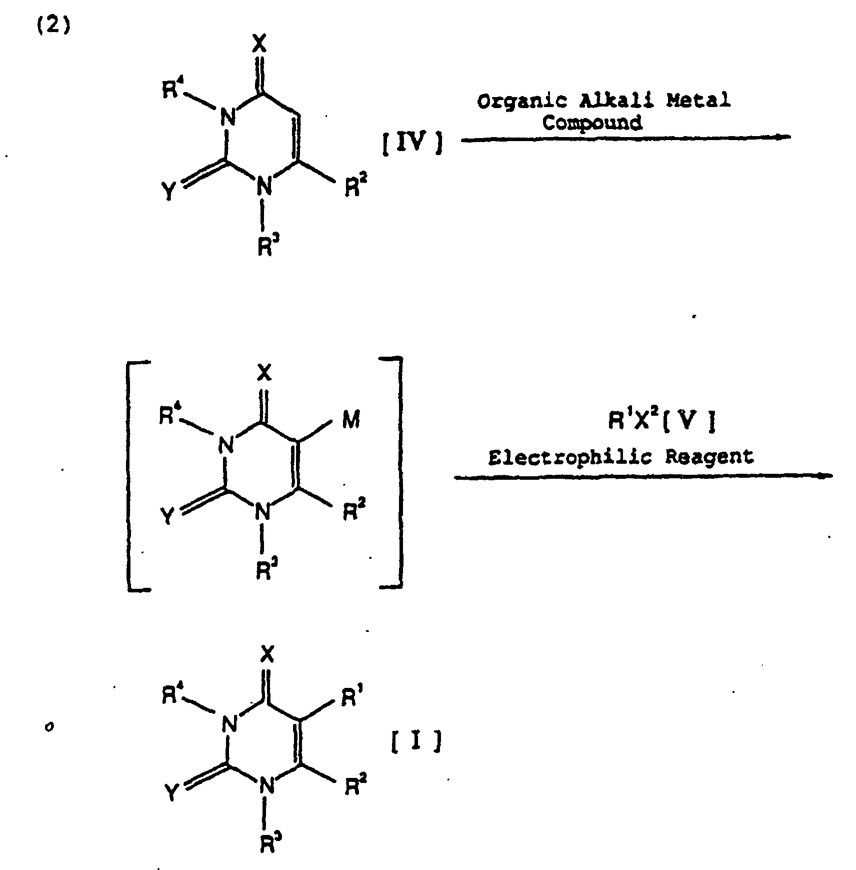 wherein R¹, R², R³, R⁴, X and Y have the same meanings defined hereinbefore, X¹ and X² represent a halogen atom, arylthio, alkoxy group or the like, and M represents an alkaline metal.

Firstly, the compound of the formula II or IV is treated with an organic alkali metal compound in an ether solvent such as diethyl ether and tetrahydrofuran at a temperature of -80 to -10°C for 0.2 to 10 hours.

Examples of the organic alkali metal compound include potassium bistrimethylsilylamide, sodium bistrimethylsilylamide and lithium alkylamide, and particularly preferred compounds among those are lithium diisopropylamide (LDA) and lithium 2,2,6,6-tetramethylpiperidide (LTMP). Such lithium alkylamides are preferably prepared immediately before the reaction. For example, lithium dialkylamide may be prepared by reacting a secondary amine such as diisopropylamine with an alkyl lithium such as n-butyl lithium in a solvent such as diethyl ether, dioxane, tetrahydrofuran and dimethoxyethane with stirring under the atmosphere of an inert gas such as argon at -80°C to -10°C for 0.2 to 5 hours.

The organic alkali metal compound is usually used in an amount of 1 to 5 moles per mole of the compound of the general formula II or IV.

Then, the electrophilic reagent of the general formula R²X¹ or R¹X² is added to the reaction mixture in a ratio of about 1 to 5 moles to the compound of the general formula II or IV to allow the reaction under the same condition as in the reaction with the organic alkali metal compound.

The electrophilic reagent should have a group of R¹ or R² defined above, and examples of this reagent includes various diaryl disulfides, arylsulfenyl chlorides, dialkyl disulfides, dicycloalkyl disulfides, alkyl halides, aralkyl halides such as benzyl bromide, acid halides such as benzoyl halide and isobutyric halide, acid anhydrides and esters thereof, arylcarbonylalkyl halides such as phenacyl chloride and the like.

The compounds of the general formula II can be prepared by a conventional method.

The compounds of the general formula IV can be prepared in accordance with the reaction formula (1) above (R¹ = H). 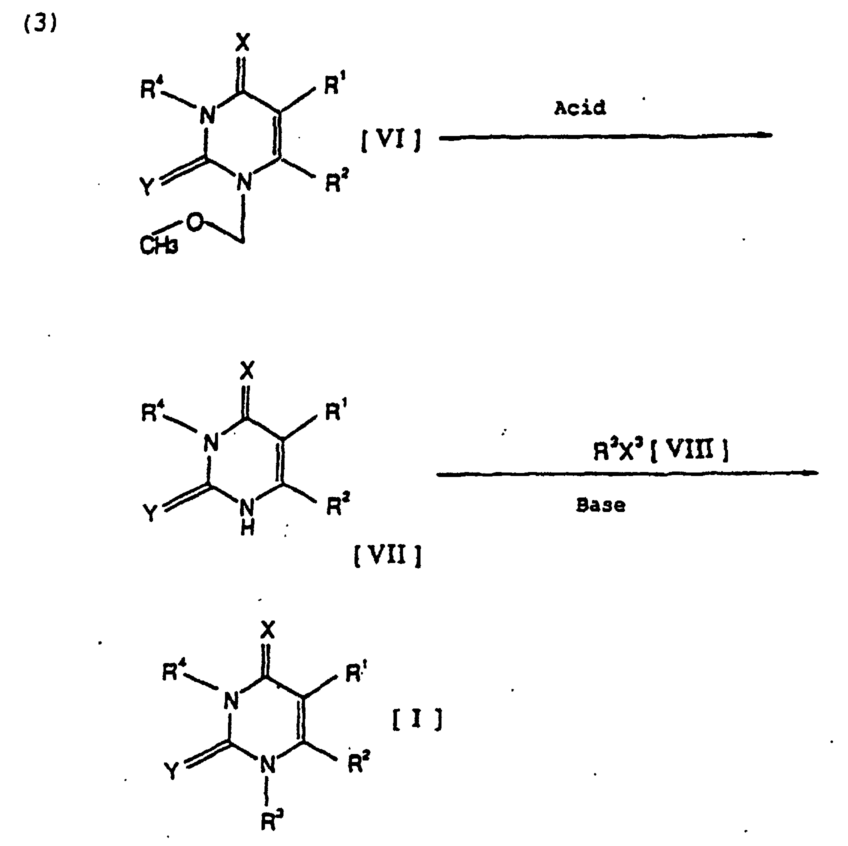 wherein R¹, R², R³, R⁴, X and Y have the same meanings defined hereinbefore and X³ represents a halogen atom such as chlorine, bromine and iodine or sulfonyloxy group such as toluenesulfonyloxy and mesyloxy groups.

The compounds of the general formula VI are treated with an acid such as hydrochloric acid and bromic acid in a suitable solvent, for example, an alcohol such as methanol and ethanol and water at an appropriate temperature of from room temperature to 100°C to obtain the compounds of the general formula VII.

Then, the compounds of the general formula VII are reacted with the compounds of the general formula VIII in a suitable solvent such as dimethylformamide, dimethyl sulfoxide, acetonitrile and tetrahydrofuran in the presence of a suitable base such as sodium hydride, sodium alkoxide, potassium alkoxide, potassium carbonate and sodium carbonate at a temperature of from ambient temperature to the boiling point of the solvent to obtain the compounds of the general formula I.

The starting compounds represented by the general formula VI can be prepared in accordance with the reaction formula (1) or (2).

When the objective compound has a hydroxyl group of R⁵ or when any intermediate compound of the reactions has a hydroxyl group, the reactions of (1) and (2) should be carried out by using a starting compound or intermediate compound of which hydroxyl group is protected by an appropriate protective group instead of the unprotected compound of the formula II or IV or the like, and the protective group is then eliminated to obtain the target compound.

Any protective groups conventionally used for the protection of hydroxyl group may be used for this purpose so long as it is not eliminated under the alkaline condition.

Examples of such protective group are aralkyl groups such as benzyl, trityl, monomethoxytrityl, dimethoxytrityl and trimethoxytrityl, silyl groups such as trimethylsilyl, triethylsilyl, t-butyldimethylsilyl and t-butyldiphenylsilyl, tetrahydropyranyl group and substituted alkyl groups such as methoxymethyl group. Among those protective groups, silyl groups are particularly preferred.

The introduction of the protective group can be carried out by a conventional method.

For example, the introduction of the protective silyl group may be carried out by reacting the compound having the hydroxyl group with 1 to 10 times by mole of silylating reagent such as trimethylsilyl chloride and t-butyldimethylsilyl chloride at a temperature of from 0 to 50°C in the presence of a base such as pyridine, picoline, diethylaniline, dimethylaniline, triethylamine and imidazole in a solvent such as dimethylformamide, acetonitrile, tetrahydrofuran and a mixture of those solvents in any combination.

The elimination of the protective group may be carried out by a conventional method corresponding to the kind of the protective group, for example, acid hydrolysis, ammonium fluoride treatment or catalytic reduction.

The compounds obtained by the reactions (1), (2) or (3) which have a nitro substituted phenylthio group at the 6-position may be converted into the compounds having an amino group by hydrogenation in accordance with the reaction formula (4) below. The hydrogenation can be carried out in an organic solvent such as alcohol and acetic acid in the presence of a catalyst such as palladium/carbon at an appropriate temperature of from room temperature to 80°C: 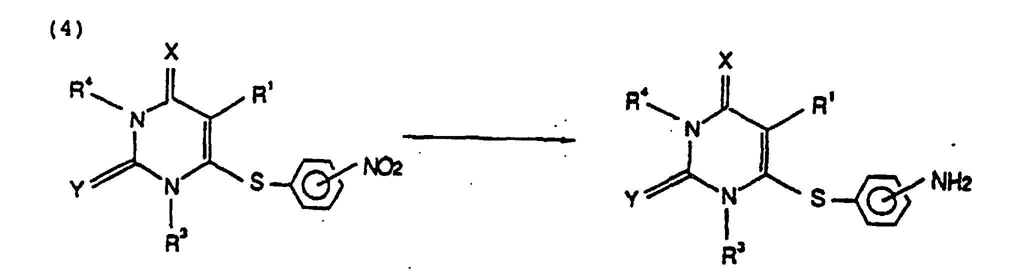 wherein the symbols have the same meanings as defined above.

The compounds having an arylthio, alkylthio or cycloalkylthio group can be converted to corresponding compounds having an arylsulfinyl, alkylsulfinyl or cycloalkylsulfinyl group by using an oxidizing agent such as hydrogen peroxide and m-chloroperbenzoic acid in accordance with the reaction formula (5) below: 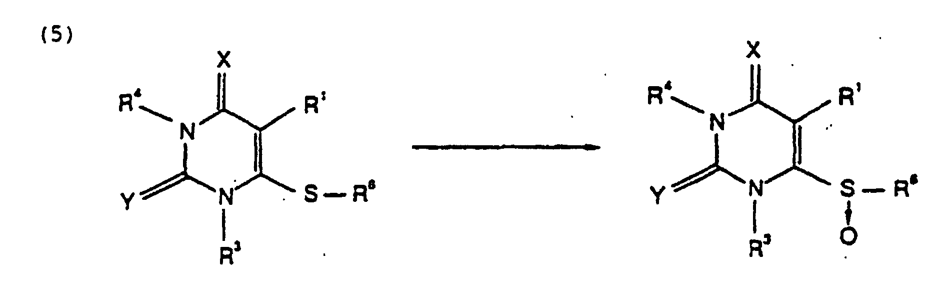 wherein R⁶ represents an aryl, alkyl or cycloalkyl group and the other symbols have the same meanings as defined above.

The compounds having phenyl sulfoxide group can be converted into corresponding compounds having a substituted arylthio or aryloxy group by reacting with sodium arylthiolate or sodium aryloxide having various substituents on the benzene ring in an organic solvent such as tetrahydrofuran, alcohol, dimethylformamide and acetonitrile at an appropriate temperature of from room temperature to 100°C in accordance with the reaction formula (6) below: 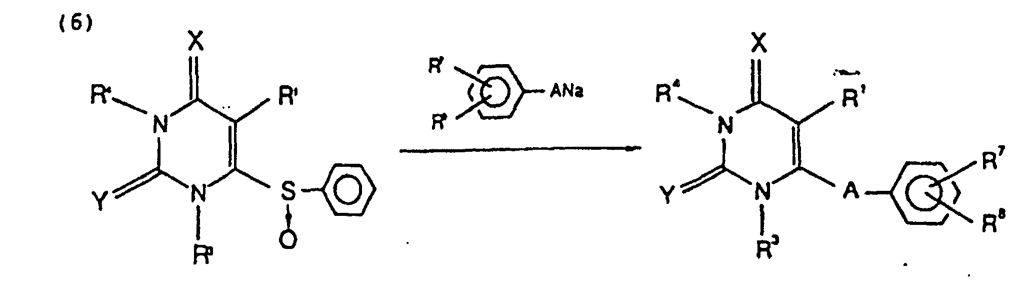 wherein A represents a sulfur or oxygen atom, R⁷ and R⁸ independently represent a halogen atom such as chlorine, bromine, fluorine and iodine, C₁ to C₅ alkyl group such as methyl, ethyl, propyl and butyl, C₁ to C₅ halogenated alkyl group such as trichloromethyl, C₁ to C₅ alkoxy group such as methoxy, ethoxy, propoxy and butoxy, hydroxyl group, nitro group, amino group, cyano group and C₂ to C₇ acyl group such as acetyl, and the other symbols have the same meanings as defined above.

The present compounds may be also prepared in accordance with, for example, the reaction formula (7) or (8) below: 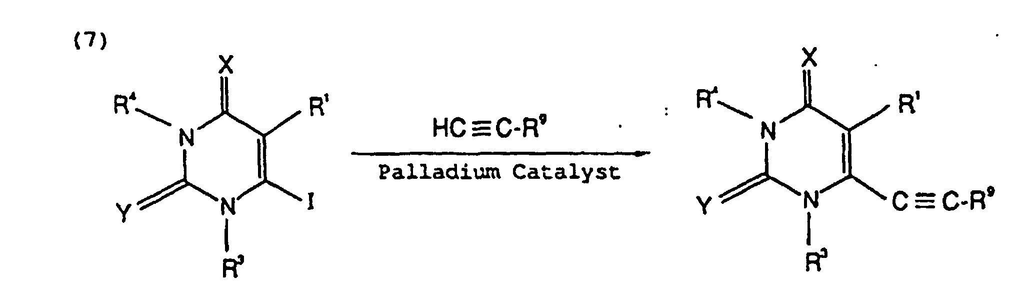 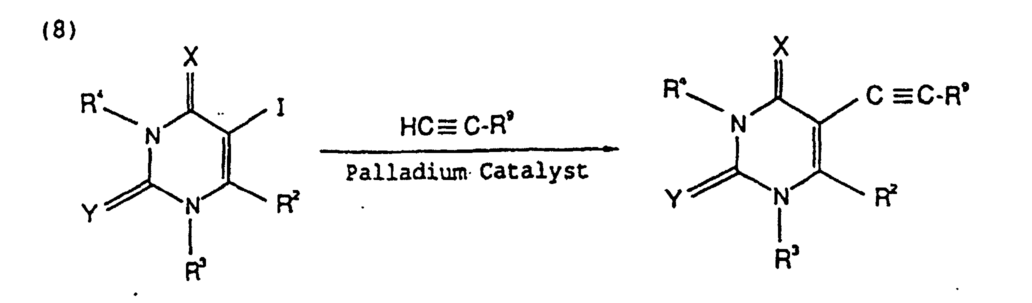 wherein R⁹ represents C₁ to C₅ alkyl group such as methyl and ethyl, C₆ to C₁₂ aryl group such as phenyl and toluyl, a protective group such as silyl group or the like, and the other symbols have the same meanings as defined above.

The reactions of the formulae (7) and (8) can be carried out in an amine solvent such as diethylamine and triethylamine in the presence of a palladium catalyst at an appropriate temperature of from room temperature to 70°C. The reactions may be carried out more homogeneously by adding another solvent such as acetonitrile. As the catalyst, a palladium catalyst of bis (triphenylphosphine)palladium dichloride, tetrakis(triphenylphosphine)palladium(0) and bis(diphenylphosphino)ethanepalladium dichloride can be used in combination with cuprous iodide.

The present compounds can be prepared also in accordance with the reaction formula (9) or (10) below, and the reactions may be carried out in the same manner as the reactions of the formulae (7) and (8) except that an olefin derivative of H₂C=CH-R₁₀ wherein R₁₀ represents C₂ to C₆ alkoxycarbonyl, nitrile, carbamoyl group and the like is used instead of the acetylene derivative in the reactions of the formulae (7) and (8) : 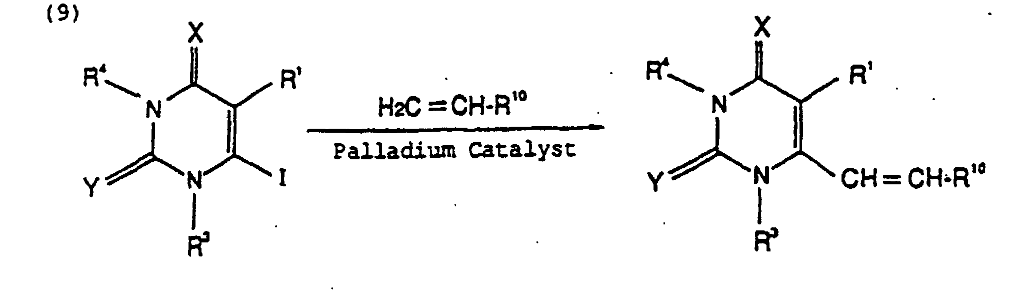 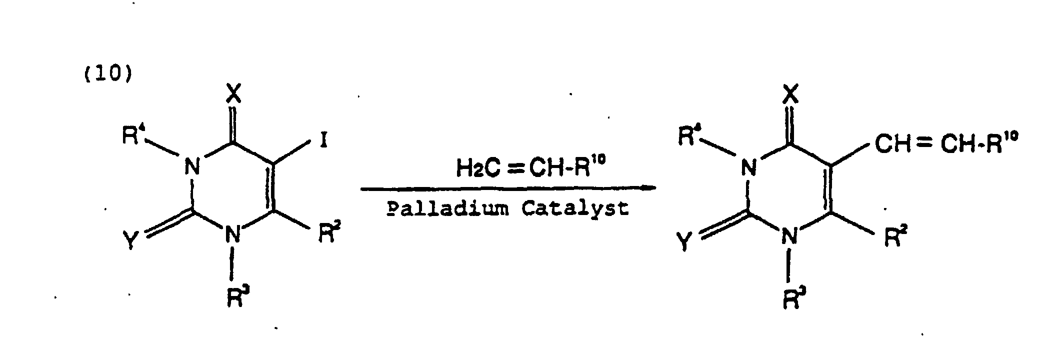 wherein the symbols have the same meanings as defined above.

The palladium catalyst may be the same as in the reaction of the formulae (7) and (8).

The compounds according to the invention can be prepared also in accordance with the reaction formula (11) below: 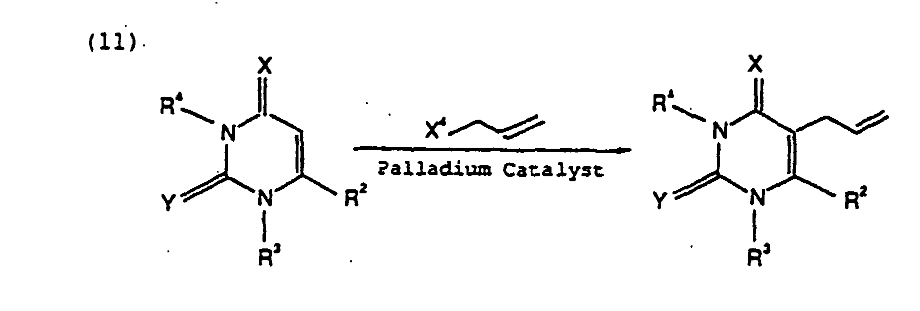 wherein X⁴ represents a halogen atom such as chlorine, bromine and iodine, and the other symbols have the same meaning as defined above.

The compounds according to the invention can be prepared also in accordance with the reaction formula (12) or (13) below: 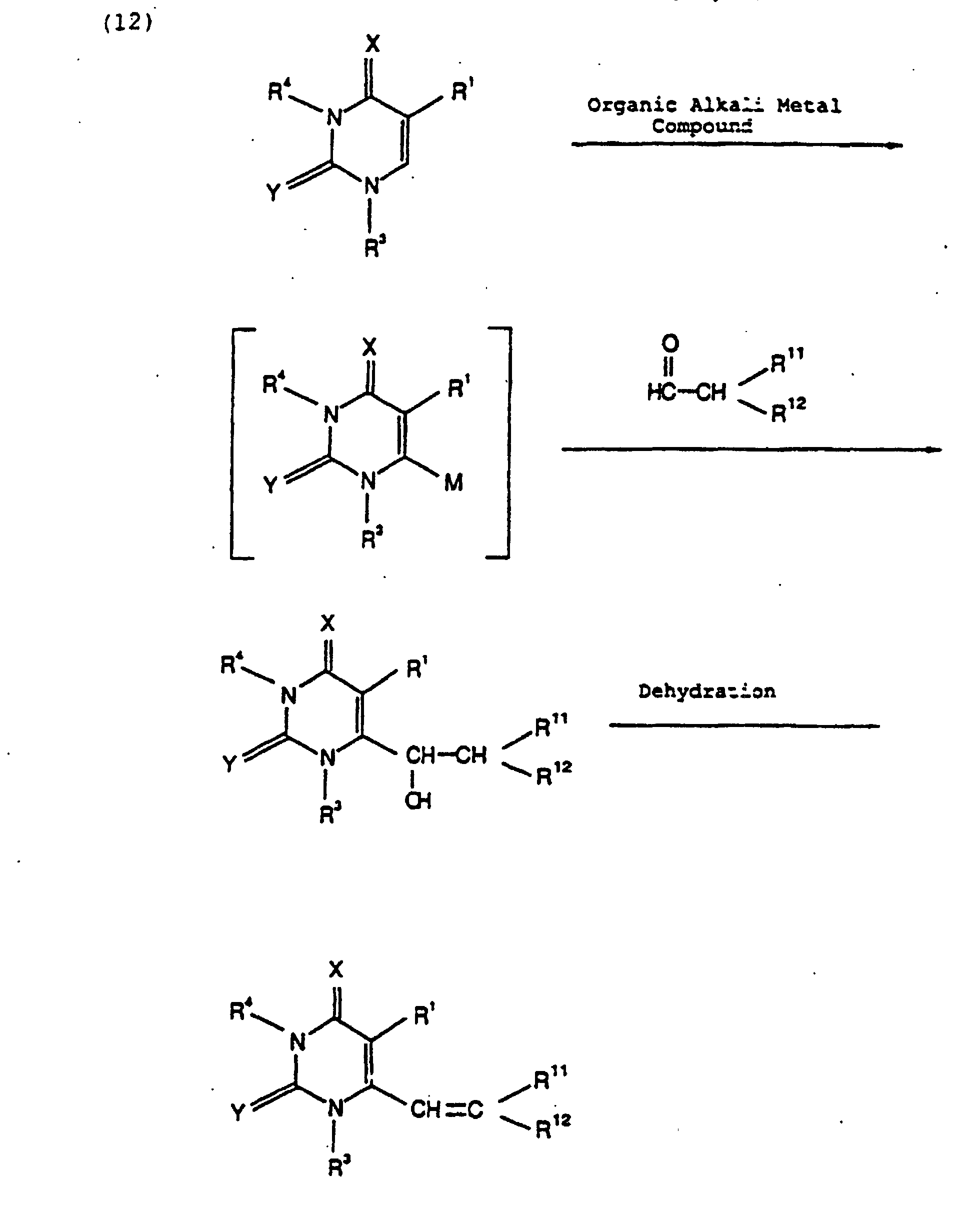 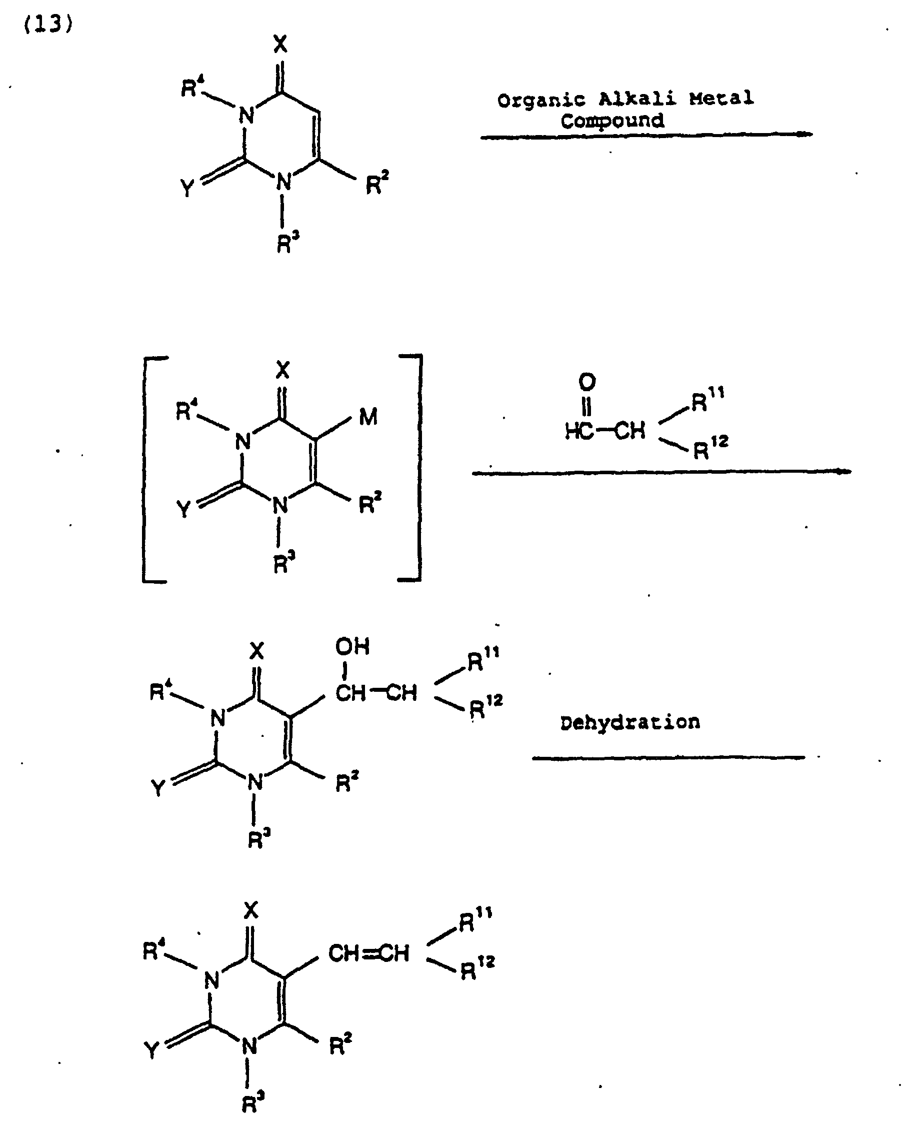 wherein the symbols have the same meanings as defined hereinbefore.

In the reactions of the formulae (12) and (13), intermediate compounds are prepared in accordance with the reaction formulae (1) and (2) as described hereinbefore except that a compound of OHC-CH(R¹¹)(R¹²) wherein R¹¹ and R¹² independently represent a hydrogen atom, C₁ to C₃ alkyl group such as methyl, ethyl and propyl or C₆ to C₁₂ aryl group such as phenyl is used instead of the compounds R¹X² and R²X¹, and then the intermediate compounds are dehydrated by a dehydrating agent such as mesyl chloride, tosyl chloride and thionyl chloride to produce the compounds according to the invention having an alkenyl group.

By hydrogenation, the alkynyl group of the compounds produced in the reactions of the formula (7) or (8) can be converted into the corresponding alkenyl or alkyl group and the alkenyl group of the compound produced in any one of the reactions formulae (9) to (13) can be converted into the corresponding alkyl group. For the reduction of alkynyl group into alkenyl group, the hydrogenation may be carried out at an appropriate temperature of from room temperature to 80°C under hydrogen atmosphere in the presence of a catalyst such as palladium/barium sulfate, palladium/calcium carbonate, palladium/calcium carbonate/lead acetate and palladium/barium sulfate/quinoline in'a solvent such as alcohol and acetic acid. For the reduction of alkenyl or alkynyl group into alkyl group, the hydrogenation may be carried out by using a catalyst such as palladium/carbon and palladium hydroxide under the same conditions as used for producing the alkenyl group.

The 6-benzyl substituted derivatives of the invention may be prepared in accordance with the reaction formula (14) below: 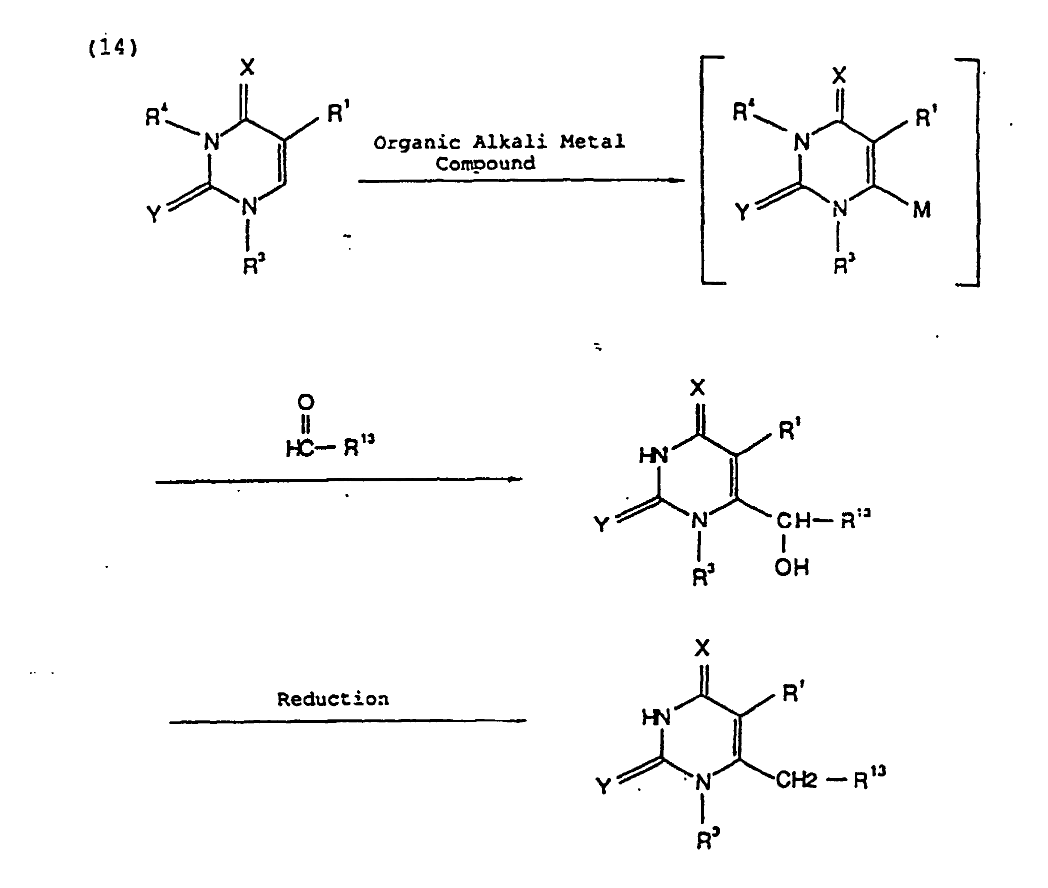 wherein the symbols have the same meanings as defined hereinbefore.

In the reactions of the formula (14), intermediate compounds are prepared in the same way as the reactions of the formula (1) using OHC-R¹³ where R¹³ represents an optionally substituted C₆ to C₁₂ aryl group such as phenyl instead of R¹X², and the intermediate compounds are reduced by a suitable reducing agent to convert the hydroxyl group into a hydrogen atom. The reduction can be carried out by using hydrogen gas in the presence of palladium/carbon or palladium hydroxide.

The 6-substituted acyclouridine or acyclothymidine derivatives obtained in the above-described reactions can be converted into 4-thio derivatives by heating them with 2,4-bis(4-methoxyphenyl)-1,3-dithia-2,4-diphosphetane-2,4-disulfide in a solvent such as toluene and xylene in accordance with the reaction formula (15) below: 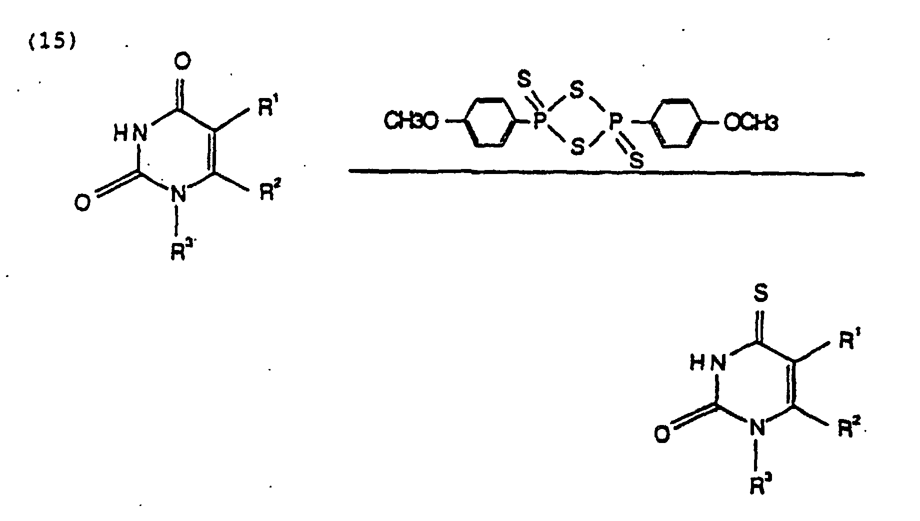 wherein the symbols have the same meanings as defined hereinbefore.

The 4-thio derivatives can be also prepared by preparing corresponding 4-chloro derivatives by chlorination of corresponding uridine or thymidine derivatives by a chlorinating agent such as phosphorous pentachloride or phosphorous oxychloride and reacting the 4-chloro derivatives with sodium bisulfide.

Further, 4-amino derivatives can be prepared by reacting the acyclouridine or thymidine derivatives with 1-(2-mesitylenesulfonyl)-3-nitro-1,2,4-triazole in the presence of diphenylphosphoric acid in a solvent such as pyridine to produce corresponding 4-(3-nitro-1,2,4-triazole) derivatives which are converted to the corresponding 4-amino derivatives by aqueous ammonia at an appropriate temperature of from room temperature to 100°C in accordance with the reaction formula (16) below: 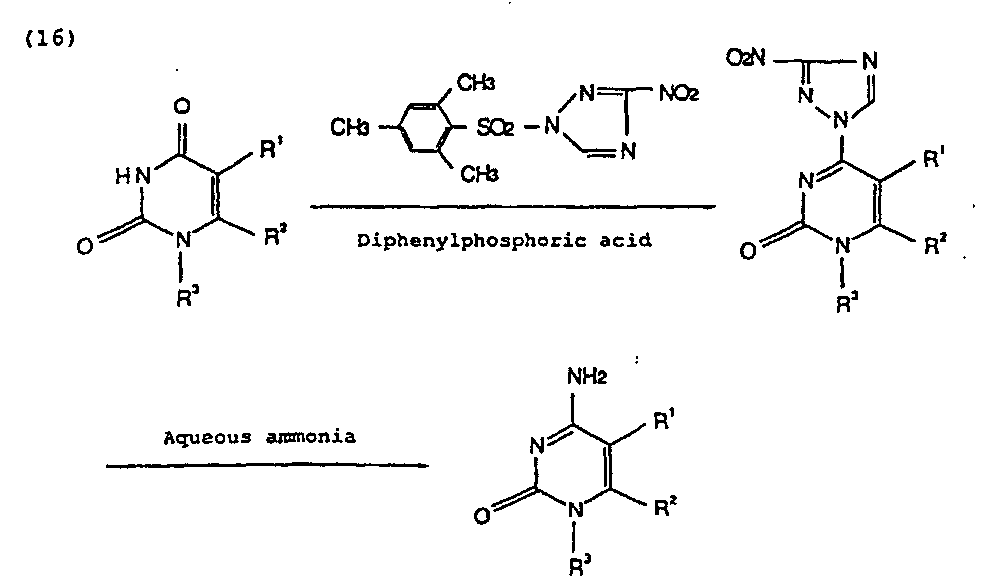 wherein the symbols have the same meanings as defined hereinbefore.

The above-obtained compounds where R⁴ is a hydrogen atom may be converted into corresponding compounds having R⁴ other than the hydrogen atom in accordance with the reaction formula (17) below: 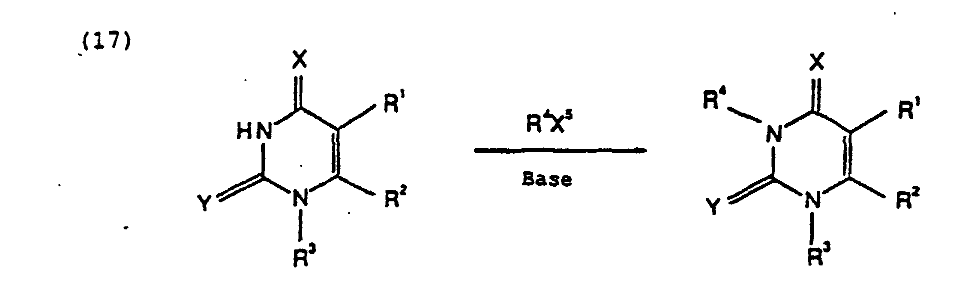 wherein X5 represents a halogen atom such as chlorine, bromine and iodine or sulfonyloxy group such as toluenesulfonyloxy and mesyloxy, and the other symbols have the same meanings as defined hereinbefore.

The reaction of the formula (17) may be carried out in a suitable solvent such as tetrahydrofuran, acetonitrile, dimethylformamide, pyridine and alcohol in the presence of a base in an amount of 1 to 2 times of the starting compound at a suitable temperature from room temperature to the boiling point of the solvent. Examples of the base include sodium alkoxide, potassium alkoxide, potassium carbonate, sodium carbonate, sodium hydride and the like.

The compounds of the invention where R⁵ is a hydroxyl group, which are obtained in any of the reactions of formula (1) to (17), may be converted into corresponding compounds having a substituted hydroxyl group in accordance with any of the reaction formulae (18) to (21) below: 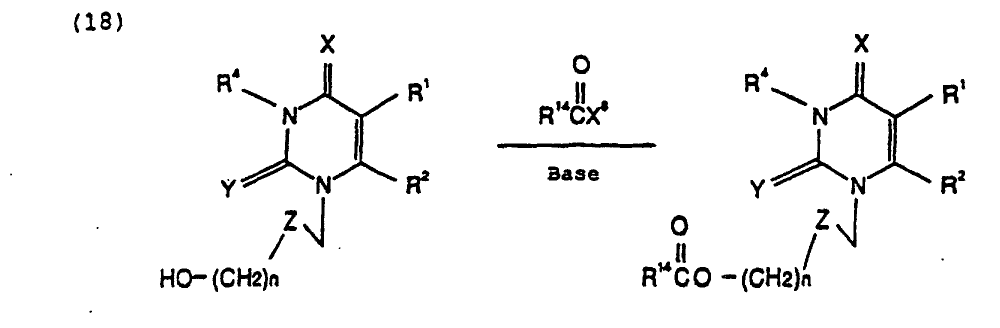 wherein R¹⁴ represents an optionally branched alkyl group, optionally substituted aryl group or heterocyclic group, X⁶ represents a halogen atom such as chlcrine, bromine and iodine or -OCOR¹⁴, and the other symbols have the same. meanings as defined hereinbefore.

The reaction of the formula (18) may be carried out in a suitable solvent such as tetrahydrofuran, acetonitrile, dimethylformamide, pyridine, dichloromethane and chloroform in the presence of a base in an amount of 1 to 2 times of the starting compound at a suitable temperature from room temperature to the boiling point of the solvent. Examples of the base include triethylamine, pyridine, imidazole, sodium carbonate, potassium carbonate, sodium hydroxide and the like. 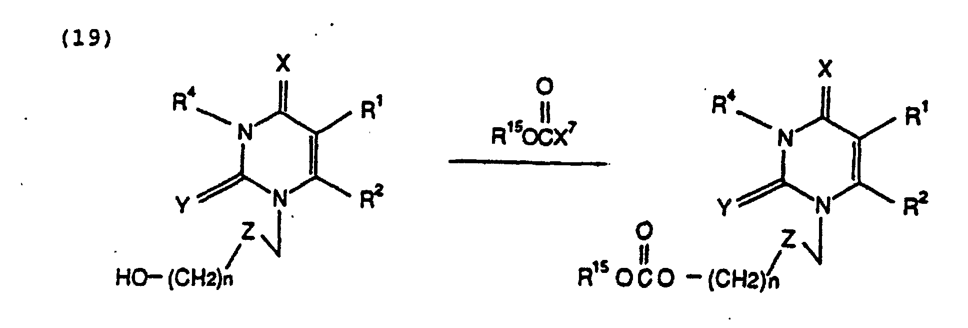 wherein R¹⁵ represents an optionally branched C₁ to C₁₀ alkyl group or C₇ to C₁₁ aralkyl group, X⁷ represents a halogen atom such as chlorine, bromine and iodine or -OCOOR¹⁵, and the other symbols have the same meanings as defined hereinbefore.

The reaction of the formula (19) may be carried out in a suitable solvent such as tetrahydrofuran, acetonitrile, dimethylformamide, pyridine, dichloromethane and chloroform in the presence of a base in an amount of 1 to 2 times of the starting compound at a suitable temperature from room temperature to the boiling point of the solvent. Examples of the base include triethylamine, pyridine, imidazole, sodium carbonate, potassium carbonate, sodium hydroxide and the like. 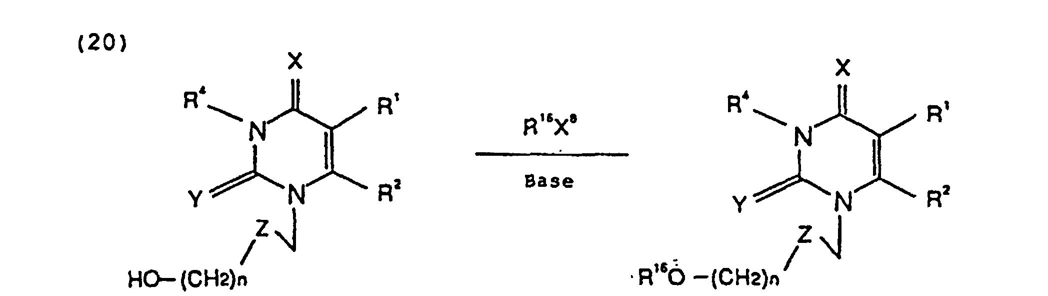 wherein R¹⁶ represents an optionally branched C₁ to C₁₀ alkyl group or C₇ to C₁₃ aralkyl group, R⁸ represents a halogen atom such as chlorine, bromine and iodine or sulfonyloxy group such as toluenesulfonyloxy and mesyloxy, and the other symbols have the same meanings as defined hereinbefore.

The reaction of the formula (20) may be carried out in a suitable solvent such as tetrahydrofuran, acetonitrile, dimethylformamide, pyridine, dichloromethane and chloroform in the presence of a base in an amount of 1 to 2 times of the starting compound at a suitable temperature from room temperature to the boiling point of the solvent. Examples of the base include triethylamine, pyridine, imidazole, sodium carbonate, potassium carbonate, sodium hydroxide and the like. 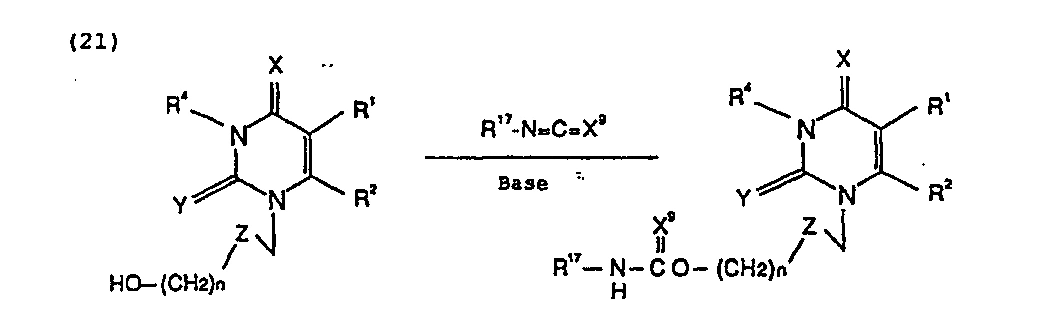 wherein R¹⁷ represents an optionally branched C₁ to C₁₀ alkyl group or C₆ to C₁₂ aryl group, X⁹ represents an oxygen or sulfur atom, and the other symbols have the same meanings as defined hereinbefore.

The reaction of formula (21) may be carried out in an appropriate solvent such as tetrahydrofuran, acetonitrile, dimethylformamide, pyridine, dichloromethane and chloroform at an appropriate temperature of from room temperature to the boiling point of the solvent.

The compounds of the present invention obtained as described hereinbefore and represented by the formula I or I' may be separated and purified by any of the conventional methods for the separation and purification of nucleosides, for example, recrystallization, adsorption chromatography, ion exchange chromatography and the like.

The compounds of the invention represented by the formula I may be converted into a pharmaceutically acceptable salt thereof by a conventional method. Such salt may be, for example, an alkali metal salt such as sodium or potassium salt, alkaline earth metal salt such as magnesium salt, ammonium salt or alkylammonium salt such as methylammonium, dimethylammonium, trimethylammonium, tetramethylammonium salt or the like.

The compounds according to the invention can be administered to human beings via any route, oral, rectal, parenteral or local for the prevention or treatment of the infection of viruses such as retrovirus. The administration dose of the compounds according to the invention may be determined according to age, physical condition, body weight and the like of a patient to be treated; however, a suitable daily does of the compounds is 1 to 100 mg/(body weight)kg, preferably 5 to 50 mg/(body weight)kg and it is administered in one to several times.

The compound of the invention is generally prepared in a pharmaceutical composition with a suitable carrier, excipient and other additives. Either a liquid carrier or solid carrier may be suitably used for the present antiviral agent.

Examples of the solid carrier are lactose, kaolin, sucrose, crystalline cellulose, corn starch, talc, agar, pectin, stearic acid, magnesium stearate, lecithin, sodium chloride and the like.

Examples of the liquid carrier are glycerin, peanut oil, polyvinyl pyrrolidone, olive oil, ethanol, benzyl alcohol, propylene glycol, water and the like.

The present antiviral agent may be made in various forms. For example, it may be in the form of a tablet, powder, granule, capsule, suppository, troche or the like when a solid carrier is used, and it may be also in the form of syrup, emulsion, soft gelatin capsule, cream, gel, paste, spray, injection solution, or the like when a liquid carrier is used.

The novel 6-substituted acyclopyrimidine nucleoside derivatives according to the present invention have an effective antiviral activity against viruses such as retrovirus and have a relatively low toxicity against the host cell, hence the derivatives of the invention are extremely useful as an active ingredient of antiviral agent.

### EXAMPLE

The present invention will be further illustrated hereinafter by way of examples, but these examples do not limit the invention and many variations and modifications can be made without departing from the scope of the present invention.

The numbers of the compounds used in the description of the examples correspond to those used in Table 1.

The starting compounds used in the examples such as
1-[(2-hydroxyethoxy)methyl]-6-phenylthio-2-thiothymine,
1-[(2-hydroxyethoxy]methyl]-6-phenylthiothymine,
1-[(2-hydroxyethoxy)methyl]-6-(m,m'-dimethylphenylthio)-thymine,
1-[(2-hydroxyethoxy)methyl]-6-(m,m'-dimethylphenylthio)-2-thiothymine,
1-[(2-hydroxyethoxy)methyl]-6-(m,m'-dichlorophenylthio)-thymine,
1-[(2-hydroxyethoxy)methyl]-6-benzylthymine,
1-[(2-hydroxyethoxy)methyl]-6-cyclohexylthiothymine,
1-[(2-hydroxyethoxy)methyl]-6-m-tolylthiothymine
and the like were produced according to the methods described in the examples of PCT International Application WO89/09213.

### EXAMPLE 1

### Preparation of 1-[(2-acetoxyethoxy)methyl]-6-phenylthio-2-thiothymine (compound No.1)

To 2 ml of pyridine, 0.31 g (1.0 mmole) of 1-[(2-hydroxyethoxy)methyl]-6-phenylthio-2-thiothymine and 0.10 ml (1.1 mmole) of acetic anhydride were added under a flow of nitrogen, allowed to react for 2 hours at room temperature, concentrated to dryness under reduced pressure and crystallized from ethanol/water to obtain 0.62 g of the target compound (Yield: 88 %).

### EXAMPLES 2 - 6

Using the following compounds in place of 1-[(2-hydroxyethoxy)methyl]-6-phenylthio-2-thiothymine in Example 1, Compounds Nos.2 to 6 in Table 1 were obtained in the same manner as Example 1:
1-[(2-hydroxyethoxy)methyl]-6-phenylthiothymine,
1-[(2-hydroxyethoxy)methyl]-6-(m,m'-dimethylphenylthio)-thymine,
1-[(2-hydroxyethoxy)methyl]-6-(m,m'-dimethylphenylthio)-2-thiothymine,
1-[(2-hydroxyethoxy)methyl]-6-(m,m'-dichlorophenylthio)-thymine, and
1-[(2-hydroxyethoxy)methyl]-6-benzylthymine.

### EXAMPLES 7 - 13

Compounds Nos.7 to 13 in Table 1 were prepared in the same manner as Example 2 by using ethyl formate, i-butyryl chloride, pivaloyl chloride, decanoyl chloride, cyclohexanecarbonyl chloride, benzoyl chloride or nicotinyl chloride respectively in place of acetic anhydride in Example 2, .

### EXAMPLE 14

Compound No.14 was prepared in the same manner as Example 2 by using t-butoxycarbonyl chloride in place of acetic anhydride in Example 2.

### EXAMPLE 15

Compound No.15 was obtained in the same manner as Example 2 by using 1-[(2-hydroxyethoxy)methyl]-6-cyclohexylthiothymine and benzyloxycarbonyl chloride in place of 1-[(2-hydroxyethoxy)-methyl]-6-phenylthiothymine and acetic anhydride in Example 2 respectively.

### EXAMPLE 16

### Preparation of 1-[(2-phenylcarbamoyloxyethoxy)methyl]-6-m-tolylthiothymine (Compound No.16)

To 2 ml of pyridine, 0.32 g (1.0 mmole) of 1-[(2-hydroxyethoxy)methyl]-6-m-tolylthiothymine and 0.12 ml (1.1 mmole) of phenyl isocyanate were added under a flow of nitrogen, allowed to react for 18 hours at room temperature. The reaction mixture was concentrated to dryness under reduced pressure and crystallized from acetone/water to obtain 0.24 g of the target compound (Yield: 54 %).

### EXAMPLE 17 and 18

Compounds Nos.17 and 18 were prepared in the same manner as Example 16 by using ethyl isocyanate or phenyl thioisocyanate respectively in place of phenyl isocyanate.

### EXAMPLE 19

### Preparation of 1-[(2-benzyloxyethoxy)methyl]-6-phenylthiothymine (Compound No.19)

To 4 ml of tetrahydrofuran, 0.17 g (4.2 mmol) of sodium hydride was added under a nitrogen flow, and stirred to form a suspension. To this suspension, a solution of 0.62 g (2.0 mmole) of 1-[(2-hydroxyethoxy)methyl]-6-phenylthiothymine in 2 ml of tetrahydrofuran was added slowly to react for 45 minutes at room temperature. The resultant was added with 0.24 ml (2.0 mmol) of benzyl bromide and 7.4 g (20 µmol) of tetrabutylammonium iodide and allowed to react for 15 hours. The reaction mixture was neutralized with acetic acid and distributed between chloroform and saturated aqueous solution of sodium hydrogencarbonate, and the chloroform layer was concentrated to dryness under reduced pressure. The residue was dissolved in a small amount of chloroform, adsorbed on a silica gel column and eluted with 1 % methanol/chloroform. The eluate was concentrated and crystallized from diethyl ether/hexane to obtain 0.64 g of the target compound (Yield: 80 %).

### EXAMPLES 20 - 21

Compounds Nos.20 and 21 were prepared in the same manner as Example 19 by using methyl bromide or bromopentane respectively in place of benzyl bromide.

### EXAMPLE 22

### Preparation of 1-(methoxymethyl)-6-phenylthiothymine (Compound No.22)

To 250 ml of methylene chloride, 25 g (0.20 mol) of thymine and 109 ml (0.44 mol) of bistrimethylsilylacetamide were added under a nitrogen flow, and stirred for 2.5 hours at room temperature. To this mixture, 24 g (0.30 mole) of chloromethyl methyl ether and 0.59 g (1.6 mmol) of tetrabutylammonium iodide were added and heated under reflux for 1.5 hours. Then, the reaction mixture was added with 400 ml of methanol and 100 ml of water slowly and concentrated under reduced pressure. The residue was crystallized from ethyl acetate to obtain 1-(methoxymethyl)-thymine. Then, 119 ml of lithium diisopropylamide (0.25 mol) solution in tetrahydrofuran (2.1 M) was added to 335 ml of tetrahydrofuran under a nitrogen flow at -70°C, to which a suspension of 17.0 g (0.10 ml) 1-(methoxymethyl)thymine in 107 ml of tetrahydrofuran was added dropwise over 30 minutes. After stirring for 2.5 hours at -70°C, the reaction mixture was added with a solution of 43.6 g of diphenyl disulfide in 49 ml of tetrahydrofuran dropwise over 20 minutes and allowed to react for 20 minutes. The reaction mixture was added with 35 ml of acetic acid, brought to room temperature and then added with 1 l of ethyl acetate. The mixture was washed with water (100 ml x 5) and saturated solution of sodium hydrogencarbonate (twice), dried on magnesium sulfate and concentrated under reduced pressure. The residue was crystallized from ethanol to obtain 20 g of the target compound (Yield: 73 %).

### EXAMPLES 23 - 26

Compounds Nos.23 to 26 were prepared in the same manner as Example 22 by using 1-(ethoxymethyl)thymine, 1-[(2-azidoethoxy)-methyl]thymine, 1-[(2-fluoroethoxy)methyl]thymine or 1-[(2-chloroethoxy)methyl]thymine respectively in place of 1-(methoxymethyl)thymine.

### EXAMPLE 27

### Preparation of 6-phenylthiothymine (Compound No.27)

To 100 ml of concentrated hydrochloric acid, 17.2 g (62 mmole) of 1-(methoxymethyl)-6-phenylthiothymine was added and allowed to react for 2 hours at 80 °C. The reaction mixture was concentrated under reduced pressure and crystallized from ethanol to obtain 3.8 g of the target compound (Yield: 26 %).

### EXAMPLE 28

### Preparation of 1-methyl-6-phenylthiothymine (Compound No.28)

To 1 ml of dimethyl sulfoxide, 20 mg (85 µmol) of 6-phenylthiothymine, 2.5 µl (40 µmol) of methyl iodide and 12 mg (85 µmol) of potassium carbonate were added and allowed to react for 6 hours at 80°C. The reaction mixture was concentrated under reduced pressure and adsorbed on a silica gel column and eluted with 1 % methanol/chloroform. The eluate was concentrated and crystallized from diisopropyl ether to obtain 5.0 mg of the target compound (Yield: 51 %).

### EXAMPLES 29 - 30

Compounds Nos.29 and 30 were prepared in the same manner as Example 28 by using ethyl tosylate or n-butyl iodide respectively in place of methyl iodide.

### EXAMPLE 31

### Preparation of 1-(methylthiomethyl)-6-phenylthiothymine (Compound No. 31)

To 4 ml of dimethylformamide, 0.17 ml (2.0 mmol) of chloromethylmethylsulfide, 0.47 g (2.0 mmol) of 6-phenylthiothymine, 0.56 ml (2.0 mmol) of triethylamine were added and allowed to react for 22 hours at room temperature. The reaction mixture was concentrated under reduced pressure and the residue was adsorbed on a silica gel column and eluted with chloroform. The eluate was concentrated and crystallized from ethyl acetate to obtain 45 mg of the target compound (Yield: 8 %).

### EXAMPLE 32

### Preparation of 1-ethoxymethyl-5-ethyl-6-phenylthiouracil (Compound No. 215)

To 100 ml of methylene chloride, 5.1 g (40 mmol) of 5-ethyluracil and 22 ml (0.88 mmol) of bistrimethylsilylacetamide were added under a nitrogen atmosphere and stirred for 40 minutes at room temperature. To this mixture, 4.1 ml (88 mmole) of chloromethyl ethyl ether and 0.15 g (0.4 mmol) of tetrabutylammonium iodide were added and heated under reflux for 15 hours. Then, the reaction mixture was poured carefully into 50 ml of saturated aqueous solution of sodium hydrogencarbonate and filtered through Celite. The organic layer was washed with water, dried on magnesium sulfate and concentrated under reduced pressure. The residue was crystallized from ethyl acetate to obtain 6.4 g of 1-ethoxymethyl-5-ethyluracil (Yield: 81%).

Then, 2.2 ml of lithium diisopropylamice (4.4 mmol) solution in tetrahydrofuran (2.1 M) was added to 6 ml of tetrahydrofuran under a nitrogen atmosphere at -70°C, to which a solution of 0.40 g (2.0 mmol) of 1-ethoxymethyl-5-ethyluracil in 3 ml of tetrahydrofuran was added dropwise over 15 minutes. After stirring for 1 hour at -70°C, the reaction mixture was added with a solution of 0.57 g of diphenyl disulfide in 2 ml of tetrahydrofuran dropwise over 10 minutes and allowed to react for 30 minutes. The reaction mixture was added with 1 ml of acetic acid, brought to room temperature and then added with 30 ml of ethyl acetate. The mixture was washed with water (3 ml x 5) and saturated aqueous solution of sodium hydrogencarbonate (twice), dried on magnesium sulfate and concentrated under reduced pressure. The residue was purified by silica gel chromatography (ethyl acetate/hexane = 3:17) and crystallized from ethyl acetate to obtain 0.61 g of 1-ethoxymethyl-5-ethyl-6-phenylthiouracil (Yield: 32 %).

### EXAMPLE 33

Compound No.312 was obtained in the same way as Example 32 by using 3,3',5,5'-tetramethylphenyl disulfide in place of diphenyl disulfide.

### EXAMPLE 34

### Preparation of 1-ethoxymethyl-5-ethyl-6-phenylthio-2-thiouracil (Compound No.313)

To 100 ml of methylene chloride, 5.1 g (40 mmol) of 2-thiouracil and 22 ml (88 mmol) of bistrimethylsilylacetamide were added under a nitrogen atmosphere, and stirred for 40 minutes at room temperature. To this mixture, 8.2 ml (88 mmole) of chloromethyl ethyl ether and 0.15 g (0.4 mmol) of tetrabutylammonium iodide were added and heated under reflux for 15 hours. Then, the reaction mixture was poured carefully into 50 ml of saturated aqueous solution of sodium hydrogencarbonate and filtered through Celite. The organic layer was washed with water, dried on magnesium sulfate and concentrated under reduced pressure. The residue was crystallized from ethyl acetate to obtain 1.1 g of 1-ethoxymethyl-2-thiouracil (Yield: 15 %).

Then, 3.3 ml of lithium diisopropylamide solution in tetrahydrofuran (2.1 M) was added to 9 ml of tetrahydrofuran under a nitrogen atmosphere at -70°C, to which a solution of 0.56 g (3.0 mmol) of 1-ethoxymethyl-2-thiouracil in 3 ml of tetrahydrofuran was added dropwise over 15 minutes. After stirring for 1 hour at -70°C, the reaction mixture was added with a solution of 0.85 g (3.9 mmol) of diphenyl disulfide in 1 ml of tetrahydrofuran dropwise over 10 minutes and allowed to react for 20 minutes. The reaction mixture was added with 1 ml of acetic acid, brought to room temperature and then added with 30 ml of ethyl acetate. The mixture was washed with water (3 ml x 5) and saturated aqueous solution of sodium hydrogencarbonate (twice), dried on magnesium sulfate and concentrated under reduced pressure. The residue was purified by silica gel chromatography (ethyl acetate/hexane = 3:17), crystallized from ethyl acetate to obtain 0.64 g of 1-ethoxymethyl-6-phenylthio-2-thiouracil (Yield: 73 %).

Then, 2.1 ml of 1.6 M butyl lithium (3.4 mmol) solution in hexane was added to a solution of 0.57 ml (3.4 mmol) 2,2,6,6-tetramethylpyperidine in 8 ml of tetrahydrofuran under a nitrogen atmosphere at -70°C, warmed to -50°C, and stirred for 20 minutes. After cooling to -70°C again, the mixture was added with a solution of 0.44 g (1.5 mmol) of 1-ethoxymethy-6-phenylthio-2-thiouracil in 4 ml tetrahydrofuran dropwise over 15 minutes, stirred for an hour, added with 1.2 ml (15 mmol) ethyl iodide and stirred for 19 hours. Then, the mixture was added with 1 ml acetic acid, brought to room temperature, added with 30 ml ethyl acetate, washed with water and saturated aqueous solution of sodium chloride, dried on magnesium sulfate and concentrated under reduced pressure. The residue was purified by silica gel chromatography (ethyl acetate/hexane = 3:17) and crystallized from ethyl acetate to obtain 96 mg of the title compound (Yield: 20 %).

### EXAMPLE 35

Compound No.314 was prepared in the same way as Example 34 by using 3,3',5,5'-tetramethyldiphenyl disulfide in place of diphenyl disulfide.

### EXAMPLE 36

Compound No.315 was prepared in the same way as Example 32 by using benzyl chloromethyl ether in place of chloromethyl ethyl ether.

### EXAMPLE 37

Compound No.316 was prepared in the same way as Example 32 by using benzyl chloromethyl ether and 3,3',5,5'-tetramethyldiphenyl disulfide respectively in place of chloromethyl ethyl ether and diphenyl disulfide.

### EXAMPLE 38

Compound No 317 was prepared in the same way as Example 32 by using thymine and benzyl chloromethyl ether in place of 5-ethyluracil and chloromethyl ethyl ether.

### EXAMPLE 39

Compound No.38 was prepared in the same way as Example 2. by using chloromethyl propyl ether in place of chloromethyl methyl ether.

### EXAMPLE 40

Compound No.440 was prepared in the same way as Example 22 by using butyl chloromethyl ether in place of chloromethyl methyl ether.

### EXAMPLE 41

Compound No.320 was prepared in the same way as Example 32 by using 3,3',5,5'-tetrachlorodiphenyl disulfide in place of diphenyl disulfide.

### EXAMPLE 42

Compound No.321 was prepared in the same way as Example 32 by using 5-ethyl-2-thiouracil and 3,3',5,5'-tetrachlorodiphenyl disulfide respectively in place of 5-ethyluracil and diphenyl disulfide.

### EXAMPLE 43

Compound No. 451 was prepared in the same way as Example 32 by using 5-isopropyluracil in place of 5-ethyluracil.

### EXAMPLE 44

Compound No.452 was prepared in the same way as Example 32 by using 5-isopropyl-2-thiouracil in place of 5-ethyluracil.

### EXAMPLE 45

Compound No.527 was prepared in the same way as Example 32 by using 5-cyclopropyluracil in place of 5-ethyluracil.

### EXAMPLE 46

Compound No.528 was prepared in the same way as Example 32 by using 5-cyclopropyl-2-thiouracil in place of 5-ethyluracil.

### EXAMPLE 47

Compound No.628 was prepared in the same way as Example 32 by using chloromethyl isopropyl ether in place of chloromethyl ethyl ether.

### EXAMPLE 48

Compound No.629 was prepared in the same way as Example 32 by using 5-ethyl-2-thiouracil and chloromethyl isopropyl ether respectively in place of 5-ethyluracil and chloromethyl ethyl ether.

### EXAMPLE 49

Compound No.638 was prepared in the same way as Example 32 by using chloromethyl cyclohexyl ether in place of chloromethyl ethyl ether.

### EXAMPLE 50

Compound No.639 was prepared in the same way as Example 32 by using 5-ethyl-2-thiouracil and chloromethyl cyclohexyl ether respectively in place of 5-ethyluracil and chloromethyl ethyl ether.

### EXAMPLE 51

Compound No.642 was prepared in the same way as Example 32 by using chloromethyl cyclohexylmethyl ether in place of chloromethyl ethyl ether.

### EXAMPLE 52

Compound No.643 was prepared in the same way as Example 32 by using 5-ethyl-2-thiouracil and chloromethyl cyclohexylmethyl ether respectively in place of 5-ethyluracil and chloromethyl ethyl ether.

### EXAMPLE 53

Compound No.465 was prepared in the same way as Example 32 by using 5-iospropyluracil and benzyl chloromethyl ether respectively in place of 5-ethyluracil and chloromethyl ethyl ether.

### EXAMPLE 54

Compound No.466 was prepared in the same way as Example 32 by using 5-isopropyl-2-thiouracil and benzyl chloromethyl ether respectively in place of 5-ethyluracil and chloromethyl ethyl ether.

### EXAMPLE 55

Compound No.701 was prepared in the same way as Example 32 by using chloromethyl phenetyl ether in place of chloromethyl ethyl ether.

### EXAMPLE 56

Compound No.702 was prepared in the same way as Example 32 by using 5-ethyl-2-thiouracil and chloromethyl phenetyl ether respectively in place of 5-ethyluracil and chloromethyl ethyl ether.

### EXAMPLE 57

Compound No.327 was prepared in the same way as Example 32 by using 5-ethyl-2-thiouracil and chloromethyl 4-methylbenzyl ether respectively in place of 5-ethyluracil and chloromethyl ethyl ether.

### EXAMPLE 58

Compound No.657 was prepared in the same way as Example 32 by using 5-ethyl-2-thiouracil and 4-chlorobenzyl chloromethyl ether respectively in place of 5-ethyluracil and chloromethyl ethyl ether.

### EXAMPLE 59

### Preparation of 6-benzyl-1-ethoxymethyl-5-ethyluracil (Compound No.427)

To 100 ml of methylene chloride, 5.1 g (40 mmol) of 5-ethyluracil and 22 ml (88 mmol) of bistrimethylsilylacetamide were added under a nitrogen atmosphere and stirred for 40 minutes at room temperature. To this mixture, 4.1 ml (88 mmole) of chloromethyl ethyl ether and 0.15 g (0.4 mmol) of tetrabutylammonium iodide were added and heated under reflux for 15 hours. Then, the reaction mixture was poured into 50 ml of saturated sodium bicarbonate solution carefully and filtered through Celite. The organic layer was washed with water, dried on magnesium sulfate and concentrated under reduced pressure. The residue was crystallized from ethyl acetate to obtain 6.4 g of 1-ethoxymethyl-5-ethyluracil (Yield: 81 %).

Then, 2.2 ml (4.4 mmol) of lithium diisopropylamide solution in tetrahydrofuran (2.1 M) was added to 6 ml of tetrahydrofuran under a nitrogen atmosphere at -70°C, to which a solution of 0.40 g (2.0 mmol) of 1-ethoxymethyl-5-ethyluracil in 3 ml of tetrahydrofuran was added dropwise over 15 minutes. After stirring for 1 hour at -70°C, the reaction mixture was added with a solution of 0.27 g (2.6 mmol) of benzaldehyde in 2 ml of tetrahydrofuran dropwise over 10 minutes and allowed to react for 30 minutes. The reaction mixture was added with 1 ml of acetic acid, brought to room temperature and then added with 30 ml of ethyl acetate. The mixture was washed with water (3 ml x 5) and saturated aqueous solution of sodium hydrogencarbonate (twice), dried on magnesium sulfate and concentrated under reduced pressure.

The residue was dissolved in 10 ml of ethanol, added with 20 mg of 20 % palladium hydroxide/carbon and stirred under a hydrogen atmosphere for a day at 55°C. Then, after removing the catalyst by filtration, the reaction mixture was concentrated. The residue was crystallized from hexane to obtain 0.28 g of 6-benzyl-1-ethoxymethyl-5-ethyluracil (Yield: 85 %).

### EXAMPLE 60

Compound No.429 was prepared in the same way as Example 59 by using 3,5-dimethylbenzaldehyde in place of benzaldehyde.

### EXAMPLE 61

### Preparation of 1-butyl-5-ethyl-6-phenylthiouracil (Compound No.220)

To a solution of 5.6 g (40 mmol) of 5-ethyluracil in 60 ml of dimethylformamide, 5.5 g (40 mmol) of potassium carbonate and 2.3 ml (20 mmol) of n-iodobutane were added and stirred for 2 hours at 120°C. The reaction mixture was concentrated under reduced pressure and distributed between dichloromethane and aqueous solution of ammonium chloride, and the organic layer was concentrated under reduced pressure. The residue was adsorbed on a silica gel column and eluted with 30 % ethyl acetate/hexane to obtain 2.7 g of 1-butyl-5-ethyluracil (Yield: 69 %).

Then, a solution of 4.4 mmol of lithium diisopropylamide in 2.8 ml of tetrahydrofuran was added dropwise to a solution of 392 mg (2.0 mmol) 1-butyl-5-ethyluracil in 9 ml of tetrahydrofuran under a nitrogen atmosphere at -70°C and stirred for 70 minutes at -70°C and further 5 minutes at -25°C. The mixture was cooled to -70°C again, added with a solution of 567 mg (2.6 mmol) diphenyl disulfide in 3 ml of tetrahydrofuran, stirred for 20 minutes, added with 1 ml of acetic acid, brought to room temperature, washed with saturated aqueous solution of sodium chloride and concentrated under reduced pressure. The residue was adsorbed on a silica gel, eluted with 10 % ethyl acetate/hexane and crystallized from hexane to obtain 40 mg of 1-butyl-5-ethyl-6-phenylthiouracil (Yield: 7 %).

### EXAMPLE 62

Compound No.318 was prepared in the same way as Example 32 by using 5-ethyl-2-thiouracil and benzyl chloromethyl ether respectively in place of 5-ethyluracil and chloromethyl ethyl ether.

### EXAMPLE 63

Compound No.319 was prepared in the same way as Example 32 by using 5-ethyl-2-thiouracil, benzyl chloromethyl ether and 3,3',5,5'-tetramethyldiphenyl disulfide respectively in place of 5-ethyluracil, chloromethyl ethyl ether and diphenyl disulfide.

### EXAMPLE 64

### Preparation of 5-(2-(E)-bromovinyl)-1-(ethoxymethyl)-6-(phenylthio)uracil (Compound No.231)

In 50 ml of dichloromethane, 4.76 g (20 mmol) of 5-iodouracil was suspended and added with 11 ml (45 mmol) of bistrimethylsilylacetamide and stirred for 15 minutes at room temperature to form a homogeneous solution. This solution was added with 2.04 ml (22 mmol) of chloromethyl ethyl ether and 60 mg of tetra-n-butylammonium iodide and heated under reflux for 3 hours. After the solvent was evaporated under reduced pressure, the residue was added with water to produce crystals, which were taken by filtration. The crystals were washed by suspending them in hot methanol and recovering them by cooling and filtration to obtain 5.43 g of 1-(ethoxy-methyl)-5-iodouracil.

Then, 1.184 g (4 mmol) of 1-(ethoxymethyl)-5-iodouracil, 870 µg (8 mmol) of ethyl acrylate, 45 mg of palladium acetate and 0.6 ml of triethylamine were dissolved in 40 ml of dimethylformamide, heated and stirred for 5 hours at 70°C. After the solvent was evaporated under reduced pressure, the residue was adsorbed on a silica gel column, eluted with a solution of dichloromethan/ethyl acetate (1:1 v/v) to recover the desired fraction, from which the solvent was evaporated under reduced pressure to obtain 798 mg of 5-(2-(E)-carboethoxyvinyl)-1-(ethoxymethyl)uracil as crystals.

Then, 0.16 g (4.0 mmol) of sodium hydroxide and 0.54 g (2.0 mmol) of 5-(2-(E)-carboethoxyvinyl)-1-(ethoxymethyl)-uracil were added to 8 ml of water, stirred for 4.5 hours, neutralized with 1N hydrochloric acid and added with 10 ml of dimethylformamide to obtain a homogeneous solution.

This solution was then added with 0.62 g (4.5 mmol) of potassium carbonate, stirred for 5 minutes at room temperature to make it a homogeneous solution, then added with 0.36 g (2.0 mmol) of N-bromosuccinimide and stirred for 30 minutes. The reaction mixture was concentrated under reduced pressure and distributed between chloroform and aqueous solution of ammonium chloride, and the organic layer was concentrated under reduced pressure. The residue was adsorbed on a silica gel column and eluted with 20 % ethyl acetate/hexane to collect the desired fraction, from which the solvent was evaporated under reduced pressure to obtain 0.15 g of 5-(2-(E)-bromovinyl)-1-(ethoxymethyl)uracil (Yield: 28 %).

Then, a solution of 0.15 g (0.56 mmol) 5-(2(E)bromovinyl)-1-(ethoxymethyl)uracil in 1.7 ml of tetrahydrofuran was added dropwise to a solution of 1.22 mmol of lithium diisopropylamide in 2.3 ml of tetrahydrofuran under a nitrogen atmosphere at -70°C over 7 minutes and stirred for 40 minutes, added with a solution of 0.16 g (0.73 mmol) diphenyl disulfide in 1 ml of tetrahydrofuran and stirred for 1 hour. The reaction mixture was washed with saturated aqueous solution of sodium chloride and concentrated under reduced pressure. The residue was adsorbed on a silica gel column, eluted with 15 % ethyl acetate/hexane to collect the desired faction, from which the solvent was evaporated under reduced pressure to obtain 11 mg of the target compound (Yield: 5 %, m.p.: 143 - 148°C).

Compounds No. 32 to 37, 39 to 214, 216 to 219, 221 to 230, 232 to 311, 322 to 326, 328 to 426, 428, 430 to 439, 441 to 450, 453 to 464, 467 to 526, 529 to 627, 630 to 637, 640, 641, 644 to 656, 658 to 700 and 703 to 710 in Table 1 may be prepared similarly according to the methods described in the working examples above.

### EXAMPLE 65

### Production of tablet

| | |
|---|---|
| 1-[(2-acetoxyethoxy)methyl]-6-phenylthiothymine | 10 g |
| Corn starch | 65 g |
| Carboxycellulose | 20 g |
| Polyvinyl pyrrolidone | 3 g |
| Calcium stearate | 2 g |
| Total weight | 100 g |

The above-mentioned components were well mixed and tablets were produced by a direct tableting method. Each tablet had a weight of 100 mg and contained 1.0 mg of 1-[(2-acetoxyethoxy)methyl]-6-phenylthiothymine.

### EXAMPLE 66

### Production of powder and encapsulated medicine

| | |
|---|---|
| 1-[(2-acetoxyethoxy)methyl]-6-phenylthiothymine | 20 g |
| Crystalline cellulose | 80 g |
| Total weight | 100 g |

Both powder components were well mixed to obtain a powder formulation. 100 mg of the thus-obtained powder was charged into a hard capsule of No.5 to obtain an encapsulated medicine.

### EXAMPLE 67

### Inhibitory activity for HIV infection

In RPMI 1640 DM culture medium containing 20 mM of Hepes buffer solution, 10 % fetal bovine serum and 20 g/ml of gentamycin, 3 x 10⁴ MT-4 cells (human T cell clone which is destroyed by the infection of HIV) were infected with HIV in an amount of 100 times as large as expected to cause 50 % infection of the cells. Immediately thereafter, a predetermined amount of sample was added to the culture medium using 50 mg/ml sample solutions in dimethyl sulfoxide and the cells were cultured at 37°C.

After 5 days of incubation, the number of existing cells was counted to determine the concentration of the compound for preventing the death of 50 % of the MT-4 cells. Separately, MT-4 cells were cultured in the same way as above except that they were not infected with HIV to determine the concentration of the compound at which 50 % of the MT-4 cells were destroyed.

Both results are shown in Table 2.

**Table 2**

| Compound No. | 50% inhibitory concentration of HIV infection (µM) | 50% cytotoxic concentration to MT-4 cells (µM) |
|---|---|---|
| 1 | 2.8 | 196 |
| 2 | 6.7 | 314 |
| 3 | < 0.8 | 236 |
| 4 | < 0.8 | 240 |
| 5 | 1.8 | 218 |
| 7 | 7.1 | 292 |
| 8 | 9.9 | 218 |
| 10 | 11 | 162 |
| 11 | 7.5 | 78 |
| 12 | 7.6 | 53 |
| 13 | 11 | 170 |
| 14 | 12 | 66 |
| 16 | 21 | 420 |
| 17 | 0.96 | 171 |
| 20 | 8.6 | 292 |
| 22 | 2.1 | 244 |
| 23 | < 0.8 | 215 |
| 24 | 5.7 | 169 |
| 25 | 1.1 | 191 |
| 26 | 1.7 | 193 |
| 29 | 4.3 | 96 |
| 30 | 1.2 | 89 |
| 31 | 1.2 | 154 |
| 38 | 5.6 | 147 |
| 215 | 0.016 | 133 |
| 220 | 0.016 | 45 |
| 312 | 0.005 | > 100 |
| 313 | 0.026 | 81 |
| 314 | 0.004 | > 100 |
| 315 | 0.0025 | 30 |
| 316 | 0.005 | > 20 |
| 317 | 0.076 | 123 |
| 318 | 0.0078 | > 10 |
| 319 | 0.0069 | > 20 |
| 320 | 0.0074 | 45 |
| 321 | 0.013 | 45 |
| 327 | 0.012 | > 20 |
| 427 | 0.041 | 245 |
| 429 | 0.0064 | > 500 |
| 440 | 4.7 | 83 |
| 451 | 0.012 | 106 |
| 452 | 0.014 | > 100 |
| 465 | 0.0027 | > 20 |
| 466 | 0.0068 | > 20 |
| 527 | 0.10 | 223 |
| 528 | 0.095 | 46 |
| 628 | 0.34 | 143 |
| 629 | 0.22 | > 100 |
| 638 | 3.8 | > 100 |
| 639 | 1.6 | 223 |
| 642 | 0.45 | 17 |
| 643 | 0.35 | > 100 |
| 657 | 0.012 | 20 |
| 701 | 0.096 | 38 |
| 702 | 0.091 | > 20 |

## Claims

1. A 6-substituted acyclopyrimidine nucleoside derivative represented by the following general formula I; 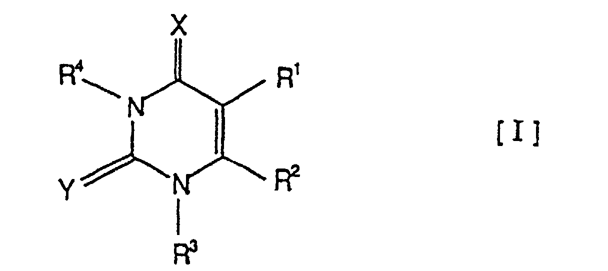 wherein:
R¹ represents C₁ to C₁₀ alkyl; C₃ to C₁₀ cycloalkyl; C₂ to C₅ alkenyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkynyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₇ to C₁₃ arylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₈ to C₁₄ arylcarbonylalkyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₆ to C₁₂ arylthio optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; or C₇ to C₁₇ aralkyl group optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups,
R² represents C₆ to C₁₂ arylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₆ to C₁₂ arylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₃ to C₁₀ cycloalkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkenyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkynyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₇ aralkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₃ arylcarbonyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₈ to C₁₄ arylcarbonylalkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; or C₆ to C₁₂ aryloxy group optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups,
R³ represents a hydrogen atom; methyl; C₃ to C₁₀ branched alkyl; or -CH₂-Z-(CH₂)ₙ-R⁵ group where R⁵ represents a hydrogen atom, halogen atom, 5- or 6-membered heterocyclic carbonyloxy, formyloxy, C₂ to C₁₁ alkylcarbonyloxy, C₄ to C₁₁ cycloalkylcarbonyloxy, C₈ to C₁₂ aralkylcarbonyloxy, C₇ to C₁₃ arylcarbonyloxy, azido, C₂ to C₁₁ alkoxycarbonyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₁ to C₁₀ alkoxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ aralkyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ branched alkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ cycloalkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, or C₆ to C₁₂ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5,
R⁴ represents a hydrogen atom, ; and
X and Y independently represent an oxygen or sulfur atom;
or a pharmaceutically acceptable salt thereof.

2. A compound according to claim 1, wherein:
R¹ represents C₁ to C₅ alkyl; C₃ to C₈ cycloalkyl; C₂ to C₅ alkenyl optionally substituted by one or more substituents selected from a halogen atom, phenyl, cyano, C₂ to C₆ alkoxycarbonyl and carbamoyl groups; C₂ to C₅ alkynyl optionally substituted by one or more substituents selected from a halogen atom, phenyl, cyano, C₂ to C₆ alkoxycarbonyl and carbamoyl groups; C₂ to C₅ alkylcarbonyl optionally substituted by one or more substituents selected from a halogen atom, phenyl, cyano, C₂ to C₆ alkoxycarbonyl and carbamoyl groups; C₇ to C₁₃ arylcarbonyl optionally substituted by one or more substituents selected from a halogen atom, phenyl, cyano, C₂ to C₆ alkoxycarbonyl and carbamoyl groups; C₈ to C₁₄ arylcarbonylalkyl optionally substituted by one or more substituents selected from a halogen atom, phenyl, cyano, C₂ to C₆ alkoxycarbonyl and carbamoyl groups; C₆ to C₁₂ arylthio optionally substituted by one or more substituents selected from a halogen atom, phenyl, cyano, C₂ to C₆ alkoxycarbonyl and carbamoyl groups; or C₇ to C₁₇ aralkyl group optionally substituted by one or more substituents selected from a halogen atom, phenyl, cyano, C₂ to C₆ alkoxycarbonyl and carbamoyl groups,
R² represents C₆ to C₁₀ arylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₁ to C₅ alkylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₆ to C₁₂ arylsulfinyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₁ to C₅ alkylsulfinyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₃ to C₁₀ cycloalkylsulfinyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₂ to C₅ alkenyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₂ to C₅ alkynyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₇ to C₁₁ aralkyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₇ to C₁₃ arylcarbonyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₈ to C₁₄ arylcarbonylalkyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; or C₆ to C₁₂ aryloxy group optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups.

3. A compound according to claim 2, wherein:
R¹ represents C₁ to C₅ alkyl; C₃ to C₈ cycloalkyl; C₂ to C₅ alkenyl optionally substituted by one or more substituents selected from a halogen atom, phenyl, cyano, C₂ to C₆ alkoxycarbonyl and carbamoyl groups; C₂ to C₅ alkynyl optionally substituted by one or more substituents selected from a halogen atom, phenyl, cyano, C₂ to C₆ alkoxycarbonyl and carbamoyl groups; C₂ to C₅ alkylcarbonyl; C₇ to C₁₃ arylcarbonyl; C₈ to C₁₄ arylcarbonylalkyl; C₆ to C₁₂ arylthio; or C₇ to C₁₇ aralkyl group,
R² represents C₆ to C₁₀ arylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₁ to C₅ alkylthio; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₆ to C₁₂ arylsulfinyl; C₁ to C₅ alkylsulfinyl; C₃ to C₁₀ cycloalkylsulfinyl; C₂ to C₅ alkenyl; C₂ to C₅ alkynyl; C₇ to C₁₁ aralkyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₇ to C₁₃ arylcarbonyl; C₈ to C₁₄ arylcarbonylalkyl; or C₆ to C₁₂ aryloxy group,
R³ represents a hydrogen atom; methyl; or -CH₂-Z-(CH₂)ₙ-R⁵ group where R⁵ represents a hydrogen atom, halogen atom, 5- or 6-membered heterocyclic carbonyloxy, formyloxy, C₂ to C₁₁ alkylcarbonyloxy, C₄ to C₁₁ cycloalkylcarbonyloxy, C₈ to C₁₂ aralkylcarbonyloxy, C₇ to C₁₃ arylcarbonyloxy, azido, C₂ to C₁₁ alkoxycarbonyloxy, C₂ to C₈ N-alkylcarbamoyloxy, C₇ to C₁₃ N-arylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₈ N-alkylthiocarbamoyloxy, C₇ to C₁₃ N-arylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₁ to C₁₀ alkoxy, C₇ to C₁₃ aralkyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₅ branched alkyl, C₅ to C₇ cycloalkyl, or C₆ to C₁₀ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5.

4. A compound according to claim 3, wherein:
R¹ represents C₁ to C₅ alkyl; C₃ to C₈ cycloalkyl; or C₂ to C₅ alkenyl optionally. substituted by one or more substituents selected from a halogen atom, phenyl, cyano, C₂ to C₆ alkoxycarbonyl and carbamoyl groups,
R² represents C₆ to C₁₀ arylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; or C₇ to C₁₁ aralkyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups,
R³ represents a hydrogen atom; methyl; or -CH₂-Z-(CH₂)ₙ-R⁵ group where R⁵ represents a hydrogen atom, halogen atom, 5- or 6-membered heterocyclic carbonyloxy, formyloxy, C₂ to C₁₁ alkylcarbonyloxy, C₄ to C₁₁ cycloalkylcarbonyloxy, C₈ to C₁₂ aralkylcarbonyloxy, C₇ to C₁₃ arylcarbonyloxy, azido, C₂ to C₁₁ alkoxycarbonyloxy, C₂ to C₈ N-alkylcarbamoyloxy, C₇ to C₁₃ N-arylcarbamoyloxy, C₂ to C₈ N-alkylthiocarbamoyloxy, C₇ to C₁₃ N-arylthiocarbamoyloxy, C₁ to C₁₀ alkoxy, C₇ to C₁₃ aralkyloxy, C₃ to C₅ branched alkyl, C₅ to C₇ cycloalkyl, or C₆ to C₁₀ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5.

5. An antiviral agent containing as an active ingredient a 6-substituted acyclopyrimidine nucleoside derivative or a pharmaceutically acceptable salt thereof according to any one of claims 1 to 4.

6. A pharmaceutical composition containing a compound of any of claims 1 to 4 or a pharmaceutically acceptable salt thereof in association with a pharmaceutical vehicle.

7. The pharmaceutical composition of claim 6 which has effective antiviral activity, particularly anti-retroviral activity.

8. The use of a 6-substituted acyclopyrimidine nucleoside derivative represented by the following general formula I; 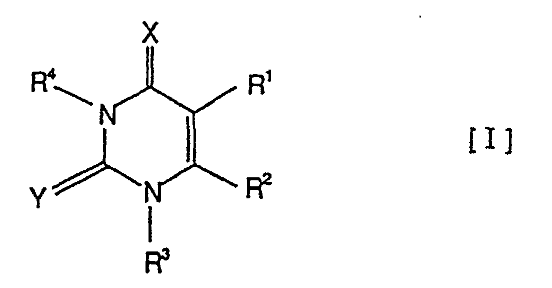 wherein:
R¹ represents a hydrogen atom; halogen atom; C₁ to C₁₀ alkyl; C₃ to C₁₀ cycloalkyl; C₂ to C₅ alkenyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkynyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₇ to C₁₃ arylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₈ to C₁₄ arylcarbonylalkyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₆ to C₁₂ arylthio optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; or C₇ to C₁₇ aralkyl group optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups,
R² represents C₆ to C₁₂ arylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylthio optionally substituted by one or more substituents selected from a halogen atom, C₁ to C₅ alkyl, C₁ to C₅ halogenated alkyl, C₁ to C₅ alkoxy, C6 to C₁₂ aryl, hydroxyl, nitro, amino, cyano and C₂ to C₇ acyl groups; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₆ to C₁₂ arylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₃ to C₁₀ cycloalkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkenyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkynyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₇ aralkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₃ arylcarbonyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₈ to C₁₄ arylcarbonylalkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; or C₆ to C₁₂ aryloxy group optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups,
R³ represents a hydrogen atom; methyl; C₃ to C₁₀ branched alkyl; or -CH₂-Z-(CH₂)ₙ-R⁵ group where R⁵ represents a hydrogen atom, halogen atom, 5- or 6-membered heterocyclic carbonyloxy, formyloxy, C₂ to C₁₁ alkylcarbonyloxy, C₄ to C₁₁ cycloalkylcarbonyloxy, C₈ to C₁₂ aralkylcarbonyloxy, C₇ to C₁₃ arylcarbonyloxy, azido, C₂ to C₁₁ alkoxycarbonyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₁ to C₁₀ alkoxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ aralkyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ branched alkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ cycloalkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, or C₆ to C₁₂ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5,
R⁴ represents a hydrogen atom, C₁ to C₁₃ alkyl or C₇ to C₁₃ aralkyl group; and
X and Y independently represent an oxygen or sulfur atom;
or a pharmaceutically acceptable salt thereof,
in a process for the production of a pharmaceutical composition for the treatment or prevention of diseases caused by viral, particularly retroviral infection, especially in human beings.

9. The use according to claim 8, wherein:
R¹ represents a hydrogen atom; halogen atom; C₁ to C₅ alkyl; C₃ to C₈ cycloalkyl; C₂ to C₅ alkenyl optionally substituted by one or more substituents selected from a halogen atom, phenyl, cyano, C₂ to C₆ alkoxycarbonyl and carbamoyl groups; C₂ to C₅ alkynyl optionally substituted by one or more substituents selected from a halogen atom, phenyl, cyano, C₂ to C₆ alkoxycarbonyl and carbamoyl groups; C₂ to C₅ alkylcarbonyl optionally substituted by one or more substituents selected from a halogen atom, phenyl, cyano, C₂ to C₆ alkoxycarbonyl and carbamoyl groups; C₇ to C₁₃ arylcarbonyl optionally substituted by one or more substituents selected from a halogen atom, phenyl, cyano, C₂ to C₆ alkoxycarbonyl and carbamoyl groups; C₈ to C₁₄ arylcarbonylalkyl optionally substituted by one or more substituents selected from a halogen atom, phenyl, cyano, C₂ to C₆ alkoxycarbonyl and carbamoyl groups; C₆ to C₁₂ arylthio optionally substituted by one or more substituents selected from a halogen atom, phenyl, cyano, C₂ to C₆ alkoxycarbonyl and carbamoyl groups; or C₇ to C₁₇ aralkyl group optionally substituted by one or more substituents selected from a halogen atom, phenyl, cyano, C₂ to C₆ alkoxycarbonyl and carbamoyl groups,
R² represents C₆ to C₁₀ arylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₁ to C₅ alkylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₆ to C₁₂ arylsulfinyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₁ to C₅ alkylsulfinyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₃ to C₁₀ cycloalkylsulfinyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₂ to C₅ alkenyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₂ to C₅ alkynyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₇ to C₁₁ aralkyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₇ to C₁₃ arylcarbonyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₈ to C₁₄ arylcarbonylalkyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; or C₆ to C₁₂ aryloxy group optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups.

10. The use according to claim 9, wherein:
R¹ represents a hydrogen atom; halogen atom; C₁ to C₅ alkyl; C₃ to C₈ cycloalkyl; C₂ to C₅ alkenyl optionally substituted by one or more substituents selected from a halogen atom, phenyl, cyano, C₂ to C₆ alkoxycarbonyl and carbamoyl groups; C₂ to C₅ alkynyl optionally substituted by one or more substituents selected from a halogen atom, phenyl, cyano, C₂ to C₆ alkoxycarbonyl and carbamoyl groups; C₂ to C₅ alkylcarbonyl; C₇ to C₁₃ arylcarbonyl; C₈ to C₁₄ arylcarbonylalkyl; C₆ to C₁₂ arylthio; or C₇ to C₁₇ aralkyl group,
R² represents C₆ to C₁₀ arylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₁ to C₅ alkylthio; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₆ to C₁₂ arylsulfinyl; C₁ to C₅ alkylsulfinyl; C₃ to C₁₀ cycloalkylsulfinyl; C₂ to C₅ alkenyl; C₂ to C₅ alkynyl; C₇ to C₁₁ aralkyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₇ to C₁₃ arylcarbonyl; C₈ to C₁₄ arylcarbonylalkyl; or C₆ to C₁₂ aryloxy group,
R³ represents a hydrogen atom; methyl; or -CH₂-Z-(CH₂)ₙ-R⁵ group where R⁵ represents a hydrogen atom, halogen atom, 5- or 6-membered heterocyclic carbonyloxy, formyloxy, C₂ to C₁₁ alkylcarbonyloxy, C₄ to C₁₁ cycloalkylcarbonyloxy, C₈ to C₁₂ aralkylcarbonyloxy, C₇ to C₁₃ arylcarbonyloxy, azido, C₂ to C₁₁ alkoxycarbonyloxy, C₂ to C₈ N-alkylcarbamoyloxy, C₇ to C₁₃ N-arylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₈ N-alkylthiocarbamoyloxy, C₇ to C₁₃ N-arylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₁ to C₁₀ alkoxy, C₇ to C₁₃ aralkyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₅ branched alkyl, C₅ to C₇ cycloalkyl, or C₆ to C₁₀ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5.

11. The use according to claim 10, wherein:
R¹ represents a hydrogen atom; halogen atom; C₁ to C₅ alkyl; C₃ to C₈ cycloalkyl; or C₂ to C₅ alkenyl optionally substituted by one or more substituents selected from a halogen atom, phenyl, cyano, C₂ to C₆ alkoxycarbonyl and carbamoyl groups,
R² represents C₆ to C₁₀ arylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups; or C₇ to C₁₁ aralkyl optionally substituted by one or more substituents selected from a halogen atom, C₁ to C₅ alkyl, C₁ to C₅ halogenated alkyl, C₁ to C₅ alkoxy, phenyl, hydroxyl, nitro, amino, cyano and C₂ to C₇ acyl groups,
R³ represents a hydrogen atom; methyl; or -CH₂-Z-(CH₂)ₙ-R⁵ group where R⁵ represents a hydrogen atom, halogen atom, 5- or 6-membered heterocyclic carbonyloxy, formyloxy, C₂ to C₁₁ alkylcarbonyloxy, C₄ to C₁₁ cycloalkylcarbonyloxy, C₈ to C₁₂ aralkylcarbonyloxy, C₇ to C₁₃ arylcarbonyloxy, azido, C₂ to C₁₁ alkoxycarbonyloxy, C₂ to C₈ N-alkylcarbamoyloxy, C₇ to C₁₃ N-arylcarbamoyloxy, C₂ to C₈ N-alkylthiocarbamoyloxy, C₇ to C₁₃ N-arylthiocarbamoyloxy, C₁ to C₁₀ alkoxy, C₇ to C₁₃ aralkyloxy, C₃ to C₅ branched alkyl, C₅ to C₇ cycloalkyl, or C₆ to C₁₀ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5.

12. A process for the preparation of a compound represented by the following general formula I; 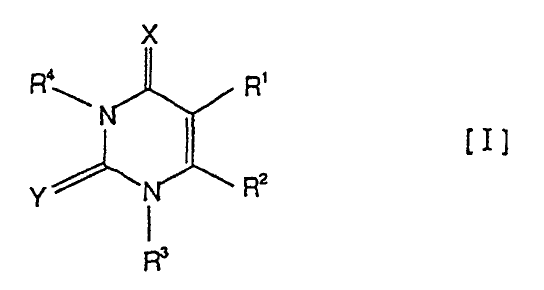 wherein:
R¹ represents a C₁ to C₁₀ alkyl; C₃ to C₁₀ cycloalkyl; C₂ to C₅ alkenyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkynyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₂ to C₅ alkylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₇ to C₁₃ arylcarbonyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₈ to C₁₄ arylcarbonylalkyl optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; C₆ to C₁₂ arylthio optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups; or C₇ to C₁₇ aralkyl group optionally substituted by one or more substituents selected from a halogen atom, C₆ to C₁₂ aryl, cyano, C₂ to C₆ alkoxycarbonyl, carbamoyl, C₁ to C₅ alkoxy, amino and nitro groups,
R² represents C₆ to C₁₂ arylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₃ to C₁₀ cycloalkylthio optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₆ to C₁₂ arylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₁ to C₁₀ alkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₃ to C₁₀ cycloalkylsulfinyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkenyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₂ to C₅ alkynyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₇ aralkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₇ to C₁₃ arylcarbonyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; C₈ to C₁₄ arylcarbonylalkyl optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups; or C₆ to C₁₂ aryloxy group optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups,
R³ represents methyl; C₃ to C₁₀ branched alkyl; or -CH₂-Z-(CH₂)ₙ-R⁵ group where R⁵ represents a hydrogen atom, halogen atom, azido, C₁ to C₁₀ alkoxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ aralkyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ branched alkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ cycloalkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, or C₆ to C₁₂ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5,
R⁴ represents a hydrogen atom ; and
X and Y independently represent an oxygen or sulfur atom;
which comprises reacting a compound represented by the following general formula II; 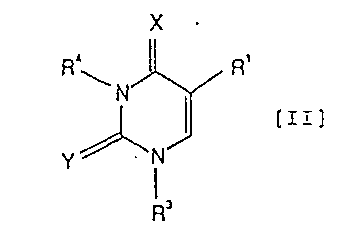 wherein R¹, R³, R⁴, X and Y have the same meanings as defined above, with an organic alkali metal compound to obtain a compound represented by the following general formula II'; 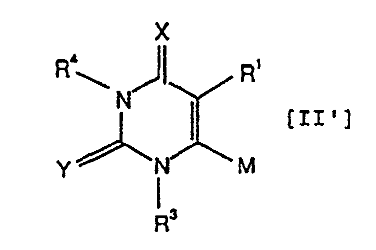 wherein R¹, R³, R⁴, X and Y have the same meanings as defined above and M represents an alkaline metal, and then reacting the obtained compound of the formula II' with a compound represented by the following general formula III;
R²X¹ [III]
wherein R² has the same meaning as defined above and X¹ represents a halogen atom, arylthio or alkoxy group, to obtain the compound of the formula I above.

13. A process for the preparation of a compound represented by the following general formula I; 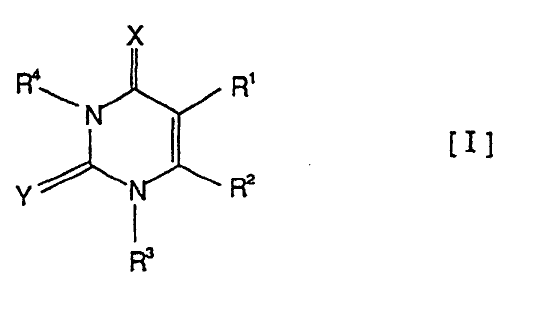 wherein R¹, R², R³, R⁴, X and Y have the same meanings as defined in claim 1, which comprises reacting a compound represented by the following general formula IV; 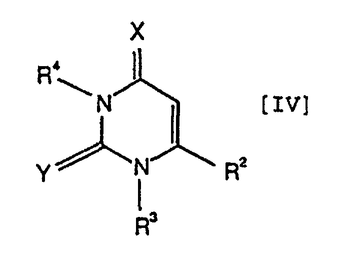 wherein R², R³, R⁴, X and Y have the same meanings as defined in claim 12, with an organic alkali metal compound to obtain a compound represented by the following general formula IV'; 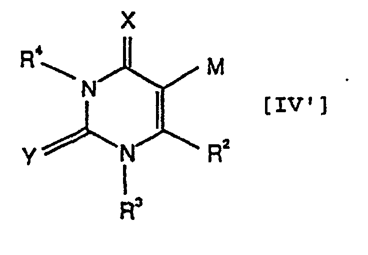 wherein R², R³, R⁴, X and Y have the same meanings as defined in claim 12 and M represents an alkali metal, and then reacting the obtained compound of the formula IV' with a compound represented by the following general formula V;
R¹X² [V]
wherein R¹ has the same meaning as defined in claim 12 and X² represents a halogen atom, arylthio or alkoxy group, to obtain the compound of the formula I above.

14. A process for the preparation of a compound represented by the following general formula I; 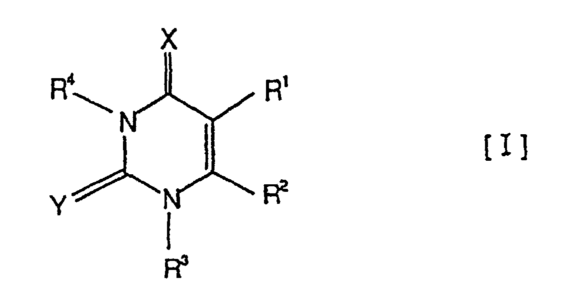 wherein R¹, R², R³, R⁴, X and Y have the same meanings as defined in claim 12, which comprises reacting a compound represented by the following general formula VI; 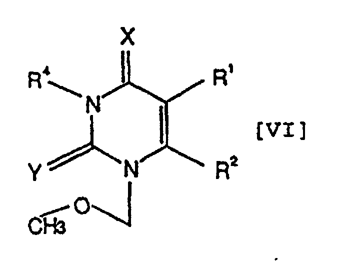 wherein R¹, R², R⁴, X and Y have the same meanings as defined in claim 12, with an acid to obtain a compound represented by the following general formula VII; 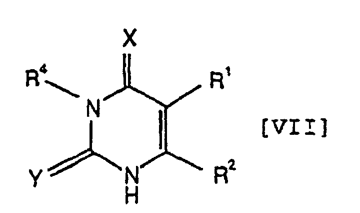 wherein R¹, R², R⁴, X and Y have the same meanings as defined in claim 12, and then reacting the obtained compound of the formula VII with a compound represented by the following general formula VIII;
R³X³ [VIII]
wherein R³ has the same meaning as defined in claim 12 and X³ represents a sulfonyloxy group, in the presence of a base to obtain the compound of the formula I above.

15. A process for the preparation of a compound represented by the following general formula X; 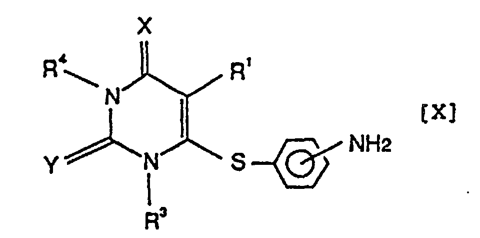 wherein R¹, R⁴, X and Y have the same meanings as defined in claim 12 and R³ represents a hydrogen atom; methyl; C₃ to C₁₀ branched alkyl; or -CH₂-Z-(CH₂)ₙ-R⁵ group where R⁵ represents a hydrogen atom, halogen atom, 5- or 6-membered heterocyclic carbonyloxy, formyloxy, C₂ to C₁₁ alkylcarbonyloxy, C₄ to C₁₁ cycloalkylcarbonyloxy, C₈ to C₁₂ aralkylcarbonyloxy, C₇ to C₁₃ arylcarbonyloxy, azido, C₂ to C₁₁ alkoxycarbonyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylcarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₂ to C₁₁ N-alkylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₇ to C₁₃ N-arylthiocarbamoyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₁ to C₁₀ alkoxy optionally substituted by one or more substituents selected from a halogen atom, C₆ to C₁₂ aryl, C₁ to C₅ alkyl; C₁ to C₅ alkoxy and C₁ to C₅ halogenated alkyl groups, C₇ to C₁₃ aralkyloxy optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ branched alkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, C₃ to C₁₀ cycloalkyl optionally substituted by one or more substituents selected from a halogen atom, C₆ to C₁₂ aryl, C₁ to C₅ alkyl, C₁ to C₅ alkoxy and C₁ to C₅ halogenated alkyl groups, or C₆ to C₁₂ aryl group optionally substituted by one or more substituents selected from a halogen atom, C₆ to C₁₂ aryl, C₁ to C₅ alkyl, C₁ to C₅ alkoxy and C₁ to C₅ halogenated alkyl groups, Z represents an oxygen, sulfur atom or methylene group, and n represents 0 or an integer of 1 to 5, which comprises hydrogenating a compound represented by the following general formula IX; 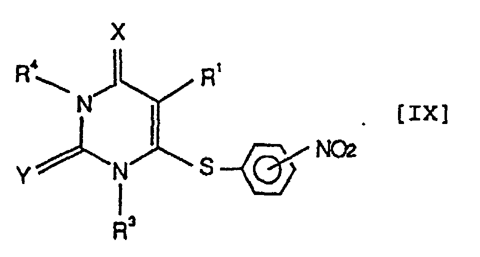 wherein R¹, R⁴, X and Y have the same meanings as defined in claim 12 and R³ has the same meaning as defined above, in the presence of a catalyst to obtain the compound of the formula X above.

16. A process for the preparation of a compound represented by the following general formula XII; 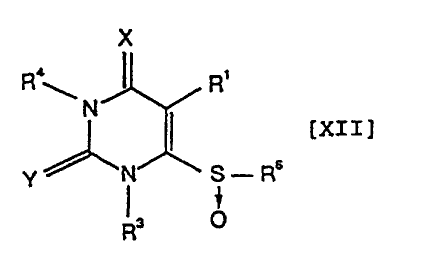 wherein R¹, R³, R⁴, X and Y have the same meanings as defined in claim 15 and R⁶ represents C₆ to C₁₂ aryl, C₁ to C₁₀ alkyl or C₃ to C₁₀ cycloalkyl group, which comprises converting a compound represented by the following general formula XI; 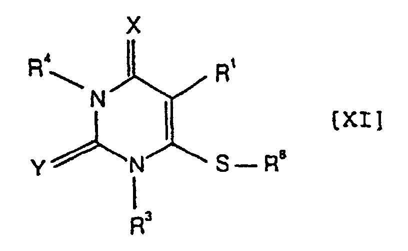 wherein R¹, R³, R⁴, X and Y have the same meanings as defined in claim 2 and R⁶ has the same meaning as defined above into the compound of the formula XII above by an action of oxidizing agent.

17. A process for the preparation of a compound represented by the following general formula XV; 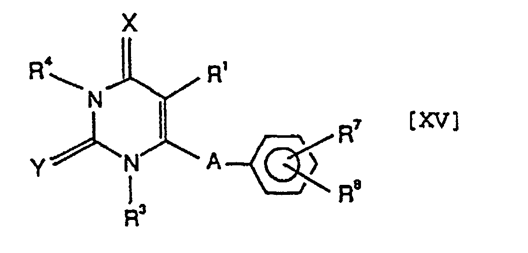 wherein R¹, R³, R⁴, X and Y have the same meanings as defined in claim 15, R⁷ and R⁸ independently represent a halogen atom, C₁ to C₅ alkyl, C₁ to C₅ halogenated alkyl, C₁ to C₅ alkoxy, hydroxyl, nitro, amino, cyano or C₂ to C₇ acyl group and A represents a sulfur or oxygen atom, which comprises reacting a compound represented by the following general formula XIII; 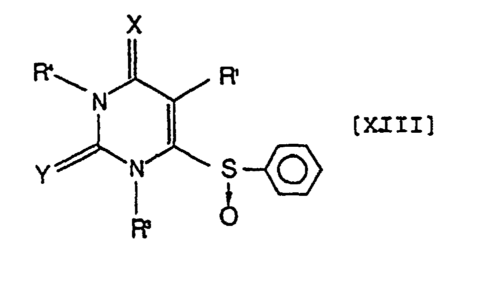 wherein R¹, R³, R⁴, X and Y have the same meanings as defined in claim 15, with a compound represented by the following general formula XIV; 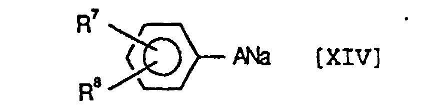 wherein R⁷ and R⁸ have the same meanings as defined above, to obtain the compound of the formula XV above.

18. A process for the preparation of a compound represented by the following general formula XVIII; 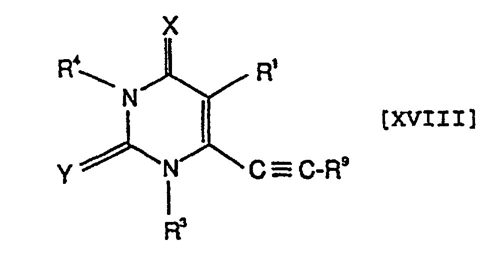 wherein R¹, R³, R⁴, X and Y have the same meanings as defined in claim 15 and R⁹ represents C₁ to C₅ alkyl, C₆ to C₁₂ aryl or silyl group, which comprises reacting a compound represented by the following general formula XVI; 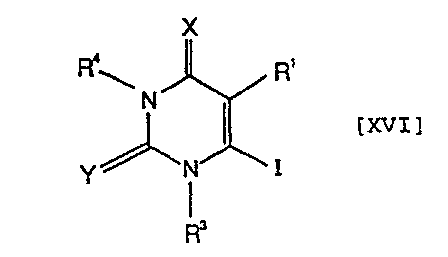 wherein R¹, R³, R⁴, X and Y have the same meanings as defined in claim 15, with a compound represented by the following general formula XVII;
HC≡C-R⁹ [XVII]
wherein R⁹ has the same meaning as defined above, in the presence of a palladium catalyst to obtain the compound of the formula XVIII above.

19. A process for the preparation of a compound represented by the following general formula XX; 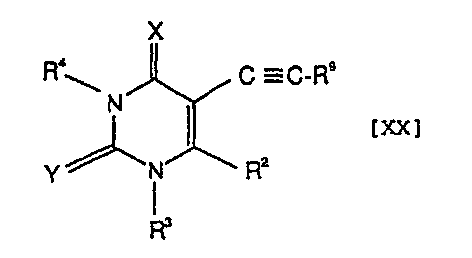 wherein R², R³, R⁴, X and Y have the same meanings as defined in claim 15 and R⁹ represents C₁ to C₅ alkyl, C₆ to C₁₂ aryl or silyl group, which comprises reacting a compound represented by the following general formula XIX; 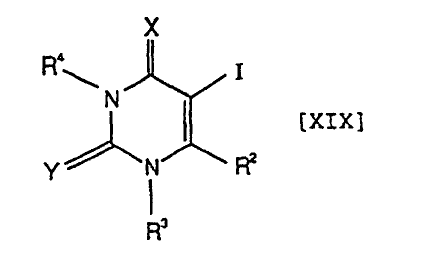 wherein R², R³, R⁴, X and Y have the same meanings as defined in claim 15, with a compound represented by the following general formula XVII;
HC≡C-R⁹ [XVII]
wherein R⁹ has the same meaning as defined above, in the presence of a palladium catalyst to obtain the compound of the formula XX above.

20. A process for the preparation of a compound represented by the following general formula XXII; 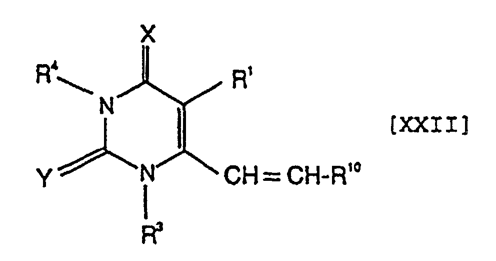 wherein R¹, R³, R⁴, X and Y have the same meanings as defined in claim 15 and R¹⁰ represents C₂ to C₆ alkoxycarbonyl, nitrile or carbamoyl group, which comprises reacting a compound represented by the following general formula XVI; 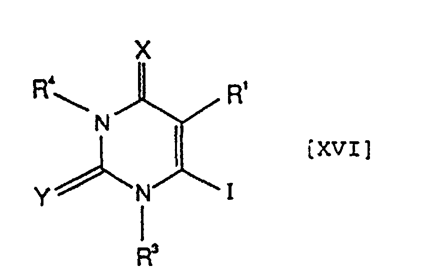 wherein R¹, R³, R⁴, X and Y have the same meanings as defined in claim 15, with a compound represented by the following general formula XXI;
H₂C=CH-R¹⁰ [XXI]
wherein R¹⁰ has the same meaning as defined above, in the presence of a palladium catalyst to obtain the compound of the formula XXII above.

21. A process for the preparation of a compound represented by the following general formula XXIII; 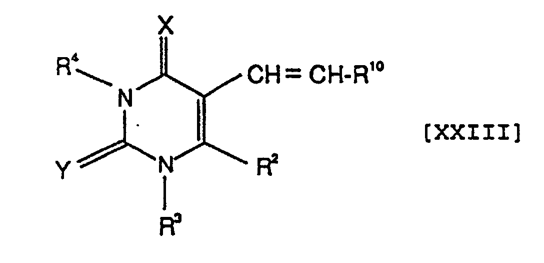 wherein R², R³, R⁴, X and Y have the same meanings as defined in claim 15 and R¹⁰ has the same meaning as defined in claim 20, which comprises reacting a compound represented by the following general formula XIX; 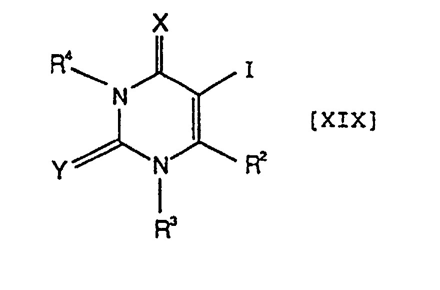 wherein R², R³, R⁴, X and Y have the same meanings as defined in claim 15, with a compound represented by the following general formula XVII;
H₂C=CH-R¹⁰ [XXI]
wherein R¹⁰ has the same meaning as defined in claim 20, in the presence of a palladium catalyst to obtain the compound of the formula XXIII above.

22. A process for the preparation of a compound represented by the following general formula XXV; 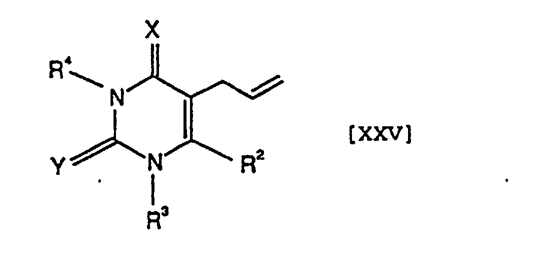 wherein R², R³, R⁴, X and Y have the same meanings as defined in claim 15, which comprises reacting a compound represented by the following general formula IV; 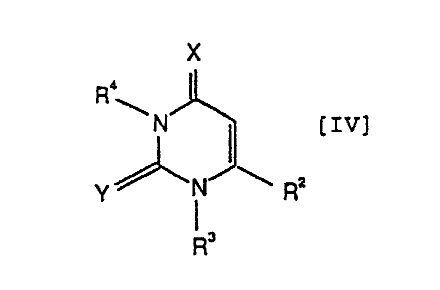 wherein R², R³, R⁴, X and Y have the same meanings as defined in claim 15, with a compound represented by the following general formula XXIV;
X⁴-CH₂CH=CH₂ [XXIV]
wherein X⁴ represents a halogen atom, in the presence of a palladium catalyst to obtain the compound of the formula XXV above.

23. A process for the preparation of a compound represented by the following general formula XXVIII; 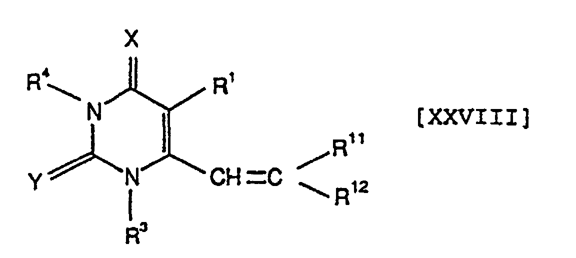 wherein R¹, R³, R⁴, X and Y have the same meanings as defined in claim 12, and R¹¹ and R¹² independently represent a hydrogen atom, C₁ to C₃ alkyl or C₆ to C₁₂ aryl group, which comprises reacting a compound represented by the following general formula II; 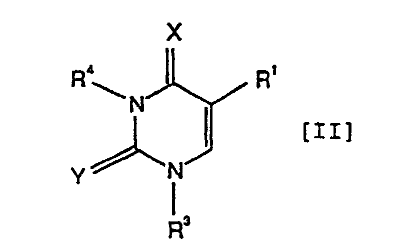 wherein R¹, R³, R⁴, X and Y have the same meanings as defined in claim 12, with an organic alkali metal compound to obtain a compound represented by the following general formula II'; 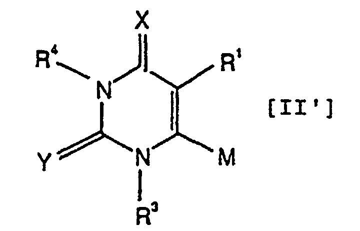 wherein R¹, R³, R⁴, X and Y have the same meanings as defined in claim 12 and M represents an alkali metal, reacting the obtained compound of the formula II' with a compound represented by the following general formula XXVI; 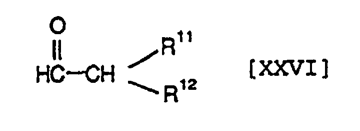 wherein R¹¹ and R¹² have the same meanings as defined above to obtain a compound represented by the following general formula XXVII; 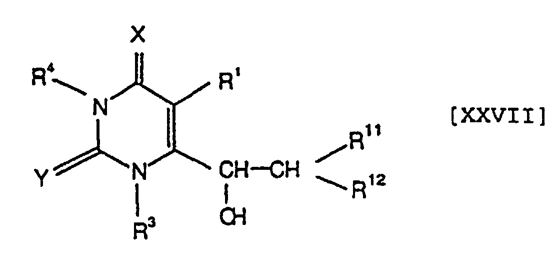 wherein R¹, R³, R⁴, X and Y have the same meanings as defined in claim 12 and R¹¹ and R¹² have the same meanings defined as above, and then dehydrating the obtained compound of the formula XXVII using a dehydrating agent to obtain the compound of the formula XXVIII above.

24. A process for the preparation of a compound represented by the following general formula XXX; 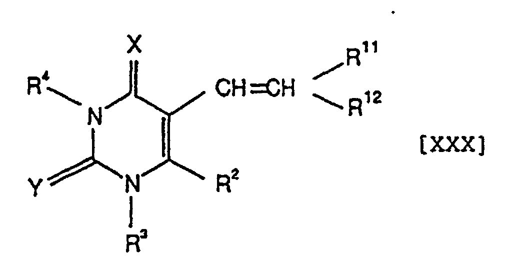 wherein R², R³, R⁴, X and Y have the same meanings as defined in claim 15 and R¹¹ and R¹² independently represent a hydrogen atom, C₁ to C₃ alkyl or C₆ to C₁₂ aryl group, which comprises reacting a compound represented by the following general formula IV; 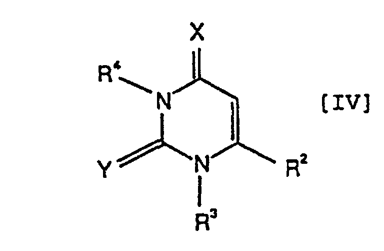 wherein R², R³, R⁴, X and Y have the same meanings as defined in claim 15, with an organic alkali metal compound to obtain a compound represented by the following general formula IV'; 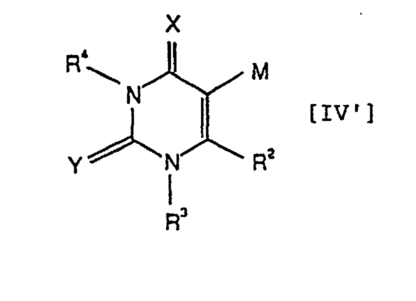 wherein R², R³, R⁴, X and Y have the same meanings as defined in claim 12 and M represents an alkali metal, reacting the obtained compound of the formula IV' with a compound represented by the following general formula XXVI; 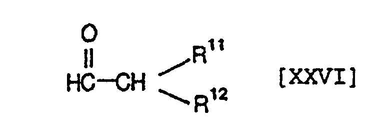 wherein R¹¹ and R¹² have the same meanings as defined above, to obtain a compound represented by the following general formula XXIX; 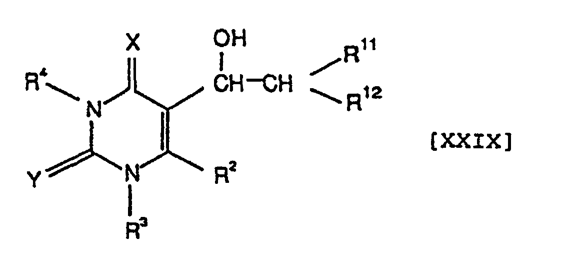 wherein R², R³, R⁴, X and Y have the same meanings as defined in claim 15 and R¹¹ and R¹² have the same meanings as defined above, and then dehydrating the obtained compound of the formula XXIX using a dehydrating agent to obtain the compound of the formula XXX above.

25. A process for the preparation of a compound represented by the following general formula XXXIII; 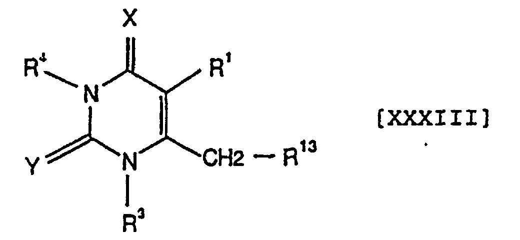 wherein R¹, R³, R⁴, X and Y have the same meanings as defined in claim 12 and R¹³ represents C₆ to C₁₂ aryl group optionally substituted by one or more substituents selected from a halogen atom, C₁ to C₅ alkyl, C₁ to C₅ halogenated alkyl, C₁ to C₅ alkoxy, C₆ to C₁₂ aryl, hydroxyl, nitro, amino, cyano and C₂ to C₇ acyl groups, which comprises reacting a compound represented by the following general formula II; 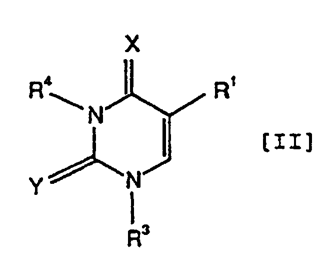 wherein R¹, R³, R⁴, X and Y have the same meanings as defined in claim 12, with an organic alkali metal compound to obtain a compound represented by the following general formula II'; 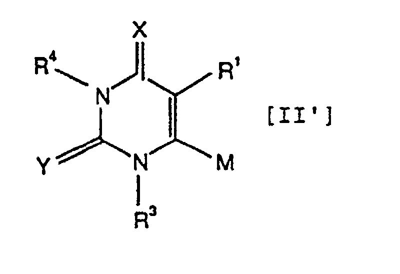 wherein R¹, R³, R⁴, X and Y have the same meanings as defined in claim 12 and M represents an alkali metal, reacting the obtained compound of the formula II' with a compound represented by the following general formula XXXI; 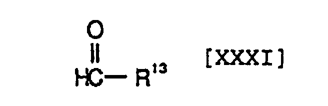 wherein R¹³ has the same meaning as defined above to obtain a compound represented by the following general formula XXXII; 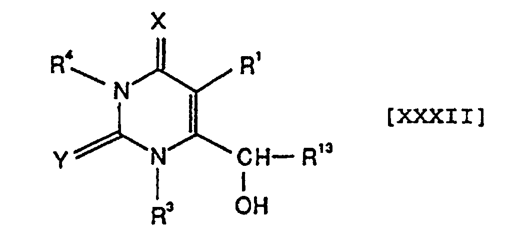 wherein R¹, R³, R⁴, X and Y have the same meanings as defined in claim 12 and R¹³ has the same meaning as defined above, and then reducing the obtained compound of the formula XXXII using a reducing agent to obtain the compound of the formula XXXIII above.

26. A process for the preparation of a compound represented by the following general formula XXXVI; 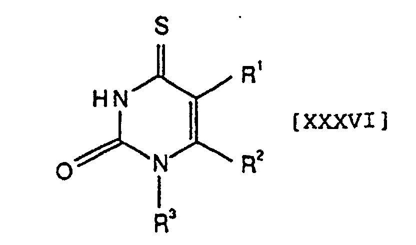 wherein R¹, R² and R³ have the same meanings as defined in claim 15, which comprises reacting a compound represented by the following general formula XXXIV; 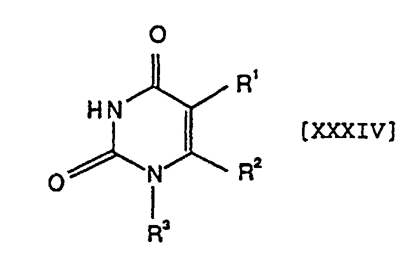 wherein R¹, R² and R³ have the same meanings as defined in claim 15 with a compound represented by the following general formula XXXV; 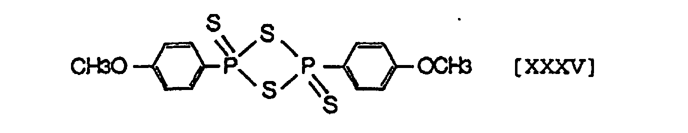 to obtain the compound of the formula XXXVI above.

27. A process for the preparation of a compound represented by the following general formula XXXX; 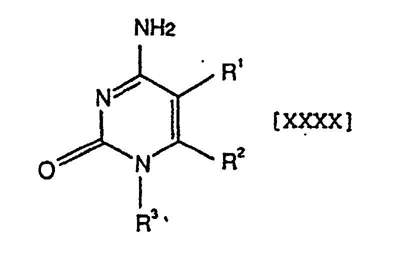 wherein R¹, R² and R³ have the same meanings as defined in claim 15, which comprises reacting a compound represented by the following general formula XXXVII; 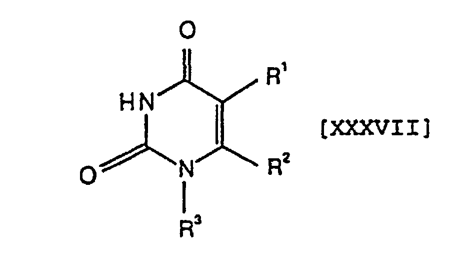 wherein R¹, R² and R³ have the same meanings as defined in claim 15, with a compound represented by the following general formula XXXVIII; 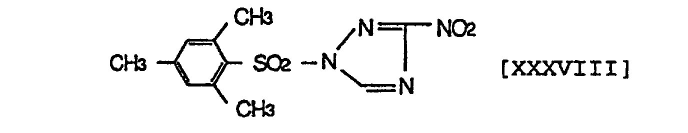 in a solvent in the presence of diphenylphosphoric acid to obtain a compound of the following general formula XXXIV; 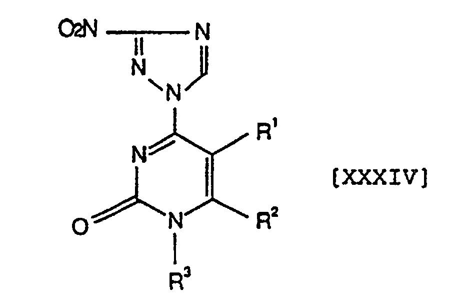 wherein R¹, R² and R³ have the same meanings as defined in claim 15, and reacting the obtained compound of the formula XXXIV with aqueous ammonia to obtain the compound of the formula XXXX above.

28. A process for the preparation of a compound represented by the following general formula I; 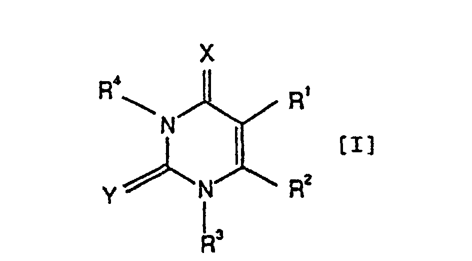 wherein R¹, R², R³, X and Y have the same meanings as defined in claim 15 and R⁴ has the same meaning as defined in claim 12, which comprises reacting a compound represented by the following general formula XXXXI; 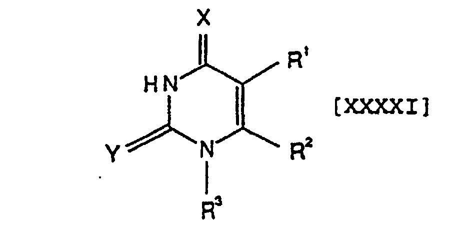 wherein R¹, R², R³, X and Y have the same meanings as defined in claim 15 with a compound represented by the following general formula XXXXII;
R⁴X⁵ [XXXXII]
wherein R⁴ have the same meanings as defined in claim 1 and X⁵ represents a halogen atom or sulfonyloxy group, in the presence of a base to obtain the compound of the formula I above.

29. A process for the preparation of a compound represented by the following general formula XXXXV; 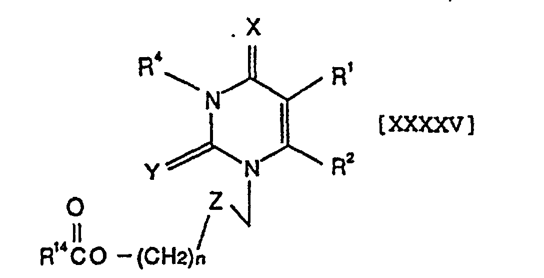 wherein R¹, R², R⁴, X, Y, Z and n have the same meanings as defined in claim 12 and R¹⁴ represents C₁ to C₁₀ alkyl, C₃ to C₁₀ cycloalkyl, C₆ to C₁₂ aryl, C₇ to C₁₁ aralkyl or 5- or 6-membered heterocyclic group, which comprises reacting a compound represented by the following general formula XXXXIII; 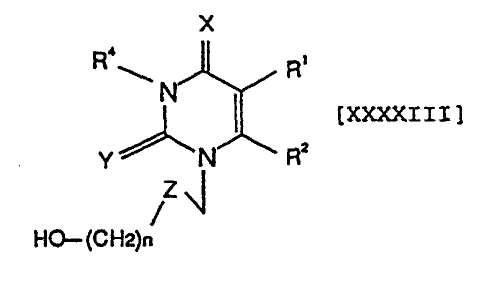 wherein R¹, R², R⁴, X, Y, Z and n have the same meanings as defined in claim 12, with a compound represented by the following general formula XXXXIV; 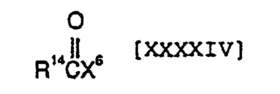 wherein R¹⁴ has the same meaning as defined above and X⁶ represents a halogen atom or -OCOR¹⁴, in a solvent in the presence of a base to obtain the compound of the formula XXXXV above.

30. A process for the preparation of a compound represented by the following general formula XXXXVII; 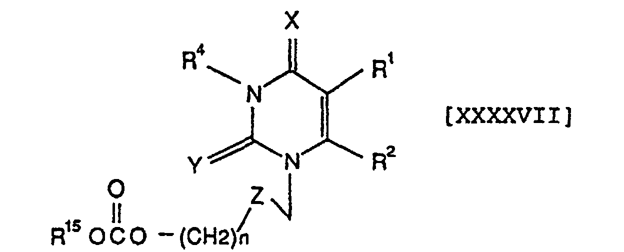 wherein R¹, R², R⁴, X, Y, Z and n have the same meanings as defined in claim 12 and R¹⁵ represents C₁ to C₁₀ alkyl, which comprises reacting a compound represented by the following general formula XXXXIII; 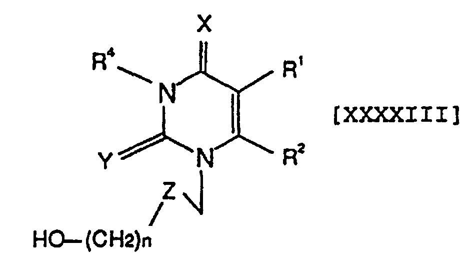 wherein R¹, R², R⁴, X, Y, Z and n have the same meanings as defined in claim 12 with a compound represented by the following general formula XXXXVI; 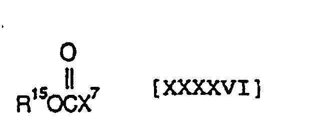 wherein R¹⁵ has the same meaning as defined above and X⁷ represents a halogen atom or -OCOOR¹⁵, in a solvent in the presence of a base to obtain the compound of the formula XXXXVII above.

31. A process for the preparation of a compound represented by the following general formula XXXXIX; 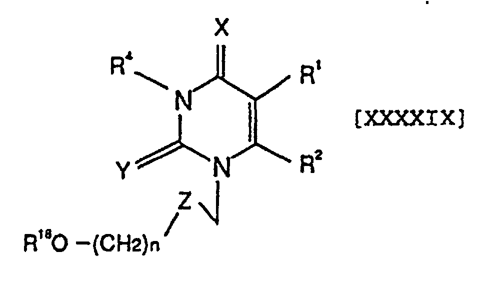 wherein R¹, R², R⁴, X, Y, Z and n have the same meanings as defined in claim 12 and R¹⁶ represents C₁ to C₁₀ alkyl, C₇ to C₁₃ aralkyl or formyl group, which comprises reacting a compound represented by the following general formula XXXXIII; 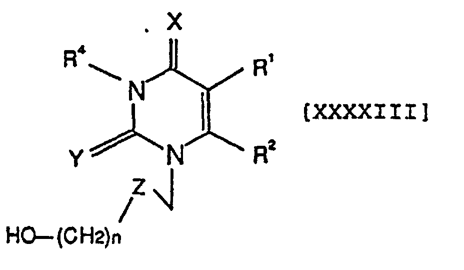 wherein R¹, R², R⁴, X, Y, Z and n have the same meanings as defined in claim 12, with a compound represented by the following general formula XXXXVIII;
R¹⁶X⁸ [XXXXVIII]
wherein R¹⁶ have the same meaning as defined above and X⁸ represents a halogen atom or sulfonyloxy group, in a solvent in the presence of a base to obtain the compound of the formula XXXXIX above.

32. A process for the preparation of a compound represented by the following general formula XXXXXI; 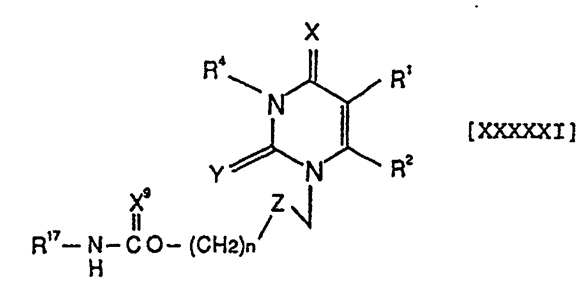 wherein R¹, R², R⁴, X, Y, Z and n have the same meanings as defined in claim 12, R¹⁷ represents C₁ to C₁₀ alkyl or C₆ to C₁₂ aryl group and X⁹ represents a sulfur or oxygen atom, which comprises reacting a compound represented by the following general formula XXXXIII; 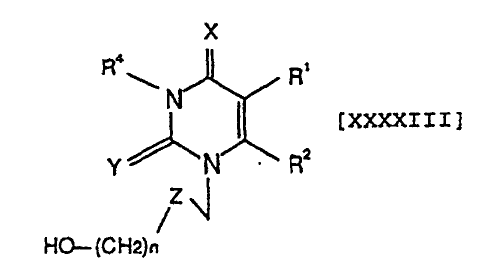 wherein R¹, R², R⁴, X, Y, Z and n have the same meanings as defined in claim 12, with a compound represented by the following general formula XXXXX;
R¹⁷-N=C=X⁹ [XXXXX]
wherein R¹⁷ and X⁹ have the same meanings as defined above, in a solvent in the presence of a base to obtain the compound of the formula XXXXXI above.

## Patentansprüche

1. 6-substituiertes Acyclopyrimidinnucleosidderivat, dargestellt durch die folgende allgemeine Formel (I): 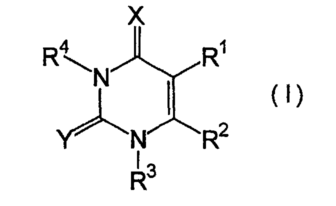 worin:
R¹ darstellt: C₁₋₁₀-Alkyl; C₃₋₁₀-Cycloalkyl; C₂₋₅-Alkenyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₂₋₅-Alkinyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₂₋₅-Alkylcarbonyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₇₋₁₃-Arylcarbonyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₈₋₁₄-Arylcarbonylalkyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₆₋₁₂-Arylthio, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; oder C₇₋₁₇-Aralkylgruppen, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen,
R² darstellt: C₆₋₁₂-Arylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₁₋₁₀-Alkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₃₋₁₀-Cycloalkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₆₋₁₂-Arylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₁₋₁₀-Alkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₃₋₁₀-Cycloalkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₂₋₅-Alkenyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₂₋₅-Alkinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₇₋₁₇-Aralkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₇₋₁₃-Arylcarbonyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₈₋₁₄-Arylcarbonylalkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; oder C₆₋₁₂-Aryloxygruppen, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen,
R³ darstellt: ein Wasserstoffatom; Methyl; verzweigtes C₃₋₁₀-Alkyl; oder eine -CH₂-Z-(CH₂)ₙ-R⁵-Gruppe, worin R⁵ darstellt: ein Wasserstoffatom, ein Halogenatom, 5- oder 6-gliedriges heterocyclisches Carbonyloxy, Formyloxy, C₂₋₁₁-Alkylcarbonyloxyl C₄₋₁₁-Cycloalkylcarbonyloxy, C₈₋₁₂-Aralkylcarbonyloxy, C₇₋₁₃-Arylcarbonyloxy, Azido, C₂₋₁₁-Alkoxycarbonyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₂₋₁₁-N-Alkylcarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-N-Arylcarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₂₋₁₁-N-Alkylthiocarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-N-Arylthiocarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₁₋₁₀-Alkoxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-Aralkyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, verzweigtem C₃₋₁₀-Alkyl, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₃₋₁₀-Cycloalkyl, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, oder C₆₋₁₂-Arylgruppen, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, Z Sauerstoff, ein Schwefelatom oder eine Methylengruppe darstellt, und n 0 oder eine ganze Zahl von 1 bis 5 darstellt,
R⁴ ein Wasserstoffatom darstellt; und
X und Y unabhängig ein Sauerstoff- oder Schwefelatom darstellen;
oder ein pharmazeutisch annehmbares Salz davon.

2. Verbindung gemäss Anspruch 1, worin:
R¹ darstellt: C₁₋₅-Alkyl; C₃₋₈-Cycloalkyl; C₂₋₅-Alkenyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₂₋₅-Alkinyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₂₋₅-Alkylcarbonyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₇₋₁₃-Arylcarbonyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₈₋₁₄-Arylcarbonylalkyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₆₋₁₂-Arylthio, gegebenenfalls substituiert durch ein oder mehrere Substituenten, ausgewählt aus einem Halogenatom, Phenyl, Cyano, C₂₋₆-Alkoxycarbonyl und Carbamoylgruppen; oder eine C₇₋₁₇-Aralkylgruppe, gegebenenfalls substituiert durch ein oder mehrere Substituenten, ausgewählt aus einem Halogenatom, Phenyl, Cyano, C₂₋₆-Alkoxycarbonyl und Carbamoylgruppen,
R² darstellt: C₆₋₁₀-Arylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₁₋₅-Alkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₃₋₁₀-Cycloalkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₆₋₁₂-Arylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₁₋₅-Alkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₃₋₁₀-Cycloalkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₂₋₅-Alkenyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₂₋₅-Alkinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₇₋₁₁-Aralkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₇₋₁₃-Arylcarbonyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₈₋₁₄-Arylcarbonylalkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; oder C₆₋₁₂-Aryloxy, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen.

3. Verbindung gemäss Anspruch 2, worin:
R¹ darstellt: C₁₋₅-Alkyl; C₃₋₈-Cycloalkyl; C₂₋₅-Alkenyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₂₋₅-Alkinyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₂₋₅-Alkylcarbonyl; C₇₋₁₃-Arylcarbonyl; C₈₋₁₄-Arylcarbonylalkyl; C₆₋₁₂-Arylthio; oder C₇₋₁₇-Aralkylgruppen,
R² darstellt: C₆₋₁₀-Arylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₁₋₅-Alkylthio; C₃₋₁₀-Cycloalkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₆₋₁₂-Arylsulfinyl; C₁₋₅-Alkylsulfinyl; C₃₋₁₀-Cycloalkylsulfinyl; C₂₋₅-Alkenyl; C₂₋₅-Alkinyl; C₇₋₁₁-Aralkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₇₋₁₃-Arylcarbonyl; C₈₋₁₄-Arylcarbonylalkyl; oder eine C₆₋₁₂-Aryloxygruppe,
R³ darstellt: ein Wasserstoffatom; Methyl; oder eine -CH₂-Z-(CH₂)ₙ-R⁵-Gruppe, worin R⁵ darstellt: ein Wasserstoffatom, Halogenatom, 5- oder 6-gliedriges heterocyclisches Carbonyloxy, Formyloxy, C₂₋₁₁-Alkylcarbonyloxy, C₄₋₁₁-Cycloalkylcarbonyloxy, C₈₋₁₂-Aralkylcarbonyloxy, C₇₋₁₃-Arylcarbonyloxy, Azido, C₂₋₁₁-Alkoxycarbonyloxy, C₂₋₈-N-Alkylcarbamoyloxy, C₇₋₁₃-N-Arylcarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₂₋₈-N-Alkylthiocarbamoyloxy, C₇₋₁₃-N-Arylthiocarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₁₋₁₀-Alkoxy, C₇₋₁₃-Aralkyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, verzweigtes C₃₋₅-Alkyl, C₅₋₇-Cycloalkyl, oder C₆₋₁₀-Arylgruppen, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, Z Sauerstoff, ein Schwefelatom oder eine Methylengruppe darstellt, und n 0 oder eine ganze Zahl von 1 bis 5 darstellt.

4. Verbindung gemäss Anspruch 3, worin:
R¹ darstellt: C₁₋₅-Alkyl; C₃₋₈-Cycloalkyl; oder C₂₋₅-Alkenyl, gegebenenfalls substituiert durch ein oder mehrere Substituenten, ausgewählt aus einem Halogenatom, Phenyl, Cyano, C₂₋₆-Alkoxycarbonyl und Carbamoylgruppen,
R² darstellt: C₆₋₁₀-Arylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₃₋₁₀-Cycloalkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; oder C₇₋₁₁-Aralkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen,
R³ darstellt: ein Wasserstoffatom; Methyl; oder eine -CH₂-Z-(CH₂)ₙ-R⁵-Gruppe, worin R⁵ darstellt: ein Wasserstoffatom, Halogenatom, 5- oder 6-gliedriges heterocyclisches Carbonyloxy, Formyloxy, C₂₋₁₁-Alkylcarbonyloxy, C₄₋₁₁-Cycloalkylcarbonyloxy, C₈₋₁₂-Aralkylcarbonyloxy, C₇₋₁₃-Arylcarbonyloxy, Azido, C₂₋₁₁-Alkoxycarbonyloxy, C₂₋₈-N-Alkylcarbamoyloxy, C₇₋₁₃-N-Arylcarbamoyloxy, C₂₋₈-N-Alkylthiocarbamoyloxy, C₇₋₁₃-N-Arylthiocarbamoyloxy, C₁₋₁₀-Alkoxy, C₇₋₁₃-Aralkyloxy, verzweigtes C₃₋₅-Alkyl, C₅₋₇-Cycloalkyl, oder eine C₆₋₁₀-Arylgruppe, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, Z Sauerstoff, ein Schwefelatom oder eine Methylengruppe darstellt, und n 0 oder eine ganze Zahl von 1 bis 5 darstellt.

5. Antivirales Mittel, das als Wirkstoff ein 6-substituiertes Acyclopyrimidinnukleosidderivat oder ein pharmazeutisch annehmbares Salz davon gemäss einem der Ansprüche 1 bis 4 enthält.

6. Pharmazeutische Zusammensetzung, die eine Verbindung gemäss einem der Ansprüche 1 bis 4 oder ein pharmazeutisch annehmbares Salz davon in Verbindung mit einem pharmazeutischen Träger enthält.

7. Pharmazeutische Zusammensetzung gemäss Anspruch 6, das eine effektive antivirale Wirkung, insbesondere antiretrovirale Wirkung, aufweist.

8. Verwendung eines 6-substituierten Acyclopyrimidinnucleosidderivats, dargestellt durch die folgende allgemeine Formel (I) 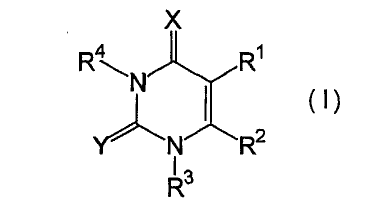 worin:
R¹ darstellt: ein Wasserstoff; Halogenatom; C₁₋₁₀-Alkyl; C₃₋₁₀-Cycloalkyl; C₂₋₅-Alkenyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₂₋₅-Alkinyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₂₋₅-Alkylcarbonyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₇₋₁₃-Arylcarbonyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₈₋₁₄-Arylcarbonylalkyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₆₋₁₂-Arylthio, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; oder C₇₋₁₇-Aralkylgruppen, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen,
R² darstellt: C₆₋₁₂-Arylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₁₋₁₀-Alkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₃₋₁₀-Cycloalkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₆₋₁₂-Arylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₁₋₁₀-Alkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₃₋₁₀-Cycloalkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₂₋₅-Alkenyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₂₋₅-Alkinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₇₋₁₇-Aralkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₇₋₁₃-Arylcarbonyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₈₋₁₄-Arylcarbonylalkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; oder C₆₋₁₂-Aryloxygruppen, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen,
R³ darstellt: ein Wasserstoffatom; Methyl; verzweigtes C₃₋₁₀-Alkyl; oder eine -CH₂-Z-(CH₂)ₙ-R⁵-Gruppe, worin R⁵ darstellt: ein Wasserstoffatom, ein Halogenatom, 5- oder 6-gliedriges heterocyclisches Carbonyloxy, Formyloxy, C₂₋₁₁-Alkylcarbonyloxy, C₄₋₁₁-Oycloalkylcarbonyloxy, C₈₋₁₂-Aralkylcarbonyloxy, C₇₋₁₃-Arylcarbonyloxy, Azido, C₂₋₁₁-Alkoxycarbonyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₂₋₁₁-N-Alkylcarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-N-Arylcarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₂₋₁₁-N-Alkylthiocarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-N-Arylthiocarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₁₋₁₀-Alkoxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-Aralkyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, verzweigtem C₃₋₁₀-Alkyl, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₃₋₁₀-Cycloalkyl, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, oder C₆₋₁₂-Arylgruppen, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, Z Sauerstoff, ein Schwefelatom oder eine Methylengruppe darstellt, und n 0 oder eine ganze Zahl von 1 bis 5 darstellt,
R⁴ ein Wasserstoffatom, C₁₋₁₃-Alkyl oder eine C₇₋₁₃-Aralkylgruppe darstellt; und
X und Y unabhängig ein Sauerstoff- oder Schwefelatom darstellen;
oder ein pharmazeutisch annehmbares Salz davon,
in einem Verfahren zur Herstellung einer pharmazeutischen Zusammensetzung zur Behandlung oder Prävention von Erkrankungen, die durch virale, insbesondere retrivorale Infektion, insbesondere bei Menschen, verursacht werden.

9. Verwendung gemäss Anspruch 8, worin:
R¹ darstellt: ein Wasserstoffatom; Halogenatom; C₁₋₅-Alkyl; C₃₋₈-Cycloalkyl; C₂₋₅-Alkenyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₂₋₅-Alkinyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₂₋₅-Alkylcarbonyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₇₋₁₃-Arylcarbonyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₈₋₁₄-Arylcarbonylalkyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₆₋₁₂-Arylthio, gegebenenfalls substituiert durch ein oder mehrere Substituenten, ausgewählt aus einem Halogenatom, Phenyl, Cyano, C₂₋₆-Alkoxycarbonyl und Carbamoylgruppen; oder eine C₇₋₁₇-Aralkylgruppe, gegebenenfalls substituiert durch ein oder mehrere Substituenten, ausgewählt aus einem Halogenatom, Phenyl, Cyano, C₂₋₆-Alkoxycarbonyl und Carbamoylgruppen,
R² darstellt: C₆₋₁₀-Arylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₁₋₅-Alkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₃₋₁₀-Cycloalkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₆₋₁₂-Arylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₁₋₅-Alkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₃₋₁₀-Cycloalkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₂₋₅-Alkenyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₂₋₅-Alkinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₇₋₁₁-Aralkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₇₋₁₃-Arylcarbonyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₈₋₁₄-Arylcarbonylalkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; oder C₆₋₁₂-Aryloxy, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen.

10. Verwendung gemäss Anspruch 9, worin:
R¹ darstellt: ein Wasserstoffatom; Halogenatom; C₁₋₅-Alkyl; C₃₋₈-Cycloalkyl; C₂₋₅-Alkenyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₂₋₅-Alkinyl, gegebenenfalls substituiert durch ein oder mehrere Substituenten, ausgewählt aus einem Halogenatom, Phenyl, Cyano, C₂₋₆-Alkoxycarbonyl und Carbamoylgruppen; C₂₋₅-Alkylcarbonyl; C₇₋₁₃-Arylcarbonyl; C₈₋₁₄-Arylcarbonylalkyl; C₆₋₁₂-Arylthio; oder C₇₋₁₇-Aralkylgruppen,
R² darstellt: C₆₋₁₀-Arylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₁₋₅-Alkylthio; C₃₋₁₀-Cycloalkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₆₋₁₂-Arylsulfinyl; C₁₋₅-Alkylsulfinyl; C₃₋₁₀-Cycloalkylsulfinyl; C₂₋₅-Alkenyl; C₂₋₅-Alkinyl; C₇₋₁₁-Aralkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₇₋₁₃-Arylcarbonyl; C₈₋₁₄-Arylcarbonylalkyl; oder eine C₆₋₁₂-Aryloxygruppe,
R³ darstellt: ein Wasserstoffatom; Methyl; oder eine -CH₂-Z-(CH₂)ₙ-R⁵-Gruppe, worin R⁵ darstellt: ein Wasserstoffatom, Halogenatom, 5- oder 6-gliedriges heterocyclisches Carbonyloxy, Formyloxy, C₂₋₁₁-Alkylcarbonyloxy, C₄₋₁₁-Cycloalkylcarbonyloxy, C₈₋₁₂-Aralkylcarbonyloxy, C₇₋₁₃-Arylcarbonyloxy, Azido, C₂₋₁₁-Alkoxycarbonyloxy, C₂₋₈-N-Alkylcarbamoyloxy, C₇₋₁₃-N-Arylcarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₂₋₈-N-Alkylthiocarbamoyloxy, C₇₋₁₃-N-Arylthiocarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₁₋₁₀-Alkoxy, C₇₋₁₃-Aralkyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, verzweigtes C₃₋₅-Alkyl, C₅₋₇-Cycloalkyl, oder C₆₋₁₀-Arylgruppen, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, Z Sauerstoff, ein Schwefelatom oder eine Methylengruppe darstellt, und n 0 oder eine ganze Zahl von 1 bis 5 darstellt.

11. Verwendung gemäss Anspruch 10, worin:
R¹ darstellt: ein Wasserstoffatom; Halogenatom; C₁₋₅-Alkyl; C₃₋₈-Cycloalkyl; oder C₂₋₅-Alkenyl, gegebenenfalls substituiert durch ein oder mehrere Substituenten, ausgewählt aus einem Halogenatom, Phenyl, Cyano, C₂₋₆-Alkoxycarbonyl und Carbamoylgruppen,
R² darstellt: C₆₋₁₀-Arylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; C₃₋₁₀-Cycloalkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen; oder C₇₋₁₁-Aralkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, Phenyl, Hydroxy, Nitro, Amino, Cyano und C₂₋₇-Acylgruppen,
R³ darstellt: ein Wasserstoffatom; Methyl; oder eine -CH₂-Z-(CH₂)ₙ-R⁵-Gruppe, worin R⁵ darstellt: ein Wasserstoffatom, Halogenatom, 5- oder 6-gliedriges heterocyclisches Carbonyloxy, Formyloxy, C₂₋₁₁-Alkylcarbonyloxy, C₄₋₁₁-Cycloalkylcarbonyloxy, C₈₋₁₂-Aralkylcarbonyloxy, C₇₋₁₃-Arylcarbonyloxy, Azido, C₂₋₁₁-Alkoxycarbonyloxy, C₂₋₈-N-Alkylcarbamoyloxy, C₇₋₁₃-N-Arylcarbamoyloxy, C₂₋₈-N-Alkylthiocarbamoyloxy, C₇₋₁₃-N-Arylthiocarbamoyloxy, C₁₋₁₀-Alkoxy, C₇₋₁₃-Aralkyloxy, verzweigtes C₃₋₅-Alkyl, C₅₋₇-Cycloalkyl, oder eine C₆₋₁₀-Arylgruppe, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, Z Sauerstoff, ein Schwefelatom oder eine Methylengruppe darstellt, und n 0 oder eine ganze Zahl von 1 bis 5 darstellt.

12. Verfahren zur Herstellung einer Verbindung, dargestellt durch die folgende allgemeine Formel (I): 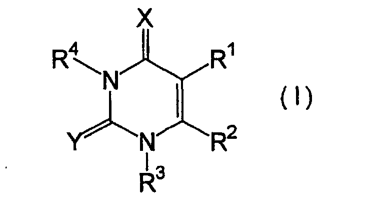 worin:
R¹ darstellt: C₁₋₁₀-Alkyl; C₃₋₁₀-Cycloalkyl; C₂₋₅-Alkenyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₂₋₅-Alkinyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₂₋₅-Alkylcarbonyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₇₋₁₃-Arylcarbonyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₈₋₁₄-Arylcarbonylalkyl, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; C₆₋₁₂-Arylthio, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen; oder C₇₋₁₇-Aralkylgruppen, gegebenenfalls substituiert durch ein oder mehrere Substituenten, ausgewählt aus einem Halogenatom, C₆₋₁₂-Aryl, Cyano, C₂₋₆-Alkoxycarbonyl, Carbamoyl, C₁₋₅-Alkoxy, Amino und Nitrogruppen,
R² darstellt: C₆₋₁₂-Arylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₁₋₁₀-Alkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₃₋₁₀-Cycloalkylthio, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₆₋₁₂-Arylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₁₋₁₀-Alkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₃₋₁₀-Cycloalkylsulfinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₂₋₅-Alkenyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₂₋₅-Alkinyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₇₋₁₇-Aralkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₇₋₁₃-Arylcarbonyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; C₈₋₁₄-Arylcarbonylalkyl, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen; oder C₆₋₁₂-Aryloxygruppen, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen,
R³ darstellt: Methyl; verzweigtes C₃₋₁₀-Alkyl; oder eine -CH₂-Z-(CH₂)ₙ-R⁵-Gruppe, worin R⁵ darstellt: ein Wasserstoffatom, ein Halogenatom, Azido, C₁₋₁₀-Alkoxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-Aralkyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, verzweigtem C₃₋₁₀-Alkyl, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₃₋₁₀-Cycloalkyl, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, oder C₆₋₁₂-Arylgruppen, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, Z Sauerstoff, ein Schwefelatom oder eine Methylengruppe darstellt, und n 0 oder eine ganze Zahl von 1 bis 5 darstellt,
R⁴ ein Wasserstoffatom darstellt; und
X und Y unabhängig ein Sauerstoff- oder Schwefelatom darstellen,
das umfasst: Umsetzen einer Verbindung, dargestellt durch die folgende allgemeine Formel (II) 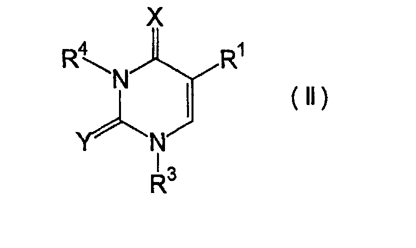 worin R¹, R³, R⁴, X und Y die gleichen Bedeutungen wie oben definiert haben, mit einer organischen Alkalimetallverbindung zum Erhalt einer Verbindung, dargestellt durch die folgende allgemeinen Formel (II'): 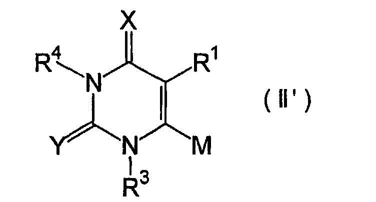 worin R¹, R³, R⁴, X und Y die gleichen Bedeutungen wie oben definiert haben, und M ein Alkalimetall darstellt, und dann Umsetzen der erhaltenen Verbindung der Formel (II') mit einer Verbindung, dargestellt durch die folgende allgemeine Formel (III):
R²X¹ (III)
worin R² die gleiche Bedeutung wie oben definiert hat, und X¹ ein Halogenatom, Arylthio oder eine Alkoxygruppe darstellt, zum Erhalt der Verbindung der obigen Formel (I).

13. Verfahren zur Herstellung einer Verbindung, dargestellt durch die folgende allgemeine Formel (I): 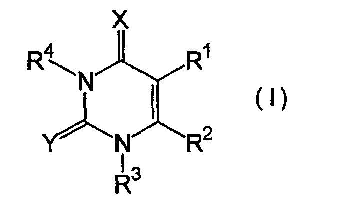 worin R¹, R², R³, R⁴, X und Y die gleichen Bedeutungen wie in Anspruch 1 definiert haben, das umfasst: Umsetzen einer Verbindung, dargestellt durch die folgende allgemeine Formel (IV): 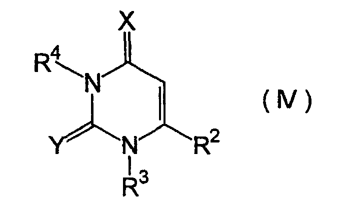 worin R², R³, R⁴, X und Y die gleichen Bedeutungen wie in Anspruch 12 definiert haben, mit einer organischen Alkalimetallverbindung zum Erhalt einer Verbindung, dargestellt durch die folgende allgemeine Formel (IV'): 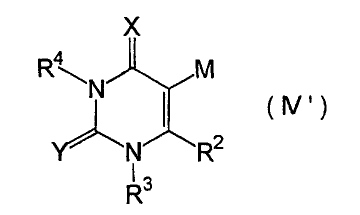 worin R², R³, R⁴, X und Y die gleichen Bedeutungen wie in Anspruch 12 definiert haben und M ein Alkalimetall darstellt, und dann Umsetzen der erhaltenen Verbindung der Formel (IV') mit einer Verbindung, dargestellt durch die folgende allgemeine Formel (V):
R¹X² (V)
worin R¹ die gleiche Bedeutung wie in Anspruch 12 definiert hat und X² ein Halogenatom, Arylthio oder eine Alkoxygruppe darstellt, zum Erhalt der Verbindung der obigen Formel (I).

14. Verfahren zur Herstellung einer Verbindung, dargestellt durch die folgende allgemeine Formel (I): 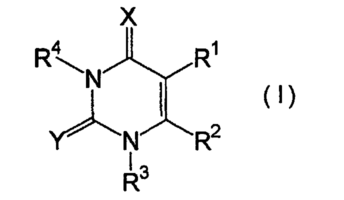 worin R¹, R², R³, R⁴, X und Y die gleichen Bedeutungen wie in Anspruch 12 definiert haben, das umfasst: Umsetzen einer Verbindung, dargestellt durch die folgende allgemeine Formel (VI): 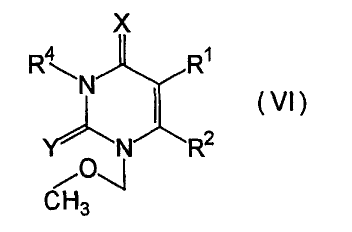 worin R¹, R², R⁴, X und Y die gleichen Bedeutungen wie in Anspruch 12 definiert haben, mit einer Säure zum Erhalt einer Verbindung, dargestellt durch die folgende allgemeine Formel (VII): 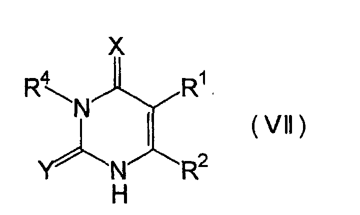 worin R¹, R², R⁴, X und Y die gleichen Bedeutungen wie in Anspruch 12 definiert haben, und dann Umsetzen der erhaltenen Verbindung der Formel (VII) mit einer Verbindung, dargestellt durch die folgende allgemeine Formel (VIII):
R³X³ (VIII)
worin R³ die gleiche Bedeutung wie in Anspruch 12 definiert hat und X³ eine Sulfonyloxygruppe darstellt, in Gegenwart einer Base zum Erhalt der Verbindung der obigen Formel (I).

15. Verfahren zur Herstellung einer Verbindung, dargestellt durch die folgende allgemeine Formel (X): 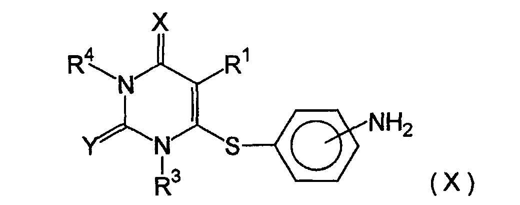 worin R¹, R⁴, X und Y die gleichen Bedeutungen wie in Anspruch 12 definiert haben und R³ darstellt: ein Wasserstoffatom; Methyl; verzweigtes C₃₋₁₀-Alkyl; oder eine -CH₂-Z-(CH₂)ₙ-R⁵-Gruppe, worin R⁵ darstellt: ein Wasserstoffatom, Halogenatom, 5- oder 6-gliedriges heterocyclisches Carbonyloxy, Formyloxy, C₂₋₁₁-Alkylcarbonyloxy, C₄₋₁₁-Cycloalkylcarbonyloxy, C₈₋₁₂-Aralkylcarbonyloxy, C₇₋₁₃-Arylcarbonyloxy, Azido, C₂₋₁₁-Alkoxycarbonyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₂₋₁₁-N-Alkylcarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-N-Arylcarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₂₋₁₁-N-Alkylthiocarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-N-Arylthiocarbamoyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₁₋₁₀-Alkoxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₇₋₁₃-Aralkyloxy, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, verzweigtes C₃₋₁₀-Alkyl, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, C₃₋₁₀-Cycloalkyl, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, oder C₆₋₁₂-Arylgruppen, gegebenenfalls substituiert durch ein oder mehrere Substituenten, ausgewählt aus einem Halogenatom, C₆₋₁₂-Aryl, C₁₋₅-Alkyl, C₁₋₅-Alkoxy und halogenierten C₁₋₅-Alkylgruppen, Z Sauerstoff, ein Schwefelatom oder eine Methylengruppe darstellt und n 0 oder eine ganze Zahl von 1 bis 5 darstellt, das umfasst: Hydrieren einer Verbindung, dargestellt durch die folgende allgemeine Formel (IX): 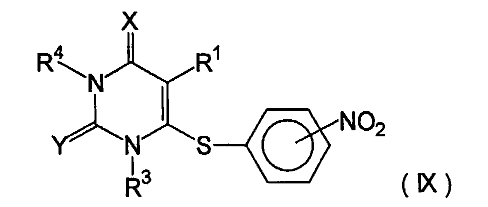 worin R¹, R⁴, X und Y die gleichen Bedeutungen wie in Anspruch 12 definiert haben und R³ die gleiche Bedeutung wie oben definiert hat, in Gegenwart eines Katalysators, zum Erhalt der Verbindung der obigen Formel (X).

16. Verfahren zur Herstellung einer Verbindung, dargestellt durch die folgende allgemeine Formel (XII): 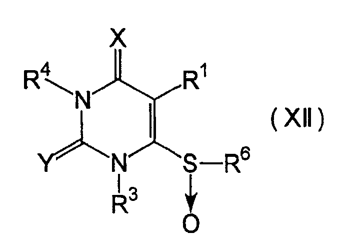 worin R¹, R³, R⁴, X und Y die gleichen Bedeutungen wie in Anspruch 15 definiert haben und R⁶ C₆₋₁₂-Aryl, C₁₋₁₀-Alkyl oder eine C₃₋₁₀-Cycloalkylgruppe darstellt, das umfasst: Umwandeln einer Verbindung, dargestellt durch die folgende allgemeine Formel (XI): 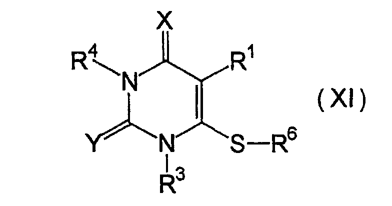 worin R¹, R³, R⁴, X und Y die gleichen Bedeutungen wie in Anspruch 2 definiert haben und R⁶ die gleiche Bedeutung wie oben definiert hat, in die Verbindung der obigen Formel (XII) durch ein Oxidationsmittel.

17. Verfahren zur Herstellung einer Verbindung, dargestellt durch die folgende allgemeine Formel (XV): 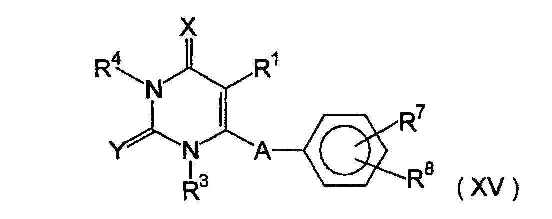 worin R¹, R³, R⁴, x und Y die gleichen Bedeutungen wie in Anspruch 15 definiert haben, R⁷ und R⁸ unabhängig ein Halogenatom, C₁₋₅-Alkyl, halogeniertes C₁₋₅-Alkyl, Hydroxy, Nitro, Amino, Cyano oder eine C₂₋₇-Acylgruppe darstellen und A ein Schwefel- oder Sauerstoffatom darstellt, das umfasst: Umsetzen einer Verbindung, dargestellt durch die folgende allgemeine Formel (XIII): 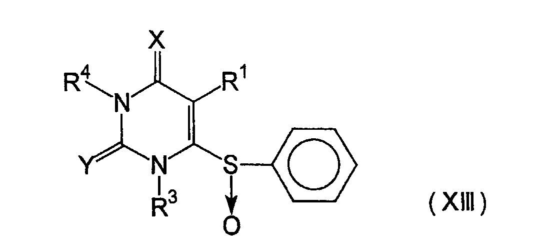 worin R¹, R³, R⁴, X und Y die gleichen Bedeutungen wie in Anspruch 15 definiert haben, mit einer Verbindung, dargestellt durch die folgende allgemeine Formel (XIV): 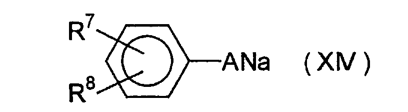 worin R¹, R³, R⁴, X und Y die gleichen Bedeutungen wie oben definiert haben, zum Erhalt der Verbindung der obigen Formel (XV).

18. Verfahren zur Herstellung einer Verbindung, dargestellt durch die folgende allgemeine Formel (XVIII): 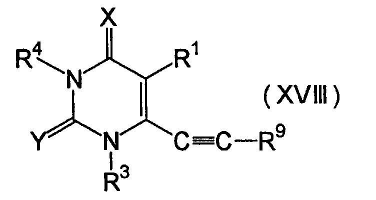 worin R¹, R³, R⁴, X und Y die gleichen Bedeutungen wie in Anspruch 15 definiert haben und R⁹ C₁₋₅-Alkyl, C₆₋₁₂-Aryl oder eine Silylgruppe darstellt, das umfasst: Umsetzen einer Verbindung, dargestellt durch die folgende allgemeine Formel (XVI): 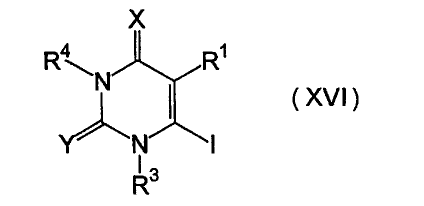 worin R¹, R³, R⁴, X und Y die gleichen Bedeutungen wie in Anspruch 15 definiert haben, mit einer Verbindung, dargestellt durch die folgende allgemeine Formel (XVII):
HC≡C-R⁹ (XVII)
worin R⁹ die gleiche Bedeutung wie oben definiert hat, in Gegenwart eines Palladiumkatalysators zum Erhalt der Verbindung der obigen Formel (XVIII).

19. Verfahren zur Herstellung einer Verbindung, dargestellt durch die folgende allgemeine Formel (XX): 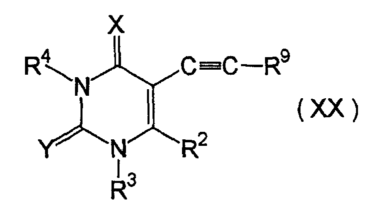 worin R², R³, R⁴, X und Y die gleichen Bedeutungen wie in Anspruch 15 definiert haben und R⁹ C₁₋₅-Alkyl, C₆₋₁₂-Aryl oder eine Silylgruppe darstellt, das umfasst: Umsetzen einer Verbindung, dargestellt durch die folgende allgemeine Formel (XIX): 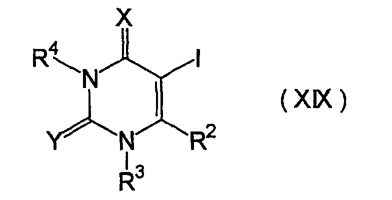 worin R², R³, R⁴, X und Y die gleichen Bedeutungen wie in Anspruch 15 definiert haben, mit einer Verbindung, dargestellt durch die folgende allgemeine Formel (XVII):
HC≡C―R⁹ (XVII)
worin R⁹ die gleiche Bedeutung wie oben definiert hat, in Gegenwart eines Palladiumkatalysators zum Erhalt der Verbindung der obigen Formel (XX).

20. Verfahren zur Herstellung einer Verbindung, dargestellt durch die folgende allgemeine Formel (XXII): 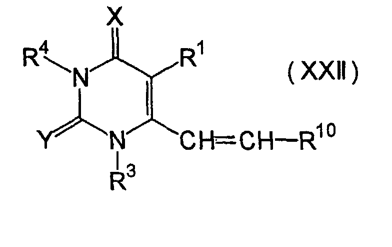 worin R¹, R³, R⁴, X und Y die gleichen Bedeutungen wie in Anspruch 15 definiert haben und R¹⁰ C₂₋₆-Alkoxycarbonyl, Nitril oder eine Carbamoylgruppe darstellt, das umfasst: Umsetzen einer Verbindung, dargestellt durch die folgende allgemeine Formel (XVI): 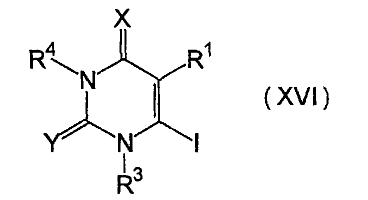 worin R¹, R³, R⁴, X und Y die gleichen Bedeutungen wie in Anspruch 15 definiert haben, mit einer Verbindung, dargestellt durch die folgende allgemeine Formel (XXI):
H₂C=CH―R¹⁰ (XXI)
worin R¹⁰ die gleiche Bedeutung wie oben definiert hat, in Gegenwart eines Palladiumkatalysators zum Erhalt der Verbindung der obigen Formel (XXII).

21. Verfahren zur Herstellung einer Verbindung, dargestellt durch die folgende allgemeine Formel (XXIII): 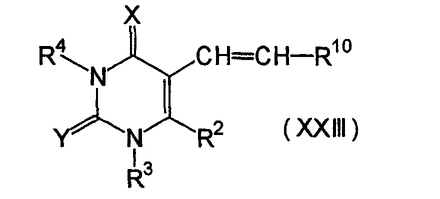 worin R², R³, R⁴, X und Y die gleichen Bedeutungen wie in Anspruch 15 definiert haben und R¹⁰ die gleiche Bedeutung wie in Anspruch 20 definiert hat, das umfasst: Umsetzen einer Verbindung, dargestellt durch die folgende allgemeine Formel (XIX): 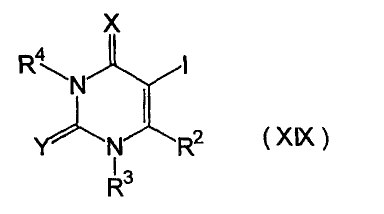 worin R², R³, R⁴, X und Y die gleichen Bedeutungen wie in Anspruch 15 definiert haben, mit einer Verbindung, dargestellt durch die folgende allgemeine Formel (XXI):
H₂C=CH―R¹⁰ (XXI)
worin R¹⁰ die gleiche Bedeutung wie in Anspruch 20 definiert hat, in Gegenwart eines Palladiumkatalysators zum Erhalt der Verbindung der obigen Formel (XXIII).

22. Verfahren zur Herstellung einer Verbindung, dargestellt durch die folgende allgemeine Formel (XXV): 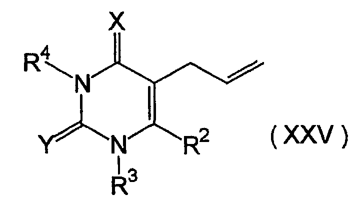 worin R², R³, R⁴, X und Y die gleichen Bedeutungen wie in Anspruch 15 definiert haben, das umfasst: Umsetzen einer Verbindung, dargestellt durch die folgende allgemeine Formel (IV): 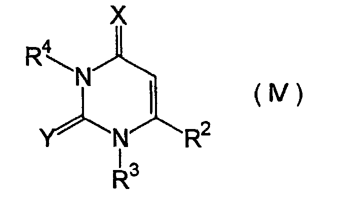 worin R², R³, R⁴, X und Y die gleichen Bedeutungen wie in Anspruch 15 definiert haben, mit einer Verbindung, dargestellt durch die folgende allgemeine Formel (XXIV):
X⁴―CH₂CH=CH₂ (XXIV)
worin X⁴ ein Halogenatom darstellt, in Gegenwart eines Palladiumkatalysators zum Erhalt der Verbindung der obigen Formel (XXV).

23. Verfahren zur Herstellung einer Verbindung, dargestellt durch die folgende allgemeine Formel (XXVIII): 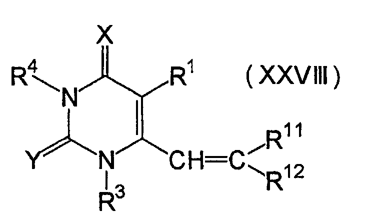 worin R¹, R³, R⁴, X und Y die gleichen Bedeutungen wie in Anspruch 12 definiert haben und R¹¹ und R¹² unabhängig ein Wasserstoffatom, C₁₋₃-Alkyl oder eine C₆₋₁₂-Arylgruppe darstellen, das umfasst: Umsetzen einer Verbindung, dargestellt durch die folgende allgemeine Formel (II): 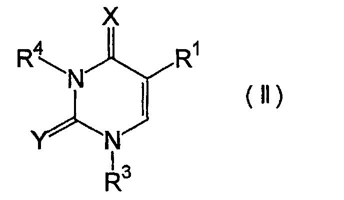 worin R¹, R³, R⁴, X und Y die gleichen Bedeutungen wie in Anspruch 12 definiert haben, mit einer organischen Alkalimetallverbindung zum Erhalt einer Verbindung, dargestellt durch die folgende allgemeine Formel (II'): 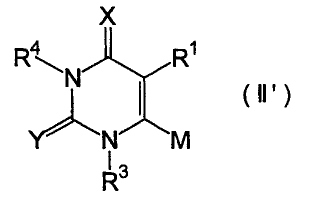 worin R¹, R³, R⁴, X und Y die gleichen Bedeutungen wie in Anspruch 12 definiert haben und M ein Alkalimetall darstellt, Umsetzen der erhaltenen Verbindung der Formel (II') mit einer Verbindung, dargestellt durch die folgende allgemeine Formel (XXVI): 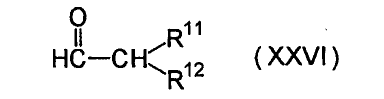 worin R¹¹ und R¹² die gleichen Bedeutungen wie oben definiert haben, zum Erhalt einer Verbindung, dargestellt durch die folgende allgemeine Formel (XXVII): 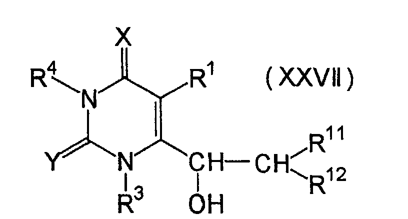 worin R¹, R³, R⁴, X und Y die gleichen Bedeutungen wie in Anspruch 12 definiert haben und R¹¹ und R¹² die gleichen Bedeutungen wie oben definiert haben, und dann Dehydrieren der erhaltenen Verbindung der Formel (XXVII) unter Verwendung eines Dehydrierungsmittels zum Erhalt der Verbindung der obigen Formel (XXVIII).

24. Verfahren zur Herstellung einer Verbindung, dargestellt durch die folgende allgemeine Formel (XXX): 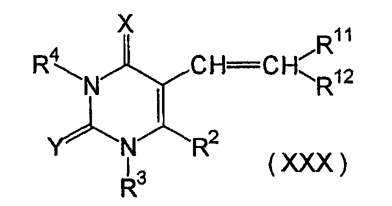 worin R², R³, R⁴, X und Y die gleichen Bedeutungen wie in Anspruch 15 haben und R¹¹ und R¹² unabhängig ein Wasserstoffatom, C₁₋₃-Alkyl oder eine C₆₋₁₂-Arylgruppe darstellen, das umfasst: Umsetzen einer Verbindung, dargestellt durch die allgemeine Formel (IV): 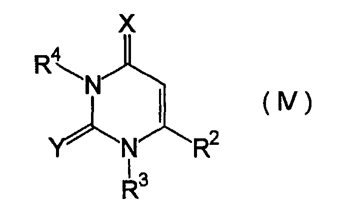 worin R², R³, R⁴, X und Y die gleichen Bedeutungen wie in Anspruch 15 definiert haben, mit einer organischen Alkalimetallverbindung zum Erhalt einer Verbindung, dargestellt durch die folgende allgemeine Formel (IV') : 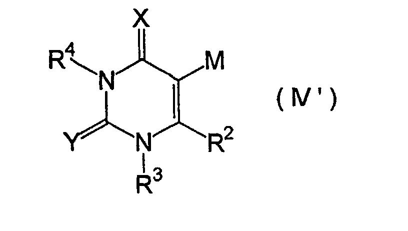 worin R², R³, R⁴, X und Y die gleichen Bedeutungen wie in Anspruch 12 definiert haben und M ein Alkalimetall darstellt, Umsetzen der erhaltenen Verbindung der Formel (IV') mit einer Verbindung, dargestellt durch die folgende allgemeine Formel (XXVI): 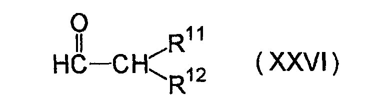 worin R¹¹ und R¹² die gleichen Bedeutungen wie oben definiert haben, zum Erhalt einer Verbindung, dargestellt durch die folgende allgemeine Formel (XXIX): 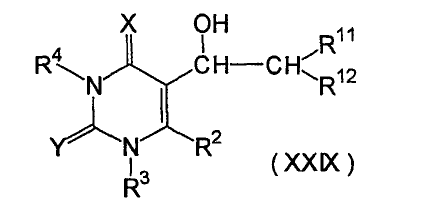 worin R², R³, R⁴, X und Y die gleichen Bedeutungen wie in Anspruch 15 definiert haben und R¹¹ und R¹² die gleichen Bedeutungen wie oben definiert haben, und dann Dehydratisieren der erhaltenen Verbindung der Formel (XXIX) unter Verwendung eines Dehydratisierungsmittels zum Erhalt der Verbindung der obigen Formel (XXX).

25. Verfahren zur Herstellung einer Verbindung, dargestellt durch die folgende allgemeine Formel (XXXIII): 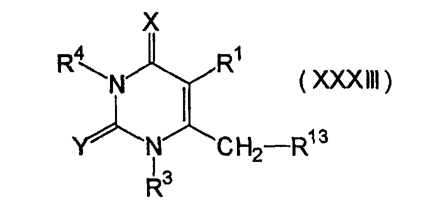 worin R¹, R³, R⁴, X und Y die gleichen Bedeutungen wie in Anspruch 12 definiert haben und R¹³ darstellt: eine C₆₋₁₂-Arylgruppe, gegebenenfalls substituiert durch ein oder mehrere Substituenten, ausgewählt aus einem Halogenatom, C₁₋₅-Alkyl, halogeniertem C₁₋₅-Alkyl, C₁₋₅-Alkoxy, C₆₋₁₂-Aryl, Hydroxy, Nitro, Amino, Cyano und C₂₋₇-Acylgruppen, das umfasst: Umsetzen einer Verbindung, dargestellt durch die folgende allgemeine Formel (II): 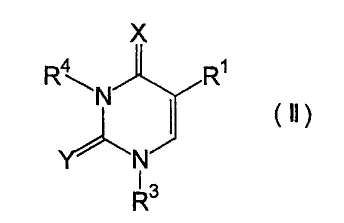 worin R¹, R³, R⁴, X und Y die gleichen Bedeutungen wie in Anspruch 12 definiert haben, mit einer organischen Alkalimetallverbindung zum Erhalt einer Verbindung, dargestellt durch die folgende allgemeine Formel (II'): 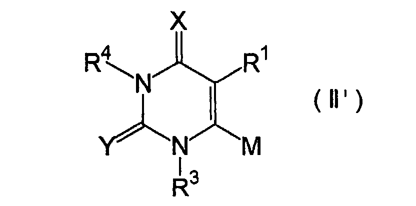 worin R¹, R³, R⁴, X und Y die gleichen Bedeutungen wie in Anspruch 12 definiert haben und M ein Alkalimetall darstellt, Umsetzen der erhaltenen Verbindung der Formel (II') mit einer Verbindung, dargestellt durch die folgende allgemeine Formel (XXXI): 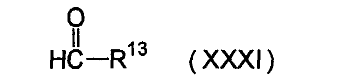 worin R¹³ die gleiche Bedeutung wie oben definiert hat, zum Erhalt einer Verbindung, dargestellt durch die folgende allgemeine Formel (XXXII): 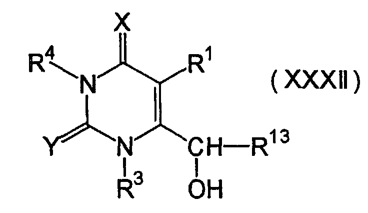 worin R¹, R³, R⁴, X und Y die gleichen Bedeutungen wie in Anspruch 12 definiert haben und R¹³ die gleiche Bedeutung wie oben definiert hat, und dann Reduzieren der erhaltenen Verbindung der Formel (XXXII) unter Verwendung eines Reduktionsmittels zum Erhalt der Verbindung der obigen Formel (XXXIII).

26. Verfahren zur Herstellung einer Verbindung, dargestellt durch die folgende allgemeine Formel (XXXVI): 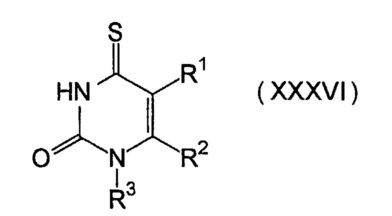 worin R¹, R² und R³ die gleichen Bedeutungen wie in Anspruch 15 definiert haben, das umfasst: Umsetzen einer Verbindung, dargestellt durch die folgende allgemeine Formel (XXXIV): 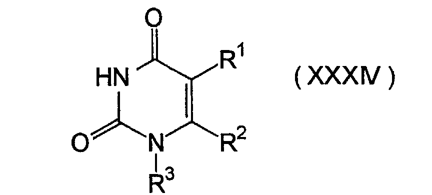 worin R¹, R² und R³ die gleichen Bedeutungen wie in Anspruch 15 definiert haben, mit einer Verbindung, dargestellt durch die folgende allgemeine Formel (XXXV): 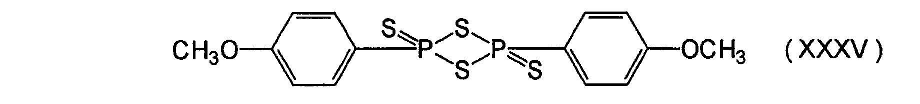 zum Erhalt der Verbindung der obigen Formel (XXXVI).

27. Verfahren zur Herstellung einer Verbindung, dargestellt durch die folgende allgemeine Formel (XXXX): 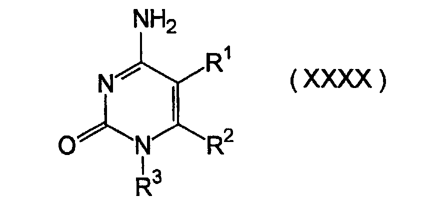 worin R¹, R² und R³ die gleichen Bedeutungen wie in Anspruch 15 definiert haben, das umfasst: Umsetzen einer Verbindung, dargestellt durch die folgende allgemeine Formel (XXXVII) : 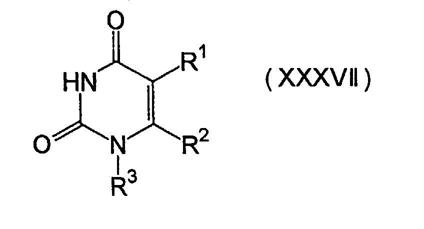 worin R¹, R² und R³ die gleichen Bedeutungen wie in Anspruch 15 definiert haben, mit einer Verbindung, dargestellt durch die folgende allgemeine Formel (XXXVIII): 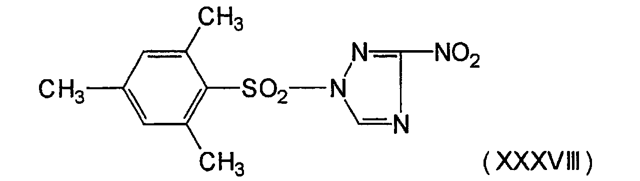 in einem Lösungsmittel in Gegenwart von Diphenylphosphorsäure zum Erhalt einer Verbindung der folgenden allgemeinen Formel (XXXIV): 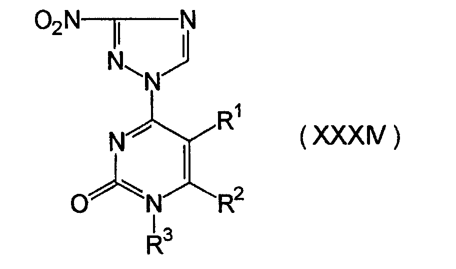 worin R¹, R² und R³ die gleichen Bedeutungen wie in Anspruch 15 definiert haben, und dann Umsetzen der erhaltenen Verbindung der Formel (XXXIV) mit wässrigem Ammoniak zum Erhalt der Verbindung der obigen Formel (XXXX).

28. Verfahren zur Herstellung einer Verbindung, dargestellt durch die folgende allgemeine Formel (I): 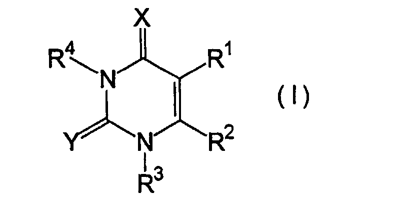 worin R¹, R², R³, x und Y die gleichen Bedeutungen wie in Anspruch 14 definiert haben und R⁴ die gleiche Bedeutung wie in Anspruch 12 definiert hat, das umfasst: Umsetzen einer Verbindung, dargestellt durch die folgende allgemeine Formel (XXXXI): 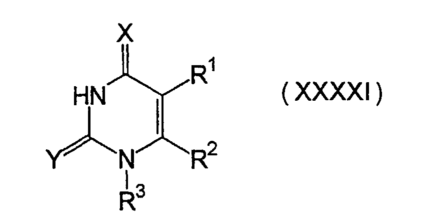 worin R¹, R², R³, X und Y die gleichen Bedeutungen wie in Anspruch 15 definiert haben, mit einer Verbindung, dargestellt durch die folgende allgemeine Formel (XXXXII) :
R⁴X⁵ (XXXXII)
worin R⁴ die gleichen Bedeutungen wie in Anspruch 1 definiert hat und X⁵ ein Halogenatom oder eine Sulfonyloxygruppe darstellt, in Gegenwart einer Base zum Erhalt der Verbindung der obigen Formel (I).

29. Verfahren zur Herstellung einer Verbindung, dargestellt durch die folgende allgemeine Formel (XXXXV): 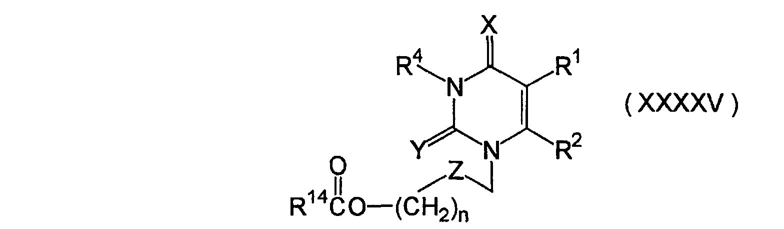 worin R¹, R², R⁴, X, Y, Z und n die gleichen Bedeutungen wie in Anspruch 12 definiert haben und R¹⁴ darstellt: C₁₋₁₀-Alkyl, C₃₋₁₀-Cycloalkyl, C₆₋₁₂-Aryl, C₇₋₁₁-Aralkyl oder eine 5- oder 6-gliedrige heterocyclische Gruppe, das umfasst: Umsetzen einer Verbindung, dargestellt durch die folgende allgemeine Formel (XXXXIII) : 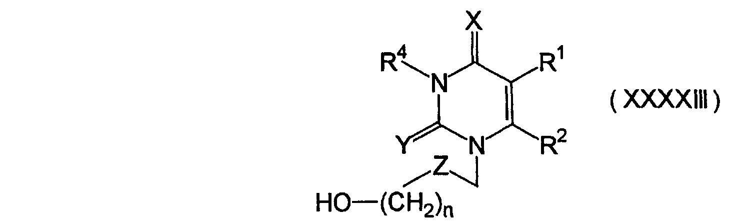 worin R¹, R², R⁴, X, Y, Z und n die gleichen Bedeutungen wie in Anspruch 12 definiert haben, mit einer Verbindung, dargestellt durch die folgende allgemeine Formel (XXXXIV): 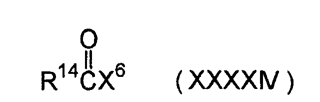 worin R¹⁴ die gleiche Bedeutung wie oben definiert hat, und X⁶ ein Halogenatom oder -OCOR¹⁴ darstellt, in einem Lösungsmittel in Gegenwart einer Base zum Erhalt der Verbindung der obigen Formel (XXXXV).

30. Verfahren zur Herstellung einer Verbindung, dargestellt durch die folgende allgemeine Formel (XXXXVII): 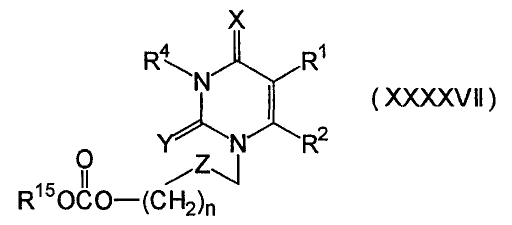 worin R¹, R², R⁴, X, Y, Z und n die gleichen Bedeutungen wie in Anspruch 12 definiert haben und R¹⁵ C₁₋₁₀-Alkyl darstellt, das umfasst: Umsetzen einer Verbindung, dargestellt durch die folgende allgemeine Formel (XXXXIII): 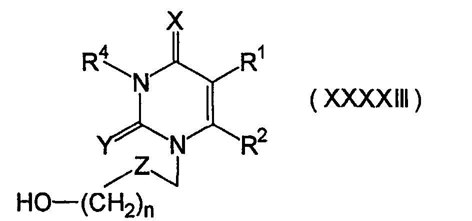 worin R¹, R², R⁴, X, Y, Z und n die gleichen Bedeutungen wie in Anspruch 12 definiert haben, mit einer Verbindung, dargestellt durch die folgende allgemeine Formel (XXXXVI): 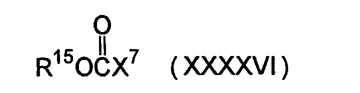 worin R¹⁵ die gleiche Bedeutung wie oben definiert hat und X⁷ ein Halogenatom oder -OCOOR¹⁵ darstellt, in einem Lösungsmittel in Gegenwart einer Base, zum Erhalt der Verbindung der obigen Formel (XXXXVII).

31. Verfahren zur Herstellung einer Verbindung, dargestellt durch die folgende allgemeine Formel (XXXXIX): 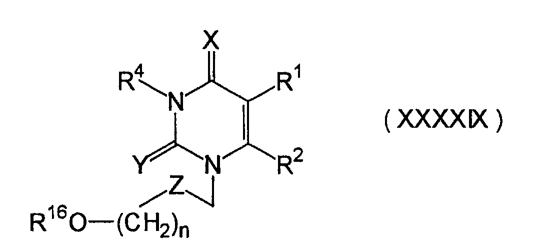 worin R¹, R², R⁴, X, Y, Z und n die gleichen Bedeutungen wie in Anspruch 12 definiert haben und R¹⁶ C₁₋₁₀-Alkyl, C₇₋₁₃-Aralkyl oder eine Formylgruppe darstellt, das umfasst: Umsetzen einer Verbindung, dargestellt durch die folgende allgemeine Formel (XXXXIII): 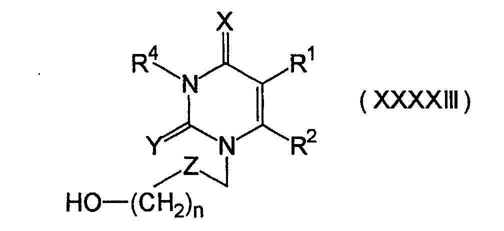 worin R¹, R², R⁴, X, Y, Z und n die gleichen Bedeutungen wie in Anspruch 12 definiert haben, mit einer Verbindung, dargestellt durch die folgende allgemeine Formel (XXXXVIII):
R¹⁶X⁸ (XXXXVIII)
worin R¹⁶ die gleiche Bedeutung wie oben definiert hat und X⁸ ein Halogenatom oder eine Sulfonyloxygruppe darstellt, in einem Lösungsmittel in Gegenwart einer Base zum Erhalt der Verbindung der obigen Formel (XXXXIX).

32. Verfahren zur Herstellung einer Verbindung, dargestellt durch die folgende allgemeine Formel (XXXXXI): 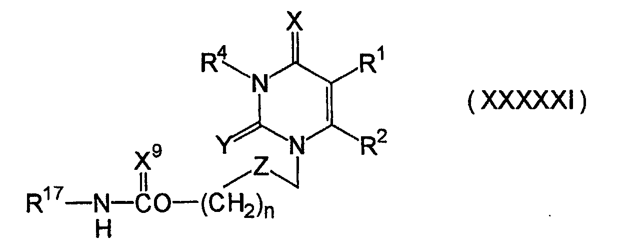 worin R¹, R², R⁴, X, Y, Z und n die gleichen Bedeutungen wie in Anspruch 12 definiert haben, R¹⁷ C₁₋₁₀-Alkyl oder eine C₆₋₁₂-Arylgruppe darstellt und X⁹ ein Schwefel- oder Sauerstoffatom darstellt, das umfasst: Umsetzen einer Verbindung, dargestellt durch die folgende allgemeine Formel (XXXXIII): 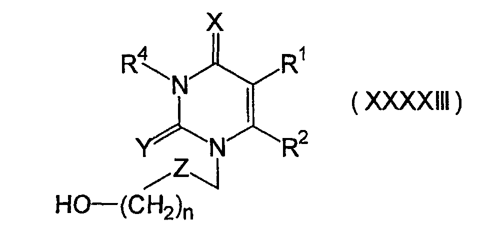 worin R¹, R², R⁴, X, Y, Z und n die gleichen Bedeutungen wie in Anspruch 12 definiert haben, mit einer Verbindung, dargestellt durch die folgende allgemeinen Formel (XXXXX):
R¹⁷―N=C=X⁹ (XXXXX)
worin R¹⁷ und X⁹ die gleichen Bedeutungen wie oben definiert haben, in einem Lösungsmittel in Gegenwart einer Base zum Erhalt der Verbindung der obigen Formel (XXXXXI).

## Revendications

1. Dérivé d'acyclopyrimidine nucléoside substitué en 6 représenté par la formule générale I suivante : 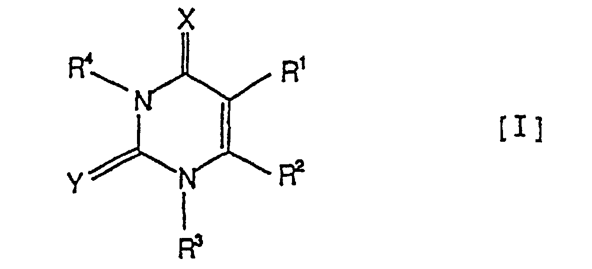 dans laquelle
R¹ représente un groupe alkyle en C₁ à C₁₀ ; un groupe cycloalkyle en C₃ à C₁₀ ; un groupe alkényle en C₂ à C₅ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe alkynyle en C₂ à C₅ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro; un groupe alkylcarbonyle en C₂ à C₅ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe arylcarbonyle en C₇ à C₁₃ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe arylcarbonylalkyle en C₈ à C₁₄ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe arylthio en C₆ à C₁₂ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; ou un groupe aralkyle en C₇ à C₁₇ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro,
R² représente un groupe arylthio en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe alkylthio en C₁ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe cycloalkylthio en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇; un groupe arylsulfinyle en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇; un groupe alkylsulfinyle en C₁ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe cycloalkylsulfinyle en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe alkényle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe alkynyle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe aralkyle en C₇ à C₁₇ facultativement substitué par un ou plusieurs substituants choisis parmi un atome d'halogène et les groupes alkyle en C₁ à C₅, allcyle halogéné en C₁ à C₅, alkoxy en C₁ à C₅, aryle en C₆ à C₁₂, hydroxyle, nitro, amino, cyano et acyle en C₂ à C₇ ; un groupe arylcarbonyle en C₇ à C₁₃ facultativement substitué par un ou plusieurs substituants choisis parmi un atome d'halogène et les groupes allcyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe arylcarbonylalkyle en C₈ à C₁₄ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; ou un groupe aryloxy en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇,
R³ représente un atome d'hydrogène ; un groupe méthyle ; un groupe alkyle ramifié en C₃ à C₁₀ ; ou un groupe -CH₂-Z-(CH₂)ₙ-R⁵, où R⁵ représente un atome d'hydrogène, un atome d'halogène, un groupe carbonyloxy hétérocyclique de 5 ou 6 membres, formyloxy, alkylcarbonyloxy en C₂ à C₁₁, cycloalkylcarbonyloxy en C₄ à C₁₁, aralkylcarbonyloxy en C₈ à C₁₂, arylcarbonyloxy en C₇ à C₁₃, azido, alkoxycarbonyloxy en C₂ à C₁₁ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-alkylcarbamoyloxy en C₂ à C₁₁ facultativement substitué par un ou plusieurs substituants choisis parmi un atome d'halogène et les groupes aryle en C₆ à C₁₂, allcyle en C₁ à C₅, alkoxy en C₁ à C₅ et alkyle halogéné en C₁ à C₅, N-arylcarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-alkylthiocarbamoyloxy en C₂ à C₁₁ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-arylthiocarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkoxy en C₁ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, aralkyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkyle ramifié en C₃ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, cycloalkyle en C₃ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, ou aryle en C₆ à C₁₂ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, et Z représente un atome d'oxygène ou de soufre ou un groupe méthylène, et n représente 0 ou un nombre entier de 1 à 5,
R⁴ représente un atome d'hydrogène ; et
X et Y représentent indépendamment un atome d'oxygène ou un atome de soufre ;
ou l'un de ses sels acceptables du point de vue pharmaceutique.

2. Composé selon la revendication 1, dans lequel :
R¹ représente un groupe alkyle en C₁ à C₅ ; un groupe cycloalkyle en C₃ à C₈ ; un groupe alkényle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe alkynyle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe alkylcarbonyle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe arylcarbonyle en C₇ à C₁₃ facultativement substitué par un ou plusieurs substituants choisis parmi un atome d'halogène et les groupes phényle, cyano, alkoxycarbonyle en C₂ à C₆ et carbamoyle ; un groupe arylcarbonylalkyle en C₈ à C₁₄ facultativement substitué par un ou plusieurs substituants choisis parmi un atome d'halogène et les groupes phényle, cyano, alkoxycarbonyle en C₂ à C₆ et carbamoyle ; un groupe arylthio en C₆ à C₁₂ facultativement substitué par un ou plusieurs substituants choisis parmi un atome d'halogène et les groupes phényle, cyano, alkoxycarbonyle en C₂ à C₆ et carbamoyle ; ou un groupe aralkyle en C₇ à C₁₇ facultativement substitué par un ou plusieurs substituants choisis parmi un atome d'halogène et les groupes phényle, cyano, alkoxycarbonyle en C₂ à C₆ et carbamoyle ;
R² représente un groupe arylthio en C₆ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkylthio en C₁ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe cycloalkylthio en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylsulfinyle en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkylsulfinyle en C₁ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe cycloalkylsulfinyle en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkényle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkynyle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe aralkyle en C₇ à C₁₁ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylcarbonyle en C₇ à C₁₃ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylcarbonylalkyle en C₈ à C₁₄ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, mitro, amino, cyano et acyle en C₂ à C₇ ; ou un groupe aryloxy en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇.

3. Composé selon la revendication 2, dans lequel :
R¹ représente un groupe alkyle en C₁ à C₅ ; un groupe cycloalkyle en C₃ à C₈ ; un groupe alkényle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe alkynyle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe alkylcarbonyle en C₂ à C₅ ; un groupe arylcarbonyle en C₇ à C₁₃ ; un groupe arylcarbonylalkyle en C₈ à C₁₄ ; un groupe arylthio en C₆ à C₁₂ ; ou un groupe aralkyle en C₇ à C₁₇ ;
R² représente un groupe arylthio en C₆ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkylthio en C₁ à C₅ ; un groupe cycloalkylthio en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylsulfinyle en C₆ à C₁₂ ; un groupe alkylsulfinyle en C₁ à C₅ ; un groupe cycloalkylsulfinyle en C₃ à C₁₀ ; un groupe alkényle en C₂ à C₅ ; un groupe alkynyle en C₂ à C₅ ; un groupe aralkyle en C₇ à C₁₁ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylcarbonyle en C₇ à C₁₃ ; un groupe arylcarbonylalkyle en C₈ à C₁₄ ; ou un groupe aryloxy en C₆ à C₁₂ ;
R³ représente un atome d'hydrogène ; un groupe méthyle ; ou un groupe ―CH₂-Z-(CH₂)ₙ-R⁵, où R⁵ représente un atome d'hydrogène, un atome d'halogène, un groupe carbonyloxy hétérocyclique de 5 ou 6 membres, formyloxy, alkylcarbonyloxy en C₂ à C₁₁, cycloalkylcarbonyloxy en C₄ à C₁₁, aralkylcarbonyloxy en C₈ à C₁₂, arylcarbonyloxy en C₇ à C₁₃, azido, alkoxycarbonyloxy en C₂ à C₁₁, N-alkylcarbamoyloxy en C₂ à C₈, N-arylcarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-alkylthiocarbamoyloxy en C₂ à C₈, N-arylthiocarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkoxy en C₁ à C₁₀, aralkyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkyle ramifié en C₃ à C₅, cycloalkyle en C₅ à C₇, ou aryle en C₆ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, et Z représente un atome d'oxygène, un atome de soufre ou un groupe méthylène, et n représente 0 ou un nombre entier de 1 à 5.

4. Composé selon la revendication 3, dans lequel:
R¹ représente un groupe alkyle en C₁ à C₅ ; un groupe cycloalkyle en C₃ à C₈ ; un groupe alkényle en C₂ à C₅ facultativement substitué par un ou plusieurs substituants choisis parmi un atome d'halogène et les groupes phényle, cyano, alkoxycarbonyle en C₂ à C₆ et carbamoyle ;
R² représente un groupe arylthio en C₆ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkylthio en C₁ à C₅ ; un groupe cycloalkylthio en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; ou un groupe aralkyle en C₇ à C₁₁ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ;
R³ représente un atome d'hydrogène ; un groupe méthyle ; ou un groupe ―CH₂-Z-(CH₂)ₙ-R⁵, où R⁵ représente un atome d'hydrogène, un atome d'halogène, un groupe carbonyloxy hétérocyclique de 5 ou 6 membres, formyloxy, alkylcarbonyloxy en C₂ à C₁₁, cycloalkylcarbonyloxy en C₄ à C₁₁, aralkylcarbonyloxy en C₈ à C₁₂, arylcarbonyloxy en C₇ à C₁₃, azido, alkoxycarbonyloxy en C₂ à C₁₁, N-alkylcarbamoyloxy en C₂ à C₈, N-arylcarbamoyloxy en C₇ à C₁₃, N-alkylthiocarbamoyloxy en C₂ à C₈, N-arylthiocarbamoyloxy en C₇ à C₁₃, alkoxy en C₁ à C₁₀, aralkyloxy en C₇ à C₁₃, alkyle ramifié en C₃ à C₅, cycloalkyle en C₅ à C₇, ou aryle en C₆ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, et Z représente un atome d'oxygène, un atome de soufre ou un groupe méthylène, et n représente 0 ou un nombre entier de 1 à 5.

5. Agent antiviral contenant en tant qu'un ingrédient actif un dérivé d'acyclopyrimidine nucléoside substitué en 6 ou l'un de ses sels acceptable du point de vue pharmaceutique selon l'une quelconque des revendications 1 à 4.

6. Composition pharmaceutique contenant un composé selon l'une quelconque des revendications 1 à 4 ou l'un de ses sels acceptable du point de vue pharmaceutique en association avec un véhicule pharmaceutique.

7. Composition pharmaceutique selon la revendication 6 qui possède une activité antivirale efficace, en particulier une activité antirétrovirale.

8. Utilisation d'un dérivé d'acyclopyrimidine nucléoside substitué en 6 représenté par la formule générale I suivante : 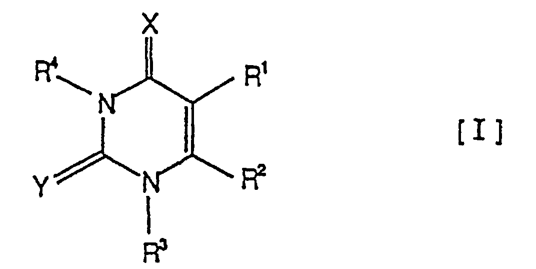 dans laquelle
R¹ représente un atome d'hydrogène ; un atome d'halogène ; un groupe alkyle en C₁ à C₁₀ ; un groupe cycloalkyle en C₃ à C₁₀ ; un groupe alkényle en C₂ à C₅ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe alkynyle en C₂ à C₅ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe alkylcarbonyle en C₂ à C₅ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe arylcarbonyle en C₇ à C₁₃ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe arylcarbonylalkyle en C₈ à C₁₄ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe arylthio en C₆ à C₁₂ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; ou un groupe aralkyle en C₇ à C₁₇ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro,
R² représente un groupe arylthio en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe alkylthio en C₁ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe cycloalkylthio en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇; un groupe arylsulfinyle en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇; un groupe alkylsulfinyle en C₁ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇; un groupe cycloalkylsulfinyle en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇; un groupe alkényle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇; un groupe alkynyle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe aralkyle en C₇ à C₁₇ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇; un groupe arylcarbonyle en C₇ à C₁₃ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe arylcarbonylalkyle en C₈ à C₁₄ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇; ou un groupe aryloxy en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇,
R³ représente un atome d'hydrogène ; un groupe méthyle ; un groupe alkyle ramifié en C₃ à C₁₀ ; ou un groupe -CH₂-Z-(CH₂)ₙ-R⁵, où R⁵ représente un atome d'hydrogène, un atome d'halogène, un groupe carbonyloxy hétérocyclique de 5 ou 6 membres, formyloxy, alkylcarbonyloxy en C₂ à C₁₁, cycloalkylcarbonyloxy en C₄ à C₁₁, aralkylcarbonyloxy en C₈ à C₁₂, arylcarbonyloxy en C₇ à C₁₃, azido, alkoxycarbonyloxy en C₂ à C₁₁ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-alkylcarbamoyloxy en C₂ à C₁₁ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-arylcarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-alkylthiocarbamoyloxy en C₂ à C₁₁ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-ayylthiocarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkoxy en C₁ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, aralkyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkyle ramifié en C₃ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, cycloalkyle en C₃ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, ou aryle en C₆ à C₁₂ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, et Z représente un atome d'oxygène, un atome de soufre ou un groupe méthylène, et n représente 0 ou un nombre entier de 1 à 5,
R⁴ représente un atome d'hydrogène, un groupe alkyle en C₁ à C₁₃ ou aralkyle en C₇ à C₁₃ ; et
X et Y représentent indépendamment un atome d'oxygène ou un atome de soufre ;
ou l'un de ses sels acceptable du point de vue pharmaceutique, dans un procédé de production d'une composition pharmaceutique destinée au traitement ou à la prévention de maladies causées par une infection virale, en particulier une infection rétrovirale, en particulier chez l'homme.

9. Utilisation selon la revendication 8, dans laquelle :
R¹ représente un atome d'hydrogène; un atome d'halogène; un groupe alkyle en C₁ à C₅; un groupe cycloalkyle en C₃ à C₈ ; un groupe alkényle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe alkynyle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe alkylcarbonyle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe arylcarbonyle en C₇ à C₁₃ facultativement substitué par un ou plusieurs substituants choisis parmi un atome d'halogène et les groupes phényle, cyano, alkoxycarbonyle en C₂ à C₆ et carbamoyle ; un groupe arylcarbonylalkyle en C₈ à C₁₄ facultativement substitué par un ou plusieurs substituants choisis parmi un atome d'halogène et les groupes phényle, cyano, alkoxycarbonyle en C₂ à C₆ et carbamoyle; un groupe arylthio en C₆ à C₁₂ facultativement substitué par un ou plusieurs substituants choisis parmi un atome d'halogène et les groupes phényle, cyano, alkoxycarbonyle en C₂ à C₆ et carbamoyle ; ou un groupe aralkyle en C₇ à C₁₇ facultativement substitué par un ou plusieurs substituants choisis parmi un atome d'halogène et les groupes phényle, cyano, alkoxycarbonyle en C₂ à C₆ et carbamoyle ;
R² représente un groupe arylthio en C₆ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkylthio en C₁ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe cycloalkylthio en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylsulfinyle en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkylsulfinyle en C₁ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe cycloalkylsulfinyle en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkényle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkynyle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe aralkyle en C₇ à C₁₁ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylcarbonyle en C₇ à C₁₃ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylcarbonylalkyle en C₈ à C₁₄ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; ou un groupe aryloxy en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇.

10. Utilisation selon la revendication 9, dans laquelle :
R¹ représente un atome d'hydrogène, un atome d'halogène, un groupe alkyle en C₁ à C₅ ; un groupe cycloalkyle en C₃ à C₈ ; un groupe alkényle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe alkynyle en C₂ à C₅ facultativement substitué par un ou plusieurs substituants choisis parmi un atome d'halogène et les groupes phényle, cyano, alkoxycarbonyle en C₂ à C₆ et carbamoyle ; un groupe alkylcarbonyle en C₂ à C₅ ; un groupe arylcarbonyle en C₇ à C₁₃ ; un groupe arylcarbonylalkyle en C₈ à C₁₄ ; un groupe arylthio en C₆ à C₁₂ ; ou un groupe aralkyle en C₇ à C₁₇ ;
R² représente un groupe arylthio en C₆ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe alkylthio en C₁ à C₅ ; un groupe cycloalkylthio en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylsulfinyle en C₆ à C₁₂ ; un groupe alkylsulfinyle en C₁ à C₅ ; un groupe cycloalkylsulfinyle en C₃ à C₁₀ ; un groupe alkényle en C₂ à C₅ ; un groupe alkynyle en C₂ à C₅ ; un groupe aralkyle en C₇ à C₁₁ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe arylcarbonyle en C₇ à C₁₃ ; un groupe arylcarbonylalkyle en C₈ à C₁₄ ; ou un groupe aryloxy en C₆ à C₁₂ ;
R³ représente un atome d'hydrogène ; un groupe méthyle ; ou un groupe ―CH₂-Z-(CH₂)ₙ-R⁵, où R⁵ représente un atome d'hydrogène, un atome d'halogène, un groupe carbonyloxy hétérocyclique de 5 ou 6 membres, formyloxy, alkylcarbonyloxy en C₂ à C₁₁, cycloalkylcarbonyloxy en C₄ à C₁₁, aralkylcarbonyloxy en C₈ à C₁₂, arylcarbonyloxy en C₇ à C₁₃, azido, alkoxycarbonyloxy en C₂ à C₁₁, N-alkylcarbamoyloxy en C₂ à C₈, N-arylcarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-alkylthiocarbamoyloxy en C₂ à C₈, N-arylthiocarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkoxy en C₁ à C₁₀, aralkyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkyle ramifié en C₃ à C₅, cycloalkyle en C₅ à C₇, ou aryle en C₆ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, et Z représente un atome d'oxygène, un atome de soufre ou un groupe méthylène, et n représente 0 ou un nombre entier de 1 à 5.

11. Utilisation selon la revendication 10, dans laquelle :
R¹ représente un atome d'hydrogène, un atome d'halogène, un groupe alkyle en C₁ à C₅ ; un groupe cycloalkyle en C₃ à C₈ ; un groupe alkényle en C₂ à C₅ facultativement substitué par un ou plusieurs substituants choisis parmi un atome d'halogène et les groupes phényle, cyano, alkoxycarbonyle en C₂ à C₆ et carbamoyle ;
R² représente un groupe arylthio en C₆ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; un groupe cycloalkylthio en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇ ; ou un groupe aralkyle en C₇ à C₁₁ facultativement substitué par un ou plusieurs substituants choisis parmi un atome d'halogène et les groupes alkyle en C₁ à C₅, alkyle halogéné en C₁ à C₅, alkoxy en C₁ à C₅, phényle, hydroxyle, nitro, amino, cyano et acyle en C₂ à C₇;
R³ représente un atome d'hydrogène ; un groupe méthyle ; ou un groupe ―CH₂-Z-(CH₂)ₙ-R⁵, où R⁵ représente un atome d'hydrogène, un atome d'halogène, un groupe carbonyloxy hétérocyclique de 5 ou 6 membres, formyloxy, alkylcarbonyloxy en C₂ à C₁₁, cycloalkylcarbonyloxy en C₄ à C₁₁, aralkylcarbonyloxy en C₈ à C₁₂, arylcarbonyloxy en C₇ à C₁₃, azido, alkoxycarbonyloxy en C₂ à C₁₁, N-alkylcarbamoyloxy en C₂ à C₈, N-arylcarbamoyloxy en C₇ à C₁₃, N-alkylthiocarbamoyloxy en C₂ à C₈, N-arylthiocarbamoyloxy en C₇ à C₁₃, alkoxy en C₁ à C₁₀, aralkyloxy en C₇ à C₁₃, alkyle ramifié en C₃ à C₅, cycloalkyle en C₅ à C₇, ou aryle en C₆ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et allyle halogéné en C₁ à C₅, et Z représente un atome d'oxygène, un atome de soufre ou un groupe méthylène, et n représente 0 ou un nombre entier de 1 à 5.

12. Procédé de préparation d'un composé représenté par la formule générale I suivante : 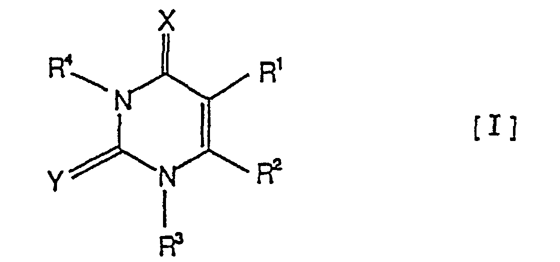 dans laquelle
R¹ représente un groupe alkyle en C₁ à C₁₀ ; un groupe cycloalkyle en C₃ à C₁₀ ; un groupe alkényle en C₂ à C₅ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe alkynyle en C₂ à C₅ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe alkylcarbonyle en C₂ à C₅ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe arylcarbonyle en C₇ à C₁₃ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe arylcarbonylalkyle en C₈ à C₁₄ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; un groupe arylthio en C₆ à C₁₂ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro ; ou un groupe aralkyle en C₇ à C₁₇ facultativement substitué par un ou plusieurs substituants choisis parmi un atome d'halogène et les groupes aryle en C₆ à C₁₂, cyano, alkoxycarbonyle en C₂ à C₆, carbamoyle, alkoxy en C₁ à C₅, amino et nitro,
R² représente un groupe arylthio en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe alkylthio en C₁ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe cycloalkylthio en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe arylsulfinyle en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe alkylsulfinyle en C₁ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe cycloalkylsulfinyle en C₃ à C₁₀ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe alkényle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe alkynyle en C₂ à C₅ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe aralkyle en C₇ à C₁₇ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe arylcarbonyle en C₇ à C₁₃ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; un groupe arylcarbonylalkyle en C₈ à C₁₄ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇ ; ou un groupe aryloxy en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇,
R³ représente un groupe méthyle ; un groupe alkyle ramifié en C₃ à C₁₀ ; ou un groupe ―CH₂-Z-(CH₂)ₙ-R⁵, où R⁵ représente un atome d'hydrogène, un atome d'halogène, azido, un groupe alkoxy en C₁ à C₁₀, facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, en C₃ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, cycloalkyle en C₃ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, aryle en C₆ à C₁₂ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkoxy en C₁ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, Z représente un atome d'oxygène ou de soufre ou un groupe méthylène, et n représente 0 ou un nombre entier de 1 à 5,
R⁴ représente un atome d'hydrogène ; et
X et Y représentent indépendamment un atome d'oxygène ou un atome de soufre ;
lequel procédé comprend la mise en réaction d'un composé représenté par la formule générale II suivante : 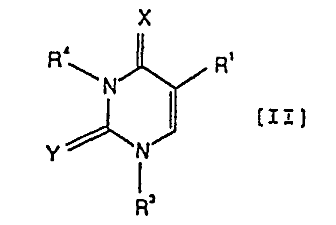 dans laquelle R¹, R³, R⁴, X et Y ont les mêmes significations que celles définies ci-dessus, avec un composé organique de métal alcalin de manière à obtenir un composé représenté par la formule générale II' suivante : 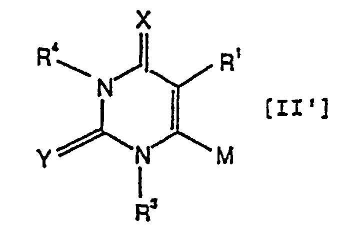 dans laquelle R¹, R³, R⁴, X et Y ont les mêmes significations que celles définies ci-dessus et M représente un métal alcalin, suivie de la mise en réaction du composé de formule II' ainsi obtenu avec un composé représenté par la formule générale III suivante:
R²X¹ [III]
dans laquelle R² a la même signification que celle définie ci-dessus et X¹ représente un atome d'halogène, un groupe arylthio ou alkoxy, de manière à obtenir le composé de formule I ci-dessus.

13. Procédé de préparation d'un composé représenté par la formule générale I suivante : 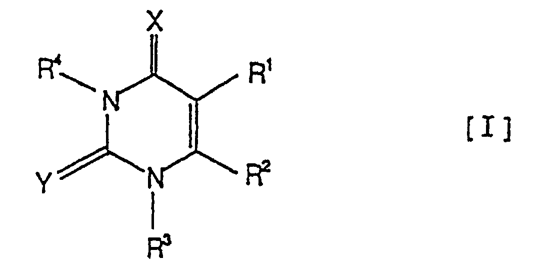 dans laquelle R¹, R², R³, R⁴, X et Y ont les mêmes significations que celles définies dans la revendication 1, lequel procédé comprend la mise en réaction d'un composé représenté par la formule générale IV suivante : 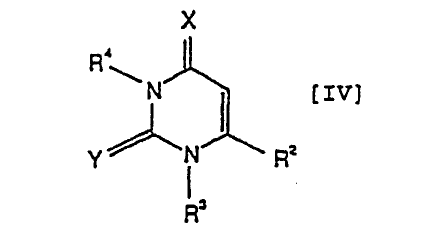 dans laquelle R², R³, R⁴, X et Y ont les mêmes significations que celles définies dans la revendication 12, avec un composé organique de métal alcalin de manière à obtenir un composé représenté par la formule générale IV' suivante : 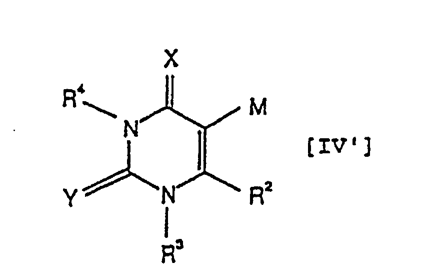 dans laquelle R², R³, R⁴, X et Y ont les mêmes significations que celles définies dans la revendication 12 et M représente un métal alcalin, suivie de la mise en réaction du composé de formule IV' ainsi obtenu avec un composé représenté par la formule générale V suivante :
R¹X² [V]
dans laquelle R¹ a la même signification que celle définie dans la revendication 12 et X² représente un atome d'halogène, un groupe arylthio ou alkoxy, de manière à obtenir le composé de formule I ci-dessus.

14. Procédé de préparation d'un composé représenté par la formule générale I suivante : 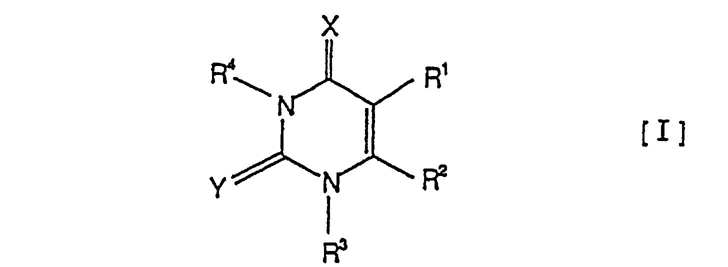 dans laquelle R¹, R², R³, R⁴, X et Y ont les mêmes significations que celles définies dans la revendication 12, lequel procédé comprend la mise en réaction d'un composé représenté par la formule générale VI suivante : 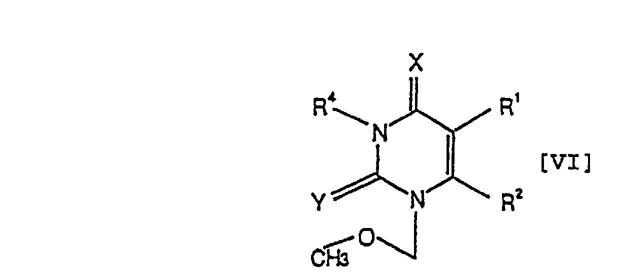 dans laquelle R¹, R², R⁴, X et Y ont les mêmes significations que celles définies dans la revendication 12, avec un acide de manière à obtenir un composé représenté par la formule générale VII suivante : 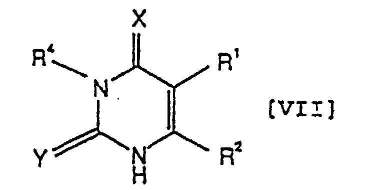 dans laquelle R¹, R², R⁴, X et Y ont les mêmes significations que celles définies dans la revendication 12, suivie de la mise en réaction du composé de formule VII ainsi obtenu avec un composé représenté par la formule générale VIII suivante :
R³X³ [VIII]
dans laquelle R³ a la même signification que celle définie dans la revendication 12 et X³ représente un groupe sulfonyloxy, en présence d'une base, de manière à obtenir le composé de formule I ci-dessus.

15. Procédé de préparation d'un composé représenté par la formule générale X suivante : 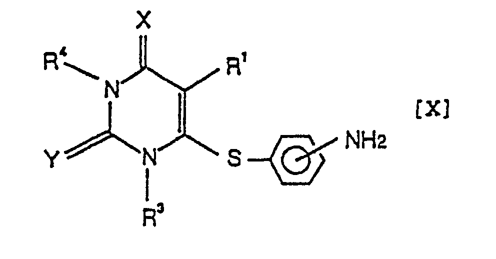 dans laquelle R¹, R⁴, X et Y ont les mêmes significations que celles définies dans la revendication 12, et
R³ représente un atome d'hydrogène ; un groupe méthyle ; un groupe alkyle ramifié en C₃ à C₁₀ ; ou un groupe ―CH₂-Z-(CH₂)ₙ-R⁵, où R⁵ représente un atome d'hydrogène, un atome d'halogène, un groupe carbonyloxy hétérocyclique de 5 ou 6 membres, formyloxy, alkylcarbonyloxy en C₂ à C₁₁, cycloalkylcarbonyloxy en C₄ à C₁₁, aralkylcarbonyloxy en C₈ à C₁₂, arylcarbonyloxy en C₇ à C₁₃, azido, alkoxycarbonyloxy en C₂ à C₁₁ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-alkylcarbamoyloxy en C₂ à C₁₁ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-arylcarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-alkylthiocarbamoyloxy en C₂ à C₁₁ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, N-arylthiocarbamoyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkoxy en C₁ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, aralkyloxy en C₇ à C₁₃ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, alkyle ramifié en C₃ à C₁₀ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, cycloalkyle en C₃ à C₁₀ facultativement substitué par un ou plusieurs substituants choisis parmi un atome d'halogène et les groupes aryle en C₆ à C₁₂, allyle en C₁ à C₅, alkoxy en C₁ à C₅ et alkyle halogéné en C₁ à C₅, ou aryle en C₆ à C₁₂ facultativement substitué par un ou plusieurs substituants choisis parmi un atome d'halogène et les groupes aryle en C₆ à C₁₂, alkyle en C₁ à C₅, alkoxy en C₁ à C₅ et alkyle halogéné en C₁ à C₅, et Z représente un atome d'oxygène, un atome de soufre ou un groupe méthylène, et n représente 0 ou un nombre entier de 1 à 5, qui comprend l'hydrogénation d'un composé représenté par la formule générale IX suivante : 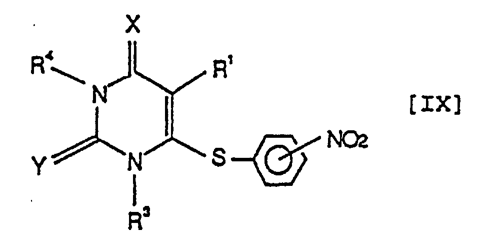 dans laquelle R¹, R⁴, X et Y ont les mêmes significations que celles définies dans la revendication 12 et R³ a la même signification que celle définie ci-dessus, en présence d'un catalyseur, de manière à obtenir le composé de formule X ci-dessus.

16. Procédé de préparation d'un composé représenté par la formule générale XII suivante : 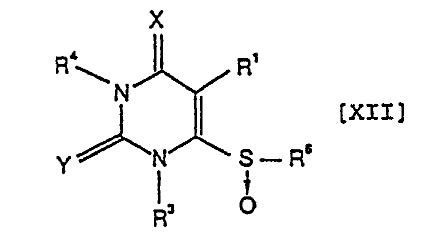 dans laquelle R¹, R³, R⁴, X et Y ont les mêmes significations que celles définies dans la revendication 15 et R⁶ représente un groupe aryle en C₆ à C₁₂, un groupe alkyle en C₁ à C₁₀ ou un groupe cycloalkyle en C₃ à C₁₀, qui comprend la conversion d'un composé représenté par la formule générale XI suivante : 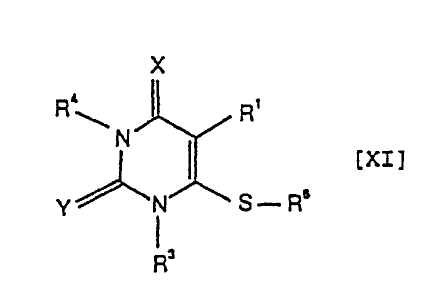 dans laquelle R¹, R³, R⁴, X et Y ont les mêmes significations que celles définies dans la revendication 2 et R⁶ a la même signification que celle définie ci-dessus, en le composé de formule XII ci-dessus grâce à l'action d'un agent oxydant.

17. Procédé de préparation d'un composé représenté par la formule générale XV suivante : 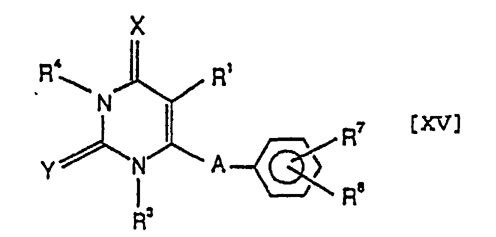 dans laquelle R¹, R³, R⁴, X et Y ont les mêmes significations que celles définies dans la revendication 15, R⁷ et R⁸ représentent indépendamment un atome d'halogène, un groupe alkyle en C₁ à C₅, un groupe alkyle halogéné en C₁ à C₅, un groupe alkoxy en C₁ à C₅, hydroxyle, nitro, amino, cyano ou acyle en C₂ à C₇, et A représente un atome de soufre ou d'oxygène, qui comprend la mise en réaction d'un composé représenté par la formule générale XIII suivante : 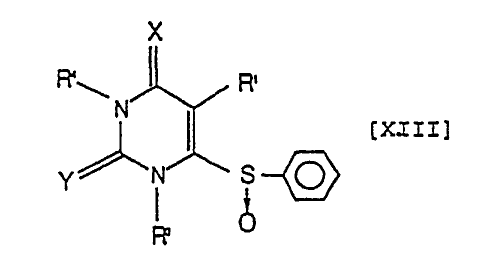 dans laquelle R¹, R³, R⁴, X et Y ont les mêmes significations que celles définies dans la revendication 15, avec un composé représenté par la formule générale XIV suivante : 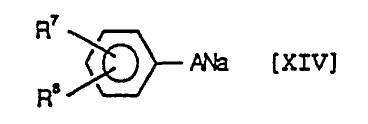 dans laquelle R⁷ et R⁸ ont les mêmes significations que celles définies ci-dessus, de manière à obtenir le composé de formule XV ci-dessus.

18. Procédé de préparation d'un composé représenté par la formule générale VIII suivante : 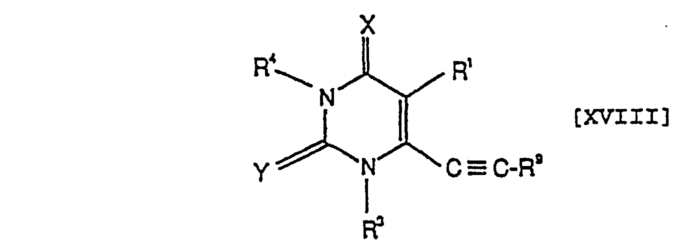 dans laquelle R¹, R³, R⁴, X et Y ont les mêmes significations que celles définies dans la revendication 15 et R⁹ représente un groupe alkyle en C₁ à C₅, un groupe aryle ou silyle en C₆ à C₁₂, qui comprend la mise en réaction d'un composé représenté par la formule générale XVI suivante : 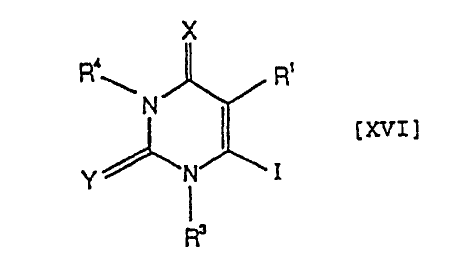 dans laquelle R¹, R³, R⁴, X et Y ont les mêmes significations que celles définies dans la revendication 15, avec un composé représenté par la formule générale XVII suivante :
HC≡C-R⁹ [XVII]
dans laquelle R⁹ a la même signification que celle définie ci-dessus, en présence d'un catalyseur à base de palladium, de manière à obtenir le composé de formule XVIII ci-dessus.

19. Procédé de préparation d'un composé représenté par la formule générale XX suivante : 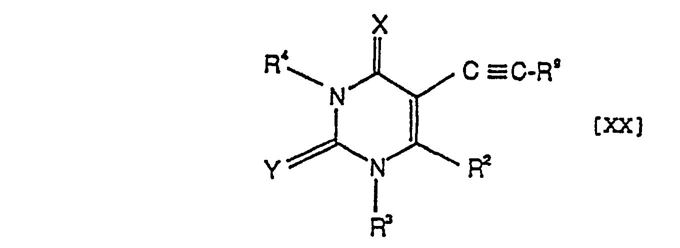 dans laquelle R², R³, R⁴, X et Y ont les mêmes significations que celles définies dans la revendication 15 et R⁹ représente un groupe alkyle en C₁ à C₅, un groupe aryle ou silyle en C₆ à C₁₂, qui comprend la mise en réaction d'un composé représenté par la formule générale XIX suivante : 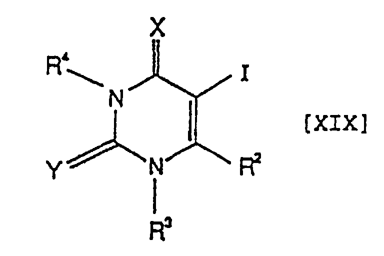 dans laquelle R², R³, R⁴, X et Y ont les mêmes significations que celles définies dans la revendication 15, avec un composé représenté par la formule générale XVII suivante :
HC≡CH-R⁹ [XVII]
dans laquelle R⁹ a la même signification que celle définie ci-dessus, en présence d'un catalyseur à base de palladium, de manière à obtenir le composé de formule XX ci-dessus.

20. Procédé de préparation d'un composé représenté par la formule générale XXII suivante : 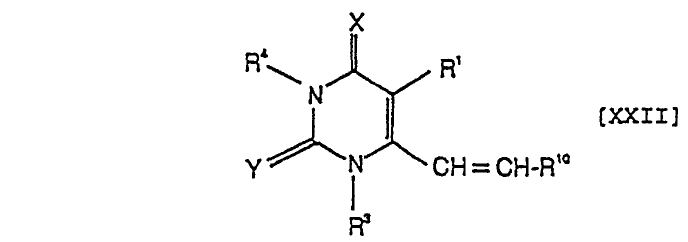 dans laquelle R¹, R³, R⁴, X et Y ont les mêmes significations que celles définies dans la revendication 15 et R¹⁰ représente un groupe alkoxycarbonyle en C₂ à C₆, nitrile ou carbamoyle, qui comprend la mise en réaction d'un composé représenté par la formule générale XVI suivante : 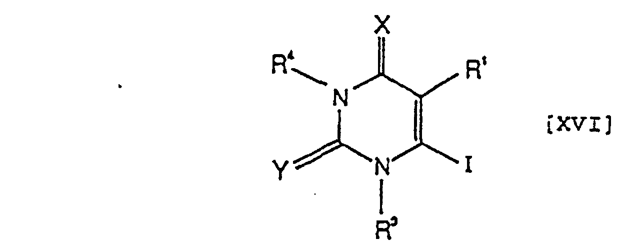 dans laquelle R¹, R³, R⁴, X et Y ont les mêmes significations que celles définies dans la revendication 15, avec un composé représenté par la formule générale XXI suivante:
H₂C=CH-R¹⁰ [XXI]
dans laquelle R¹⁰ a la même signification que celle définie ci-dessus, en présence d'un catalyseur à base de palladium, de manière à obtenir le composé de formule XXII ci-dessus.

21. Procédé de préparation d'un composé représenté par la formule générale XXIII suivante: 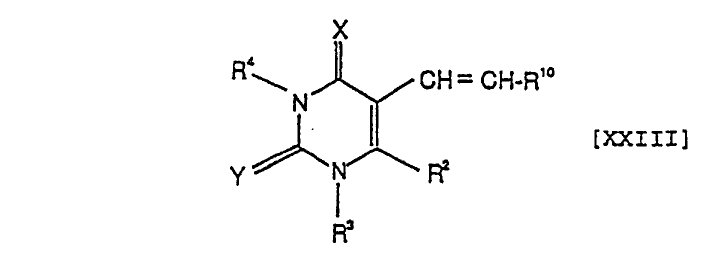 dans laquelle R², R³, R⁴, X et Y ont les mêmes significations que celles définies dans la revendication 15 et R¹⁰ a la même signification que celle définie dans la revendication 20, qui comprend la mise en réaction d'un composé représenté par la formule générale XIX suivante : 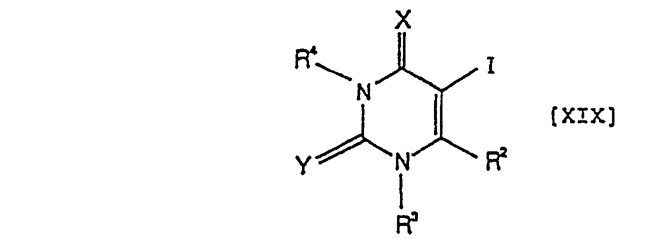 dans laquelle R², R³, R⁴, X et Y ont les mêmes significations que celles définies dans la revendication 15, avec un composé représenté par la formule générale XXI suivante :
H₂C=CH-R¹⁰ [XXI]
dans laquelle R¹⁰ a la même signification que celle définie dans la revendication 20, en présence d'un catalyseur à base de palladium, de manière à obtenir le composé de formule XXIII ci-dessus.

22. Procédé de préparation d'un composé représenté par la formule générale XXV suivante : 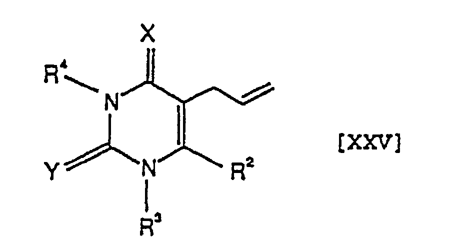 dans laquelle R², R³, R⁴, X et Y ont les mêmes significations que celles définies dans la revendication 15, qui comprend la mise en réaction d'un composé représenté par la formule générale IV suivante : 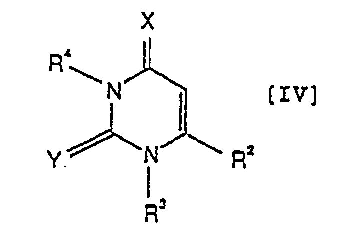 dans laquelle R², R³, R⁴, X et Y ont les mêmes significations que celles définies dans la revendication 15, avec un composé représenté par la formule générale XXIV suivante :
X⁴-CH₂CH=CH₂ [XXIV]
dans laquelle X⁴ représente un atome d'halogène, en présence d'un catalyseur à base de palladium, de manière à obtenir le composé de formule XXV ci-dessus.

23. Procédé de préparation d'un composé représenté par la formule générale XXVIII suivante : 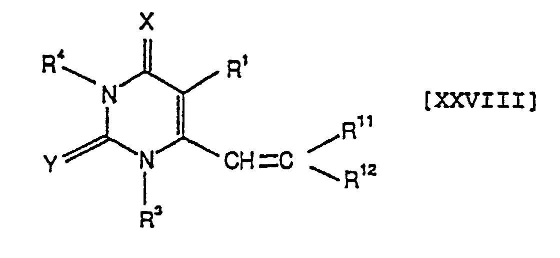 dans laquelle R¹, R³, R⁴, X et Y ont les mêmes significations que celles définies dans la revendication 12 et R¹¹ et R¹² représentent indépendamment un atome d'hydrogène, un groupe alkyle en C₁ à C₃ ou un groupe aryle en C₆ à C₁₂, qui comprend la mise en réaction d'un composé représenté par la formule générale II suivante : 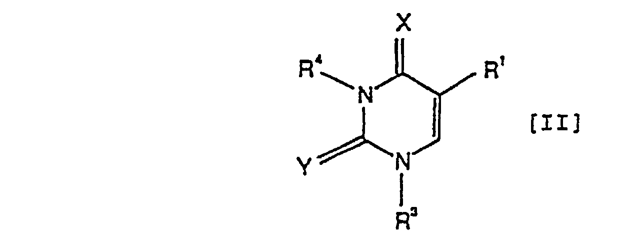 dans laquelle R¹, R³, R⁴, X et Y ont les mêmes significations que celles définies dans la revendication 12, avec un composé organique de métal alcalin de manière à obtenir un composé représenté par la formule générale II' suivante : 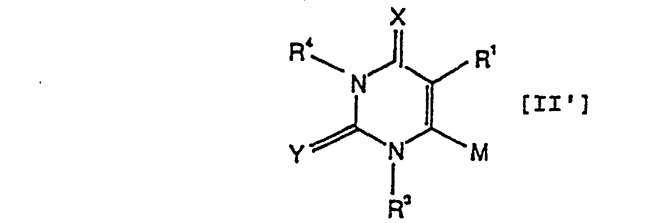 dans laquelle R¹, R³, R⁴, X et Y ont les mêmes significations que celles définies dans la revendication 12 et M représente un métal alcalin, la mise en réaction du composé de formule II' ainsi obtenu avec un composé représenté par la formule générale XXVI suivante : 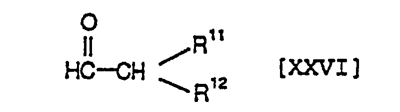 dans laquelle R¹¹ et R¹² ont les mêmes significations que celles définies ci-dessus de manière à obtenir un composé représenté par la formule générale XXVII suivante : 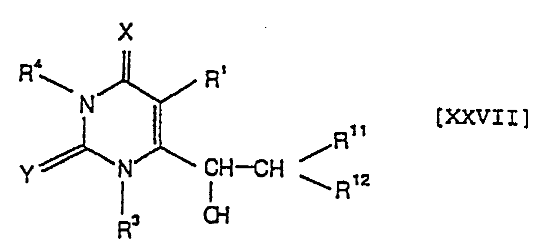 dans laquelle R¹, R³, R⁴, X et Y ont les mêmes significations que celles définies dans la revendication 12 et R¹¹ et R¹² ont les mêmes significations que celles définies ci-dessus, suivie par la déshydratation du composé de formule XXVII ainsi obtenu au moyen d'un agent déshydratant de manière à obtenir le composé de formule générale XXVIII ci-dessus.

24. Procédé de préparation d'un composé représenté par la formule générale XXX suivante : 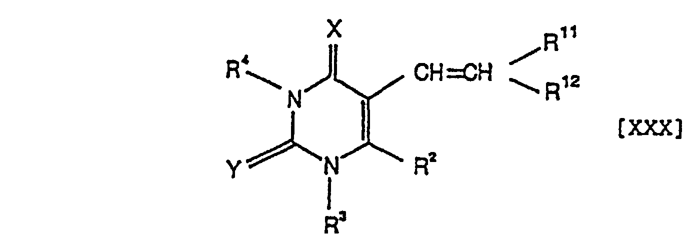 dans laquelle R², R³, R⁴, X et Y ont les mêmes significations que celles définies dans la revendication 15 et R¹¹ et R¹² représentent indépendamment un atome d'hydrogène, un groupe alkyle en C₁ à C₃ ou un groupe aryle en C₆ à C₁₂, qui comprend la mise en réaction d'un composé représenté par la formule générale IV suivante : 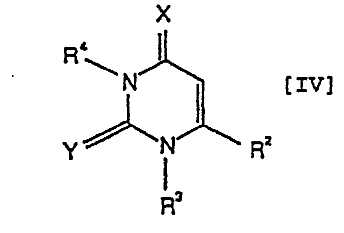 dans laquelle R², R³, R⁴, X et Y ont les mêmes significations que celles définies dans la revendication 15, avec un composé organique de métal alcalin de manière à obtenir un composé représenté par la formule générale IV' suivante : 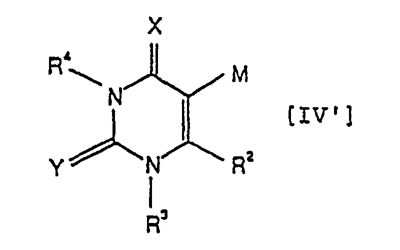 dans laquelle R², R³, R⁴, X et Y ont les mêmes significations que celles définies dans la revendication 12 et M représente un métal alcalin, la mise en réaction du composé de formule IV' ainsi obtenu avec un composé représenté par la formule générale XXVI suivante : 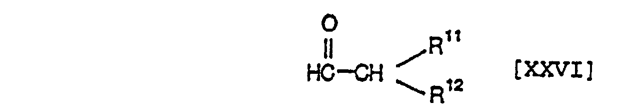 dans laquelle R¹¹ et R¹² ont les mêmes significations que celles définies ci-dessus, de manière à obtenir un composé représenté par la formule générale XXIX suivante : 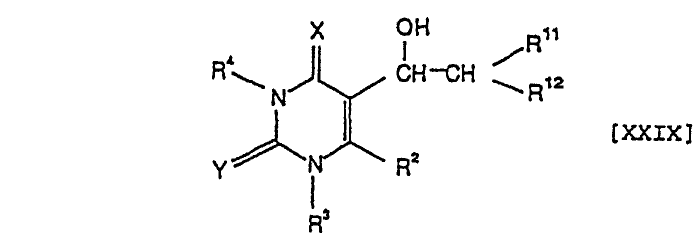 dans laquelle R², R³, R⁴, X et Y ont les mêmes significations que celles définies dans la revendication 15 et R¹¹ et R¹² ont les mêmes significations que celles définies ci-dessus, suivie par la déshydratation du composé de formule XXIX ainsi obtenu au moyen d'un agent déshydratant de manière à obtenir le composé de formule générale XXX ci-dessus.

25. Procédé de préparation d'un composé représenté par la formule générale XXXIII suivante : 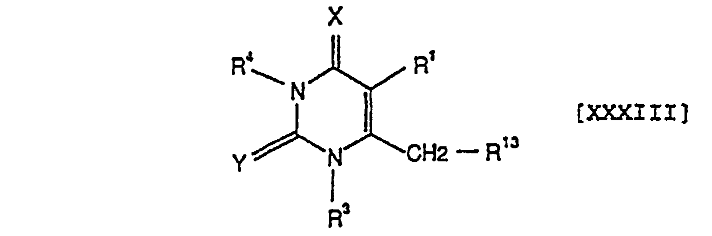 dans laquelle R¹, R³, R⁴, X et Y ont les mêmes significations que celles définies dans la revendication 12 et R¹³ représente un groupe aryle en C₆ à C₁₂ facultativement substitué par un ou plusieurs substituants choisis parmi un atome d'halogène et les groupes alkyle en C₁ à C₅, alkyle halogéné en C₁ à C₅, alkoxy en C₁ à C₅, aryle en C₆ à C₁₂, hydroxyle, nitro, amino, cyano et acyle en C₂ à C₇, qui comprend la mise en réaction d'un composé représenté par la formule générale II suivante : 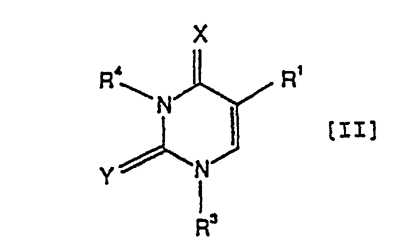 dans laquelle R¹, R³, R⁴, X et Y ont les mêmes significations que celles définies dans la revendication 12, avec un composé organique de métal alcalin de manière à obtenir un composé représenté par la formule générale II' suivante : 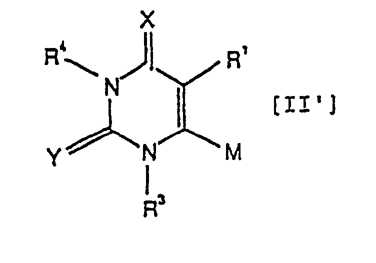 dans laquelle R¹, R³, R⁴, X et Y ont les mêmes significations que celles définies dans la revendication 12 et M représente un métal alcalin, la mise en réaction du composé de formule II' ainsi obtenu avec un composé représenté par la formule générale XXXI suivante : 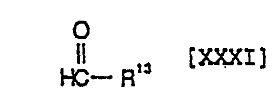 dans laquelle R¹³ a la même signification que celle définie ci-dessus de manière à obtenir un composé représenté par la formule générale XXXII suivante : 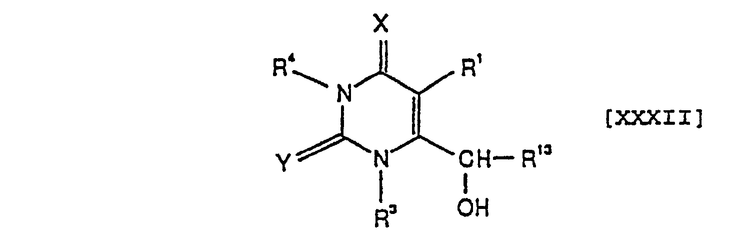 dans laquelle R¹, R³, R⁴, X et Y ont les mêmes significations que celles définies dans la revendication 12 et R¹³ a la même signification que celle définie ci-dessus, suivie par la réduction du composé de formule XXXII ainsi obtenu au moyen d'un agent réducteur de manière à obtenir le composé de formule générale XXXIII ci-dessus.

26. Procédé de préparation d'un composé représenté par la formule générale XXXVI suivante : 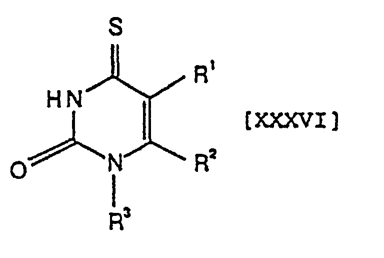 dans laquelle R¹, R² et R³ ont les mêmes significations que celles définies dans la revendication 15, qui comprend la mise en réaction d'un composé représenté par la formule générale XXXIV suivante : 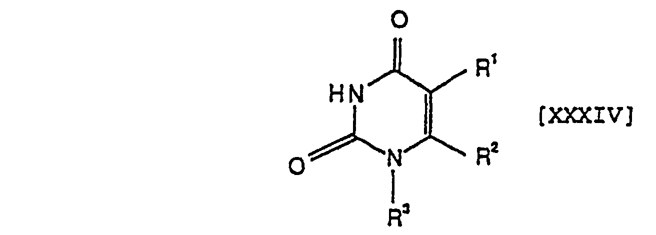 dans laquelle R¹, R² et R³ ont les mêmes significations que celles définies dans la revendication 15, avec un composé représenté par la formule générale XXXV suivante : 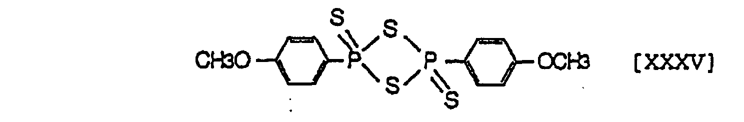 de manière à obtenir le composé de formule générale XXXVI ci-dessus.

27. Procédé de préparation d'un composé représenté par la formule générale XXXX suivante : 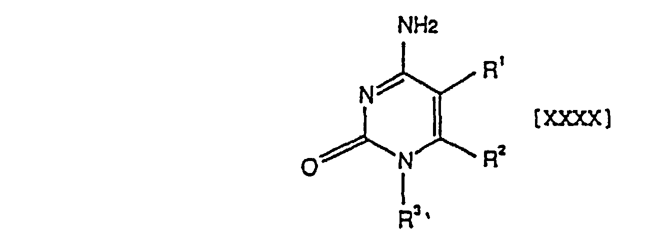 dans laquelle R¹, R² et R³ ont les mêmes significations que celles définies dans la revendication 15, qui comprend la mise en réaction d'un composé représenté par la formule générale XXXVII suivante : 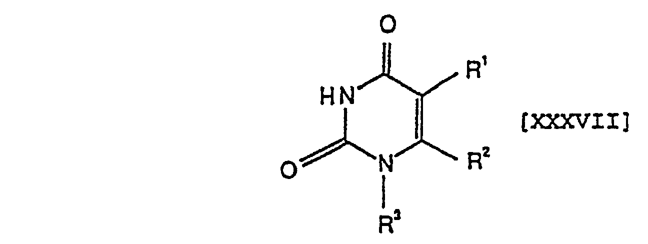 dans laquelle R¹, R² et R³ ont les mêmes significations que celles définies dans la revendication 15, avec un composé représenté par la formule générale XXXVIII suivante : 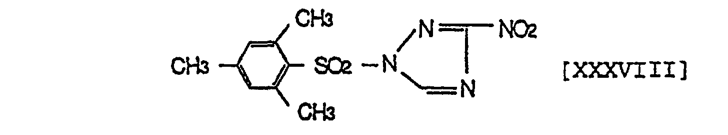 dans un solvant en présence d'acide diphénylphosphorique de manière à obtenir un composé représenté par la formule générale XXXIV suivante : 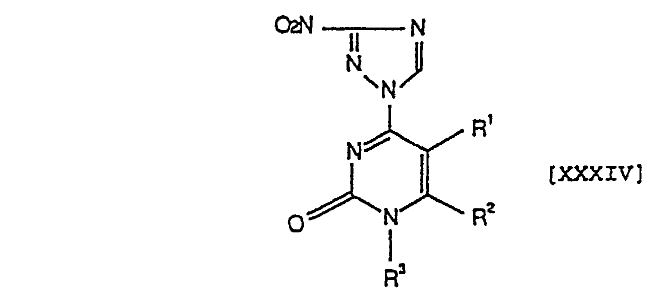 dans laquelle R¹, R² et R³ ont les mêmes significations que celles définies dans la revendication 15, et la mise en réaction du composé de formule XXXIV ainsi obtenu avec de l'ammoniaque aqueux de manière à obtenir le composé de formule XXXX ci-dessus.

28. Procédé de préparation d'un composé représenté par la formule générale I suivante : 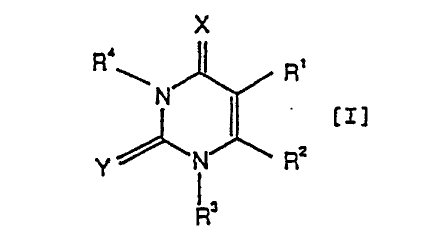 dans laquelle R¹, R² et R³ ont les mêmes significations que celles définies dans la revendication 15 et R⁴ a la même signification que celle définie dans la revendication 12, qui comprend la mise en réaction d'un composé représenté par la formule générale XXXXI suivante : 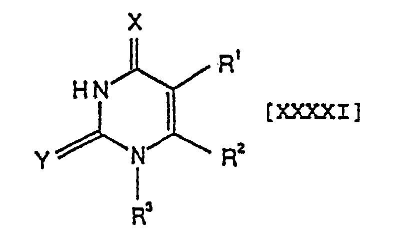 dans laquelle R¹, R², R³, X et Y ont les mêmes significations que celles définies dans la revendication 15, avec un composé représenté par la formule générale XXXXII suivante:
R⁴X⁵ [XXXXII]
dans laquelle R⁴ a la même signification que celle définie dans la revendication 1 et X⁵ représente un atome d'halogène ou un groupe sulfonyloxy, en présence d'une base, de manière à obtenir le composé de formule I ci-dessus.

29. Procédé de préparation d'un composé représenté par la formule générale XXXXV suivante : 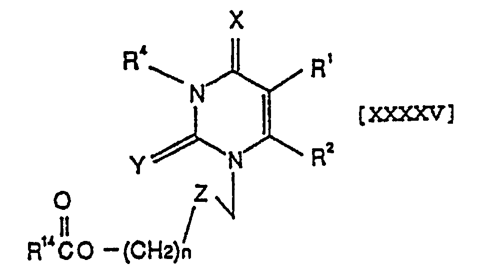 dans laquelle R¹, R², R⁴, X, Y, Z et n ont les mêmes significations que celles définies dans la revendication 12 et R¹⁴ représente un groupe alkyle en C₁ à C₁₀, cycloalkyle en C₃ à C₁₀, aryle en C₆ à C₁₂, aralkyle en C₇ à C₁₁ ou un groupe hétérocyclique de 5 ou 6 membres, qui comprend la mise en réaction d'un composé représenté par la formule générale XXXXIII suivante : 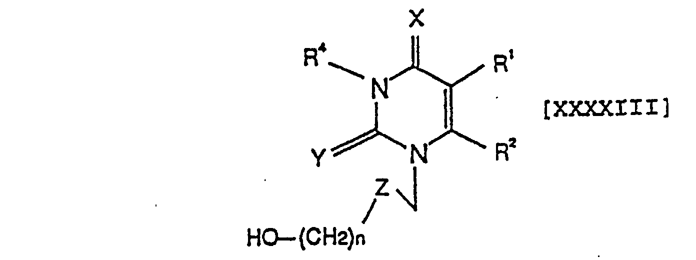 dans laquelle R¹, R², R⁴, X, Y, Z et n ont les mêmes significations que celles définies dans la revendication 12, avec un composé représenté par la formule générale XXXXIV suivante : 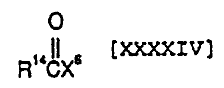 dans laquelle R¹⁴ a la même signification que celle définie ci-dessus et X⁶ représente un atome d'halogène ou ―OCOR¹⁴, dans un solvant, en présence d'une base, de manière à obtenir le composé de formule XXXXV ci-dessus.

30. Procédé de préparation d'un composé représenté par la formule générale XXXXVII suivante : 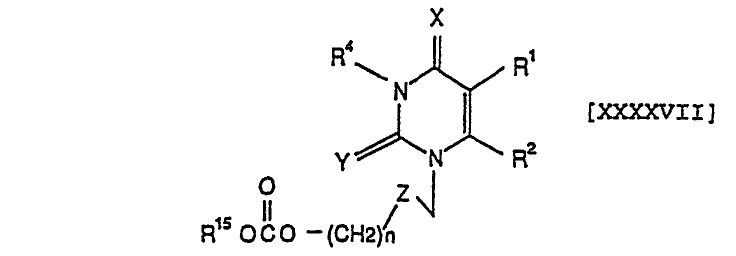 dans laquelle R¹, R², R⁴, X, Y, Z et n ont les mêmes significations que celles définies dans la revendication 12 et R¹⁵ représente un groupe alkyle en C₁ à C₁₀, qui comprend la mise en réaction d'un composé représenté par la formule générale XXXXIII suivante : 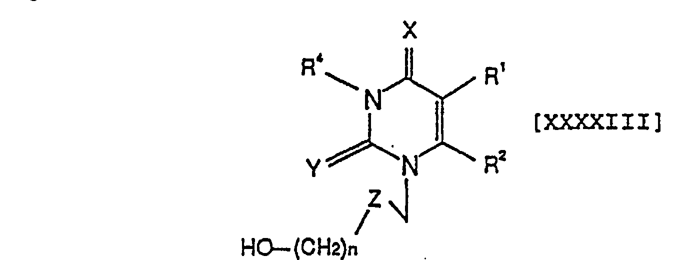 dans laquelle R¹, R², R⁴, X, Y, Z et n ont les mêmes significations que celles définies dans la revendication 12, avec un composé représenté par la formule générale XXXXVI suivante : 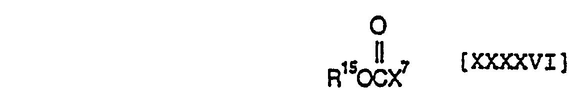 dans laquelle R¹⁵ a la même signification que celle définie ci-dessus et X⁷ représente un atome d'halogène ou ―OCOOR¹⁵, dans un solvant, en présence d'une base, de manière à obtenir le composé de formule XXXXVII ci-dessus.

31. Procédé de préparation d'un composé représenté par la formule générale XXXXIX suivante : 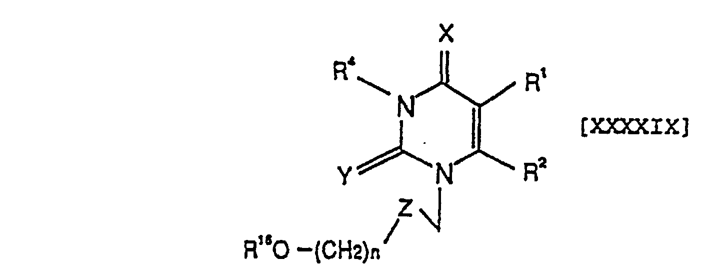 dans laquelle R¹, R², R⁴, X, Y, Z et n ont les mêmes significations que celles définies dans la revendication 12 et R¹⁶ représente un groupe alkyle en C₁ à C₁₀, aralkyle en C₇ à C₁₃ ou un groupe formyle, qui comprend la mise en réaction d'un composé représenté par la formule générale XXXXIII suivante : 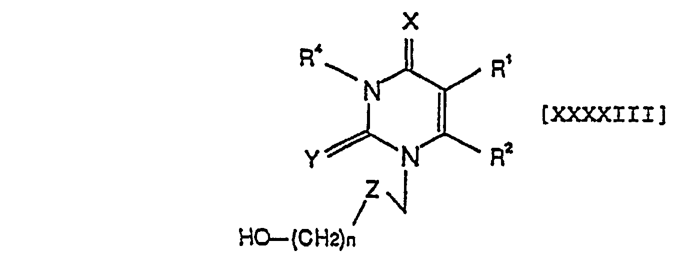 dans laquelle R¹, R², R⁴, X, Y, Z et n ont les mêmes significations que celles définies dans la revendication 12, avec un composé représenté par la formule générale XXXXVIII suivante :
R¹⁶X⁸ [XXXXVIII]
dans laquelle R¹⁶ a la même signification que celle définie ci-dessus et X⁸ représente un atome d'halogène ou un groupe sulfonyloxy, dans un solvant, en présence d'une base, de manière à obtenir le composé de formule XXXXIX ci-dessus.

32. Procédé de préparation d'un composé représenté par la formule générale XXXXXI suivante : 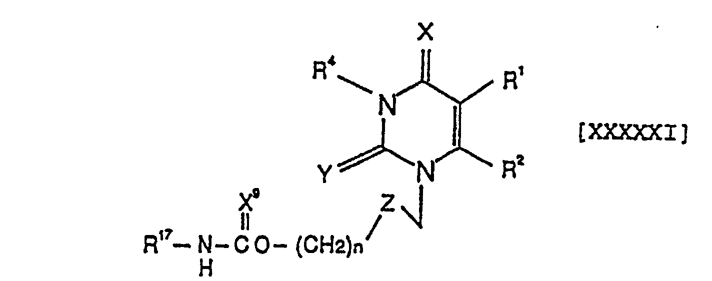 dans laquelle R¹, R², R⁴, X, Y, Z et n ont les mêmes significations que celles définies dans la revendication 12, R¹⁷ représente un groupe alkyle en C₁ à C₁₀ ou un groupe aryle en C₆ à C₁₂, et X⁹ représente un atome de soufre ou d'oxygène, qui comprend la mise en réaction d'un composé représenté par la formule générale XXXXIII suivante : 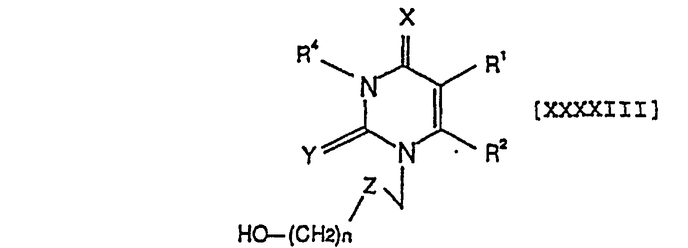 dans laquelle R¹, R², R⁴, X, Y, Z et n ont les mêmes significations que celles définies dans la revendication 12, avec un composé représenté par la formule générale XXXXX suivante :
R¹⁷-N=C=X⁹ [XXXXX]
dans laquelle R¹⁷ et X⁹ ont les mêmes significations que celles définies ci-dessus, dans un solvant, en présence d'une base, de manière à obtenir le composé de formule XXXXXI ci-dessus.
